(19) Europäisches Patentamt
European Patent Office
Office européen des brevets

(11) EP 1 679 382 A2

(12) # EUROPEAN PATENT APPLICATION

(43) Date of publication:
**12.07.2006 Bulletin 2006/28**

(51) Int Cl.:
***C12Q 1/68*** *(2006.01)*

(21) Application number: **06075499.1**

(22) Date of filing: **31.03.2003**

(84) Designated Contracting States:
**AT BE BG CH CY CZ DE DK EE ES FI FR GB GR HU IE IT LI LU MC NL PT RO SE SI SK TR**

(30) Priority: **29.03.2002 US 368667 P**

(62) Document number(s) of the earlier application(s) in accordance with Art. 76 EPC:
**03252023.1 / 1 355 150**

(71) Applicant: **ORTHO-CLINICAL DIAGNOSTICS, INC.**
**Rochester,**
**New York 14626-5101 (US)**

(72) Inventor: **Wang, Yixin**
**San Diego, CA 92130 (US)**

(74) Representative: **Mercer, Christopher Paul et al**
**Carpmaels & Ransford,**
**43-45 Bloomsbury Square**
**London WC1A 2RA (GB)**

Remarks:

- This application was filed on 03 - 03 - 2006 as a divisional application to the application mentioned under INID code 62.
- The sequence listing, which is published as annex to the application documents, was filed after the date of filing. The applicant has declared that it does not include matter which goes beyond the content of the application as filed.

(54) **Panel of nucleic acid sequences for cancer diagnosis**

(57) A method of diagnosing cancer by identifying differential modulation of each gene (relative to the expression of the same genes in a normal population) in a combination of genes selected from two groups of genes.

Gene expression portfolios and kits for employing the method are further aspects of the invention.

EP 1 679 382 A2

SEQUENCE LISTING

<110> WANG, YIXIN

<120> CANCER DIAGNOSTIC PANEL

<130> P033681EP

<140> 06075499.1
<141> 2003-03-03

<150> US 60/368,667
<151> 2002-03-29

<160> 100

<170> PatentIn version 3.1

<210> 1
<211> 1466
<212> DNA
<213> Homo sapiens

<400> 1
agccccaagc ttaccacctg cacccggaga gctgtgtgtc accatgtggg tcccggttgt 60

cttcctcacc ctgtccgtga cgtggattgg tgctgcaccc ctcatcctgt ctcggattgt 120

gggaggctgg gagtgcgaga agcattccca accctggcag gtgcttgtgg cctctcgtgg 180

cagggcagtc tgcggcggtg ttctggtgca cccccagtgg gtcctcacag ctgcccactg 240

catcaggaac aaaagcgtga tcttgctggg tcggcacagc ctgtttcatc ctgaagacac 300

aggccaggta tttcaggtca gccacagctt cccacacccg ctctacgata tgagcctcct 360

gaagaatcga ttcctcaggc caggtgatga ctccagccac gacctcatgc tgctccgcct 420

gtcagagcct gccgagctca cggatgctgt gaaggtcatg gacctgccca cccaggagcc 480

agcactgggg accacctgct acgcctcagg ctggggcagc attgaaccag aggagttctt 540

gaccccaaag aaacttcagt gtgtggacct ccatgttatt tccaatgacg tgtgtgcgca 600

agttcaccct cagaaggtga ccaagttcat gctgtgtgct ggacgctgga caggggggcaa 660

aagcacctgc tcgggtgatt ctgggggccc acttgtctgt aatggtgtgc ttcaaggtat 720

cacgtcatgg ggcagtgaac catgtgccct gcccgaaagg ccttccctgt acaccaaggt 780

ggtgcattac cggaagtgga tcaaggacac catcgtggcc aacccctgag cacccctatc 840

aacccctat tgtagtaaac ttggaacctt ggaaatgacc aggccaagac tcaagcctcc 900

ccagttctac tgacctttgt ccttaggtgt gaggtccagg gttgctagga aaagaaatca 960

gcagacacag gtgtagacca gagtgtttct taaatggtgt aattttgtcc tctctgtgtc 1020

ctggggaata ctggccatgc ctggagacat atcactcaat ttctctgagg acacagatag 1080

gatggggtgt ctgtgttatt tgtggggtac agagatgaaa gaggggtggg atccacactg 1140

agagagtgga gagtgacatg tgctggacac tgtccatgaa gcactgagca gaagctggag 1200

gcacaacgca ccagacactc acagcaagga tggagctgaa aacataaccc actctgtcct 1260

ggaggcactg ggaagcctag agaaggctgt gagccaagga gggagggtct tcctttggca 1320

tgggatgggg atgaagtaag gagagggact ggaccccctg gaagctgatt cactatgggg 1380

ggaggtgtat tgaagtcctc cagacaaccc tcagatttga tgatttccta gtagaactca 1440

cagaaataaa gagctgttat actgtg 1466

<210> 2
<211> 786
<212> DNA
<213> Homo sapiens

<400> 2
atgtgggacc tggttctctc catcgccttg tctgtggggt gcactggtgc cgtgcccctc 60

atccagtctc ggattgtggg aggctgggag tgtgagaagc attcccaacc ctggcaggtg 120

gctgtgtaca gtcatggatg ggcacactgt gggggtgtcc tggtgcaccc ccagtgggtg 180

ctcacagctg cccattgcct aaagaagaat agccaggtct ggctgggtcg gcacaacctg 240

tttgagcctg aagacacagg ccagagggtc cctgtcagcc acagcttccc acacccgctc 300

tacaatatga gccttctgaa gcatcaaagc cttagaccag atgaagactc cagccatgac 360

ctcatgctgc tccgcctgtc agagcctgcc aagatcacag atgttgtgaa ggtcctgggc 420

ctgcccaccc aggagccagc actggggacc acctgctacg cctcaggctg gggcagcatc 480

gaaccagagg agttcttgcg ccccaggagt cttcagtgtg tgagcctcca tctcctgtcc 540

aatgacatgt gtgctagagc ttactctgag aaggtgacag agttcatgtt gtgtgctggg 600

ctctggacag gtggtaaaga cacttgtggg ggtgattctg gggggtccact tgtctgtaat 660

ggggtgcttc aaggtatcac atcatggggc cctgagccat gtgccctgcc tgaaaagcct 720

gctgtgtaca ccaaggtggt gcattaccgg aagtggatca aggacaccat cgcagccaac 780

ccctga 786

<210> 3
<211> 503
<212> DNA
<213> Homo sapiens

<400> 3
gacagcggct tccttgatcc ttgccacccg cgactgaaca ccgacagcag cagcctcacc 60

atgaagttgc tgatggtcct catgctggcg gccctctccc agcactgcta cgcaggctct 120

ggctgcccct tattggagaa tgtgatttcc aagacaatca atccacaagt gtctaagact 180

gaatacaaag aacttcttca agagttcata gacgacaatg ccactacaaa tgccatagat 240

gaattgaagg aatgttttct taaccaaacg gatgaaactc tgagcaatgt tgaggtgttt 300

atgcaattaa tatatgacag cagtctttgt gatttatttt aactttctgc aagacctttg 360

gctcacagaa ctgcagggta tggtgagaaa ccaactacgg attgctgcaa accacacctt 420

ctctttctta tgtcttttta ctacaaacta caagacaatt gttgaaacct gctatacatg 480

tttattttaa taaattgatg gca 503

<210> 4
<211> 2560
<212> DNA
<213> Homo sapiens

<400> 4
gaattccggc cagaagaaat ctggcctcgg aacacgccat tctccgcgcc gcttccaata 60

accactaaca tccctaacga gcatccgagc cgagggctct gctcggaaat cgtcctggcc 120

caactcggcc cttcgagctc tcgaagatta ccgcatctat tttttttttc ttttttttct 180

tttcctagcg cagataaagt gagcccggaa agggaaggag ggggcgggga caccattgcc 240

ctgaaagaat aaataagtaa ataaacaaac tggctcctcg ccgcagctgg acgcggtcgg 300

ttgagtccag gttgggtcgg acctgaaccc ctaaaagcgg aaccgcctcc cgccctcgcc 360

atcccggagc tgagtcgccg gcggcggtgg ctgctgccag acccggagtt tcctctttca 420

ctggatggag ctgaactttg ggcggccaga gcagcacagc tgtccgggga tcgctgcacg 480

ctgagctccc tcggcaagac ccagcggcgg ctcgggattt ttttgggggg gcggggacca 540

gccccgcgcc ggcaccatgt tcctggcgac cctgtacttc gcgctgccgc tcttggactt 600

gctcctgtcg gccgaagtga gcggcggaga ccgcctggat tgcgtgaaag ccagtgatca 660

gtgcctgaag gagcagagct gcagcaccaa gtaccgcacg ctaaggcagt gcgtggcggg 720

caaggagacc aacttcagcc tggcatccgg cctggaggcc aaggatgagt gccgcagcgc 780

catggaggcc ctgaagcaga agtcgctcta caactgccgc tgcaagcggg gtatgaagaa 840

ggagaagaac tgcctgcgca tttactggag catgtaccag agcctgcagg gaaatgatct 900

gctggaggat tccccatatg aaccagttaa cagcagattg tcagatatat tccgggtggt 960

cccattcata tcagatgttt ttcagcaagt ggagcacatt cccaaaggga acaactgcct 1020

ggatgcagcg aaggcctgca acctcgacga catttgcaag aagtacaggt cggcgtacat 1080

cacccgtgc accaccagcg tgtccaacga tgtctgcaac cgccgcaagt gccacaaggc 1140

cctccggcag ttctttgaca aggtcccggc caagcacagc tacggaatgc tcttctgctc 1200

ctgccgggac atcgcctgca cagagcggag gcgacagacc atcgtgcctg tgtgctccta 1260

tgaagagagg gagaagccca actgtttgaa tttgcaggac tcctgcaaga cgaattacat 1320
ctgcagatct cgccttgcgg attttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg ggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160
aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 5
<211> 2560
<212> DNA
<213> Homo sapiens

<400> 5
gaattccggc cagaagaaat ctggcctcgg aacacgccat tctccgcgcc gcttccaata 60
accactaaca tccctaacga gcatccgagc cgagggctct gctcggaaat cgtcctggcc 120
caactcggcc cttcgagctc tcgaagatta ccgcatctat ttttttttc ttttttttct 180
tttcctagcg cagataaagt gagcccggaa agggaaggag gggcgggga caccattgcc 240
ctgaaagaat aaataagtaa ataaacaaac tggctcctcg ccgcagctgg acgcggtcgg 300

ttgagtccag gttgggtcgg acctgaaccc ctaaaagcgg aaccgcctcc cgccctcgcc 360
atcccggagc tgagtcgccg gcggcggtgg ctgctgccag acccggagtt tcctctttca 420
ctggatggag ctgaactttg ggcggccaga gcagcacagc tgtccgggga tcgctgcacg 480
ctgagctccc tcggcaagac ccagcggcgg ctcgggattt ttttgggggg gcggggacca 540
gccccgcgcc ggcaccatgt tcctggcgac cctgtacttc gcgctgccgc tcttggactt 600
gctcctgtcg gccgaagtga gcggcggaga ccgcctggat tgcgtgaaag ccagtgatca 660
gtgcctgaag gagcagagct gcagcaccaa gtaccgcacg ctaaggcagt gcgtggcggg 720
caaggagacc aacttcagcc tggcatccgg cctggaggcc aaggatgagt gccgcagcgc 780
catggaggcc ctgaagcaga agtcgctcta caactgccgc tgcaagcggg gtatgaagaa 840
ggagaagaac tgcctgcgca tttactggag catgtaccag agcctgcagg gaaatgatct 900
gctggaggat tccccatatg aaccagttaa cagcagattg tcagatatat tccgggtggt 960
cccattcata tcagatgttt ttcagcaagt ggagcacatt cccaaaggga acaactgcct 1020
ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260
tgaagagagg gagaagccca actgtttgaa tttgcaggac tcctgcaaga cgaattacat 1320
ctgcagatct cgccttgcgg attttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg gggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160

aaactcagaa ggctttggga tatgctgtat tttaaaggga cagtttgtaa cttgggctgt 2220

aaagcaaact ggggctgtgt tttcgatgat gatgatcatc atgatcatga tgattttaac 2280

agttttactt ctggcctttc ctagctagag aaggagttaa tatttctaag gtaactccca 2340

tatctccttt aatgacattg atttctaatg atataaattt cagcctacat tgatgccaag 2400

ctttttttgcc acaaagaaga ttcttaccaa gagtgggctt tgtggaaaca gctggtactg 2460

atgttcacct ttatatatgt actagcattt tccacgctga tgtttatgta ctgtaaacag 2520

ttctgcactc ttgtacaaaa gaaaaaacca cccggaattc 2560

<210> 6
<211> 2439
<212> DNA
<213> Homo sapiens

<400> 6
cagcacccag ctccccgcca ccgccatggt ccccgacacc gcctgcgttc ttctgctcac 60

cctggctgcc ctcggcgcgt ccggacaggg ccagagcccg ttgggctcag acctgggccc 120

gcagatgctt cgggaactgc aggaaaccaa cgcggcgctg caggacgtgc gggactggct 180

gcggcagcag gtcagggaga tcacgttcct gaaaaacacg gtgatggagt gtgacgcgtg 240

cgggatgcag cagtcagtac gcaccggcct acccagcgtg cggcccctgc tccactgcgc 300

gcccggcttc tgcttccccg gcgtggcctg catccagacg gagagcggcg gccgctgcgg 360

cccctgcccc gcgggcttca cgggcaacgg ctcgcactgc accgacgtca acgagtgcaa 420

cgcccacccc tgcttccccc gagtccgctg tatcaacacc agcccggggt tccgctgcga 480

ggcttgcccg ccggggtaca gcggccccac ccaccagggc gtggggctgg ctttcgccaa 540

ggccaacaag caggtttgca cggacatcaa cgagtgtgag accgggcaac ataactgcgt 600

ccccaactcc gtgtgcatca acacccgggg ctccttccag tgcggcccgt gccagcccgg 660

cttcgtgggc gaccaggcgt ccggctgcca gcgcggcgca cagcgcttct gccccgacgg 720

ctcgcccagc gagtgccacg agcatgcaga ctgcgtccta gagcgcgatg gctcgcggtc 780

gtgcgtgtgt cgcgttggct gggccggcaa cgggatcctc tgtggtcgcg acactgacct 840

agacggcttc ccggacgaga agctgcgctg cccggagccg cagtgccgta aggacaactg 900

cgtgactgtg cccaactcag ggcaggagga tgtggaccgc gatggcatcg gagacgcctg 960

cgatccggat gccgacgggg acggggtccc caatgaaaag gacaactgcc cgctggtgcg 1020

gaacccagac cagcgcaaca cggacgagga caagtggggc gatgcgtgcg acaactgccg 1080

gtcccagaag aacgacgacc aaaaggacac agaccaggac ggccggggcg atgcgtgcga 1140

cgacgacatc gacggcgacc ggatccgcaa ccaggccgac aactgcccta gggtacccaa 1200

```
ctcagaccag aaggacagtg atggcgatgg tataggggat gcctgtgaca actgtcccca   1260

gaagagcaac ccggatcagg cggatgtgga ccacgacttt gtgggagatg cttgtgacag   1320

cgatcaagac caggatggag acggacatca ggactctcgg gacaactgtc ccacggtgcc   1380

taacagtgcc caggaggact cagaccacga tggccagggt gatgcctgcg acgacgacga   1440

cgacaatgac ggagtccctg acagtcggga caactgccgc ctggtgccta accccggcca   1500

ggaggacgcg gacagggacg gcgtgggcga cgtgtgccag gacgactttg atgcagacaa   1560

ggtggtagac aagatcgacg tgtgtccgga gaacgctgaa gtcacgctca ccgacttcag   1620

ggccttccag acagtcgtgc tggacccgga gggtgacgcg cagattgacc ccaactgggt   1680

ggtgctcaac cagggaaggg agatcgtgca gacaatgaac agcgacccag gcctggctgt   1740

gggttacact gccttcaatg gcgtggactt cgagggcacg ttccatgtga acacggtcac   1800

ggatgacgac tatgcgggct tcatctttgg ctaccaggac agctccagct tctacgtggt   1860

catgtggaag cagatggagc aaacgtattg gcaggcgaac cccttccgtg ctgtggccga   1920

gcctggcatc caactcaagg ctgtgaagtc ttccacaggc cccggggaac agctgcggaa   1980

cgctctgtgg catacaggag acacagagtc ccaggtgcgg ctgctgtgga aggacccgcg   2040

aaacgtgggt tggaaggaca agaagtccta tcgttggttc ctgcagcacc ggccccaagt   2100

gggctacatc agggtgcgat tctatgaggg ccctgagctg gtggccgaca gcaacgtggt   2160

cttggacaca accatgcggg gtggccgcct ggggtcttc tgcttctccc aggagaacat   2220

catctgggcc aacctgcgtt accgctgcaa tgacaccatc ccagaggact atgagaccca   2280

tcagctgcgg caagcctagg gaccagggtg aggacccgcc ggatgacagc caccctcacc   2340

gcggctggat gggggctctg cacccagccc aaggggtggc cgtcctgagg gggaagtgag   2400

aagggctcag agaggacaaa ataaagtgtg tgtgcaggg                          2439
```

<210> 7
<211> 2439
<212> DNA
<213> Homo sapiens

<400> 7

```
cagcacccag ctccccgcca ccgccatggt ccccgacacc gcctgcgttc ttctgctcac     60

cctggctgcc ctcggcgcgt ccggacaggg ccagagcccg ttgggctcag acctgggccc    120

gcagatgctt cgggaactgc aggaaaccaa cgcggcgctg caggacgtgc gggactggct    180

gcggcagcag gtcagggaga tcacgttcct gaaaaacacg gtgatggagt gtgacgcgtg    240

cgggatgcag cagtcagtac gcaccggcct acccagcgtg cggcccctgc tccactgcgc    300

gcccggcttc tgcttccccg gcgtggcctg catccagacg gagagcggcg gccgctgcgg    360
```

```
ccccctgcccc gcgggcttca cgggcaacgg ctcgcactgc accgacgtca acgagtgcaa    420
cgcccacccc tgcttccccc gagtccgctg tatcaacacc agcccggggt tccgctgcga    480
ggcttgcccg ccggggtaca gcggccccac ccaccagggc gtggggctgg ctttcgccaa    540
ggccaacaag caggtttgca cggacatcaa cgagtgtgag accgggcaac ataactgcgt    600
ccccaactcc gtgtgcatca acacccgggg ctccttccag tgcggcccgt gccagcccgg    660
cttcgtgggc gaccaggcgt ccggctgcca gcgcggcgca cagcgcttct gccccgacgg    720
ctcgcccagc gagtgccacg agcatgcaga ctgcgtccta gagcgcgatg gctcgcggtc    780
gtgcgtgtgt cgcgttggct gggccggcaa cgggatcctc tgtggtcgcg acactgacct    840
agacggcttc ccggacgaga agctgcgctg cccggagccg cagtgccgta aggacaactg    900
cgtgactgtg cccaactcag ggcaggagga tgtggaccgc gatggcatcg gagacgcctg    960
cgatccggat gccgacgggg acggggtccc caatgaaaag gacaactgcc cgctggtgcg   1020
gaacccagac cagcgcaaca cggacgagga caagtggggc gatgcgtgcg acaactgccg   1080
gtcccagaag aacgacgacc aaaaggacac agaccaggac ggccggggcg atgcgtgcga   1140
cgacgacatc gacggcgacc ggatccgcaa ccaggccgac aactgcccta gggtacccaa   1200
ctcagaccag aaggacagtg atggcgatgg tataggggat gcctgtgaca actgtcccca   1260
gaagagcaac ccggatcagg cggatgtgga ccacgacttt gtgggagatg cttgtgacag   1320
cgatcaagac caggatggag acggacatca ggactctcgg gacaactgtc ccacggtgcc   1380
taacagtgcc caggaggact cagaccacga tggccagggt gatgcctgcg acgacgacga   1440
cgacaatgac ggagtccctg acagtcggga caactgccgc ctggtgccta accccggcca   1500
ggaggacgcg gacagggacg gcgtgggcga cgtgtgccag gacgactttg atgcagacaa   1560
ggtggtagac aagatcgacg tgtgtccgga gaacgctgaa gtcacgctca ccgacttcag   1620
ggccttccag acagtcgtgc tggacccgga gggtgacgcg cagattgacc ccaactgggt   1680
ggtgctcaac cagggaaggg agatcgtgca gacaatgaac agcgacccag gcctggctgt   1740
gggttacact gccttcaatg gcgtggactt cgagggcacg ttccatgtga acacggtcac   1800
ggatgacgac tatgcgggct tcatctttgg ctaccaggac agctccagct tctacgtggt   1860
catgtggaag cagatggagc aaacgtattg gcaggcgaac cccttccgtg ctgtggccga   1920
gcctggcatc caactcaagg ctgtgaagtc ttccacaggc cccggggaac agctgcggaa   1980
cgctctgtgg catacaggag acacagagtc ccaggtgcgg ctgctgtgga aggacccgcg   2040
aaacgtgggt tggaaggaca agaagtccta tcgttggttc ctgcagcacc ggccccaagt   2100
gggctacatc agggtgcgat tctatgaggg ccctgagctg gtggccgaca gcaacgtggt   2160
```

cttggacaca accatgcggg gtggccgcct gggggtcttc tgcttctccc aggagaacat 2220

catctgggcc aacctgcgtt accgctgcaa tgacaccatc ccagaggact atgagaccca 2280

tcagctgcgg caagcctagg gaccagggtg aggacccgcc ggatgacagc caccctcacc 2340

gcggctggat gggggctctg cacccagccc aaggggtggc cgtcctgagg gggaagtgag 2400

aagggctcag agaggacaaa ataaagtgtg tgtgcaggg 2439

<210> 8
<211> 1700
<212> DNA
<213> Homo sapiens

<400> 8
haggtgagcg gttgctcgtc gtcggggcgg ccggcagcgg cggctccagg gcccagcatg 60

cgcgggggac cccgcggcca ccatgtatgt gggctatgtg ctggacaagg attcgcccgt 120

gtaccccggc ccagccaggc cagccagcct cggcctgggc ccggcaaact acggcccccc 180

ggccccgccc ccggcgcccc cgcagtaccc cgacttctcc agctactctc acgtggagcc 240

ggcccccgcg cccccgacgg cctggggggc gcccttccct gcgcccaagg acgactgggc 300

cgccgcctac ggcccgggcc ccgcggcccc tgccgccagc ccagcttcgc tggcattcgg 360

gccccctcca gactttagcc cggtgccggc gccccctggg cccggcccgg gcctcctggc 420

gcagcccctc gggggcccgg gcacaccgtc ctcgcccgga gcgcagaggc cgacgcccta 480

cgagtggatg cggcgcagcg tggcggccgg aggcggcggt ggcagcggta agactcggac 540

caaggacaag taccgcgtgg tctacaccga ccaccaacgc ctggagctgg agaaggagtt 600

tcattacagc cgttacatca caatccggcg gaaatcagag ctggctgcca atctggggct 660

cactgaacgg caggtgaaga tctggttcca aaaccggcgg gcaaaggagc gcaaagtgaa 720

caagaagaaa cagcagcagc aacagccccc acagccgccg atggcccacg acatcacggc 780

caccccagcc gggccatccc tgggggggcct gtgtcccagc aacaccagcc tcctggccac 840

ctcctctcca atgcctgtga aagaggagtt tctgccatag ccccatgccc agcctgtgcg 900

ccgggggacc tggggactcg ggtgctggga gtgtggctcc tgtgggccca ggaggtctgg 960

tccgagtctc agccctgacc ttctgggaca tggtggacag tcacctatcc accctctgca 1020

tccccttggc ccattgtgtg cagtaagcct gttggataaa gaccttccag ctcctgtgtt 1080

ctagacctct gggggataag ggagtccagg gtggatgatc tcaatctccc gtgggcatct 1140

caagccccaa atggttgggg gaggggccta gacaaggctc caggccccac ctcctcctcc 1200

atacgttcag aggtgcagct ggaggcctgt gtggggacca cactgatcct ggagaaaagg 1260

gatggagctg aaaaagatgg aatgcttgca gagcatgacc tgaggaggga ggaacgtggt 1320

caactcacac ctgcctcttc tgcagcctca cctctacctg cccccatcat aagggcactg 1380

agcccttccc aggctggata ctaagcacaa agcccatagc actgggctct gatggctgct 1440

ccactgggtt acagaatcac agccctcatg atcattctca gtgagggctc tggattgaga 1500

gggaggccct gggaggagag aagggggcag agtcttccct accaggtttc tacacccccg 1560

ccaggctgcc catcagggcc cagggagccc ccagaggact ttattcggac caagcagagc 1620

tcacagctgg acaggtgttg tatatagagt ggaatctctt ggatgcagct tcaagaataa 1680

atttttcttc tcttttcaaa 1700

<210> 9
<211> 4098
<212> DNA
<213> Homo sapiens

<400> 9
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agctatggag ccgcggccca 180

cggcgccctc ctccggcgcc ccgggactgg ccggggtcgg ggagacgccg tcagccgctg 240

cgctggccgc agccagggtg gaactgcccg gcacggctgt gccctcggtg ccggaggatg 300

ctgcgcccgc gagccgggac ggcggcgggg tccgcgatga gggccccgcg gcggccgggg 360

acgggctggg cagacccttg ggcccaccc cgagccagag ccgtttccag gtggacctgg 420

tttccgagaa cgccgggcgg gccgctgctg cggcggcggc ggcggcggcg gcagcggcgg 480

cggctggtgc tggggcgggg gccaagcaga cccccgcgga cggggaagcc agcggcgaga 540

gcgagccagc taaaggcagc gaggaagcca agggccgctt ccgcgtgaac ttcgtggacc 600

cagctgcctc ctcgtcggct gaagacagcc tgtcagatgc tgccggggtc ggagtcgacg 660

ggcccaacgt gagcttccag aacggcgggg acacggtgct gagcgagggc agcagcctgc 720

actccggcgg cggcggcggc agtgggcacc accagcacta ctattatgat acccacacca 780

acacctacta cctgcgcacc ttcggccaca acaccatgga cgctgtgccc aggatcgatc 840

actaccggca cacagccgcg cagctgggcg agaagctgct ccggcctagc ctggcggagc 900

tccacgacga gctggaaaag gaaccttttg aggatggctt tgcaaatggg gaagaaagta 960

ctccaaccag agatgctgtg gtcacgtata ctgcagaaag taaaggagtc gtgaagtttg 1020

gctggatcaa gggtgtatta gtacgttgta tgttaaacat ttggggtgtg atgcttttca 1080

ttagattgtc atggattgtg ggtcaagctg gaataggtct atcagtcctt gtaataatga 1140

tggccactgt tgtgacaact atcacaggat tgtctacttc agcaatagca actaatggat 1200

ttgtaagagg aggaggagca tattatttaa tatctagaag tctagggcca gaatttggtg 1260

gtgcaattgg tctaatcttc gcctttgcca acgctgttgc agttgctatg tatgtggttg 1320

gatttgcaga aaccgtggtg gagttgctta aggaacattc catacttatg atagatgaaa 1380

tcaatgatat ccgaattatt ggagccatta cagtcgtgat tcttttaggt atctcagtag 1440

ctggaatgga gtgggaagca aaagctcaga ttgttctttt ggtgatccta cttcttgcta 1500

ttggtgattt cgtcatagga acatttatcc cactggagag caagaagcca aaagggtttt 1560

ttggttataa atctgaaata tttaatgaga actttgggcc cgattttcga gaggaagaga 1620

ctttcttttc tgtatttgcc atcttttttc ctgctgcaac tggtattctg gctggagcaa 1680

atatctcagg tgatcttgca gatcctcagt cagccatacc caaaggaaca ctcctagcca 1740

ttttaattac tacattggtt tacgtaggaa ttgcagtatc tgtaggttct tgtgttgttc 1800

gagatgccac tggaaacgtt aatgacacta tcgtaacaga gctaacaaac tgtacttctg 1860

cagcctgcaa attaaacttt gatttttcat cttgtgaaag cagtccttgt tcctatggcc 1920

taatgaacaa cttccaggta atgagtatgg tgtcaggatt tacaccacta atttctgcag 1980

gtatattttc agccactctt tcttcagcat tagcatccct agtgagtgct cccaaaatat 2040

ttcaggctct atgtaaggac aacatctacc cagctttcca gatgtttgct aaaggttatg 2100

ggaaaaataa tgaacctctt cgtggctaca tcttaacatt cttaattgca cttggattca 2160

tcttaattgc tgaactgaat gttattgcac caattatctc aaacttcttc cttgcatcat 2220

atgcattgat caatttttca gtattccatg catcacttgc aaaatctcca ggatggcgtc 2280

ctgcattcaa atactacaac atgtggatat cacttcttgg agcaattctt tgttgcatag 2340

taatgttcgt cattaactgg tgggctgcat tgctaacata tgtgatagtc cttgggctgt 2400

atatttatgt tacctacaaa aaaccagatg tgaattgggg atcctctaca caagccctga 2460

cttacctgaa tgcactgcag cattcaattc gtctttctgg agtggaagac cacgtgaaaa 2520

actttaggcc acagtgtctt gttatgacag gtgctccaaa ctcacgtcca gctttacttc 2580

atcttgttca tgatttcaca aaaaatgttg gtttgatgat ctgtggccat gtacatatgg 2640

gtcctcgaag acaagccatg aaagagatgt ccatcgatca agccaaatat cagcgatggc 2700

ttattaagaa caaaatgaag gcattttatg ctccagtaca tgcagatgac ttgagagaag 2760

gtgcacagta tttgatgcag gctgctggtc ttggtcgtat gaagccaaac acacttgtcc 2820

ttggatttaa gaaagattgg ttgcaagcag atatgaggga tgtggatatg tatataaact 2880

tatttcatga tgcttttgac atacaatatg gagtagtggt tattcgccta aaagaaggtc 2940

tggatatatc tcatcttcaa ggacaagaag aattattgtc atcacaagag aaatctcctg 3000

gcaccaagga tgtggtagta agtgtggaat atagtaaaaa gtccgattta gatacttcca 3060

aaccactcag tgaaaaacca attacacaca aagttgagga agaggatggc aagactgcaa 3120

ctcaaccact gttgaaaaaa gaatccaaag gccctattgt gcctttaaat gtagctgacc 3180

aaaagcttct tgaagctagt acacagtttc agaaaaaaca aggaaagaat actattgatg 3240

tctggtggct ttttgatgat ggaggtttga ccttattgat accttacctt ctgacgacca 3300

agaaaaaatg gaaagactgt aagatcagag tattcattgg tggaaagata aacagaatag 3360

accatgaccg gagagcgatg gctactttgc ttagcaagtt ccggatagac ttttctgata 3420

tcatggttct aggagatatc aataccaaac caaagaaaga aaatattata gcttttgagg 3480

aaatcattga gccatacaga cttcatgaag atgataaaga gcaagatatt gcagataaaa 3540

tgaaagaaga tgaaccatgg cgaataacag ataatgagct tgaactttat aagaccaaga 3600

cataccggca gatcaggtta aatgagttat taaaggaaca ttcaagcaca gctaatatta 3660

ttgtcatgag tctcccagtt gcacgaaaag gtgctgtgtc tagtgctctc tacatggcat 3720

ggttagaagc tctatctaag gacctaccac caatcctcct agttcgtggg aatcatcaga 3780

gtgtccttac cttctattca taaatgttct atacagtgga cagccctcca gaatggtact 3840

tcagtgccta gtgtagtaac ctgaaatctt caatgacaca ttaacatcac aatggcgaat 3900

ggtgactttt ctttcacgat ttcattaatt tgaaagcaca caggaaagct tgctccattg 3960

ataacgtgta tggagacttc ggttttagtc aattccatat ctcaatctta atggtgattc 4020

ttctctgttg aactgaagtt tgtgagagta gttttccttt gctacttgaa tagcaataaa 4080

agcgtgttaa ctttttgg 4098

<210> 10
<211> 4098
<212> DNA
<213> Homo sapiens

<400> 10
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agctatggag ccgcggccca 180

cggcgccctc ctccggcgcc ccgggactgg ccggggtcgg ggagacgccg tcagccgctg 240

cgctggccgc agccagggtg gaactgcccg gcacggctgt gccctcggtg ccggaggatg 300

ctgcgcccgc gagccgggac ggcggcgggg tccgcgatga gggccccgcg gcggccgggg 360

acgggctggg cagacccttg gggcccaccc cgagccagag ccgtttccag gtggacctgg 420

tttccgagaa cgccgggcgg gccgctgctg cggcggcggc ggcggcggcg gcagcggcgg 480

cggctggtgc tggggcgggg gccaagcaga cccccgcgga cggggaagcc agcggcgaga 540

gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atctttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340

taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct ttttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840
tcagtgccta gtgtagtaac ctgaaatctt caatgacaca ttaacatcac aatggcgaat 3900
ggtgactttt ctttcacgat ttcattaatt tgaaagcaca caggaaagct tgctccattg 3960
ataacgtgta tggagacttc ggttttagtc aattccatat ctcaatctta atggtgattc 4020
ttctctgttg aactgaagtt tgtgagagta gttttccttt gctacttgaa tagcaataaa 4080
agcgtgttaa ctttttgg 4098

<210> 11
<211> 3311
<212> DNA
<213> Homo sapiens

<400> 11
tgctaatgct tttggtacaa atggatgtgg aatataattg aatattttct tgtttaaggg 60

gagcatgaag aggtgttgag gttatgtcaa gcatctggca cagctgaagg cagatggaaa 120

tatttacaag tacgcaattt gagactaaga tattgttatc attctcctat tgaagacaag 180

agcaatagta aaacacatca ggtcaggggg ttaaagacct gtgataaacc acttccgata 240

agttggaaac gtgtgtctat attttcatat ctgtatatat ataatggtaa agaaagacac 300

cttcgtaacc cgcattttcc aaagagagga atcacaggga gatgtacagc aatggggcca 360

tttaagagtt ctgtgttcat cttgattctt caccttctag aaggggccct gagtaattca 420

ctcattcagc tgaacaacaa tggctatgaa ggcattgtcg ttgcaatcga ccccaatgtg 480

ccagaagatg aaacactcat tcaacaaata aaggacatgg tgacccaggc atctctgtat 540

ctgtttgaag ctacaggaaa gcgattttat ttcaaaaatg ttgccatttt gattcctgaa 600

acatggaaga caaaggctga ctatgtgaga ccaaaacttg agacctacaa aaatgctgat 660

gttctggttg ctgagtctac tcctccaggt aatgatgaac cctacactga gcagatgggc 720

aactgtggag agaagggtga aaggatccac ctcactcctg atttcattgc aggaaaaaag 780

ttagctgaat atggaccaca aggtaaggca tttgtccatg agtgggctca tctacgatgg 840

ggagtatttg acgagtacaa taatgatgag aaattctact tatccaatgg aagaatacaa 900

gcagtaagat gttcagcagg tattactggt acaaatgtag taaagaagtg tcagggaggc 960

agctgttaca ccaaaagatg cacattcaat aaagttacag gactctatga aaaaggatgt 1020

gagtttgttc tccaatcccg ccagacggag aaggcttcta taatgtttgc acaacatgtt 1080

gattctatag ttgaattctg tacagaacaa aaccacaaca aagaagctcc aaacaagcaa 1140

aatcaaaaat gcaatctccg aagcacatgg gaagtgatcc gtgattctga ggactttaag 1200

aaaaccactc ctatgacaac acagccacca aatcccacct tctcattgct gcagattgga 1260

caaagaattg tgtgtttagt ccttgacaaa tctggaagca tggcgactgg taaccgcctc 1320

aatcgactga atcaagcagg ccagcttttc ctgctgcaga cagttgagct ggggtcctgg 1380

gttgggatgg tgacatttga cagtgctgcc catgtacaaa gtgaactcat acagataaac 1440

agtggcagtg acagggacac actcgccaaa agattacctg cagcagcttc aggagggacg 1500

tccatctgca gcgggcttcg atcggcattt actgtgatta ggaagaaata tccaactgat 1560

ggatctgaaa ttgtgctgct gacggatggg gaagacaaca ctataagtgg gtgctttaac 1620

gaggtcaaac aaagtggtgc catcatccac acagtcgctt tggggccctc tgcagctcaa 1680

gaactagagg agctgtccaa aatgacagga ggtttacaga catatgcttc agatcaagtt 1740

cagaacaatg gcctcattga tgcttttggg gccctttcat caggaaatgg agctgtctct 1800

cagcgctcca tccagcttga gagtaaggga ttaaccctcc agaacagcca gtggatgaat 1860

ggcacagtga tcgtggacag caccgtggga aaggacactt tgtttcttat cacctggaca 1920

acgcagcctc cccaaatcct tctctgggat cccagtggac agaagcaagg tggctttgta 1980

gtggacaaaa acaccaaaat ggcctacctc caaatcccag gcattgctaa ggttggcact 2040

tggaaataca gtctgcaagc aagctcacaa accttgaccc tgactgtcac gtcccgtgcg 2100

tccaatgcta ccctgcctcc aattacagtg acttccaaaa cgaacaagga caccagcaaa 2160

ttccccagcc ctctggtagt ttatgcaaat attcgccaag gagcctcccc aattctcagg 2220

gccagtgtca cagccctgat tgaatcagtg aatggaaaaa cagttacctt ggaactactg 2280

gataatggag caggtgctga tgctactaag gatgacggtg tctactcaag gtatttcaca 2340

acttatgaca cgaatggtag atacagtgta aaagtgcggg ctctgggagg agttaacgca 2400

gccagacgga gagtgatacc ccagcagagt ggagcactgt acatacctgg ctggattgag 2460

aatgatgaaa tacaatggaa tccaccaaga cctgaaatta ataaggatga tgttcaacac 2520

aagcaagtgt gtttcagcag aacatcctcg ggaggctcat ttgtggcttc tgatgtccca 2580

aatgctccca tacctgatct cttcccacct ggccaaatca ccgacctgaa ggcggaaatt 2640

cacgggggca gtctcattaa tctgacttgg acagctcctg gggatgatta tgaccatgga 2700

acagctcaca agtatatcat tcgaataagt acaagtattc ttgatctcag agacaagttc 2760

aatgaatctc ttcaagtgaa tactactgct ctcatcccaa aggaagccaa ctctgaggaa 2820

gtctttttgt ttaaaccaga aaacattact tttgaaaatg gcacagatct tttcattgct 2880

attcaggctg ttgataaggt cgatctgaaa tcagaaatat ccaacattgc acgagtatct 2940

ttgtttattc ctccacagac tccgccagag acacctagtc ctgatgaaac gtctgctcct 3000

tgtcctaata ttcatatcaa cagcaccatt cctggcattc acattttaaa aattatgtgg 3060

aagtggatag gagaactgca gctgtcaata gcctagggct gaatttttgt cagataaata 3120

aaataaatca ttcatccttt ttttgattat aaaattttct aaaatgtatt ttagacttcc 3180

tgtagggggc gatatactaa atgtatatag tacatttata ctaaatgtat tcctgtaggg 3240

ggcgatatac taaatgtatt ttagacttcc tgtagggggc gataaaataa aatgctaaac 3300

aactgggtaa a 3311

<210> 12
<211> 3311
<212> DNA

<213> Homo sapiens

<400> 12

```
tgctaatgct tttggtacaa atggatgtgg aatataattg aatattttct tgtttaaggg 60
gagcatgaag aggtgttgag gttatgtcaa gcatctggca cagctgaagg cagatggaaa 120
tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt ggggccctc tgcagctcaa 1680
gaactagagg agctgtccaa aatgacagga ggtttacaga catatgcttc agatcaagtt 1740
```

cagaacaatg gcctcattga tgcttttggg gccctttcat caggaaatgg agctgtctct 1800

cagcgctcca tccagcttga gagtaaggga ttaaccctcc agaacagcca gtggatgaat 1860

ggcacagtga tcgtggacag caccgtggga aaggacactt tgtttcttat cacctggaca 1920

acgcagcctc cccaaatcct tctctgggat cccagtggac agaagcaagg tggctttgta 1980

gtggacaaaa acaccaaaat ggcctacctc caaatcccag gcattgctaa ggttggcact 2040

tggaaataca gtctgcaagc aagctcacaa accttgaccc tgactgtcac gtcccgtgcg 2100

tccaatgcta ccctgcctcc aattacagtg acttccaaaa cgaacaagga caccagcaaa 2160

ttccccagcc ctctggtagt ttatgcaaat attcgccaag gagcctcccc aattctcagg 2220

gccagtgtca cagccctgat tgaatcagtg aatggaaaaa cagttacctt ggaactactg 2280

gataatggag caggtgctga tgctactaag gatgacggtg tctactcaag gtatttcaca 2340

acttatgaca cgaatggtag atacagtgta aaagtgcggg ctctgggagg agttaacgca 2400

gccagacgga gagtgatacc ccagcagagt ggagcactgt acatacctgg ctggattgag 2460

aatgatgaaa tacaatggaa tccaccaaga cctgaaatta ataaggatga tgttcaacac 2520

aagcaagtgt gtttcagcag aacatcctcg ggaggctcat ttgtggcttc tgatgtccca 2580

aatgctccca tacctgatct cttcccacct ggccaaatca ccgacctgaa ggcggaaatt 2640

cacgggggca gtctcattaa tctgacttgg acagctcctg gggatgatta tgaccatgga 2700

acagctcaca agtatatcat tcgaataagt acaagtattc ttgatctcag agacaagttc 2760

aatgaatctc ttcaagtgaa tactactgct ctcatcccaa aggaagccaa ctctgaggaa 2820

gtctttttgt ttaaaccaga aaacattact tttgaaaatg gcacagatct tttcattgct 2880

attcaggctg ttgataaggt cgatctgaaa tcagaaatat ccaacattgc acgagtatct 2940

ttgtttattc ctccacagac tccgccagag acacctagtc ctgatgaaac gtctgctcct 3000

tgtcctaata ttcatatcaa cagcaccatt cctggcattc acattttaaa aattatgtgg 3060

aagtggatag gagaactgca gctgtcaata gcctagggct gaatttttgt cagataaata 3120

aaataaatca ttcatccttt ttttgattat aaaattttct aaaatgtatt ttagacttcc 3180

tgtagggggc gatatactaa atgtatatag tacatttata ctaaatgtat tcctgtaggg 3240

ggcgatatac taaatgtatt ttagacttcc tgtagggggc gataaaataa aatgctaaac 3300

aactgggtaa a 3311

<210> 13
<211> 2609
<212> DNA
<213> Homo sapiens

<220>

```
<221>  misc_feature
<222>  (2025)..(2025)
<223>  any kind of base

<220>
<221>  misc_feature
<222>  (2036)..(2036)
<223>  any kind of base

<220>
<221>  misc_feature
<222>  (2164)..(2164)
<223>  any kind of base

<220>
<221>  misc_feature
<222>  (2264)..(2264)
<223>  any kind of base

<400>  13
gctgatagca cagttctgtc cagagaagga aggcggaata aacttattca ttcccaggaa      60

ctcttggggt aggtgtgtgt ttttcacatc ttaaaggctc acagaccctg cgctggacaa     120

atgttccatt cctgaaggac ctctccagaa tccggattgc tgaatcttcc ctgttgccta     180

gaagggctcc aaaccacctc ttgacaatgg gaaactgggt ggttaaccac tggttttcag     240

ttttgtttct ggttgtttgg ttagggctga atgttttcct gtttgtggat gccttcctga     300

aatatgagaa ggccgacaaa tactactaca caagaaaaat ccttgggtca acattggcct     360

gtgcccgagc gtctgctctc tgcttgaatt ttaacagcac gctgatcctg cttcctgtgt     420

gtcgcaatct gctgtccttc ctgaggggca cctgctcatt ttgcagccgc acactgagaa     480

agcaattgga tcacaacctc accttccaca agctggtggc ctatatgatc tgcctacata     540

cagctattca catcattgca cacctgttta actttgactg ctatagcaga agccgacagg     600

ccacagatgg ctcccttgcc tccattctct ccagcctatc tcatgatgag aaaaaggggg     660

gttcttggct aaatcccatc cagtcccgaa acacgacagt ggagtatgtg acattcacca     720

gcgttgctgg tctcactgga gtgatcatga caatagcctt gattctcatg gtaacttcag     780

ctactgagtt catccggagg agttattttg aagtcttctg gtatactcac cacctttta      840

tcttctatat ccttggctta gggattcacg gcattggtgg aattgtccgg ggtcaaacag     900

aggagagcat gaatgagagt catcctcgca agtgtgcaga gtcttttgag atgtgggatg     960

atcgtgactc ccactgtagg cgccctaagt ttgaagggca tccccctgag tcttggaagt    1020

ggatccttgc accggtcatt ctttatatct gtgaaaggat cctccggttt taccgctccc    1080

agcagaaggt tgtgattacc aaggttgtta tgcacccatc caaagttttg gaattgcaga    1140
```

tgaacaagcg tggcttcagc atggaagtgg ggcagtatat ctttgttaat tgcccctcaa 1200

tctctctcct ggaatggcat ccttttactt tgacctctgc tccagaggaa gatttcttct 1260

ccattcatat ccgagcagca ggggactgga cagaaaatct cataagggct ttcgaacaac 1320

aatattcacc aattcccagg attgaagtgg atggtccctt tggcacagcc agtgaggatg 1380

ttttccagta tgaagtggct gtgctggttg gagcaggaat tggggtcacc ccctttgctt 1440

ctatcttgaa atccatctgg tacaaattcc agtgtgcaga ccacaacctc aaaacaaaaa 1500

agatctattt ctactggatc tgcagggaga caggtgcctt ttcctggttc aacaacctgt 1560

tgacttccct ggaacaggag atggaggaat taggcaaagt gggttttcta aactaccgtc 1620

tcttcctcac cggatgggac agcaatattg ttggtcatgc agcattaaac tttgacaagg 1680

ccactgacat cgtgacaggt ctgaaacaga aaacctcctt tgggagacca atgtgggaca 1740

atgagttttc tacaatagct acctcccacc ccaagtctgt agtgggagtt ttcttatgtg 1800

gccctcggac tttggcaaag agcctgcgca aatgctgtca ccgatattcc agtctggatc 1860

ctagaaaggt tcaattctac ttcaacaaag aaaatttttg agttatagga ataaggacgg 1920

taatctgcat tttgtctctt tgtatcttca gtaattgagt tataggaata aggacggtaa 1980

tctgcatttt gtctctttgt atcttcagta atttacttgg tctcntcagg tttgancagt 2040

cactttagga taagaatgtg cctctcaagc cttgactccc tggtattctt tttttgattg 2100

cattcaactt cgttacttga gcttcagcaa cttaagaact tctgaagttc ttaaagttct 2160

gaanttctta aagcccatgg atcctttctc agaaaaataa ctgtaaatct ttctggacag 2220

ccatgactgt agcaaggctt gatagcagaa gtttggtggt tcanaattat acaactaatc 2280

ccaggtgatt ttatcaattc cagtgttacc atctcctgag ttttggtttg taatcttttg 2340

tccctcccac ccccacagaa gattttaagt agggtgactt tttaaataaa aatttattga 2400

ataattaatg ataaaacata ataataaaca taaataataa acaaaattac cgagaacccc 2460

atccccatat aacaccaaca gtgtacatgt ttactgtcac ttttgatatg gtttatccag 2520

tgtgaacagc aatttattat ttttgctcat caaaaaataa aggatttttt ttcacttgaa 2580

aaaaaaaaaa aaaaaaaaaa aaaaaaaaa 2609

<210> 14
<211> 15720
<212> DNA
<213> Homo sapiens

<400> 14
caacccacac cgcccctgcc agccaccatg gggctgccac tagcccgcct ggcggctgtg 60

tgcctggccc tgtctttggc agggggctcg gagctccaga cagagggcag aacccgatac 120

```
cacggccgca acgtctgcag cacctggggc aacttccact acaagacctt cgacggggac     180
gtcttccgct tccccggcct ctgcgactac aacttcgcct ccgactgccg aggctcctac     240
aaggaatttg ctgtgcacct gaagcggggt ccgggccagg ctgaggcccc cgccggggtg     300
gagtccatcc tgctgaccat caaggatgac accatctacc tcacccgcca cctggctgtg     360
cttaacgggg ccgtggtcag caccccgcac tacagccccg ggctgctcat tgagaagagc     420
gatgcctaca ccaaagtcta ctcccgcgcc ggcctcaccc tcatgtggaa ccgggaggat     480
gcactcatgc tggagctgga cactaagttc cggaaccaca cctgtggcct ctgcggggac     540
tacaacggcc tgcagagcta ttcagaattc ctctctgacg gcgtgctctt cagtcccctg     600
gagtttggga acatgcagaa gatcaaccag cccgatgtgg tgtgtgagga tcccgaggag     660
gaggtggccc ccgcatcctg ctccgagcac cgcgccgagt gtgagaggct gctgaccgcc     720
gaggccttcg cggactgtca ggacctggtg ccgctggagc cgtatctgcg cgcctgccag     780
caggaccgct gccggtgccc gggcggtgac acctgcgtct gcagcaccgt ggccgagttc     840
tcccgccagt gctcccacgc cggcggccgg cccgggaact ggaggaccgc cacgctctgc     900
cccaagacct gccccgggaa cctggtgtac ctggagagcg gctcgccctg catggacacc     960
tgctcacacc tggaggtgag cagcctgtgc gaggagcacc gcatggacgg ctgtttctgc    1020
ccagaaggca ccgtatatga cgacatcggg gacagtggct gcgttcctgt gagccagtgc    1080
cactgcaggc tgcacggaca cctgtacaca ccgggccagg agatcaccaa tgactgcgag    1140
cagtgtgtct gtaacgctgg ccgctgggtg tgcaaagacc tgccctgccc cggcacctgt    1200
gccctggaag gcggctccca catcaccacc ttcgatggga agacgtacac cttccacggg    1260
gactgctact atgtcctggc caagggtgac cacaacgatt cctacgctct cctgggcgag    1320
ctggcccccct gtggctccac agacaagcag acctgcctga agacggtggt gctgctggct    1380
gacaagaaga agaatgcggt ggtcttcaag tccgatggca gtgtactgct caaccagctg    1440
caggtgaacc tgccccacgt gaccgcgagc ttctctgtct tccgcccgtc ttcctaccac    1500
atcatggtga gcatggccat tggcgtccgg ctgcaggtgc agctggcccc agtcatgcaa    1560
ctctttgtga cactggacca ggcctcccag ggggcaggtgc agggcctctg cgggaacttc    1620
aacggcctgg aaggtgacga cttcaagacg gccagcgggc tggtggaggc cacgggggcc    1680
ggctttgcca acacctggaa ggcacagtca acctgccatg acaagctgga ctggttggac    1740
gatccctgct ccctgaacat cgagagcgcc aactacgccg agcactggtg ctccctcctg    1800
aagaagacag agacccccctt tggcaggtgc cactcggctg tggaccctgc tgagtattac    1860
aagaggtgca aatatgacac gtgtaactgt cagaacaatg aggactgcct gtgcgccgcc    1920
```

ctgtcctcct acgcgcgcgc ctgcaccgcc aagggcgtca tgctgtgggg ctggcgggag 1980
catgtctgca acaaggatgt gggctcctgc cccaactcgc aggtcttcct gtacaacctg 2040
accacctgcc agcagacctg ccgctccctc tccgaggccg acagccactg tctcgagggc 2100
tttgcgcctg tggacggctg cggctgccct gaccacacct tcctggacga gaagggccgc 2160
tgcgtacccc tggccaagtg ctcctgttac caccgcggtc tctacctgga ggcgggggat 2220
gtggtcgtca ggcaggaaga acgatgtgtg tgccgggatg ggcggctgca ctgtaggcag 2280
atccggctga tcggccagag ctgcacggcc ccaaagatcc acatggactg cagcaacctg 2340
actgcactgg ccacctcgaa gccccgagcc ctcagctgcc agacgctggc cgccggctat 2400
taccacacag agtgtgtcag tggctgtgtg tgccccgacg ggctgatgga tgacggccgg 2460
ggtggctgcg tggtggagaa ggaatgccct tgcgtccata acaacgacct gtattcttcc 2520
ggcgccaaga tcaaggtgga ctgcaatacc tgcacctgca agagaggacg ctgggtgtgc 2580
acccaggctg tgtgccatgg cacctgctcc atttacggga gtggccacta catcaccttt 2640
gatgggaagt actacgactt tgacggacac tgctcctacg tggctgttca ggactactgc 2700
ggccagaact cctcactggg ctcattcagc atcatcaccg agaacgtccc ctgtggcact 2760
acgggcgtca cctgctccaa ggccatcaag atcttcatgg ggaggacgga gctgaagttg 2820
gaagacaagc accgtgtggt gatccagcgt gatgagggtc accacgtggc ctacaccacg 2880
cgggaggtgg gccagtacct ggtggtggag tccagcacgg gcatcatcgt catctgggac 2940
aagaggacca ccgtgttcat caagctggct ccctcctaca agggcaccgt gtgtggcctg 3000
tgtgggaact ttgaccaccg ctccaacaac gacttcacca cgcgggacca catggtggtg 3060
agcagcgagc tggacttcgg gaacagctgg aaggaggccc ccacctgccc agatgtgagc 3120
accaaccccg agccctgcag cctgaacccg caccgccgct cctgggccga gaagcagtgc 3180
agcatcctca aaagcagcgt gttcagcatc tgccacagca aggtggaccc caagcccttc 3240
tacgaggcct gtgtgcacga ctcgtgctcc tgtgacacgg gtggggactg tgagtgcttc 3300
tgctctgccg tggcctccta cgcccaggag tgtaccaaag agggggcctg cgtgttctgg 3360
aggacgccgg acctgtgccc catattctgc gactactaca accctccgca tgagtgtgag 3420
tggcactatg agccatgtgg gaaccggagc ttcgagacct gcaggaccat caacggcatc 3480
cactccaaca tctccgtgtc ctacctggag ggctgctacc cccggtgccc caaggacagg 3540
cccatctatg aggaggatct gaagaagtgt gtcactgcag acaagtgtgg ctgctatgtc 3600
gaggacaccc actacccacc tggagcatcg gttcccaccg aggagacctg caagtcctgc 3660
gtgtgtacca actcctccca agtcgtctgc aggccggagg aaggaaagat tcttaaccag 3720
acccaggatg gcgccttctg ctactgggag atctgtggcc ccaacgggac ggtggagaag 3780

cacttcaaca tctgttccat tacgacacgc ccgtccaccc tgaccacctt caccaccatc 3840
accctcccca ccaccccac ctccttcacc actaccacca ccaccaccac cccgacctcc 3900
agcacagttt tatcaacaac tccgaagctg tgctgcctct ggtctgactg gatcaatgag 3960
gaccacccca gcagtggcag cgacgacggt gaccgagaac catttgatgg ggtctgcggg 4020
gcccctgagg acatcgagtg caggtcggtc aaggatcccc acctcagctt ggagcagcat 4080
ggccagaagg tgcagtgtga tgtctctgtt gggttcattt gcaagaatga agaccagttt 4140
ggaaatggac catttggact gtgttacgac tacaagatac gtgtcaattg ttgctggccc 4200
atggataagt gtatcaccac tcccagccct ccaactacca ctcccagccc tccaccaacc 4260
acgacgacca cccttccacc aaccaccacc cccagccctc caaccaccac cacaaccacc 4320
cctccaccaa ccaccacccc cagccctcca ataaccacca cgaccacccc tctaccaacc 4380
accactccca gccctccaat aagcaccaca accacccctc caccaaccac cactcccagc 4440
cctccaacca ccactcccag ccctccaacc accactccca gccctccaac aaccaccaca 4500
accacccctc caccaaccac cactcccagc cctccaatga ctacgcccat cactccacca 4560
gccagcacta ccacccttcc accaaccacc actcccagcc ctccaacaac caccacaacc 4620
acccctccac caaccaccac tcccagtcct ccaacgacta cgcccatcac tccaccaacc 4680
agcactacta cccttccacc aaccaccact cccagccctc caccaaccac cacaaccacc 4740
cctccaccaa ccaccactcc cagccctcca acaaccacca ctcccagtcc tccaacaatc 4800
accacaacca cccctccacc aaccaccact cccagccctc caacaacgac cacaaccacc 4860
cctccaccaa ccaccactcc cagccctcca acgactacac ccatcactcc accaaccagc 4920
actaccaccc ttccaccaac caccactccc agccctccac caaccaccac aaccacccct 4980
ccaccaacca ccactcccag ccctccaaca accaccactc ccagccctcc aataaccacc 5040
acaaccaccc ctccaccaac caccactccc agctctccaa taaccaccac tcccagccct 5100
ccaacaacca ccatgaccac cccttcacca accaccaccc ccagctctcc aataaccacc 5160
acaaccaccc cttcctcaac taccactccc agccctccac caaccaccat gaccacccct 5220
tcaccaacca ccactcccag ccctccaaca accaccatga ccacccttcc accaaccacc 5280
acttccagcc ctctaacaac tactcctcta cctccatcaa taactcctcc tacattttca 5340
ccattctcaa cgacaacccc tactacccca tgcgtgcctc tctgcaattg gactggctgg 5400
ctggattctg gaaaacccaa ctttcacaaa ccaggtggag acacagaatt gattggagac 5460
gtctgtggac caggctgggc agctaacatc tcttgcagag ccaccatgta tcctgatgtt 5520
cccattggac agcttggaca aacagtggtg tgtgatgtct ctgtggggct gatatgcaaa 5580

aatgaagacc aaaagccagg tggggtcatc cctatggcct tctgcctcaa ctacgagatc 5640
aacgttcagt gctgtgagtg tgtcacccaa cccaccacca tgacaaccac caccacagag 5700
aacccaactc cgccaaccac gacacccatc accaccacca ctacggtgac cccaacccca 5760
acacccaccg gcacacagac cccaaccacg acacccatca ccaccaccac tacggtgacc 5820
ccaaccccaa cacccaccgg cacacagacc ccaaccacga cacccatcac caccaccact 5880
acggtgaccc caaccccaac acccaccggc acacagaccc caaccacgac acccatcacc 5940
accaccacta cggtgacccc aaccccaaca cccaccggca cacagacccc aaccacgaca 6000
cccatcacca ccaccactac ggtgacccca accccaacac ccaccggcac acagacccca 6060
accacgacac ccatcaccac caccactacg gtgaccccaa ccccaacacc caccggcaca 6120
cagaccccaa ccacgacacc catcaccacc accactacgg tgaccccaac cccaacaccc 6180
accggcacac agaccccaac cacgacaccc atcaccacca ccactacggt gaccccaacc 6240
ccaacaccca ccggcacaca gaccccaacc acgacaccca tcaccaccac cactacggtg 6300
accccaaccc caacacccac cggcacacag accccaacca cgacacccat caccaccacc 6360
actacggtga ccccaacccc aacacccacc ggcacacaga ccccaaccac gacacccatc 6420
accaccacca ctacggtgac cccaacccca acacccaccg gcacacagac cccaaccacg 6480
acacccatca ccaccaccac tacggtgacc ccaaccccaa cacccaccgg cacacagacc 6540
ccaaccacga cacccatcac caccaccact acggtgaccc caaccccaac acccaccggc 6600
acacagaccc caaccacgac acccatcacc accaccacta cggtgacccc aaccccaaca 6660
cccaccggca cacagacccc aaccacgaca cccatcacca ccaccactac ggtgacccca 6720
accccaacac ccaccggcac acagacccca accacgacac ccatcaccac caccactacg 6780
gtgaccccaa ccccaacacc caccggcaca cagaccccaa ccacgacacc catcaccacc 6840
accactacgg tgaccccaac cccaacaccc accggcacac agaccccaac cacgacaccc 6900
atcaccacca ccactacggt gaccccaacc ccaacaccca ccggcacaca gaccccaacc 6960
acgacaccca tcaccaccac cactacggtg accccaaccc caacacccac cggcacacag 7020
accccaacca cgacacccat caccaccacc actacggtga ccccaacccc aacacccacc 7080
ggcacacaga ccccaaccac gacacccatc accaccacca ctacggtgac cccaacccca 7140
acacccaccg gcacacagac cccaaccacg acacccatca ccaccaccac tacggtgacc 7200
ccaaccccaa cacccaccgg cacacagacc ccaaccacga cacccatcac caccaccact 7260
acggtgaccc caaccccaac acccaccggc acacagaccc caaccacgac acccatcacc 7320
accaccacta cggtgacccc aaccccaaca cccaccggca cacagacccc aaccacgaca 7380
cccatcacca ccaccactac ggtgacccca accccaacac ccaccggcac acagacccca 7440

accacgacac ccatcaccac caccactacg gtgaccccaa ccccaacacc caccggcaca 7500

cagaccccaa ccacgacacc catcaccacc accactacgg tgaccccaac cccaacaccc 7560

accggcacac agaccccaac cacgacaccc atcaccacca ccactacggt gaccccaacc 7620

ccaacaccca ccggcacaca gaccccaacc acgacaccca tcaccaccac cactacggtg 7680

accccaaccc caacacccac cggcacacag accccaacca cgacacccat caccaccacc 7740

actacggtga ccccaacccc aacacccacc ggcacacaga ccccaaccac gacacccatc 7800

accaccacca ctacggtgac cccaacccca acacccaccg gcacacagac cccaaccacg 7860

acacccatca ccaccaccac tacggtgacc ccaaccccaa cacccaccgg cacacagacc 7920

ccaaccacga cacccatcac caccaccact acggtgaccc caaccccaac acccaccggc 7980

acacagaccc caaccacgac acccatcacc accaccacta cggtgacccc aaccccaaca 8040

cccaccggca cacagacccc aaccacgaca cccatcacca ccaccactac ggtgacccca 8100

accccaacac ccaccggcac acagacccca accacgacac ccatcaccac caccactacg 8160

gtgaccccaa ccccaacacc caccggcaca cagaccccaa ccacgacacc catcaccacc 8220

accactacgg tgaccccaac cccaacaccc accggcacac agaccccaac cacgacaccc 8280

atcaccacca ccactacggt gaccccaacc ccaacaccca ccggcacaca gaccccaacc 8340

acgacaccca tcaccaccac cactacggtg accccaaccc caacacccac cggcacacag 8400

accccaacca cgacacccat caccaccacc actacggtga ccccaacccc aacacccacc 8460

ggcacacaga ccccaaccac gacacccatc accaccacca ctacggtgac cccaacccca 8520

acacccaccg gcacacagac cccaaccacg acacccatca ccaccaccac tacggtgacc 8580

ccaaccccaa cacccaccgg cacacagacc ccaaccacga cacccatcac caccaccact 8640

acggtgaccc caaccccaac acccaccggc acacagaccc caaccacgac acccatcacc 8700

accaccacta cggtgacccc aaccccaaca cccaccggca cacagacccc aaccacgaca 8760

cccatcacca ccaccactac ggtgacccca accccaacac ccaccggcac acagacccca 8820

accacgacac ccatcaccac caccactacg gtgaccccaa ccccaacacc caccggcaca 8880

cagaccccaa ccacgacacc catcaccacc accactacgg tgaccccaac cccaacaccc 8940

accggcacac agaccccaac cacgacaccc atcaccacca ccactacggt gaccccaacc 9000

ccaacaccca ccggcacaca gaccccaacc acgacaccca tcaccaccac cactacggtg 9060

accccaaccc caacacccac cggcacacag accccaacca cgacacccat caccaccacc 9120

actacggtga ccccaacccc aacacccacc ggcacacaga ccccaaccac gacacccatc 9180

accaccacca ctacggtgac cccaacccca acacccaccg gcacacagac cccaaccacg 9240

acacccatca ccaccaccac tacggtgacc ccaaccccaa cacccaccgg cacacagacc 9300
ccaaccacga cacccatcac caccaccact acggtgaccc caaccccaac acccaccggc 9360
acacagaccc caaccacgac acccatcacc accaccacta cggtgacccc aaccccaaca 9420
cccaccggca cacagacccc aaccacgaca cccatcacca ccaccactac ggtgacccca 9480
accccaacac ccaccggcac acagacccca accacgacac ccatcaccac caccactacg 9540
gtgaccccaa ccccaacacc caccggcaca cagaccccaa ccacgacacc catcaccacc 9600
accactacgg tgaccccaac cccaacaccc accggcacac agaccccaac cacgacaccc 9660
atcaccacca ccactacggt gaccccaacc ccaacaccca ccggcacaca gaccccaacc 9720
acgacaccca tcaccaccac cactacggtg accccaaccc caacacccac cggcacacag 9780
accccaacca cgacacccat caccaccacc actacggtga ccccaacccc aacacccacc 9840
ggcacacaga ccccaaccac gacacccatc accaccacca ctacggtgac cccaacccca 9900
acacccaccg gcacacagac cccaaccacg acacccatca ccaccaccac tacggtgacc 9960
ccaaccccaa cacccaccgg cacacagacc ccaaccacga cacccatcac caccaccact 10020
acggtgaccc caaccccaac acccaccggc acacagaccc caaccacgac acccatcacc 10080
accaccacta cggtgacccc aaccccaaca cccaccggca cacagacccc aaccacgaca 10140
cccatcacca ccaccactac ggtgacccca accccaacac ccaccggcac acagacccca 10200
accacgacac ccatcaccac caccactacg gtgaccccaa ccccaacacc caccggcaca 10260
cagaccccaa ccacgacacc catcaccacc accactacgg tgaccccaac cccaacaccc 10320
accggcacac agaccccaac cacgacaccc atcaccacca ccactacggt gaccccaacc 10380
ccaacaccca ccggcacaca gaccccaacc acgacaccca tcaccaccac cactacggtg 10440
accccaaccc caacacccac cggcacacag accccaacca cgacacccat caccaccacc 10500
actacggtga ccccaacccc aacacccacc ggcacacaga ccccaaccac gacacccatc 10560
accaccacca ctacggtgac cccaacccca acacccaccg gcacacagac cccaaccacg 10620
acacccatca ccaccaccac tacggtgacc ccaaccccaa cacccaccgg cacacagacc 10680
ccaaccacga cacccatcac caccaccact acggtgaccc caaccccaac acccaccggc 10740
acacagaccc caaccacgac acccatcacc accaccacta cggtgacccc aaccccaaca 10800
cccaccggca cacagacccc aaccacgaca cccatcacca ccaccactac ggtgacccca 10860
accccaacac ccaccggcac acagacccca accacgacac ccatcaccac caccactacg 10920
gtgaccccaa ccccaacacc caccggcaca cagaccccaa ccacgacacc catcaccacc 10980
accactacgg tgaccccaac cccaacaccc accggcacac agaccccaac cacgacaccc 11040
atcaccacca ccactacggt gaccccaacc ccaacaccca ccggcacaca gaccccaacc 11100

acgacaccca tcaccaccac cactacggtg accccaaccc caacacccac cggcacacag 11160
accccaacca cgacacccat caccaccacc actacggtga ccccaacccc aacacccacc 11220
ggcacacaga ccccaaccac gacacccatc accaccacca ctacggtgac cccaacccca 11280
acacccaccg gcacacagac cccaaccacg acacccatca ccaccaccac tacggtgacc 11340
ccaaccccaa cacccaccgg cacacagacc ccaaccacga cacccatcac caccaccact 11400
acggtgaccc caaccccaac acccaccggc acacagaccc caaccacgac acccatcacc 11460
accaccacta cggtgacccc aaccccaaca cccaccggca cacagacccc aaccacgaca 11520
cccatcacca ccaccactac ggtgacccca accccaacac ccaccggcac acagacccca 11580
accacgacac ccatcaccac caccactacg gtgaccccaa ccccaacacc caccggcaca 11640
cagaccccaa ccacgacacc catcaccacc accactacgg tgaccccaac cccaacaccc 11700
accggcacac agaccccaac cacgacaccc atcaccacca ccactacggt gaccccaacc 11760
ccaacaccca ccggcacaca gaccccaacc acgacaccca tcaccaccac cactacggtg 11820
accccaaccc caacacccac cggcacacag accccaacca cgacacccat caccaccacc 11880
actacggtga ccccaacccc aacacccacc ggcacacaga ccccaaccac gacacccatc 11940
accaccacca ctacggtgac cccaacccca acacccaccg gcacacagac cccaaccacg 12000
acacccatca ccaccaccac tacggtgacc ccaaccccaa cacccaccgg cacacagacc 12060
ccaaccacga cacccatcac caccaccact acggtgaccc caaccccaac acccaccggc 12120
acacagaccc caaccacgac acccatcacc accaccacta cggtgacccc aaccccaaca 12180
cccaccggca cacagacccc aaccacgaca cccatcacca ccaccactac ggtgacccca 12240
accccaacac ccaccggcac acagacccca accacgacac ccatcaccac caccactacg 12300
gtgaccccaa ccccaacacc caccggcaca cagaccccaa ccacgacacc catcaccacc 12360
accactacgg tgaccccaac cccaacaccc accggcacac agaccccaac cacgacaccc 12420
atcaccacca ccactacggt gaccccaacc ccaacaccca ccggcacaca gaccccaacc 12480
acgacaccca tcaccaccac cactacggtg accccaaccc caacacccac cggcacacag 12540
accccaacca cgacacccat caccaccacc actacggtga ccccaacccc aacacccacc 12600
ggcacacaga ccgggccccc cacccacaca agcacagcac cgattgctga gttgaccaca 12660
tccaatcctc cgcctgagtc ctcaacccct cagacctctc ggtccacctc ttcccctctc 12720
acggagtcaa ccaccccttct gagtacccta ccacctgcca ttgagatgac cagcacggcc 12780
ccaccctcca cacccacggc acccacgacc acgagcggag gccacacact gtctccaccg 12840
cccagcacca ccacgtcccc tccaggcacc cccactcgcg gtaccacgac cgggtcatct 12900

tcagcccccca cccccagcac tgtgcagacg accaccacca gtgcctggac cccaacgccg 12960

accccactct ccacacccag catcatcagg accacaggcc tgaggcccta cccttcctct 13020

gtgcttatct gctgtgtcct gaacgacacc tactacgcac caggtgagga ggtgtacaac 13080

ggcacatacg gagacacctg ttatttcgtc aactgctcac tgagctgtac gttggagttc 13140

tataactggt cctgcccatc cacgccctcc ccaacaccca cgccctccaa gtcgacgccc 13200

acgccttcca agccatcgtc cacgccctcc aagccgacgc ccggcaccaa gcccccccgag 13260

tgcccagact ttgatcctcc cagacaggag aacgagactt ggtggctgtg cgactgcttc 13320

atggccacgt gcaagtacaa caacacggtg gagatcgtga aggtggagtg tgagccgccg 13380

cccatgccca cctgctccaa cggcctccaa cccgtgcgcg tcgaggaccc cgacggctgc 13440

tgctggcact gggagtgcga ctgctactgc acgggctggg gcgacccgca ctatgtcacc 13500

ttcgacggac tctactacag ctaccagggc aactgcacct acgtgctggt ggaggagatc 13560

agcccctccg tggacaactt cggagtttac atcgacaact accactgcga tcccaacgac 13620

aaggtgtcct gtccccgcac cctcatcgtg cgccacgaga cccaggaggt gctgatcaag 13680

accgtgcata tgatgcccat gcaggtgcag gtgcaggtga acaggcaggc ggtggcactg 13740

ccctacaaga agtacgggct ggaggtgtac cagtctggca tcaactacgt ggtggacatc 13800

cccgagctgg gtgtcctcgt ctcctacaat ggcctgtcct tctccgtcag gctgccctac 13860

caccggtttg gcaacaacac caagggccag tgtggcacct gcaccaacac cacctccgac 13920

gactgcattc tgcccagcgg ggagatcgtc tccaactgtg aggctgcggc tgaccagtgg 13980

ctggtgaacg acccctccaa gccacactgc cccacagca gctccacgac caagcgcccg 14040

gccgtcactg tgcccggggg cggtaaaacg accccacaca aggactgcac cccatctccc 14100

ctctgccagc tcatcaagga cagcctgttt gcccagtgcc acgcactggt gcccccgcag 14160

cactactacg atgcctgcgt gttcgacagc tgcttcatgc cgggctcgag cctggagtgc 14220

gccagtctgc aggcctacgc agccctctgt gcccagcaga acatctgcct cgactggcgg 14280

aaccacacgc atggggcctg cttggtggag tgcccatctc acagggagta ccaggcctgt 14340

ggccctgcag aagagcccac gtgcaaatcc agctcctccc agcagaacaa cacagtcctg 14400

gtggaaggct gcttctgtcc tgagggcacc atgaactacg ctcctggctt tgatgtctgc 14460

gtgaagacct gcggctgtgt gggacctgac aatgtgccca gagagtttgg ggagcacttc 14520

gagttcgact gcaagaactg tgtctgcctg gagggtggaa gtggcatcat ctgccaaccc 14580

aagaggtgca gccagaagcc cgttacccac tgcgtggaag acggcaccta cctcgccacg 14640

gaggtcaacc ctgccgacac ctgctgcaac attaccgtct gcaagtgcaa caccagcctg 14700

tgcaaagaga agccctccgt gtgcccgctg ggattcgaag tgaagagcaa gatggtgcct 14760

```
ggaaggtgct gtcccttcta ctggtgtgag tccaaggggg tgtgtgttca cgggaatgct  14820
gagtaccagc ccggttctcc agtttattcc tccaagtgcc aggactgcgt gtgcacggac  14880
aaggtggaca acaacaccct gctcaacgtc atcgcctgca cccacgtgcc ctgcaacacc  14940
tcctgcagcc ctggcttcga actcatggag gcccccgggg agtgctgtaa gaagtgtgaa  15000
cagacgcact gtatcatcaa acggcccgac aaccagcacg tcatcctgaa gcccggggac  15060
ttcaagagcg acccgaagaa caactgcaca ttcttcagct gcgtgaagat ccacaaccag  15120
ctcatctcgt ccgtctccaa catcacctgc cccaactttg atgccagcat ttgcatcccg  15180
ggctccatca cattcatgcc caatggatgc tgcaagacct gcacccctcg caatgagacc  15240
agggtgccct gctccaccgt ccccgtcacc acggaggttt cgtacgccgg ctgcaccaag  15300
accgtcctca tgaatcattg ctccgggtcc tgcgggacat ttgtcatgta ctcggccaag  15360
gcccaggccc tggaccacag ctgctcctgc tgcaaagagg agaaaaccag ccagcgtgag  15420
gtggtcctga gctgccccaa tggcggctcg ctgacacaca cctacaccca catcgagagc  15480
tgccagtgcc aggacaccgt ctgcgggctc ccccaccggca cctcccgccg ggcccggcgc  15540
tcccctaggc atctggggag cgggtgagcg gggtgggcac agccccctc actgccctcg  15600
acagctttac ctcccccgga ccctctgagc ctcctaagct cggcttcctc tcttcagata  15660
tttattgtct gagtctttgt tcagtccttg ctttccaata ataaactcag gggacatgc  15720


<210>  15
<211>  3697
<212>  DNA
<213>  Homo sapiens

<400>  15
agggagtgtt cccgggggag atactccagt cgtagcaaga gtctcgacca ctgaatggaa     60
gaaaaggact tttaaccacc attttgtgac ttacagaaag gaatttgaat aaagaaaact    120
atgatacttc aggcccatct tcactccctg tgtcttctta tgctttattt ggcaactgga    180
tatggccaag aggggaagtt tagtggaccc ctgaaaccca tgacattttc tatttatgaa    240
ggccaagaac cgagtcaaat tatattccag tttaaggcca atcctcctgc tgtgactttt    300
gaactaactg gggagacaga caacatattt gtgatagaac gggagggact tctgtattac    360
aacagagcct tggacaggga aacaagatct actcacaatc tccaggttgc agccctggac    420
gctaatggaa ttatagtgga gggtccagtc cctatcacca tagaagtgaa ggacatcaac    480
gacaatcgac ccacgtttct ccagtcaaag tacgaaggct cagtaaggca gaactctcgc    540
ccaggaaagc ccttcttgta tgtcaatgcc acagacctgg atgatccggc cactcccaat    600
ggccagcttt attaccagat tgtcatccag cttcccatga tcaacaatgt catgtacttt    660
```

cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag accctttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccaccct tggaaggcat tgtttcttta 2400
ccagttacat tctgcagttg tgtggaagga agttgtttcc ggccagcagg tcaccagact 2460

gggataccca ctgtgggcat ggcagttggt atactgctga ccacccttct ggtgattggt 2520

ataattttag cagttgtgtt tatccgcata aagaaggata aaggcaaaga taatgttgaa 2580

agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700

tgcattataa ttttttaaac agatattccc tcttgtcctt taatatttgc taaatatttc 2760

tttttttgagg tggagtcttg ctctgtcgcc caggctggag tacagtggtg tgatcccagc 2820

tcactgcaac ctccgcctcc tgggttcaca tgattctcct gcctcagctt cctaagtagc 2880

tgggtttaca ggcacccacc accatgccca gctaattttt gtatttttaa tagagacggg 2940

gtttcgccat ttggccaggc tggtcttgaa ctcctgacgt caagtgatct gcctgccttg 3000

gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tccctttttgg ggcaagacag actcattaaa tattctgtac atttttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 16
<211> 3697
<212> DNA
<213> Homo sapiens

<400> 16
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420
gctaatggaa ttatagtgga gggtccagtc cctatcacca tagaagtgaa ggacatcaac 480
gacaatcgac ccacgtttct ccagtcaaag tacgaaggct cagtaaggca gaactctcgc 540
ccaggaaagc ccttcttgta tgtcaatgcc acagacctgg atgatccggc cactcccaat 600
ggccagcttt attaccagat tgtcatccag cttcccatga tcaacaatgt catgtacttt 660
cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
gggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220

ccccatttta cattttccct cggcagtgga agcttacaaa acgactggga agtttccaaa 2280

atcaatggta ctcatgcccg actgtctacc aggcacacag agtttgagga gagggagtat 2340

gtcgtcttga tccgcatcaa tgatgggggt cggccaccct tggaaggcat tgtttcttta 2400

ccagttacat tctgcagttg tgtggaagga agttgtttcc ggccagcagg tcaccagact 2460

gggataccca ctgtgggcat ggcagttggt atactgctga ccacccttct ggtgattggt 2520

ataattttag cagttgtgtt tatccgcata aagaaggata aaggcaaaga taatgttgaa 2580

agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700

tgcattataa ttttttaaac agatattccc tcttgtcctt taatatttgc taaatatttc 2760

ttttttgagg tggagtcttg ctctgtcgcc caggctggag tacagtggtg tgatcccagc 2820

tcactgcaac ctccgcctcc tgggttcaca tgattctcct gcctcagctt cctaagtagc 2880

tgggtttaca ggcacccacc accatgccca gctaattttt gtatttttaa tagagacggg 2940

gtttcgccat ttggccaggc tggtcttgaa ctcctgacgt caagtgatct gcctgccttg 3000

gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tccctttgg ggcaagacag actcattaaa tattctgtac atttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 17
<211> 1597
<212> DNA
<213> Homo sapiens

<400> 17
aacaaactgc acccactgaa ctccgcagct agcatccaaa tcagcccttg agatttgagg 60

ccttggagac tcaggagttt tgagagcaaa atgacaacac ccagaaattc agtaaatggg 120

```
actttcctgg cagagccaat gaaaggccct attgctatgc aatctggtcc aaaaccactc    180
ttcaggagga tgtcttcact ggtgggcccc acgcaaagct tcttcatgag ggaatctaag    240
actttggggg ctgtccagat tatgaatggg ctcttccaca ttgccctggg gggtcttctg    300
atgatcccag cagggatcta tgcacccatc tgtgtgactg tgtggtaccc tctctgggga    360
ggcattatgt atattatttc cggatcactc ctggcagcaa cggagaaaaa ctccaggaag    420
tgtttggtca aaggaaaaat gataatgaat tcattgagcc tctttgctgc catttctgga    480
atgattcttt caatcatgga catacttaat attaaaattt cccatttttt aaaaatggag    540
agtctgaatt ttattagagc tcacacacca tatattaaca tatacaactg tgaaccagct    600
aatccctctg agaaaaactc cccatctacc caatactgtt acagcataca atctctgttc    660
ttgggcattt tgtcagtgat gctgatcttt gccttcttcc aggaacttgt aatagctggc    720
atcgttgaga atgaatggaa aagaacgtgc tccagaccca aatctaacat agttctcctg    780
tcagcagaag aaaaaaaaga acagactatt gaaataaaag aagaagtggt tgggctaact    840
gaaacatctt cccaaccaaa gaatgaagaa gacattgaaa ttattccaat ccaagaagag    900
gaagaagaag aaacagagac gaactttcca gaacctcccc aagatcagga atcctcacca    960
atagaaaatg acagctctcc ttaagtgatt tcttctgttt tctgtttcct tttttaaaca   1020
ttagtgttca tagcttccaa gagacatgct gactttcatt tcttgaggta ctctgcacat   1080
acgcaccaca tctctatctg gcctttgcat ggagtgacca tagctccttc tctcttacat   1140
tgaatgtaga gaatgtagcc attgtagcag cttgtgttgt cacgcttctt cttttgagca   1200
actttcttac actgaagaaa ggcagaatga gtgcttcaga atgtgatttc ctactaacct   1260
gttccttgga taggcttttt agtatagtat tttttttgt cattttctcc atcagcaacc    1320
agggagactg cacctgatgg aaaagatata tgactgcttc atgacattcc taaactatct   1380
tttttttatt ccacatctac gtttttggtg gagtcccttt tgcatcattg ttttaaggat   1440
gataaaaaaa aaataacaac tagggacaat acagaaccca ttccatttat ctttctacag   1500
ggctgacatt gtggcacatt cttagagtta ccacacccca tgagggaagc tctaaatagc   1560
caacacccat ctgtttttg taaaaacagc atagctt                             1597
```

```
<210>  18
<211>  2907
<212>  DNA
<213>  Homo sapiens

<400>  18
gccatctggg cccaggcccc atgccccgag gaggggtggt ctgaagccca ccagagcccc     60
ctgccagact gtctgcctcc cttctgactg tggccgcttg gcatggccag caacagcagc    120
```

tcctgcccga cacctggggg cgggcacctc aatgggtacc cggtgcctcc ctacgccttc 180
ttcttccccc ctatgctggg tggactctcc ccgccaggcg ctctgaccac tctccagcac 240
cagcttccag ttagtggata tagcacacca tccccagcca ccattgagac ccagagcagc 300
agttctgaag agatagtgcc cagccctccc tcgccacccc ctctaccccg catctacaag 360
ccttgctttg tctgtcagga caagtcctca ggctaccact atggggtcag cgcctgtgag 420
ggctgcaagg gcttcttccg ccgcagcatc cagaagaaca tggtgtacac gtgtcaccgg 480
gacaagaact gcatcatcaa caaggtgacc cggaaccgct gccagtactg ccgactgcag 540
aagtgctttg aagtgggcat gtccaaggag tctgtgagaa acgaccgaaa caagaagaag 600
aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg ccccctcacc 1020
gacctggtct ttgccttcgc caaccagctg ctgcccctgg agatggatga tgcggagacg 1080
gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca aggggggctga gcgggtgatc acgctgaaga tggagatccc gggctccatg 1320
ccgcctctca tccaggaaat gttggagaac tcagagggcc tggacactct gagcggacag 1380
ccgggggggtg gggggcggga cgggggtggc ctggcccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680
gcctgctccc acagcctggg ctgacgtcag aggccgaggc caggaactga gtgaggcccc 1740
tggtcctggg tctcaggatg ggtcctgggg gcctcgtgtt catcaagaca cccctctgcc 1800
cagctcacca catcttcatc accagcaaac gccaggactt ggctccccca tcctcagaac 1860
tcacaagcca ttgctcccca gctggggaac ctcaacctcc cccctgcctc ggttggtgac 1920

agagggggtg ggacaggggc gggggttcc ccctgtacat accctgccat accaacccca 1980

ggtattaatt ctcgctggtt ttgtttttat tttaattttt ttgttttgat ttttttaata 2040

agaattttca ttttaagcac atttatactg aaggaatttg tgctgtgtat tgggggggagc 2100

tggatccaga gctggagggg gtgggtccgg gggagggagt ggctcggaag gggcccccac 2160

tctcctttca tgtccctgtg ccccccagtt ctcctcctca gccttttcct cctcagtttt 2220

ctctttaaaa ctgtgaagta ctaactttcc aaggcctgcc ttcccctccc tcccactgga 2280

gaagccgcca gcccctttct ccctctgcct gaccactggg tgtggacggt gtggggcagc 2340

cctgaaagga caggctcctg gccttggcac ttgcctgcac ccaccatgag gcatggagca 2400

gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg ccccctcctt accccgcagg acgggcctac agggggggtct cccctcaccc 2760

ctgcaccccc agctgggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 19
<211> 2907
<212> DNA
<213> Homo sapiens

<400> 19
gccatctggg cccaggcccc atgccccgag gaggggtggt ctgaagccca ccagagcccc 60

ctgccagact gtctgcctcc cttctgactg tggccgcttg gcatggccag caacagcagc 120

tcctgcccga cacctggggg cgggcacctc aatgggtacc cggtgcctcc ctacgccttc 180

ttcttccccc ctatgctggg tggactctcc ccgccaggcg ctctgaccac tctccagcac 240

cagcttccag ttagtggata tagcacacca tccccagcca ccattgagac ccagagcagc 300

agttctgaag agatagtgcc cagccctccc tcgccacccc ctctaccccg catctacaag 360

ccttgctttg tctgtcagga caagtcctca ggctaccact atggggtcag cgcctgtgag 420

ggctgcaagg gcttcttccg ccgcagcatc cagaagaaca tggtgtacac gtgtcaccgg 480

gacaagaact gcatcatcaa caaggtgacc cggaaccgct gccagtactg ccgactgcag 540

aagtgctttg aagtgggcat gtccaaggag tctgtgagaa acgaccgaaa caagaagaag 600

```
aaggaggtgc ccaagcccga gtgctctgag agctacacgc tgacgccgga ggtgggggag    660
ctcattgaga aggtgcgcaa agcgcaccag gaaaccttcc ctgccctctg ccagctgggc    720
aaatacacta cgaacaacag ctcagaacaa cgtgtctctc tggacattga cctctgggac    780
aagttcagtg aactctccac caagtgcatc attaagactg tggagttcgc caagcagctg    840
cccggcttca ccaccctcac catcgccgac cagatcaccc tcctcaaggc tgcctgcctg    900
gacatcctga tcctgcggat ctgcacgcgg tacacgcccg agcaggacac catgaccttc    960
tcggacgggc tgaccctgaa ccggacccag atgcacaacg ctggcttcgg cccccctcacc   1020
gacctggtct ttgccttcgc caaccagctg ctgcccctgg agatggatga tgcggagacg   1080
gggctgctca gcgccatctg cctcatctgc ggagaccgcc aggacctgga gcagccggac   1140
cgggtggaca tgctgcagga gccgctgctg gaggcgctaa aggtctacgt gcggaagcgg   1200
aggcccagcc gcccccacat gttccccaag atgctaatga agattactga cctgcgaagc   1260
atcagcgcca aggggggctga gcgggtgatc acgctgaaga tggagatccc gggctccatg   1320
ccgcctctca tccaggaaat gttggagaac tcagagggcc tggacactct gagcggacag   1380
ccgggggggtg gggggcggga cgggggtggc ctggcccccc cgccaggcag ctgtagcccc   1440
agcctcagcc ccagctccaa cagaagcagc ccggccaccc actccccgtg accgcccacg   1500
ccacatggac acagccctcg ccctccgccc cggcttttct ctgcctttct accgaccatg   1560
tgaccccgca ccagccctgc ccccacctgc cctcccgggc agtactgggg accttccctg   1620
ggggacgggg agggaggagg cagcgactcc ttggacagag gcctgggccc tcagtggact   1680
gcctgctccc acagcctggg ctgacgtcag aggccgaggc caggaactga gtgaggcccc   1740
tggtcctggg tctcaggatg ggtcctgggg gcctcgtgtt catcaagaca cccctctgcc   1800
cagctcacca catcttcatc accagcaaac gccaggactt ggctccccca tcctcagaac   1860
tcacaagcca ttgctcccca gctggggaac ctcaacctcc cccctgcctc ggttggtgac   1920
agagggggtg ggacaggggc gggggggttcc ccctgtacat accctgccat accaacccca   1980
ggtattaatt ctcgctggtt ttgtttttat tttaattttt ttgttttgat ttttttaata   2040
agaattttca ttttaagcac atttatactg aaggaatttg tgctgtgtat tgggggggagc   2100
tggatccaga gctggagggg gtgggtccgg gggagggagt ggctcggaag gggcccccac   2160
tctcctttca tgtccctgtg ccccccagtt ctcctcctca gccttttcct cctcagtttt   2220
ctctttaaaa ctgtgaagta ctaactttcc aaggcctgcc ttcccctccc tcccactgga   2280
gaagccgcca gccccttttct ccctctgcct gaccactggg tgtggacggt gtggggcagc   2340
cctgaaagga caggctcctg gccttggcac ttgcctgcac ccaccatgag gcatggagca   2400
gggcagagca agggccccgg gacagagttt tcccagacct ggctcctcgg cagagctgcc   2460
```

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

caccccc gtg cccccctcctt accccgcagg acgggcctac agggggggtct cccctcaccc 2760

ctgcaccccc agctgggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 20
<211> 1347
<212> DNA
<213> Homo sapiens

<400> 20
atggatccca aatatttcat cttaattttg ttttgtggac acctgaacaa tacatttttt 60

tcaaagacag agacaattac aacagagaag cagtcacagc ctaccttata cacatcatca 120

atgtcacagg tattggctaa ttctcaaaac acaacaggga atcctttggg tcaaccaaca 180

caattcagcg acactttttc tggacaatca atatcacctg ccaaagtcac tgctggacaa 240

ccaacaccag ctgtctatac ctcttctgaa aaaccagaag cacatacttc tgctggacaa 300

ccacttgcct acaacaccaa acaaccaaca ccaatagcca acacctcctc ccagcaagcc 360

gtgttcacct ctgccagaca actaccatct gcccgtactt ctaccacaca accaccaaag 420

tcatttgtct atacttttac tcaacaatca tcatctgtcc agatcccttc tagaaaacaa 480

ataactgttc ataatccatc cacacaacca acatcaactg tcaaaaattc acctaggagt 540

acaccaggat ttatcttaga tactaccagt aacaaacaaa ccccacaaaa aaacaattat 600

aattcaatag ctgccatact aattggtgta cttctgactt ctatgttggt agctataatc 660

atcattgtac tttggaaatg cttaaggaaa ccagttttaa atgatcaaaa ttgggcaggt 720

agatctccat ttgctgatgg agaaacccct gacatttgta tggataacat cagagaaaat 780

gaaatatcca caaaacgtac atcaatcatt tcacttacac cctggaaacc aagcaaaagc 840

acacttttag cagatgactt agaaattaag ttgtttgaat caagtgaaaa cattgaagac 900

tccaacaacc ccaaaacaga gaaaataaaa gatcaagtaa atggtacatc agaagatagt 960

gctgatggtt caacagttgg aactgctgtt tcttcttcag atgatgcaga tctgcctcca 1020

ccacctcccc ttctggattt ggaaggacag gaaagtaacc aatctgacaa acccacaatg 1080

acaattgtat ctcctcttcc aaatgattct actagtctcc ctccatctct ggactgtctc 1140

aatcaagact gtggagatca taaatctgag ataatacaat catttccacc gcttgactca 1200

cttaacttgc ccctgccacc agtagatttt atgaaaaacc aagaagattc caaccttgag 1260

atccagtgtc aggagttctc tattcctccc aactctgatc aagatcttaa tgaatccctg 1320

ccacctccac ctgcagaact gttataa 1347

<210> 21
<211> 2170
<212> DNA
<213> Homo sapiens

<400> 21
ctgagggctc atccctctgc agagcgcggg gtcaccggga ggagacgcca tgacgcccgc 60

cctcacagcc ctgctctgcc ttgggctgag tctgggcccc aggacccgcg tgcaggcagg 120

gcccttcccc aaacccaccc tctgggctga gccaggctct gtgatcagct gggggagccc 180

cgtgaccatc tggtgtcagg ggagcctgga ggcccaggag taccgactgg ataaagaggg 240

aagcccagag cccttggaca gaaataaccc actggaaccc aagaacaagg ccagattctc 300

catcccatcc atgacagagc accatgcggg gagataccgc tgccactatt acagctctgc 360

aggctggtca gagcccagcg acccccctgga gctggtgatg acaggattct acaacaaacc 420

caccctctca gccctgccca gccctgtggt ggcctcaggg gggaatatga ccctccgatg 480

tggctcacag aagggatatc accattttgt tctgatgaag gaaggagaac accagctccc 540

ccggaccctg gactcacagc agctccacag tggggggttc caggccctgt tccctgtggg 600

ccccgtgaac cccagccaca ggtggaggtt cacatgctat tactattata tgaacacccc 660

ccaggtgtgg tcccacccca gtgacccccct ggagattctg ccctcaggcg tgtctaggaa 720

gccctccctc ctgaccctgc agggccctgt cctggcccct gggcagagcc tgaccctcca 780

gtgtggctct gatgtcggct acgacagatt tgttctgtat aaggaggggg aacgtgactt 840

cctccagcgc cctggccagc agccccaggc tgggctctcc caggccaact tcaccctggg 900

ccctgtgagc cctcccacgg ggggccagta caggtgctat ggtgcacaca acctctcctc 960

cgagtggtcg gcccccagcg accccctgaa catcctgatg gcaggacaga tctatgacac 1020

cgtctccctg tcagcacagc cgggccccac agtggcctca ggagagaacg tgaccctgct 1080

gtgtcagtca tggtggcagt ttgacacttt ccttctgacc aaagaagggg cagcccatcc 1140

cccactgcgt ctgagatcaa tgtacggagc tcataagtac caggctgaat tccccatgag 1200

tcctgtgacc tcagcccacg cggggaccta caggtgctac ggctcataca gctccaaccc 1260

ccacctgctg tctttcccca gtgagcccct ggaactcatg gtctcaggac actctggagg 1320

ctccagcctc ccacccacag ggccgccctc cacacctggt ctgggaagat acctggaggt 1380

tttgattggg gtctcggtgg ccttcgtcct gctgctcttc ctcctcctct tcctcctcct 1440

ccgacgtcag cgtcacagca aacacaggac atctgaccag agaaagactg atttccagcg 1500

tcctgcaggg gctgcggaga cagagcccaa ggacaggggc ctgctgagga ggtccagccc 1560

agctgctgac gtccaggaag aaaacctcta tgctgccgtg aaggacacac agtctgagga 1620

cagggtggag ctggacagtc agagcccaca cgatgaagac ccccaggcag tgacgtatgc 1680

cccggtgaaa cactccagtc ctaggagaga aatggcctct cctccctcct cactgtctgg 1740

ggaattcctg gacacaaagg acagacaggt ggaagaggac aggcagatgg acactgaggc 1800

tgctgcatct gaagcctccc aggatgtgac ctacgcccag ctgcacagct tgacccttag 1860

acggaaggca actgagcctc ctccatccca ggaaggggaa cctccagctg agcccagcat 1920

ctacgccact ctggccatcc actagcccgg ggggtacgca gaccccacac tcagcagaag 1980

gagactcagg actgctgaag gcacgggagc tgcccccagt ggacaccagt gaaccccagt 2040

cagcctggac ccctaacaca gaccatgagg agacgctggg aacttgtggg actcacctga 2100

ctcaaagatg actaatatcg tcccattttg gaaataaagc aacagacttc tcaacaatca 2160

atgagttaat 2170

<210> 22
<211> 1453
<212> DNA
<213> Homo sapiens

<400> 22
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc cagtgattgc cgagctgcct cccaaagtga gcgtcttcgt cccaccccgc 360

gacggcttct tcggcaaccc ccgcaagtcc aagctcatct gccaggccac gggtttcagt 420

ccccggcaga ttcaggtgtc ctggctgcgc gaggggaagc aggtggggtc tggcgtcacc 480

acggaccagg tgcaggctga ggccaaagag tctgggccca cgacctacaa ggtgaccagc 540

acactgacca tcaaagagag cgactggctc agccagagca tgttcacctg ccgcgtggat 600

cacaggggcc tgaccttcca gcagaatgcg tcctccatgt gtgtccccga tcaagacaca 660

gccatccggg tcttcgccat ccccccatcc tttgccagca tcttcctcac caagtccacc 720

aagttgacct gcctggtcac agacctgacc acctatgaca gcgtgaccat ctcctggacc 780

cgccagaatg gcgaagctgt gaaaacccac accaacatct ccgagagcca ccccaatgcc 840

actttcagcg ccgtgggtga ggccagcatc tgcgaggatg actggaattc cggggagagg 900

ttcacgtgca ccgtgaccca cacagacctg ccctcgccac tgaagcagac catctcccgg 960

cccaaggggg tggccctgca caggcccgat gtctacttgc tgccaccagc ccgggagcag 1020

ctgaacctgc gggagtcggc caccatcacg tgcctggtga cgggcttctc tcccgcggac 1080

gtcttcgtgc agtggatgca gagggggcag cccttgtccc cggagaagta tgtgaccagc 1140

gccccaatgc ctgagcccca ggccccaggc cggtacttcg cccacagcat cctgaccgtg 1200

tccgaagagg aatggaacac gggggagacc tacacctgcg tggtggccca tgaggccctg 1260

cccaacaggg tcaccgagag gaccgtggac aagtccaccg gtaaacccac cctgtacaac 1320

gtgtccctgg tcatgtccga cacagctggc acctgctact gaccctgctg gcctgcccac 1380

aggctcgggg cggctggccg ctctgtgtgt gcatgcaaac taacccgtgt caacggggtg 1440

agatgttgca tct 1453

<210> 23
<211> 1192
<212> DNA
<213> Homo sapiens

<400> 23
cagatccatc aggtccaagc tgtgttgact accactgctt ttcccttcgt ctcaattatg 60

tcttggaaga aggctttgcg gatccctgga ggccttcggg tagcaactgt gaccttgatg 120

ctggcgatgc tgagcacccc ggtggctgag ggcagagact ctcccgagga tttcgtgtac 180

cagtttaagg gcatgtgcta cttcaccaac gggacggagc gcgtgcgtct tgtgaccaga 240

tacatctata accgagagga gtacgcacgc ttcgacagcg acgtgggggt gtatcgggcg 300

gtgacgccgc tggggccgcc tgacgccgag tactggaaca gccagaagga agtcctggag 360

aggacccggg cggagttgga cacggtgtgc agacacaact accagttgga gctccgcacg 420

accttgcagc ggcgagtgga gcccacagtg accatctccc catccaggac agaggccctc 480

aaccaccaca acctgctggt ctgctcagtg acagatttct atccagccca gatcaaagtc 540

cggtggtttc ggaatgacca ggaggagaca actggcgttg tgtccacccc ccttattagg 600

aacggtgact ggaccttcca gatcctggtg atgctggaaa tgactcccca gcgtggagac 660

gtctacacct gccacgtgga gcaccccagc ctccagaacc ccatcatcgt ggagtggcgg 720

gctcagtctg aatctgccca gagcaagatg ctgagtggca ttggaggctt cgtgctgggg 780

ctgatcttcc tcgggctggg ccttattatc catcacagga gtcagaaagg gctcctgcac 840

tgactcctga gactatttta actgggattg gttatcactt ttctgtaacg cctgcttgtc 900

cctgcccaga attcccagct gcctgtgtca gcctgtcccc cgagatcaga gtcctaccgt 960

ggctgtcacg cagccaccag gtcatctcct ttcatcccca cctcaaggct gatggctgtg 1020

accctgcttc ctgcactgac ccagagcctc tgcctgtgca cggccagctg cgtctactga 1080

ggccccaagg ggtttctgtt tctattctct cctcagactg ctcaagagaa gcacatgaaa 1140

accattacct gactttagag ctttttttaca taattaaaca tgatcctgag tt 1192

<210> 24
<211> 2048
<212> DNA
<213> Homo sapiens

<400> 24
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tattttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 25
<211> 2048
<212> DNA
<213> Homo sapiens

<400> 25
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tattttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 26
<211> 2816
<212> DNA
<213> Homo sapiens

<400> 26
tcgttgatat caaagacagt tgaaggaaat gaattttgaa acttcacggt gtgccacccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160

gctgtgtacc tgcctctgcc actgtatgtt ggcatctgtt atgctaaagt ttttcttgta 2220

catgaaaccc tggaagacct actacaaaaa aactgttgtt tggcccccat agcaggtgaa 2280

ctcattttgt gcttttaata gaaagacaaa tccaccccag taatattgcc cttacgtagt 2340

tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 27
<211> 2816
<212> DNA
<213> Homo sapiens

<400> 27
tcgttgatat caaagacagt tgaaggaaat gaattttgaa acttcacggt gtgccaccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420

agtccctata acacagacca cgcgcagaac agcgtcacgg cgccctcgcc ctacgcacag 480

cccagctcca ccttcgatgc tctctctcca tcacccgcca tcccctccaa caccgactac 540

ccaggcccgc acagtttcga cgtgtccttc cagcagtcga gcaccgccaa gtcggccacc 600

tggacgtatt ccactgaact gaagaaactc tactgccaaa ttgcaaagac atgccccatc 660

cagatcaagg tgatgacccc acctcctcag ggagctgtta tccgcgccat gcctgtctac 720

aaaaaagctg agcacgtcac ggaggtggtg aagcggtgcc ccaaccatga gctgagccgt 780

gaattcaacg agggacagat tgcccctcct agtcatttga ttcgagtaga ggggaacagc 840

catgcccagt atgtagaaga tcccatcaca ggaagacaga gtgtgctggt accttatgag 900

ccaccccagg ttggcactga attcacgaca gtcttgtaca atttcatgtg taacagcagt 960

tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt ttttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460
ctgatactgt tcagtgcatt tagccaggag acttacgttt tgagtaagtg agatccaagc 2520
agacgtgtta aaatcagcac tcctggactg gaaattaaag attgaaaggg tagactactt 2580
ttcttttttt tactcaaaag tttagagaat ctctgtttct ttccatttta aaaacatatt 2640
ttaagataat agcataaaga ctttaaaaat gttcctcccc tccatcttcc cacacccagt 2700
caccagcact gtattttctg tcaccaagac aatgatttct tgttattgag gctgttgctt 2760
ttgtggatgt gtgattttaa ttttcaataa acttttgcat cttggtttaa aagaaa 2816

<210> 28
<211> 3803
<212> DNA
<213> Homo sapiens

<400> 28
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300

aaacgcgctg ggcgtctgct ccgacaccgc agcgcccctg gccgcggtgg acctcaagtg 360

ggagcacaac cccgctgtgt tcctgggccc cggctgcgtg tacgccgccg ccccagtggg 420

gcgcttcacc gcgcactggc gggtcccgct gctgaccgcc ggcgccccgg cgctgggctt 480

cggtgtcaag gacgagtatg cgctgaccac ccgcgcgggg cccagctacg ccaagctggg 540

ggacttcgtg gcggcgctgc accgacggct gggctgggag cgccaagcgc tcatgctcta 600

cgcctaccgg ccgggtgacg aagagcactg cttcttcctc gtggaggggc tgttcatgcg 660

ggtccgcgac cgcctcaata ttacggtgga ccacctggag ttcgccgagg acgacctcag 720

ccactacacc aggctgctgc ggaccatgcc gcgcaaaggc cgagttatct acatctgcag 780

ctcccctgat gccttcagaa ccctcatgct cctggccctg gaagctggct tgtgtgggga 840

ggactacgtt ttcttccacc tggatatctt tgggcaaagc ctgcaaggtg gacagggccc 900

tgctccccgc aggccctggg agagagggga tgggcaggat gtcagtgccc gccaggcctt 960

tcaggctgcc aaaatcatta catataaaga cccagataat cccgagtact tggaattcct 1020

gaagcagtta aaacacctgg cctatgagca gttcaacttc accatggagg atggcctggt 1080

gaacaccatc ccagcatcct tccacgacgg gctcctgctc tatatccagg cagtgacgga 1140

gactctggca catgggggaa ctgttactga tggggagaac atcactcagc ggatgtggaa 1200

ccgaagcttt caaggtgtga caggatacct gaaaattgat agcagtggcg atcgggaaac 1260

agacttctcc ctctgggata tggatcccga gaatggtgcc ttcagggttg tactgaacta 1320

caatgggact tcccaagagc tggtggctgt gtcggggcgc aaactgaact ggcccctggg 1380

gtaccctcct cctgacatcc ccaaatgtgg ctttgacaac gaagacccag catgcaacca 1440

agatcacctt tccaccctgg aggtgctggc tttggtgggc agcctctcct tgctcggcat 1500

tctgattgtc tccttcttca tatacaggaa gatgcagctg gagaaggaac tggcctcgga 1560

gctgtggcgg gtgcgctggg aggacgttga gcccagtagc cttgagaggc acctgcggag 1620

tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg ggctatctg ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160
cctgcgaatg gcttcacccc ctgtgcgggg ctcccaggct ggtgacgtat acagctttgg 2220
gatcatcctt caggagattg ccctgaggag tggggtcttc cacgtggaag gtttggacct 2280
gagccccaaa gagatcatcg agcgggtgac tcggggtgag cagcccccct tccggccctc 2340
cctggccctg cagagtcacc tggaggagtt ggggctgctc atgcagcggt gctgggctga 2400
ggacccacag gagaggccac cattccagca gatccgcctg acgttgcgca aatttaacag 2460
ggagaacagc agcaacatcc tggacaacct gctgtcccgc atggagcagt acgcgaacaa 2520
tctggaggaa ctggtggagg agcggaccca ggcatacctg gaggagaagc gcaaggctga 2580
ggccctgctc taccagatcc tgcctcactc agtggctgag cagctgaagc gtggggagac 2640
ggtgcaggcc gaagcctttg acagtgttac catctacttc agtgacattg tgggtttcac 2700
agcgctgtcg gcggagagca cacccatgca ggtggtgacc ctgctcaatg acctgtacac 2760
ttgctttgat gctgtcatag acaactttga tgtgtacaag gtggagacaa ttggcgatgc 2820
ctacatggtg gtgtcagggc tccctgtgcg gaacgggcgg ctacacgcct gcgaggtagc 2880
ccgcatggcc ctggcactgc tggatgctgt gcgctccttc cgaatccgcc accggcccca 2940
ggagcagctg cgcttgcgca ttggcatcca cacaggacct gtgtgtgctg gagtggtggg 3000
actgaagatg ccccgttact gtctctttgg ggatacagtc aacacagcct caagaatgga 3060
gtctaatggg gaagccctga agatccactt gtcttctgag accaaggctg tcctggagga 3120
gtttggtggt ttcgagctgg agcttcgagg ggatgtagaa atgaagggca aaggcaaggt 3180
tcggacctac tggctccttg ggagaggggg gagtagcacc cgaggctgac ctgcctcctc 3240
tcctatccct ccacacctcc cctaccctgt gccagaagca acagaggtgc caggcctcag 3300
cctcacccac agcagcccca tcgccaaagg atggaagtaa tttgaatagc tcaggtgtgc 3360
tgaccccagt gaagacacca gataggacct ctgagagggg actggcatgg ggggatctca 3420

gagcttacag gctgagccaa gcccacggcc atgcacaggg acactcacac aggcacacgc 3480

acctgctctc cacctggact caggccgggc tgggctgtgg atccttgatc ccctcccctc 3540

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gaccccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 29
<211> 3802
<212> DNA
<213> Homo sapiens

<400> 29
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300

aaacgcgctg ggcgtctgct ccgacaccgc agcgcccctg gccgcggtgg acctcaagtg 360

ggagcacaac cccgctgtgt tcctgggccc cggctgcgtg tacgccgccg ccccagtggg 420

gcgcttcacc gcgcactggc gggtcccgct gctgaccgcc ggcgccccgg cgctgggctt 480

cggtgtcaag gacgagtatg cgctgaccac ccgcgcgggg cccagctacg ccaagctggg 540

ggacttcgtg gcggcgctgc accgacggct gggctgggag cgccaagcgc tcatgctcta 600

cgcctaccgg ccgggtgacg aagagcactg cttcttcctc gtggaggggc tgttcatgcg 660

ggtccgcgac cgcctcaata ttacggtgga ccacctggag ttcgccgagg acgacctcag 720

ccactacacc aggctgctgc ggaccatgcc gcgcaaaggc cgagttatct acatctgcag 780

ctcccctgat gccttcagaa ccctcatgct cctggccctg gaagctggct tgtgtgggga 840

ggactacgtt ttcttccacc tggatatctt tgggcaaagc ctgcaaggtg gacagggccc 900

tgctccccgc aggccctggg agagagggga tgggcaggat gtcagtgccc gccaggcctt 960

tcaggctgcc aaaatcatta catataaaga cccagataat cccgagtact tggaattcct 1020

gaagcagtta aaacacctgg cctatgagca gttcaacttc accatggagg atggcctggt 1080

gaacaccatc ccagcatcct tccacgacgg gctcctgctc tatatccagg cagtgacgga 1140

gactctggca catgggggaa ctgttactga tggggagaac atcactcagc ggatgtggaa 1200

ccgaagcttt caaggtgtga caggatacct gaaaattgat agcagtggcg atcgggaaac 1260

agacttctcc ctctgggata tggatcccga gaatggtgcc ttcagggttg tactgaacta 1320

caatgggact tcccaagagc tggtggctgt gtcggggcgc aaactgaact ggcccctggg 1380

gtaccctcct cctgacatcc ccaaatgtgg ctttgacaac gaagacccag catgcaacca 1440

agatcacctt tccaccctgg aggtgctggc tttggtgggc agcctctcct tgctcggcat 1500

tctgattgtc tccttcttca tatacaggaa gatgcagctg gagaaggaac tggcctcgga 1560

gctgtggcgg gtgcgctggg aggacgttga gcccagtagc cttgagaggc acctgcggag 1620

tgcaggcagc cggctgaccc tgagcgggag aggctccaat tacggctccc tgctaaccac 1680

agagggccag ttccaagtct ttgccaagac agcatattat aagggcaacc tcgtggctgt 1740

gaaacgtgtg aaccgtaaac gcattgagct gacacgaaaa gtcctgtttg aactgaagca 1800

tatgcgggat gtgcagaatg aacacctgac caggtttgtg ggagcctgca ccgacccccc 1860

caatatctgc atcctcacag agtactgtcc ccgtgggagc ctgcaggaca ttctggagaa 1920

tgagagcatc accctggact ggatgttccg gtactcactc accaatgaca tcgtcaaggg 1980

catgctgttt ctacacaatg ggctatctgt ttcccatggg aacctcaagt catccaactg 2040

cgtggtagat gggcgctttg tgctcaagat caccgactat gggctggaga gcttcaggga 2100

cctggaccca gagcaaggac acaccgttta tgccaaaaag ctgtggacgg cccctgagct 2160

cctgcgaatg gcttcacccc ctgtgcgggg ctcccaggct ggtgacgtat acagctttgg 2220

gatcatcctt caggagattg ccctgaggag tggggtcttc cacgtggaag gtttggacct 2280

gagccccaaa gagatcatcg agcgggtgac tcggggtgag cagcccccct tccggccctc 2340

cctggccctg cagagtcacc tggaggagtt ggggctgctc atgcagcggt gctgggctga 2400

ggacccacag gagaggccac cattccagca gatccgcctg acgttgcgca aatttaacag 2460

ggagaacagc agcaacatcc tggacaacct gctgtcccgc atggagcagt acgcgaacaa 2520

tctggaggaa ctggtggagg agcggaccca ggcatacctg gaggagaagc gcaaggctga 2580

ggccctgctc taccagatcc tgcctcactc agtggctgag cagctgaagc gtggggagac 2640

ggtgcaggcc gaagcctttg acagtgttac catctacttc agtgacattg tgggtttcac 2700

agcgctgtcg gcggagagca cacccatcag gtggtgaccc tgctcaatga cctgtacact 2760

tgctttgatg ctgtcataga caactttgat gtgtacaagg tggagacaat tggcgatgcc 2820

tacatggtgg tgtcagggct ccctgtgcgg aacgggcggc tacacgcctg cgaggtagcc 2880

cgcatggccc tggcactgct ggatgctgtg cgctccttcc gaatccgcca ccggccccag 2940

gagcagctgc gcttgcgcat tggcatccac acaggacctg tgtgtgctgg agtggtggga 3000

ctgaagatgc cccgttactg tctctttggg gatacagtca acacagcctc aagaatggag 3060

```
tctaatgggg aagccctgaa gatccacttg tcttctgaga ccaaggctgt cctggaggag   3120

tttggtggtt tcgagctgga gcttcgaggg gatgtagaaa tgaagggcaa aggcaaggtt   3180

cggacctact ggctccttgg ggagaggggg agtagcaccc gaggctgacc tgcctcctct   3240

cctatccctc cacacctccc ctaccctgtg ccagaagcaa cagaggtgcc aggcctcagc   3300

ctcacccaca gcagccccat cgccaaagga tggaagtaat ttgaatagct caggtgtgct   3360

gaccccagtg aagacaccag ataggacctc tgagagggga ctggcatggg gggatctcag   3420

agcttacagg ctgagccaag cccacggcca tgcacaggga cactcacaca ggcacacgca   3480

cctgctctcc acctggactc aggccgggct gggctgtgga tccttgatcc cctcccctcc   3540

ccatgctctc ctccctcagc cttgctaccc tgtgacttac tgggaggaga gtcacctgaa   3600

ggggaacatg aaaagagact aggtgaagag agggcagggg agcccacatc tggggctggc   3660

ccacaatacc tgctcccccg accccctcca cccagcagta gacacagtgc acaggggaga   3720

agaggggtgg cgcagaaggg ttgggggcct gtatgccttg cttctaccat gagcagagac   3780

aattaaaatc tttattccag tg                                             3802
```

<210> 30
<211> 2970
<212> DNA
<213> Homo sapiens

<400> 30

```
ggggtaagga gttcaaggca gcgcccacac ccgggggctc tccgcaaccc gaccgcctgt     60

ccgctccccc acttcccgcc ctccctccca cctactcatt cacccaccca cccacccaga    120

gccgggacgg cagcccaggc gcccgggccc cgccgtctcc tcgccgcgat cctggacttc    180

ctcttgctgc aggacccggc ttccacgtgt gtcccggagc cggcgtctca gcacacgctc    240

cgctccgggc ctgggtgcct acagcagcca gagcagcagg gagtccggga cccgggcggc    300

atctgggcca agttaggcgc cgccgaggcc agcgctgaac gtctccaggg ccggaggagc    360

cgcggggcgt ccgggtctga gcctcagcaa atgggctccg acgtgcggga cctgaacgcg    420

ctgctgcccg ccgtcccctc cctgggtggc ggcggcggct gtgccctgcc tgtgagcggc    480

gcggcgcagt gggcgccggt gctggacttt gcgcccccgg gcgcttcggc ttacgggtcg    540

ttgggcggcc ccgcgccgcc accggctccg ccgccacccc cgccgccgcc gcctcactcc    600

ttcatcaaac aggagccgag ctggggcggc gcggagccgc acgaggagca gtgcctgagc    660

gccttcactg tccacttttc cggccagttc actggcacag ccggagcctg tcgctacggg    720

cccttcggtc ctcctccgcc cagccaggcg tcatccggcc aggccaggat gtttcctaac    780

gcgccctacc tgcccagctg cctcgagagc cagcccgcta ttcgcaatca gggttacagc    840
```

acggtcacct tcgacgggac gcccagctac ggtcacacgc cctcgcacca tgcggcgcag 900
ttccccaacc actcattcaa gcatgaggat cccatgggcc agcagggctc gctgggtgag 960
cagcagtact cggtgccgcc cccggtctat ggctgccaca cccccaccga cagctgcacc 1020
ggcagccagg ctttgctgct gaggacgccc tacagcagtg acaatttata ccaaatgaca 1080
tcccagcttg aatgcatgac ctggaatcag atgaacttag gagccacctt aaagggccac 1140
agcacagggt acgagagcga taaccacaca acgcccatcc tctgcggagc ccaatacaga 1200
atacacacgc acggtgtctt cagaggcatt caggatgtgc gacgtgtgcc tggagtagcc 1260
ccgactcttg tacggtcggc atctgagacc agtgagaaac gccccttcat gtgtgcttac 1320
ccaggctgca ataagagata ttttaagctg tcccacttac agatgcacag caggaagcac 1380
actggtgaga aaccatacca gtgtgacttc aaggactgtg aacgaaggtt ttctcgttca 1440
gaccagctca aaagacacca aaggagacat acaggtgtga aaccattcca gtgtaaagct 1500
tgtcagcgaa agttctcccg gtccgaccac ctgaagaccc acaccaggac tcatacaggt 1560
gaaaagccct tcagctgtcg gtggccaagt tgtcagaaaa agtttgcccg gtcagatgaa 1620
ttagtccgcc atcacaacat gcatcagaga aacatgacca aactccagct ggcgctttga 1680
ggggtctccc tcggggaccg ttcagtgtcc caggcagcac agtgtgtgaa ctgctttcaa 1740
gtctgactct ccactcctcc tcactaaaaa ggaaacttca gttgatcttc ttcatccaac 1800
ttccaagaca agataccggt gcttctggaa actaccaggt gtgcctggaa gagttggtct 1860
ctgccctgcc tacttttagt tgactcacag gccctggaga agcagctaac aatgtctggt 1920
tagttaaaag cccattgcca tttggtctgg attttctact gtaagaagag ccatagctga 1980
tcatgtcccc ctgacccttc ccttcttttt ttatgctcgt tttcgctggg gatggaatta 2040
ttgtaccatt ttctatcatg gaatatttat aggccagggc atgtgtatgt gtctgctaat 2100
gtaaactttg tcatggtttc catttactaa cagcaacagc aagaaataaa tcagagagca 2160
aggcatcggg ggtgaatctt gtctaacatt cccgaggtca gccaggctgc taacctggaa 2220
agcaggatgt agttctgcca ggcaactttt aaagctcatg catttcaagc agctgaagaa 2280
agaatcagaa ctaaccagta cctctgtata gaaatctaaa agaattttac cattcagtta 2340
attcaatgtg aacactggca cactgctctt aagaaactat gaagatctga gatttttttg 2400
tgtatgtttt tgactctttt gagtggtaat catatgtgtc tttatagatg tacatacctc 2460
cttgcacaaa tggaggggaa ttcattttca tcactgggag tgtccttagt gtataaaaac 2520
catgctggta tatggcttca agttgtaaaa atgaaagtga ctttaaaaga aaatagggga 2580
tggtccagga tctccactga taagactgtt tttaagtaac ttaaggacct ttgggtctac 2640

aagtatatgt gaaaaaaatg agacttactg ggtgaggaaa tccattgttt aaagatggtc 2700

gtgtgtgtgt gtgtgtgtgt gtgtgtgttg tgttgtgttt tgttttttaa gggagggaat 2760

ttattattta ccgttgcttg aaattactgt gtaaatatat gtctgataat gatttgctct 2820

ttgacaacta aaattaggac tgtataagta ctagatgcat cactgggtgt tgatcttaca 2880

agatattgat gataacactt aaaattgtaa cctgcatttt tcactttgct ctcaattaaa 2940

gtctattcaa aaggaaaaaa aaaaaaaaaa 2970

<210> 31
<211> 2422
<212> DNA
<213> Homo sapiens

<400> 31
gtcagcctcc cttccaccgc catattgggc cactaaaaaa agggggctcg tcttttcggg 60

gtgtttttct ccccctcccc tgtccccgct tgctcacggc tctgcgactc cgacgccggc 120

aaggtttgga gagcggctgg gttcgcggga cccgcgggct tgcacccgcc cagactcgga 180

cgggctttgc caccctctcc gcttgcctgg tcccctctcc tctccgccct cccgctcgcc 240

agtccatttg atcagcggag actcggcggc cgggccgggg cttccccgca gcccctgcgc 300

gctcctagag ctcgggccgt ggctcgtcgg ggtctgtgtc ttttggctcc gagggcagtc 360

gctgggcttc cgagaggggt tcgggccgcg taggggcgct ttgttttgtt cggttttgtt 420

tttttgagag tgcgagagag gcggtcgtgc agacccggga gaaagatgtc aaacgtgcga 480

gtgtctaacg ggagccctag cctggagcgg atggacgcca ggcaggcgga gcaccccaag 540

ccctcggcct gcaggaacct cttcggcccg gtggaccacg aagagttaac ccgggacttg 600

gagaagcact gcagagacat ggaagaggcg agccagcgca agtggaattt cgattttcag 660

aatcacaaac ccctagaggg caagtacgag tggcaagagg tggagaaggg cagcttgccc 720

gagttctact acagaccccc gcggcccccc aaaggtgcct gcaaggtgcc ggcgcaggag 780

agccaggatg tcagcgggag ccgcccggcg gcgcctttaa ttggggctcc ggctaactct 840

gaggacacgc atttggtgga cccaaagact gatccgtcgg acagccagac ggggttagcg 900

gagcaatgcg caggaataag gaagcgacct gcaaccgacg attcttctac tcaaaacaaa 960

agagccaaca gaacagaaga aaatgtttca gacggttccc caaatgccgg ttctgtggag 1020

cagacgccca agaagcctgg cctcagaaga cgtcaaacgt aaacagctcg aattaagaat 1080

atgtttcctt gtttatcaga tacatcactg cttgatgaag caaggaagat atacatgaaa 1140

attttaaaaa tacatatcgc tgacttcatg gaatggacat cctgtataag cactgaaaaa 1200

caacaacaca ataacactaa aattttaggc actcttaaat gatctgcctc taaaagcgtt 1260

ggatgtagca ttatgcaatt aggtttttcc ttatttgctt cattgtacta cctgtgtata 1320

tagtttttac cttttatgta gcacataaac tttggggaag ggagggcagg gtggggctga 1380

ggaactgacg tggagcgggg tatgaagagc ttgctttgat ttacagcaag tagataaata 1440

tttgacttgc atgaagagaa gcaattttgg ggaagggttt gaattgtttt ctttaaagat 1500

gtaatgtccc tttcagagac agctgatact tcatttaaaa aaatcacaaa aatttgaaca 1560

ctggctaaag ataattgcta tttattttta caagaagttt attctcattt gggagatctg 1620

gtgatctccc aagctatcta aagtttgtta gatagctgca tgtggctttt ttaaaaaagc 1680

aacagaaacc tatcctcact gccctcccca gtctctctta aagttggaat ttaccagtta 1740

attactcagc agaatggtga tcactccagg tagtttgggg caaaaatccg aggtgcttgg 1800

gagttttgaa tgttaagaat tgaccatctg cttttattaa atttgttgac aaaattttct 1860

cattttcttt tcacttcggg ctgtgtaaac acagtcaaaa taattctaaa tccctcgata 1920

tttttaaaga tctgtaagta acttcacatt aaaaaatgaa atattttta atttaaagct 1980

tactctgtcc atttatccac aggaaagtgt tatttttaaa ggaaggttca tgtagagaaa 2040

agcacacttg taggataagt gaaatggata ctacatcttt aaacagtatt tcattgcctg 2100

tgtatggaaa aaccatttga agtgtacctg tgtacataac tctgtaaaaa cactgaaaaa 2160

ttatactaac ttatttatgt taaaagattt tttttaatct agacaatata caagccaaag 2220

tggcatgttt tgtgcatttg taaatgctgt gttgggtaga ataggttttc ccctcttttg 2280

ttaaataata tggctatgct taaaaggttg catactgagc caagtataat tttttgtaat 2340

gtgtgaaaaa gatgccaatt attgttacac attaagtaat caataaagaa aacttccata 2400

gctaaaaaaa aaaaaaaaaa aa 2422

<210> 32
<211> 1181
<212> DNA
<213> Homo sapiens

<400> 32
ggcacgaggc ccgggccccc caaagtcccg gccgggccga gggtcggcgg ccgccggcgg 60

gccgggcccg cgcacagcgc ccgcatgtac aacatgatgg agacggagct gaagccgccg 120

ggcccgcagc aaacttcggg gggcggcggc ggcaactcca ccgcggcggc ggccggcggc 180

aaccagaaaa acagcccgga ccgcgtcaag cggcccatga atgccttcat ggtgtggtcc 240

cgcgggcagc ggcgcaagat ggcccaggag aaccccaaga tgcacaactc ggagatcagc 300

aagcgcctgg gcgccgagtg gaaacttttg tcggagacgg agaagcggcc gttcatcgac 360

gaggctaagc ggctgcgagc gctgcacatg aaggagcacc cggattataa ataccggccc 420

```
cggcggaaaa ccaagacgct catgaagaag gataagtaca cgctgcccgg cgggctgctg    480
gcccccggcg gcaatagcat ggcgagcggg gtcggggtgg gcgccggcct gggcgcgggc    540
gtgaaccagc gcatggacag ttacgcgcac atgaacggct ggagcaacgg cagctacagc    600
atgatgcagg accagctggg ctacccgcag cacccgggcc tcaatgcgca cggcgcagcg    660
cagatgcagc ccatgcaccg ctacgacgtg agcgccctgc agtacaactc catgaccagc    720
tcgcagacct acatgaacgg ctcgcccacc tacagcatgt cctactcgca gcagggcacc    780
cctggcatgg ctcttggctc catgggttcg gtggtcaagt ccgaggccag ctccagcccc    840
cctgtggtta cctcttcctc ccactccagg gcgccctgcc aggccgggga cctccgggac    900
atgatcagca tgtatctccc cggcgccgag gtgccggaac ccgccgcccc cagcagactt    960
cacatgtccc agcactacca gagcggcccg gtgcccggca cggccattaa cggcacactg   1020
cccctctcac acatgtgagg gccggacagc gaactggagg ggggagaaat tttcaaagaa   1080
aaacgaggga aatgggaggg gtgcaaaaga ggagagtaag aaacagcatg gagaaaaccc   1140
ggtacgctca aaaaaaaaaa aaaaaaaaaa aaaaaaaaaa a                       1181
```

```
<210>  33
<211>  1305
<212>  DNA
<213>  Homo sapiens

<400>  33
ggggccccgc gccggcccgc gccctgccca gtgcggcctc cttccacccg ccgctgcctg     60
gcccgcgccg tccggccgag ctgcccggcg ggctggtccc cgcgcccgag ccgcccggcc    120
gggaccccga acaaggccga gatgacttcc aaggaggacg gcaaggcggc gccgggggag    180
gagcggcgcc gcagcccgct ggaccacctg cctccgcctg ccaactccaa caagccagac    240
gccgttcagc atcgaggaca tcctcaacaa gccgtctgtg cggagaagtt actcgctgcg    300
tggggcggcg cacctgctgg ccgccgcgga caagcacgcg cagggcggct tgccctggcg    360
ggccgcgcgc tgctctcgaa gacctcgccg ctgtgcgcgc tggaggagct cgccagcaag    420
acgtttaagg ggctggaggt cagcgttctg caggcagccg aaggccgcga cggtatgacc    480
atctttgggc agcggcagac ccctaagaag cggcgaaagt cgcgcacggc cttcaccaac    540
caccagatct atgaattgga aaagcgcttt ctataccaga agtacctgtc ccccgccgat    600
cgcgaccaaa tcgcgcagca gctgggcctc accaacgcgc aagtcatcac ctggttccag    660
aatcggcgcg ctaagctcaa gcgggaactg gaggagatga aggccgacgt ggagtccccc    720
aagaaactgg gccccagcgg gcagatggac atcgtggcgc tggccgaact cgagcagaac    780
tcggaggcca cagccggcgg tggcggcggc tgcggcaggg ccaagtcgag gcccggctct    840
```

```
ccggtcctcc ccccaggcgc cccgaaggcc cccgggcgct gcgccctgca gctctcgcct    900
gcctctccgc tcacggacca gccggccagc agccaggact gctcggagga cgaggaagac    960
gaagagatcg acgtggacga ttgagcggcg ccccgggtct tccgccgccc tgggctccta   1020
gcgctcgaaa gcccaacgcc tcccggaccg gaccgccgag gggagctggg acctcctctg   1080
ccactcccgc ctcctcccct gtccccggac tcggctcctg gcagccgcct cttccctctc   1140
gaagcaataa acccaggctg gccggccggg ccggccgcca ccagcggcct ccgccgcccc   1200
ggaagccctc gccgagcaat tctgtatggc ttctatataa atatttaaac ctatatagcg   1260
ggttctcccc aaaaaaaaaa aaaaaaaaaa aaaaaaaaaa aaaaa                   1305
```

<210> 34
<211> 1926
<212> DNA
<213> Homo sapiens

<400> 34

```
gctggagcat cccgctctgg tgccgctgca gccggcagag atggttgagc tcatgttccc     60
gctgttgctc ctccttctgc ccttccttct gtatatggct gcgccccaaa tcaggaaaat    120
gctgtccagt ggggtgtgta catcaactgt tcagcttcct gggaaagtag ttgtggtcac    180
aggagctaat acaggtatcg ggaaggagac agccaaagag ctggctcaga gaggagctcg    240
agtatattta gcttgccggg atgtggaaaa gggggaattg gtggccaaag agatccagac    300
cacgacaggg aaccagcagg tgttggtgcg gaaactggac ctgtctgata ctaagtctat    360
tcgagctttt gctaagggct tcttagctga ggaaaagcac ctccacgttt tgatcaacaa    420
tgcaggagtg atgatgtgtc cgtactcgaa gacagcagat ggctttgaga tgcacatagg    480
agtcaaccac ttgggtcact tcctcctaac ccatctgctg ctagagaaac taaaggaatc    540
agccccatca aggatagtaa atgtgtcttc cctcgcacat cacctgggaa ggatccactt    600
ccataacctg cagggcgaga aattctacaa tgcaggcctg gcctactgtc acagcaagct    660
agccaacatc ctcttcaccc aggaactggc ccggagacta aaaggctctg gcgttacgac    720
gtattctgta caccctggca cagtccaatc tgaactggtt cggcactcat ctttcatgag    780
atggatgtgg tggcttttct cctttttcat caagactcct cagcagggag cccagaccag    840
cctgcactgt gccttaacag aaggtcttga gattctaagt gggaatcatt tcagtgactg    900
tcatgtggca tgggtctctg cccaagctcg taatgagact atagcaaggc ggctgtggga    960
cgtcagttgt gacctgctgg gcctcccaat agactaacag gcagtgcagt tggacccaag   1020
agaagactgc agcagactac acagtacttc ttgtcaaaat gattctcctt caaggttttc   1080
aaaaccttta gcacaaagag agcaaaacct tccagccttg cctgcttggt gtccagttaa   1140
```

```
aactcagtgt actgccagat tcgtctaaat gtctgtcatg tccagattta ctttgcttct   1200

gttactgcca gagttactag agatatcata ataggataag aagaccctca tatgacctgc   1260

acagctcatt ttccttctga aagaaactac tacctaggag aatctaagct atagcaggga   1320

tgatttatgc aaatttgaac tagcttcttt gttcacaatt cagttcctcc caaccaacca   1380

gtcttcactt caagagggcc acactgcaac ctcagcttaa catgaataac aaagactggc   1440

tcaggagcag ggcttgccca ggcatggtgg atcaccggag tcagtagttc aagaccagcc   1500

tggccaacat ggtgaaaccc cacctctact aaaaattgtg tatatctttg tgtgtcttcc   1560

tgtttatgtg tgccaaggga gtattttcac aaagttcaaa acagccacaa taatcagaga   1620

tggagcaaac cagtgccatc cagtctttat gcaaatgaaa tgctgcaaag ggaagcagat   1680

tctgtatatg ttggtaacta cccaccaaga gcacatgggt agcagggaag aagtaaaaaa   1740

agagaaggag aatactggaa gataatgcac aaaatgaagg gactagttaa ggattaacta   1800

gccctttaag gattaactag ttaaggatta atagcaaaag acattaaata tgctaacata   1860

gctatggagg aattgagggc aagcacccag gactgatgag gtcttaacaa aaaccagtgt   1920

ggcaaa                                                              1926


<210>  35
<211>  1195
<212>  DNA
<213>  Homo sapiens

<400>  35
ccgagactca cggtcaagct aaggcgaaga gtgggtggct gaagccatac tattttatag     60

aattaatgga aagcagaaaa gacatcacaa accaagaaga actttggaaa atgaagccta    120

ggagaaattt agaagaagac gattatttgc ataaggacac gggagagacc agcatgctaa    180

aaagacctgt gcttttgcat ttgcaccaaa cagcccatgc tgatgaattt gactgccctt    240

cagaacttca gcacacacag gaactctttc cacagtggca cttgccaatt aaaatagctg    300

ctattatagc atctctgact tttctttaca ctcttctgag ggaagtaatt caccctttag    360

caacttccca tcaacaatat ttttataaaa ttccaatcct ggtcatcaac aaagtcttgc    420

caatggtttc catcactctc ttggcattgg tttacctgcc aggtgtgata gcagcaattg    480

tccaacttca taatggaacc aagtataaga agtttccaca ttggttggat aagtggatgt    540

taacaagaaa gcagtttggg cttctcagtt tctttttgc tgtactgcat gcaatttata    600

gtctgtctta cccaatgagg cgatcctaca gatacaagtt gctaaactgg gcatatcaac    660

aggtccaaca aaataaagaa gatgcctgga ttgagcatga tgtttggaga atggagattt    720

atgtgtctct gggaattgtg ggattggcaa tactggctct gttggctgtg acatctattc    780
```

catctgtgag tgactctttg acatggagag aatttcacta tattcagagc aagctaggaa 840

ttgtttccct tctactgggc acaatacacg cattgatttt tgcctggaat aagtggatag 900

atataaaaca atttgtatgg tatacacctc caacttttat gatagctgtt ttccttccaa 960

ttgttgtcct gatatttaaa agcatactat tcctgccatg cttgaggaag aagatactga 1020

agattagaca tggttgggaa gacgtcacca aaattaacaa aactgagata tgttcccagt 1080

tgtagaatta ctgtttacac acatttttgt tcaatattga tatattttat caccaacatt 1140

tcaagtttgt atttgttaat aaaatgatta ttcaaggaaa aaaaaaaaaa aaaaa 1195

<210> 36
<211> 3414
<212> DNA
<213> Homo sapiens

<400> 36
gcttacacag tatggccggc gacattagct agcgctcgct ctactctctc taacgggaaa 60

gcagcggaat acaagagact gaactgtatc tgcctctatt tccaaaagac tcacgttcaa 120

ctttcgctca cacaaagccg ggaaaatttt attagtcctt tttttaaaaa aagttaatat 180

aaaattatag caaaaaaaaa aaggaacctg aactttagta acacagctgg aacaatcgca 240

gcggcggcgg cagcggcggg agaagaggtt taatttagtt gattttctgt ggttgttggt 300

tgttcgctag tctcacggtg atggaagctg cacatttttt cgaagggacc gagaagctgc 360

tggaggtttg gttctcccgg cagcagcccg acgcaaacca aggatctggg gatcttcgca 420

ctatcccaag atctgagtgg gacatacttt tgaaggatgt gcaatgttca atcataagtg 480

tgacaaaaac tgacaagcag gaagcttatg tactcagtga gagtagcatg tttgtctcca 540

agagacgttt cattttgaag acatgtggta ccaccctctt gctgaaagca ctggttcccc 600

tgttgaagct tgctagggat tacagtgggt ttgactcaat tcaaagcttc ttttattctc 660

gtaagaattt catgaagcct tctcaccaag ggtacccaca ccggaatttc caggaagaaa 720

tagagtttct taatgcaatt ttcccaaatg gagcaggata ttgtatggga cgtatgaatt 780

ctgactgttg gtacttatat actctggatt tcccagagag tcgggtaatc agtcagccag 840

atcaaacctt ggaaattctg atgagtgagc ttgacccagc agttatggac cagttctaca 900

tgaaagatgg tgttactgca aaggatgtca ctcgtgagag tggaattcgt gacctgatac 960

caggttctgt cattgatgcc acaatgttca atccttgtgg gtattcgatg aatggaatga 1020

aatcggatgg aacttattgg actattcaca tcactccaga accagaattt tcttatgtta 1080

gctttgaaac aaacttaagt cagacctcct atgatgacct gatcaggaaa gttgtagaag 1140

tcttcaagcc aggaaaattt gtgaccacct tgtttgttaa tcagagttct aaatgtcgca 1200

cagtgcttgc ttcgccccag aagattgaag gttttaagcg tcttgattgc cagagtgcta 1260
tgttcaatga ttacaatttt gtttttacca gttttgctaa gaagcagcaa caacagcaga 1320
gttgattaag aaaaatgaag aaaaaacgca aaaagagaac acatgtagaa ggtggtggat 1380
gctttctaga tgtcgatgct gggggcagtg ctttccataa ccaccactgt gtagttgcag 1440
aaagccctag atgtaatgat agtgtaatca ttttgaattg tatgcattat tatatcaagg 1500
agttagatat cttgcatgaa tgctctcttc tgtgtttagg tattctctgc cactcttgct 1560
gtgaaattga agtggatgta gaaaaaacct tttactatat gaaactttac aacacttgtg 1620
aaagcaactc aatttggttt atgcacagtg taatatttct ccaagtatca tccaaaattc 1680
cccacagaca aggctttcgt cctcattagg tgttggcctc agcctaaccc tctaggactg 1740
ttctattaaa ttgctgccag aattttacat ccagttacct ccactttcta gaacatattc 1800
tttactaatg ttattgaaac caatttctac ttcatactga tgtttttgga aacagcaatt 1860
aaagtttttc ttccatagtt gagtccttag aaaatgattc cagttactca ttttgcatat 1920
tgctatttaa cattattgga ccctgcattt atagtccttt gatttcttcc ctctccctgg 1980
tgtctccccc aagaccccaa ataaagcaat accctgttaa cactgtgggt ttatatacta 2040
attctatacc ccagatgggg aattaggggg gagatggtcc ctgggcttaa tattctttaa 2100
agggcatggg aatttagcct ctcttttatt gtaatgtgct cttttggaaa atagttggtt 2160
agcagggaga ccagagttgt agattgagat tgggtgtact ggctgttctg tggaaaacat 2220
acattctgtg ttcctcgaat aagtgaaatt gagcttctaa tgagatgcac ccctttacta 2280
acttgatgat gatataaaat tcatttttat ttagttaatt accagagaga tttagcataa 2340
ttttgcttct ggattcagta aatcaagtca gcttggatca ttcaccttaa cttttccttt 2400
agcagccctt tccactagtt tcccattaag tagtgttcta taaactttga tccaaagcag 2460
aatcaatgtc ttttccatct cgtgacttaa agttctgtga ctgtgatgca tgtgagtgtt 2520
ccgacttcat ctgttcctct taactacggt gtttccctta ccgatcggca ttcataggat 2580
gaaatgaatg actgtcccag aatgagaatt tgtccagatt attcagataa acatcataaa 2640
gagaataaca ttataaataa gtagaatatg aataaataga ataataaaat tccaaaatac 2700
tcaatgggaa atgactagta atataggctt tcaagagttg gtacctttac gtatatttgc 2760
agattctctg ggattttaag gaactgagaa aacagaaaag ttgactaaat tttatatttc 2820
ttgtcctcta aatattttga taatttctgg attgatgcag tgatgttttt cgttccttgt 2880
atttataaat gaaaaccttt ttttggtgtt tctaaaccta aaatctactt ggtttgaaat 2940
caagtggttg gaacactgtt tgacttttat ttgaagcatg ttgttgattg aaaatttcat 3000
tgaggaagtt ttcaatcagt gtgatcagtt tgattctgta atgagcacag cacctaatat 3060

tttgaggagc tctgttttga ggaccaatgc ttaaggtgga ccttgttgct aaacaatatc 3120

ccaatagatt tgttgacttg aggtctggtt tggttttgtt tttgttttgt tttggttttg 3180

ttttgtttcc caatagaatt aagaattcta atgttgaaaa actgtataaa tttttatggg 3240

acaaagccta gaaaagagaa atgtagtttg aatcataatc taaatcatcg tatgatagga 3300

aggaaaagtt ttggtgccat aatttctcct ttcactggtg ttggacttaa atcagttgaa 3360

atgtatttct gtaccacaat ttacgcttca ataaaagttt aattgtctag tgag 3414

<210> 37
<211> 3287
<212> DNA
<213> Homo sapiens

<400> 37
agactgaggc ggaggcagcc ccgcgccgcg ccggacccga gcatatttca ttttctgtca 60

ttggactttg agccattaga accatgagca actacagtgt gtcactggtt ggcccagctc 120

cttggggttt ccggctgcag ggcggtaagg atttcaacat gcctctgaca atctctagtc 180

taaaagatgg cggcaaggca gcccaggcaa atgtaagaat aggcgatgtg gttctcagca 240

ttgatggaat aaatgcacaa ggaatgactc atcttgaagc ccagaataag attaagggtt 300

gtacaggctc tttgaatatg actctgcaaa gagcatctgc tgcacccaag cctgagccgg 360

ttcctgttca aaagggagaa cctaaagaag tagttaaacc tgtgcccatt acatctcctg 420

ctgtgtccaa agtcacttcc acaaacaaca tggcctacaa taaggcacca cggccttttg 480

gttctgtgtc ttcaccaaaa gtcacatcca tcccatcacc atcgtctgcc ttcaccccag 540

cccatgcgac cacctcatca catgcttccc cttcacccgt ggctgccgtc actcctcccc 600

tgttcgctgc atctggactg catgctaatg ccaatcttag tgctgaccag tctccatctg 660

cactgagcgc tggtaaaact gcagttaatg tcccacggca gcccacagtc accagcgtgt 720

gttccgagac ttctcaggag ctagcagagg gacagagaag aggatcccag ggtgacagta 780

aacagcaaaa tggcccacca agaaaacaca ttgtggagcg ctatacagag ttttatcatg 840

tacccactca cagtgatgcc agcaagaaga gactgattga ggatactgaa gactggcgtc 900

caagaactgg aacaactcag tctcgctctt tccgaatcct tgcccagatc actgggactg 960

aacatttgaa agaatctgaa gccgataata caaagaaggc aaataactct caggagcctt 1020

ctccgcagtt ggcttccttg gtagcttcca cacggagcat gcccgagagc ctggacagcc 1080

caacctctgg cagaccaggg gttaccagcc tcacaactgc agctgccttc aagcctgtag 1140

gatccactgg cgtcatcaag tcaccaagct ggcaacggcc aaaccaagga gtaccttcca 1200

ctggaagaat ctcaaacagc gctacttact caggatcagt ggcaccagcc aactcagctt 1260

tgggacaaac ccagccaagt gaccaggaca ctttagtgca aagagctgag cacattccag 1320

cagggaaacg aactccgatg tgcgcccatt gtaaccaggt catcagagga ccattcttag 1380

tggcactggg gaaatcttgg cacccagaag aattcaactg cgctcactgc aaaaatacaa 1440

tggcctacat tggatttgta gaggagaaag gagccctgta ttgtgagctg tgctatgaga 1500

aattctttgc ccctgaatgt ggtcgatgcc aaaggaagat ccttggagaa gtcatcaatg 1560

cgttgaaaca aacttggcat gtttcctgtt ttgtgtgtgt agcctgtgga aagcccattc 1620

ggaacaatgt ttttcacttg gaggatggtg aaccctactg tgagactgat tattatgccc 1680

tctttggtac tatatgccat ggatgtgaat ttcccataga agctggtgac atgttcctgg 1740

aagctctggg ctacacctgg catgacactt gctttgtatg ctcagtgtgt tgtgaaagtt 1800

tggaaggtca gaccttttc tccaagaagg acaagcccct gtgtaagaaa catgctcatt 1860

ctgtgaattt ttgaaagtca acagttcagg agaagagaag gaatttgaag agaaaaagga 1920

aaattaaaat tactaattaa tttttagatt caatatttat atggagtttt gaaaaataat 1980

agtggccctg aaggaataaa ttccagcttt aaaaaccaag tctgaggaaa tatttggctt 2040

cataaagtaa agagacggtt tggcatttat tattactttt tcctgtattt tatgcccata 2100

aaataagctt tataaaaacc aatttcctga tggactatta aattcatctt agaataaatt 2160

agtgaagaat ttaattttag aataaataat ccaatctgaa ataattatac cttctttcct 2220

tgttaggtag ttatgagtaa atctgcaaaa ggcaatgaaa atgccttaaa ttttatcaat 2280

aacagaatta ttgtatttaa aaaaaaacta atacttatct ttaaaatagt aaataggatt 2340

ttaaacagag aattttatca gtaataggtg tcagttttta aaaaattgct tgtaggctga 2400

gcgcggtggc tcacgcctgt aatcccagca ctttgggagg ccaaggtggg tggaccacat 2460

gaggtcagga gtttgagatc agcctggcca acatggtgaa accccatctc tactaaaaat 2520

acaaaaatta gccggacgca gtggcacgcg cctgtaatcc cagctactca agaggctgag 2580

gcacgagaat cacttgaacc cgggagggag aggttgcagt gagccaagat cgtaccactg 2640

cactccagcc tgggtgacag agtgagactc cgtctccaaa aaaaaacttt gcttgtatat 2700

tattttttgcc ttacagtgga tcattctagt aggaaaggac aataagattt tttatcaaaa 2760

tgtgtcatgc cagtaagaga tgttatattc ttttcttatt tcttccccac ccaaaaataa 2820

gctaccatat agcttataag tctcaaattt ttgcctttta ctaaaatgtg attgtttctg 2880

ttcattgtgt atgcttcatc acctatatta ggcaaattcc atttttttccc ttgcgctaag 2940

gtaaagattt aattaaataa ttttggcctc tcatagtttt ctctctcttt aaagagaata 3000

aatagagggc caggtgtggt ggctcacgcc tgtgatccca gcactttggg aggccaagac 3060

gggcggatca tgaggtcaag agatcaagat catcctggcc aacatggtga aaccctgtct 3120

ctactaaaaa tacaaaaatg agctgggcat ggtggggcgt gcctgtagtc ccatgtactt 3180

gggaggctga ggcaggaaaa ttcttgaacc caggagacgg aagttgcagt gagctgagat 3240

cacaccactg cactccagcc tggtgacaga gcaagactcc ggctctt 3287

<210> 38
<211> 1254
<212> DNA
<213> Homo sapiens

<400> 38
atggcagtgg agaccacggt ccacactcac ctctctgcgt ctccaccgca gggctctccc 60

tacgaccaca cacccggcat ggcgggctcc ttggggtacc atccttacgc ggcgcccctg 120

ggatcgtacc cttacgggga cccagcgtac cggaagaacg ccacaaggga cgccacggct 180

accctcaagg cctggctcaa cgagcaccgc aagaacccct accccaccaa gggcgagaag 240

atcatgctgg ccatcatcac caagatgacc ctcacccagg tgtccacctg gttcgccaac 300

gcgcgccggc gcctcaagaa agagaataaa atgacgtgga cgccgcggaa ccgcagcgag 360

gacgaggaag aggaggagaa cattgacctg gagaagaacg acgaggacga gccccagaag 420

cccgaggaca agggcgaccc cgagggcccc gaagcaggag gagctgagca gaaggcggct 480

tcgggctgcg aacggcttca gggaccaccc accccctgcag gcaaggagac ggagggcagc 540

ctcagcgact cggattttaa ggagccgccc tcggagggcc gcctcgacgc gctgcagggc 600

cccccccgca ccggcgggcc ctccccggct gggccagcgg cggcgcggct ggcggaggac 660

ccggcccctc actaccccgc cggagcgccg gcgcccggcc cgcatccagc cgcgggcgag 720

gtgcctccgg gtcccggcgg gccctcggtt atccattcgc cgcctccgcc gccgcctcct 780

gcggtgctcg ccaagcccaa actgtggtct ttggcagaga tcgccacatt gtcggacaag 840

gtcaaggacg gggcggcgg gaacgagggc tctccatgcc caccgtgtcc cgggcccata 900

gccgggcaag ccctaggagg cagccgggcg tcgccggccc cggcgccgtc acgctcgccc 960

tcggcgcagt gtccttttcc aggcgggacg gtgctgtccc ggcctctcta ctacaccgcg 1020

cccttctatc ccggctacac gaactatggc tccttcggac accttcatgg ccacccgggg 1080

cccgggccag gccccacaac cggtccgggg tctcatttca atggattaaa ccagaccgtg 1140

ttgaaccgag cggacgcttt ggctaaagac ccgaaaatgt tgcggagcca gtctcagcta 1200

gacctgtgca aagactctcc ctatgaattg aagaaaggta tgtccgacat ttaa 1254

<210> 39
<211> 2560
<212> DNA

<213> Homo sapiens

<400> 39

```
gaattccggc cagaagaaat ctggcctcgg aacacgccat tctccgcgcc gcttccaata     60
accactaaca tccctaacga gcatccgagc cgagggctct gctcggaaat cgtcctggcc    120
caactcggcc cttcgagctc tcgaagatta ccgcatctat tttttttttc tttttttttct    180
tttcctagcg cagataaagt gagcccggaa agggaaggag gggcgggga caccattgcc    240
ctgaaagaat aaataagtaa ataaacaaac tggctcctcg ccgcagctgg acgcggtcgg    300
ttgagtccag gttgggtcgg acctgaaccc ctaaaagcgg aaccgcctcc cgccctcgcc    360
atcccggagc tgagtcgccg gcggcggtgg ctgctgccag acccggagtt tcctctttca    420
ctggatggag ctgaactttg ggcggccaga gcagcacagc tgtccgggga tcgctgcacg    480
ctgagctccc tcggcaagac ccagcggcgg ctcgggattt ttttgggggg gcggggacca    540
gccccgcgcc ggcaccatgt tcctggcgac cctgtacttc gcgctgccgc tcttggactt    600
gctcctgtcg gccgaagtga gcggcggaga ccgcctggat tgcgtgaaag ccagtgatca    660
gtgcctgaag gagcagagct gcagcaccaa gtaccgcacg ctaaggcagt gcgtggcggg    720
caaggagacc aacttcagcc tggcatccgg cctggaggcc aaggatgagt gccgcagcgc    780
catggaggcc ctgaagcaga agtcgctcta caactgccgc tgcaagcggg gtatgaagaa    840
ggagaagaac tgcctgcgca tttactggag catgtaccag agcctgcagg gaaatgatct    900
gctggaggat tccccatatg aaccagttaa cagcagattg tcagatatat tccgggtggt    960
cccattcata tcagatgttt ttcagcaagt ggagcacatt cccaaaggga acaactgcct   1020
ggatgcagcg aaggcctgca acctcgacga catttgcaag aagtacaggt cggcgtacat   1080
caccccgtgc accaccagcg tgtccaacga tgtctgcaac cgccgcaagt gccacaaggc   1140
cctccggcag ttctttgaca aggtcccggc caagcacagc tacggaatgc tcttctgctc   1200
ctgccgggac atcgcctgca cagagcggag gcgacagacc atcgtgcctg tgtgctccta   1260
tgaagagagg gagaagccca actgtttgaa tttgcaggac tcctgcaaga cgaattacat   1320
ctgcagatct cgccttgcgg attttttttac caactgccag ccagagtcaa ggtctgtcag   1380
cagctgtcta aaggaaaact acgctgactg cctcctcgcc tactcggggc ttattggcac   1440
agtcatgacc cccaactaca tagactccag tagcctcagt gtggccccat ggtgtgactg   1500
cagcaacagt gggaacgacc tagaagagtg cttgaaattt ttgaatttct tcaaggacaa   1560
tacatgtctt aaaaatgcaa ttcaagcctt tggcaatggc tccgatgtga ccgtgtggca   1620
gccagccttc ccagtacaga ccaccactgc cactaccacc actgccctcc gggttaagaa   1680
caagcccctg gggccagcag ggtctgagaa tgaaattccc actcatgttt tgccaccgtg   1740
```

tgcaaattta caggcacaga agctgaaatc caatgtgtcg ggcaatacac acctctgtat 1800

ttccaatggt aattatgaaa aagaaggtct cggtgcttcc agccacataa ccacaaaatc 1860

aatggctgct cctccaagct gtggtctgag cccactgctg gtcctggtgg taaccgctct 1920

gtccacccta ttatctttaa cagaaacatc atagctgcat taaaaaaata caatatggac 1980

atgtaaaaag acaaaaacca agttatctgt ttcctgttct cttgtatagc tgaaattcca 2040

gtttaggagc tcagttgaga aacagttcca ttcaactgga acattttttt ttttcctttt 2100

aagaaagctt cttgtgatcc ttcggggctt ctgtgaaaaa cctgatgcag tgctccatcc 2160

aaactcagaa ggctttggga tatgctgtat tttaaaggga cagtttgtaa cttgggctgt 2220

aaagcaaact ggggctgtgt tttcgatgat gatgatcatc atgatcatga tgattttaac 2280

agttttactt ctggcctttc ctagctagag aaggagttaa tatttctaag gtaactccca 2340

tatctccttt aatgacattg atttctaatg atataaattt cagcctacat tgatgccaag 2400

ctttttttgcc acaaagaaga ttcttaccaa gagtgggctt tgtggaaaca gctggtactg 2460

atgttcacct ttatatatgt actagcattt tccacgctga tgtttatgta ctgtaaacag 2520

ttctgcactc ttgtacaaaa gaaaaaacca cccggaattc 2560

<210> 40
<211> 2560
<212> DNA
<213> Homo sapiens

<400> 40
gaattccggc cagaagaaat ctggcctcgg aacacgccat tctccgcgcc gcttccaata 60

accactaaca tccctaacga gcatccgagc cgagggctct gctcggaaat cgtcctggcc 120

caactcggcc cttcgagctc tcgaagatta ccgcatctat tttttttttc ttttttttct 180

tttcctagcg cagataaagt gagcccggaa agggaaggag gggcgggga caccattgcc 240

ctgaaagaat aaataagtaa ataaacaaac tggctcctcg ccgcagctgg acgcggtcgg 300

ttgagtccag gttgggtcgg acctgaaccc ctaaaagcgg aaccgcctcc cgccctcgcc 360

atcccggagc tgagtcgccg gcggcggtgg ctgctgccag acccggagtt tcctctttca 420

ctggatggag ctgaactttg ggcggccaga gcagcacagc tgtccgggga tcgctgcacg 480

ctgagctccc tcggcaagac ccagcggcgg ctcgggattt ttttgggggg gcggggacca 540

gccccgcgcc ggcaccatgt tcctggcgac cctgtacttc gcgctgccgc tcttggactt 600

gctcctgtcg gccgaagtga gcggcggaga ccgcctggat tgcgtgaaag ccagtgatca 660

gtgcctgaag gagcagagct gcagcaccaa gtaccgcacg ctaaggcagt gcgtggcggg 720

caaggagacc aacttcagcc tggcatccgg cctggaggcc aaggatgagt gccgcagcgc 780

```
catggaggcc ctgaagcaga agtcgctcta caactgccgc tgcaagcggg gtatgaagaa    840
ggagaagaac tgcctgcgca tttactggag catgtaccag agcctgcagg gaaatgatct    900
gctggaggat tccccatatg aaccagttaa cagcagattg tcagatatat tccgggtggt    960
cccattcata tcagatgttt ttcagcaagt ggagcacatt cccaaaggga acaactgcct   1020
ggatgcagcg aaggcctgca acctcgacga catttgcaag aagtacaggt cggcgtacat   1080
caccccgtgc accaccagcg tgtccaacga tgtctgcaac cgccgcaagt gccacaaggc   1140
cctccggcag ttctttgaca aggtcccggc caagcacagc tacggaatgc tcttctgctc   1200
ctgccgggac atcgcctgca cagagcggag gcgacagacc atcgtgcctg tgtgctccta   1260
tgaagagagg gagaagccca actgtttgaa tttgcaggac tcctgcaaga cgaattacat   1320
ctgcagatct cgccttgcgg atttttttac caactgccag ccagagtcaa ggtctgtcag   1380
cagctgtcta aaggaaaact acgctgactg cctcctcgcc tactcggggc ttattggcac   1440
agtcatgacc cccaactaca tagactccag tagcctcagt gtggccccat ggtgtgactg   1500
cagcaacagt gggaacgacc tagaagagtg cttgaaattt ttgaatttct tcaaggacaa   1560
tacatgtctt aaaaatgcaa ttcaagcctt tggcaatggc tccgatgtga ccgtgtggca   1620
gccagccttc ccagtacaga ccaccactgc cactaccacc actgccctcc gggttaagaa   1680
caagcccctg gggccagcag ggtctgagaa tgaaattccc actcatgttt tgccaccgtg   1740
tgcaaattta caggcacaga agctgaaatc caatgtgtcg ggcaatacac acctctgtat   1800
ttccaatggt aattatgaaa aagaaggtct cggtgcttcc agccacataa ccacaaaatc   1860
aatggctgct cctccaagct gtggtctgag cccactgctg gtcctggtgg taaccgctct   1920
gtccacccta ttatctttaa cagaaacatc atagctgcat taaaaaaata caatatggac   1980
atgtaaaaag acaaaaacca agttatctgt ttcctgttct cttgtatagc tgaaattcca   2040
gtttaggagc tcagttgaga aacagttcca ttcaactgga acattttttt ttttcctttt   2100
aagaaagctt cttgtgatcc ttcggggctt ctgtgaaaaa cctgatgcag tgctccatcc   2160
aaactcagaa ggctttggga tatgctgtat tttaaaggga cagtttgtaa cttgggctgt   2220
aaagcaaact ggggctgtgt tttcgatgat gatgatcatc atgatcatga tgattttaac   2280
agttttactt ctggcctttc ctagctagag aaggagttaa tatttctaag gtaactccca   2340
tatctccttt aatgacattg atttctaatg atataaattt cagcctacat tgatgccaag   2400
ctttttttgcc acaaagaaga ttcttaccaa gagtgggctt tgtggaaaca gctggtactg   2460
atgttcacct ttatatatgt actagcattt tccacgctga tgtttatgta ctgtaaacag   2520
ttctgcactc ttgtacaaaa gaaaaaacca cccggaattc                         2560
```

<210> 41
<211> 2439
<212> DNA
<213> Homo sapiens

<400> 41
cagcacccag ctccccgcca ccgccatggt ccccgacacc gcctgcgttc ttctgctcac 60

cctggctgcc ctcggcgcgt ccggacaggg ccagagcccg ttgggctcag acctgggccc 120

gcagatgctt cgggaactgc aggaaaccaa cgcggcgctg caggacgtgc gggactggct 180

gcggcagcag gtcagggaga tcacgttcct gaaaaacacg gtgatggagt gtgacgcgtg 240

cgggatgcag cagtcagtac gcaccggcct acccagcgtg cggcccctgc tccactgcgc 300

gcccggcttc tgcttccccg gcgtggcctg catccagacg gagagcggcg gccgctgcgg 360

cccctgcccc gcgggcttca cgggcaacgg ctcgcactgc accgacgtca acgagtgcaa 420

cgcccacccc tgcttccccc gagtccgctg tatcaacacc agcccggggt tccgctgcga 480

ggcttgcccg ccggggtaca gcggccccac ccaccagggc gtggggctgg ctttcgccaa 540

ggccaacaag caggtttgca cggacatcaa cgagtgtgag accgggcaac ataactgcgt 600

ccccaactcc gtgtgcatca acacccgggg ctccttccag tgcggcccgt gccagcccgg 660

cttcgtgggc gaccaggcgt ccggctgcca gcgcggcgca cagcgcttct gccccgacgg 720

ctcgcccagc gagtgccacg agcatgcaga ctgcgtccta gagcgcgatg gctcgcggtc 780

gtgcgtgtgt cgcgttggct gggccggcaa cgggatcctc tgtggtcgcg acactgacct 840

agacggcttc ccggacgaga agctgcgctg cccggagccg cagtgccgta aggacaactg 900

cgtgactgtg cccaactcag ggcaggagga tgtggaccgc gatggcatcg gagacgcctg 960

cgatccggat gccgacgggg acggggtccc caatgaaaag gacaactgcc cgctggtgcg 1020

gaacccagac cagcgcaaca cggacgagga caagtggggc gatgcgtgcg acaactgccg 1080

gtcccagaag aacgacgacc aaaaggacac agaccaggac ggccggggcg atgcgtgcga 1140

cgacgacatc gacggcgacc ggatccgcaa ccaggccgac aactgcccta gggtacccaa 1200

ctcagaccag aaggacagtg atggcgatgg tataggggat gcctgtgaca actgtcccca 1260

gaagagcaac ccggatcagg cggatgtgga ccacgacttt gtgggagatg cttgtgacag 1320

cgatcaagac caggatggag acggacatca ggactctcgg gacaactgtc ccacggtgcc 1380

taacagtgcc caggaggact cagaccacga tggccagggt gatgcctgcg acgacgacga 1440

cgacaatgac ggagtccctg acagtcggga caactgccgc ctggtgccta accccggcca 1500

ggaggacgcg gacagggacg gcgtgggcga cgtgtgccag gacgactttg atgcagacaa 1560

ggtggtagac aagatcgacg tgtgtccgga gaacgctgaa gtcacgctca ccgacttcag 1620

ggccttccag acagtcgtgc tggacccgga gggtgacgcg cagattgacc ccaactgggt 1680

```
ggtgctcaac cagggaaggg agatcgtgca gacaatgaac agcgacccag gcctggctgt   1740
gggttacact gccttcaatg gcgtggactt cgagggcacg ttccatgtga acacggtcac   1800
ggatgacgac tatgcgggct tcatctttgg ctaccaggac agctccagct tctacgtggt   1860
catgtggaag cagatggagc aaacgtattg gcaggcgaac cccttccgtg ctgtggccga   1920
gcctggcatc caactcaagg ctgtgaagtc ttccacaggc cccggggaac agctgcggaa   1980
cgctctgtgg catacaggag acacagagtc ccaggtgcgg ctgctgtgga aggacccgcg   2040
aaacgtgggt tggaaggaca agaagtccta tcgttggttc ctgcagcacc ggccccaagt   2100
gggctacatc agggtgcgat tctatgaggg ccctgagctg gtggccgaca gcaacgtggt   2160
cttggacaca accatgcggg gtggccgcct gggggtcttc tgcttctccc aggagaacat   2220
catctgggcc aacctgcgtt accgctgcaa tgacaccatc ccagaggact atgagaccca   2280
tcagctgcgg caagcctagg gaccagggtg aggacccgcc ggatgacagc caccctcacc   2340
gcggctggat gggggctctg cacccagccc aaggggtggc cgtcctgagg gggaagtgag   2400
aagggctcag agaggacaaa ataaagtgtg tgtgcaggg                          2439

<210>  42
<211>  2439
<212>  DNA
<213>  Homo sapiens

<400>  42
cagcacccag ctccccgcca ccgccatggt ccccgacacc gcctgcgttc ttctgctcac     60
cctggctgcc ctcggcgcgt ccggacaggg ccagagcccg ttgggctcag acctgggccc    120
gcagatgctt cgggaactgc aggaaaccaa cgcggcgctg caggacgtgc gggactggct    180
gcggcagcag gtcagggaga tcacgttcct gaaaaacacg gtgatggagt gtgacgcgtg    240
cgggatgcag cagtcagtac gcaccggcct acccagcgtg cggcccctgc tccactgcgc    300
gcccggcttc tgcttccccg gcgtggcctg catccagacg gagagcggcg gccgctgcgg    360
cccctgcccc gcgggcttca cgggcaacgg ctcgcactgc accgacgtca acgagtgcaa    420
cgcccacccc tgcttccccc gagtccgctg tatcaacacc agcccggggt tccgctgcga    480
ggcttgcccg ccggggtaca gcggccccac ccaccagggc gtggggctgg ctttcgccaa    540
ggccaacaag caggtttgca cggacatcaa cgagtgtgag accgggcaac ataactgcgt    600
ccccaactcc gtgtgcatca acacccgggg ctccttccag tgcggcccgt gccagcccgg    660
cttcgtgggc gaccaggcgt ccggctgcca gcgcggcgca cagcgcttct gccccgacgg    720
ctcgcccagc gagtgccacg agcatgcaga ctgcgtccta gagcgcgatg gctcgcggtc    780
gtgcgtgtgt cgcgttggct gggccggcaa cgggatcctc tgtggtcgcg acactgacct    840
```

agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440
cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920
gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct gggggtcttc tgcttctccc aggagaacat 2220
catctgggcc aacctgcgtt accgctgcaa tgacaccatc ccagaggact atgagaccca 2280
tcagctgcgg caagcctagg gaccagggtg aggacccgcc ggatgacagc caccctcacc 2340
gcggctggat gggggctctg cacccagccc aaggggtggc cgtcctgagg gggaagtgag 2400
aagggctcag agaggacaaa ataaagtgtg tgtgcaggg 2439

<210> 43
<211> 1385
<212> DNA
<213> Homo sapiens

<400> 43
ctccagccat tggtgtctgt gtcattacta atagagtctt gtaaacactc gttaatcacg 60

gaagccgccg gcctggggct ccgcacgcca gcctgtgcgg gtcttccccg cctctgcagc 120

ctagtgggaa ggaggtggga ggaaagaagg aagaaaggga gggagggagg aggcaggcca 180

gagggaggga ccgcctcgga ggcagaagag ccgcgaggag ccagcggagc accgcgggct 240

ggggcgcagc cacccgccgc tcctcgagtc ccctcgcccc tttcccttcg tgcccccgg 300

cagcctccag cgtcggtccc caggcagcat ggtgaggtct gctcccggtc cctcgccacc 360

atgtacgtga gctacctcct ggacaaggac gtgagcatgt accctagctc cgtgcgccac 420

tctggcggcc tcaacctggc gccgcagaac ttcgtcagcc ccccgcagta cccggactac 480

ggcggttacc acgtggcggc cgcagctgca gcggcagcga acttggacag cgcgcagtcc 540

ccggggccat cctggccggc agcgtatggc gccccactcc gggaggactg gaatggctac 600

gcgcccggag gcgccgcggc cgccgccaac gccgtggctc acggcctcaa cggtggctcc 660

ccggccgcag ccatgggcta cagcagcccc gcagactacc atccgcacca ccacccgcat 720

caccacccgc accacccggc cgcggcgcct tcctgcgctt ctgggctgct gcaaacgctc 780

aaccccggcc ctcctgggcc cgccgccacc gctgccgccg agcagctgtc tcccggcggc 840

cagcggcgga acctgtgcga gtggatgcgg aagccggcgc agcagtccct cggcagccaa 900

gtgaaaacca ggacgaaaga caaatatcga gtggtgtaca cggaccacca gcggctggag 960

ctggagaagg agtttcacta cagtcgctac atcaccatcc ggaggaaagc cgagctagcc 1020

gccacgctgg ggctctctga gaggcaggtt aaaatctggt ttcagaaccg cagagcaaag 1080

gagaggaaaa tcaacaagaa gaagttgcag cagcaacagc agcagcagcc accacagccg 1140

cctccgccgc caccacagcc tccccagcct cagccaggtc ctctgagaag tgtcccagag 1200

cccttgagtc cggtgtcttc cctgcaagcc tcagtgtctg gctctgtccc tggggttctg 1260

gggccaactg ggggggtgct aaaccccacc gtcacccagt gacccaccgg ggttctgcag 1320

cggcagagca attccaggct gagccatgag gagcgtggac tctgctagac tcctcaggag 1380

agacc 1385

<210> 44
<211> 4098
<212> DNA
<213> Homo sapiens

<400> 44
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agctatggag ccgcggccca 180

cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300
ctgcgcccgc gagccgggac ggcggcgggg tccgcgatga gggccccgcg gcggccgggg 360
acgggctggg cagacccttg gggcccaccc cgagccagag ccgtttccag gtggacctgg 420
tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540
gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atctttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980

```
gtatattttc agccactctt tcttcagcat tagcatccct agtgagtgct cccaaaatat   2040
ttcaggctct atgtaaggac aacatctacc cagctttcca gatgtttgct aaaggttatg   2100
ggaaaaataa tgaacctctt cgtggctaca tcttaacatt cttaattgca cttggattca   2160
tcttaattgc tgaactgaat gttattgcac caattatctc aaacttcttc cttgcatcat   2220
atgcattgat caatttttca gtattccatg catcacttgc aaaatctcca ggatggcgtc   2280
ctgcattcaa atactacaac atgtggatat cacttcttgg agcaattctt tgttgcatag   2340
taatgttcgt cattaactgg tgggctgcat tgctaacata tgtgatagtc cttgggctgt   2400
atatttatgt tacctacaaa aaaccagatg tgaattgggg atcctctaca caagccctga   2460
cttacctgaa tgcactgcag cattcaattc gtctttctgg agtggaagac cacgtgaaaa   2520
actttaggcc acagtgtctt gttatgacag gtgctccaaa ctcacgtcca gctttacttc   2580
atcttgttca tgatttcaca aaaaatgttg gtttgatgat ctgtggccat gtacatatgg   2640
gtcctcgaag acaagccatg aaagagatgt ccatcgatca agccaaatat cagcgatggc   2700
ttattaagaa caaaatgaag gcattttatg ctccagtaca tgcagatgac ttgagagaag   2760
gtgcacagta tttgatgcag gctgctggtc ttggtcgtat gaagccaaac acacttgtcc   2820
ttggatttaa gaaagattgg ttgcaagcag atatgaggga tgtggatatg tatataaact   2880
tatttcatga tgcttttgac atacaatatg gagtagtggt tattcgccta aaagaaggtc   2940
tggatatatc tcatcttcaa ggacaagaag aattattgtc atcacaagag aaatctcctg   3000
gcaccaagga tgtggtagta agtgtggaat atagtaaaaa gtccgattta gatacttcca   3060
aaccactcag tgaaaaacca attacacaca aagttgagga agaggatggc aagactgcaa   3120
ctcaaccact gttgaaaaaa gaatccaaag gccctattgt gcctttaaat gtagctgacc   3180
aaaagcttct tgaagctagt acacagtttc agaaaaaaca aggaaagaat actattgatg   3240
tctggtggct tttgatgat ggaggtttga ccttattgat accttacctt ctgacgacca   3300
agaaaaaatg gaaagactgt aagatcagag tattcattgg tggaaagata aacagaatag   3360
accatgaccg gagagcgatg gctactttgc ttagcaagtt ccggatagac ttttctgata   3420
tcatggttct aggagatatc aataccaaac caaagaaaga aaatattata gcttttgagg   3480
aaatcattga gccatacaga cttcatgaag atgataaaga gcaagatatt gcagataaaa   3540
tgaaagaaga tgaaccatgg cgaataacag ataatgagct tgaactttat aagaccaaga   3600
cataccggca gatcaggtta aatgagttat taaaggaaca ttcaagcaca gctaatatta   3660
ttgtcatgag tctcccagtt gcacgaaaag gtgctgtgtc tagtgctctc tacatggcat   3720
ggttagaagc tctatctaag gacctaccac caatcctcct agttcgtggg aatcatcaga   3780
gtgtccttac cttctattca taaatgttct atacagtgga cagccctcca gaatggtact   3840
```

tcagtgccta gtgtagtaac ctgaaatctt caatgacaca ttaacatcac aatggcgaat 3900

ggtgactttt ctttcacgat ttcattaatt tgaaagcaca caggaaagct tgctccattg 3960

ataacgtgta tggagacttc ggttttagtc aattccatat ctcaatctta atggtgattc 4020

ttctctgttg aactgaagtt tgtgagagta gttttccttt gctacttgaa tagcaataaa 4080

agcgtgttaa ctttttgg 4098

<210> 45
<211> 4098
<212> DNA
<213> Homo sapiens

<400> 45
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg gggtcgggc agctatggag ccgcggccca 180

cggcgccctc ctccggcgcc ccgggactgg ccggggtcgg ggagacgccg tcagccgctg 240

cgctggccgc agccagggtg gaactgcccg gcacggctgt gccctcggtg ccggaggatg 300

ctgcgcccgc gagccgggac ggcggcgggg tccgcgatga gggccccgcg gcggccgggg 360

acgggctggg cagacccttg ggcccaccc cgagccagag ccgtttccag gtggacctgg 420

tttccgagaa cgccgggcgg gccgctgctg cggcggcggc ggcggcggcg gcagcggcgg 480

cggctggtgc tggggcgggg gccaagcaga cccccgcgga cggggaagcc agcggcgaga 540

gcgagccagc taaaggcagc gaggaagcca agggccgctt ccgcgtgaac ttcgtggacc 600

cagctgcctc ctcgtcggct gaagacagcc tgtcagatgc tgccggggtc ggagtcgacg 660

ggcccaacgt gagcttccag aacggcgggg acacggtgct gagcgagggc agcagcctgc 720

actccggcgg cggcggcggc agtgggcacc accagcacta ctattatgat acccacacca 780

acacctacta cctgcgcacc ttcggccaca acaccatgga cgctgtgccc aggatcgatc 840

actaccggca cacagccgcg cagctgggcg agaagctgct ccggcctagc ctggcggagc 900

tccacgacga gctggaaaag gaaccttttg aggatggctt tgcaaatggg gaagaaagta 960

ctccaaccag agatgctgtg gtcacgtata ctgcagaaag taaaggagtc gtgaagtttg 1020

gctggatcaa gggtgtatta gtacgttgta tgttaaacat ttggggtgtg atgcttttca 1080

ttagattgtc atggattgtg ggtcaagctg gaataggtct atcagtcctt gtaataatga 1140

tggccactgt tgtgacaact atcacaggat tgtctacttc agcaatagca actaatggat 1200

ttgtaagagg aggaggagca tattatttaa tatctagaag tctagggcca gaatttggtg 1260

gtgcaattgg tctaatcttc gcctttgcca acgctgttgc agttgctatg tatgtggttg 1320

gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atctttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120

ctcaaccact gttgaaaaaa gaatccaaag gccctattgt gcctttaaat gtagctgacc 3180

aaaagcttct tgaagctagt acacagtttc agaaaaaaca aggaaagaat actattgatg 3240

tctggtggct ttttgatgat ggaggtttga ccttattgat accttacctt ctgacgacca 3300

agaaaaaatg gaaagactgt aagatcagag tattcattgg tggaaagata aacagaatag 3360

accatgaccg gagagcgatg gctactttgc ttagcaagtt ccggatagac ttttctgata 3420

tcatggttct aggagatatc aataccaaac caaagaaaga aaatattata gcttttgagg 3480

aaatcattga gccatacaga cttcatgaag atgataaaga gcaagatatt gcagataaaa 3540

tgaaagaaga tgaaccatgg cgaataacag ataatgagct tgaactttat aagaccaaga 3600

cataccggca gatcaggtta aatgagttat taaaggaaca ttcaagcaca gctaatatta 3660

ttgtcatgag tctcccagtt gcacgaaaag gtgctgtgtc tagtgctctc tacatggcat 3720

ggttagaagc tctatctaag gacctaccac caatcctcct agttcgtggg aatcatcaga 3780

gtgtccttac cttctattca taaatgttct atacagtgga cagccctcca gaatggtact 3840

tcagtgccta gtgtagtaac ctgaaatctt caatgacaca ttaacatcac aatggcgaat 3900

ggtgactttt ctttcacgat ttcattaatt tgaaagcaca caggaaagct tgctccattg 3960

ataacgtgta tggagacttc ggttttagtc aattccatat ctcaatctta atggtgattc 4020

ttctctgttg aactgaagtt tgtgagagta gttttccttt gctacttgaa tagcaataaa 4080

agcgtgttaa ctttttgg 4098

<210> 46
<211> 3311
<212> DNA
<213> Homo sapiens

<400> 46
tgctaatgct tttggtacaa atggatgtgg aatataattg aatattttct tgtttaaggg 60

gagcatgaag aggtgttgag gttatgtcaa gcatctggca cagctgaagg cagatggaaa 120

tatttacaag tacgcaattt gagactaaga tattgttatc attctcctat tgaagacaag 180

agcaatagta aaacacatca ggtcaggggg ttaaagacct gtgataaacc acttccgata 240

agttggaaac gtgtgtctat attttcatat ctgtatatat ataatggtaa agaaagacac 300

cttcgtaacc cgcattttcc aaagagagga atcacaggga gatgtacagc aatggggcca 360

tttaagagtt ctgtgttcat cttgattctt caccttctag aaggggccct gagtaattca 420

ctcattcagc tgaacaacaa tggctatgaa ggcattgtcg ttgcaatcga ccccaatgtg 480

ccagaagatg aaacactcat tcaacaaata aaggacatgg tgacccaggc atctctgtat 540

ctgtttgaag ctacaggaaa gcgattttat ttcaaaaatg ttgccatttt gattcctgaa 600

acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460

```
aatgatgaaa tacaatggaa tccaccaaga cctgaaatta ataaggatga tgttcaacac   2520

aagcaagtgt gtttcagcag aacatcctcg ggaggctcat ttgtggcttc tgatgtccca   2580

aatgctccca tacctgatct cttcccacct ggccaaatca ccgacctgaa ggcggaaatt   2640

cacgggggca gtctcattaa tctgacttgg acagctcctg gggatgatta tgaccatgga   2700

acagctcaca agtatatcat tcgaataagt acaagtattc ttgatctcag agacaagttc   2760

aatgaatctc ttcaagtgaa tactactgct ctcatcccaa aggaagccaa ctctgaggaa   2820

gtctttttgt ttaaaccaga aaacattact tttgaaaatg gcacagatct tttcattgct   2880

attcaggctg ttgataaggt cgatctgaaa tcagaaatat ccaacattgc acgagtatct   2940

ttgtttattc ctccacagac tccgccagag acacctagtc ctgatgaaac gtctgctcct   3000

tgtcctaata ttcatatcaa cagcaccatt cctggcattc acattttaaa aattatgtgg   3060

aagtggatag gagaactgca gctgtcaata gcctagggct gaatttttgt cagataaata   3120

aaataaatca ttcatccttt ttttgattat aaaattttct aaaatgtatt ttagacttcc   3180

tgtagggggc gatatactaa atgtatatag tacatttata ctaaatgtat tcctgtaggg   3240

ggcgatatac taaatgtatt ttagacttcc tgtagggggc gataaaataa aatgctaaac   3300

aactgggtaa a                                                        3311
```

```
<210>  47
<211>  3311
<212>  DNA
<213>  Homo sapiens

<400>  47
tgctaatgct tttggtacaa atggatgtgg aatataattg aatattttct tgtttaaggg     60

gagcatgaag aggtgttgag gttatgtcaa gcatctggca cagctgaagg cagatggaaa    120

tatttacaag tacgcaattt gagactaaga tattgttatc attctcctat tgaagacaag    180

agcaatagta aaacacatca ggtcaggggg ttaaagacct gtgataaacc acttccgata    240

agttggaaac gtgtgtctat attttcatat ctgtatatat ataatggtaa agaaagacac    300

cttcgtaacc cgcattttcc aaagagagga atcacaggga gatgtacagc aatggggcca    360

tttaagagtt ctgtgttcat cttgattctt caccttctag aaggggccct gagtaattca    420

ctcattcagc tgaacaacaa tggctatgaa ggcattgtcg ttgcaatcga ccccaatgtg    480

ccagaagatg aaacactcat tcaacaaata aaggacatgg tgacccaggc atctctgtat    540

ctgtttgaag ctacaggaaa gcgattttat ttcaaaaatg ttgccatttt gattcctgaa    600

acatggaaga caaaggctga ctatgtgaga ccaaaacttg agacctacaa aaatgctgat    660

gttctggttg ctgagtctac tcctccaggt aatgatgaac cctacactga gcagatgggc    720
```

aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520

aagcaagtgt gtttcagcag aacatcctcg ggaggctcat ttgtggcttc tgatgtccca 2580

aatgctccca tacctgatct cttcccacct ggccaaatca ccgacctgaa ggcggaaatt 2640

cacgggggca gtctcattaa tctgacttgg acagctcctg gggatgatta tgaccatgga 2700

acagctcaca agtatatcat tcgaataagt acaagtattc ttgatctcag agacaagttc 2760

aatgaatctc ttcaagtgaa tactactgct ctcatcccaa aggaagccaa ctctgaggaa 2820

gtctttttgt ttaaaccaga aaacattact tttgaaaatg gcacagatct tttcattgct 2880

attcaggctg ttgataaggt cgatctgaaa tcagaaatat ccaacattgc acgagtatct 2940

ttgtttattc ctccacagac tccgccagag acacctagtc ctgatgaaac gtctgctcct 3000

tgtcctaata ttcatatcaa cagcaccatt cctggcattc acattttaaa aattatgtgg 3060

aagtggatag gagaactgca gctgtcaata gcctagggct gaatttttgt cagataaata 3120

aaataaatca ttcatccttt ttttgattat aaaattttct aaaatgtatt ttagacttcc 3180

tgtagggggc gatatactaa atgtatatag tacatttata ctaaatgtat tcctgtaggg 3240

ggcgatatac taaatgtatt ttagacttcc tgtagggggc gataaaataa aatgctaaac 3300

aactgggtaa a 3311

<210> 48
<211> 3697
<212> DNA
<213> Homo sapiens

<400> 48
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420

gctaatggaa ttatagtgga gggtccagtc cctatcacca tagaagtgaa ggacatcaac 480

gacaatcgac ccacgtttct ccagtcaaag tacgaaggct cagtaaggca gaactctcgc 540

ccaggaaagc ccttcttgta tgtcaatgcc acagacctgg atgatccggc cactcccaat 600

ggccagcttt attaccagat tgtcatccag cttcccatga tcaacaatgt catgtacttt 660

cagatcaaca acaaaacggg agccatctct cttacccgag agggatctca ggaattgaat 720

cctgctaaga atccttccta taatctggtg atctcagtga aggacatggg aggccagagt 780

gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag accctttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccacccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700

tgcattataa ttttttaaac agatattccc tcttgtcctt taatatttgc taaatatttc 2760

tttttttgagg tggagtcttg ctctgtcgcc caggctggag tacagtggtg tgatcccagc 2820

tcactgcaac ctccgcctcc tgggttcaca tgattctcct gcctcagctt cctaagtagc 2880

tgggtttaca ggcacccacc accatgccca gctaattttt gtatttttaa tagagacggg 2940

gtttcgccat ttggccaggc tggtcttgaa ctcctgacgt caagtgatct gcctgccttg 3000

gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tcccttttgg ggcaagacag actcattaaa tattctgtac atttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 49
<211> 3697
<212> DNA
<213> Homo sapiens

<400> 49
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420

gctaatggaa ttatagtgga gggtccagtc cctatcacca tagaagtgaa ggacatcaac 480

gacaatcgac ccacgtttct ccagtcaaag tacgaaggct cagtaaggca gaactctcgc 540

ccaggaaagc ccttcttgta tgtcaatgcc acagacctgg atgatccggc cactcccaat 600
ggccagcttt attaccagat tgtcatccag cttcccatga tcaacaatgt catgtacttt 660
cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340

gtcgtcttga tccgcatcaa tgatgggggt cggccaccct tggaaggcat tgtttcttta 2400

ccagttacat tctgcagttg tgtggaagga agttgtttcc ggccagcagg tcaccagact 2460

gggataccca ctgtgggcat ggcagttggt atactgctga ccacccttct ggtgattggt 2520

ataattttag cagttgtgtt tatccgcata aagaaggata aaggcaaaga taatgttgaa 2580

agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700

tgcattataa ttttttaaac agatattccc tcttgtcctt taatatttgc taaatatttc 2760

ttttttgagg tggagtcttg ctctgtcgcc caggctggag tacagtggtg tgatcccagc 2820

tcactgcaac ctccgcctcc tgggttcaca tgattctcct gcctcagctt cctaagtagc 2880

tgggtttaca ggcacccacc accatgccca gctaattttt gtatttttaa tagagacggg 2940

gtttcgccat ttggccaggc tggtcttgaa ctcctgacgt caagtgatct gcctgccttg 3000

gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tccctttttgg ggcaagacag actcattaaa tattctgtac atttttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 50
<211> 3803
<212> DNA
<213> Homo sapiens

<400> 50
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300
aaacgcgctg ggcgtctgct ccgacaccgc agcgcccctg gccgcggtgg acctcaagtg 360
ggagcacaac cccgctgtgt tcctgggccc cggctgcgtg tacgccgccg ccccagtggg 420
gcgcttcacc gcgcactggc gggtcccgct gctgaccgcc ggcgccccgg cgctgggctt 480
cggtgtcaag gacgagtatg cgctgaccac ccgcgcgggg cccagctacg ccaagctggg 540
ggacttcgtg gcggcgctgc accgacggct gggctgggag cgccaagcgc tcatgctcta 600
cgcctaccgg ccgggtgacg aagagcactg cttcttcctc gtggaggggc tgttcatgcg 660
ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380
gtaccctcct cctgacatcc ccaaatgtgg ctttgacaac gaagacccag catgcaacca 1440
agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg ggctatctg ttcccatggg aacctcaagt catccaactg 2040
cgtggtagat gggcgctttg tgctcaagat caccgactat gggctggaga gcttcaggga 2100

cctggaccca gagcaaggac acaccgttta tgccaaaaag ctgtggacgg cccctgagct 2160

cctgcgaatg gcttcacccc ctgtgcgggg ctcccaggct ggtgacgtat acagctttgg 2220

gatcatcctt caggagattg ccctgaggag tggggtcttc cacgtggaag gtttggacct 2280

gagccccaaa gagatcatcg agcgggtgac tcggggtgag cagcccccct tccggccctc 2340

cctggccctg cagagtcacc tggaggagtt ggggctgctc atgcagcggt gctgggctga 2400

ggacccacag gagaggccac cattccagca gatccgcctg acgttgcgca aatttaacag 2460

ggagaacagc agcaacatcc tggacaacct gctgtcccgc atggagcagt acgcgaacaa 2520

tctggaggaa ctggtggagg agcggaccca ggcatacctg gaggagaagc gcaaggctga 2580

ggccctgctc taccagatcc tgcctcactc agtggctgag cagctgaagc gtggggagac 2640

ggtgcaggcc gaagcctttg acagtgttac catctacttc agtgacattg tgggtttcac 2700

agcgctgtcg gcggagagca cacccatgca ggtggtgacc ctgctcaatg acctgtacac 2760

ttgctttgat gctgtcatag acaactttga tgtgtacaag gtggagacaa ttggcgatgc 2820

ctacatggtg gtgtcagggc tccctgtgcg gaacgggcgg ctacacgcct gcgaggtagc 2880

ccgcatggcc ctggcactgc tggatgctgt gcgctccttc cgaatccgcc accggcccca 2940

ggagcagctg cgcttgcgca ttggcatcca cacaggacct gtgtgtgctg gagtggtggg 3000

actgaagatg ccccgttact gtctctttgg ggatacagtc aacacagcct caagaatgga 3060

gtctaatggg gaagccctga agatccactt gtcttctgag accaaggctg tcctggagga 3120

gtttggtggt ttcgagctgg agcttcgagg ggatgtagaa atgaagggca aaggcaaggt 3180

tcggacctac tggctccttg ggagagaggg gagtagcacc cgaggctgac ctgcctcctc 3240

tcctatccct ccacacctcc cctaccctgt gccagaagca acagaggtgc caggcctcag 3300

cctcacccac agcagcccca tcgccaaagg atggaagtaa tttgaatagc tcaggtgtgc 3360

tgaccccagt gaagacacca gataggacct ctgagagggg actggcatgg ggggatctca 3420

gagcttacag gctgagccaa gcccacggcc atgcacaggg acactcacac aggcacacgc 3480

acctgctctc cacctggact caggccgggc tgggctgtgg atccttgatc cctcccctc 3540

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gaccccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 51

<211> 3803
<212> DNA
<213> Homo sapiens

<400> 51

```
ccatggtagg agcgctcgcc tcgctgcggt gcccgctgag gccatgccgg ggccccggcg      60
ccccgctggc tcccgcctgc gcctgctcct gctcctgctg ctgccgccgc tgctgctgct     120
gctccggggc agccacgcgg gcaacctgac ggtagccgtg gtactgccgc tggccaatac     180
ctcgtacccc tggtcgtggg cgcgcgtggg acccgccgtg gagctggccc tggcccaggt     240
gaaggcgcgc cccgacttgc tgccgggctg gacggtccgc acggtgctgg gcagcagcga     300
aaacgcgctg ggcgtctgct ccgacaccgc agcgcccctg gccgcggtgg acctcaagtg     360
ggagcacaac cccgctgtgt tcctgggccc cggctgcgtg tacgccgccg ccccagtggg     420
gcgcttcacc gcgcactggc gggtcccgct gctgaccgcc ggcgccccgg cgctgggctt     480
cggtgtcaag gacgagtatg cgctgaccac ccgcgcgggg cccagctacg ccaagctggg     540
ggacttcgtg gcggcgctgc accgacggct gggctgggag cgccaagcgc tcatgctcta     600
cgcctaccgg ccgggtgacg aagagcactg cttcttcctc gtggaggggc tgttcatgcg     660
ggtccgcgac cgcctcaata ttacggtgga ccacctggag ttcgccgagg acgacctcag     720
ccactacacc aggctgctgc ggaccatgcc gcgcaaaggc cgagttatct acatctgcag     780
ctcccctgat gccttcagaa ccctcatgct cctggccctg gaagctggct tgtgtgggga     840
ggactacgtt ttcttccacc tggatatctt tgggcaaagc ctgcaaggtg gacagggccc     900
tgctccccgc aggccctggg agagagggga tgggcaggat gtcagtgccc gccaggcctt     960
tcaggctgcc aaaatcatta catataaaga cccagataat cccgagtact tggaattcct    1020
gaagcagtta aaacacctgg cctatgagca gttcaacttc accatggagg atggcctggt    1080
gaacaccatc ccagcatcct tccacgacgg gctcctgctc tatatccagg cagtgacgga    1140
gactctggca catgggggaa ctgttactga tggggagaac atcactcagc ggatgtggaa    1200
ccgaagcttt caaggtgtga caggatacct gaaaattgat agcagtggcg atcgggaaac    1260
agacttctcc ctctgggata tggatcccga gaatggtgcc ttcagggttg tactgaacta    1320
caatgggact tcccaagagc tggtggctgt gtcggggcgc aaactgaact ggcccctggg    1380
gtaccctcct cctgacatcc ccaaatgtgg ctttgacaac gaagacccag catgcaacca    1440
agatcacctt tccaccctgg aggtgctggc tttggtgggc agcctctcct tgctcggcat    1500
tctgattgtc tccttcttca tatacaggaa gatgcagctg gagaaggaac tggcctcgga    1560
gctgtggcgg gtgcgctggg aggacgttga gcccagtagc cttgagaggc acctgcggag    1620
tgcaggcagc cggctgaccc tgagcgggag aggctccaat tacggctccc tgctaaccac    1680
```

agagggccag ttccaagtct ttgccaagac agcatattat aagggcaacc tcgtggctgt 1740

gaaacgtgtg aaccgtaaac gcattgagct gacacgaaaa gtcctgtttg aactgaagca 1800

tatgcgggat gtgcagaatg aacacctgac caggtttgtg ggagcctgca ccgacccccc 1860

caatatctgc atcctcacag agtactgtcc ccgtgggagc ctgcaggaca ttctggagaa 1920

tgagagcatc accctggact ggatgttccg gtactcactc accaatgaca tcgtcaaggg 1980

catgctgttt ctacacaatg ggctatctg ttcccatggg aacctcaagt catccaactg 2040

cgtggtagat gggcgctttg tgctcaagat caccgactat gggctggaga gcttcaggga 2100

cctggaccca gagcaaggac acaccgttta tgccaaaaag ctgtggacgg cccctgagct 2160

cctgcgaatg gcttcacccc ctgtgcgggg ctcccaggct ggtgacgtat acagctttgg 2220

gatcatcctt caggagattg ccctgaggag tggggtcttc cacgtggaag gtttggacct 2280

gagccccaaa gagatcatcg agcgggtgac tcggggtgag cagccccct tccggccctc 2340

cctggccctg cagagtcacc tggaggagtt ggggctgctc atgcagcggt gctgggctga 2400

ggacccacag gagaggccac cattccagca gatccgcctg acgttgcgca aatttaacag 2460

ggagaacagc agcaacatcc tggacaacct gctgtcccgc atggagcagt acgcgaacaa 2520

tctggaggaa ctggtggagg agcggaccca ggcatacctg gaggagaagc gcaaggctga 2580

ggccctgctc taccagatcc tgcctcactc agtggctgag cagctgaagc gtggggagac 2640

ggtgcaggcc gaagcctttg acagtgttac catctacttc agtgacattg tgggtttcac 2700

agcgctgtcg gcggagagca cacccatgca ggtggtgacc ctgctcaatg acctgtacac 2760

ttgctttgat gctgtcatag acaactttga tgtgtacaag gtggagacaa ttggcgatgc 2820

ctacatggtg gtgtcagggc tccctgtgcg gaacgggcgg ctacacgcct gcgaggtagc 2880

ccgcatggcc ctggcactgc tggatgctgt gcgctccttc cgaatccgcc accggcccca 2940

ggagcagctg cgcttgcgca ttggcatcca cacaggacct gtgtgtgctg gagtggtggg 3000

actgaagatg ccccgttact gtctctttgg ggatacagtc aacacagcct caagaatgga 3060

gtctaatggg gaagccctga agatccactt gtcttctgag accaaggctg tcctggagga 3120

gtttggtggt ttcgagctgg agcttcgagg ggatgtagaa atgaagggca aaggcaaggt 3180

tcggacctac tggctccttg ggagaggggg gagtagcacc cgaggctgac ctgcctcctc 3240

tcctatccct ccacacctcc cctaccctgt gccagaagca acagaggtgc caggcctcag 3300

cctcacccac agcagcccca tcgccaaagg atggaagtaa tttgaatagc tcaggtgtgc 3360

tgaccccagt gaagacacca gataggacct ctgagagggg actggcatgg ggggatctca 3420

gagcttacag gctgagccaa gcccacggcc atgcacaggg acactcacac aggcacacgc 3480

acctgctctc cacctggact caggccgggc tgggctgtgg atccttgatc cctcccctc 3540

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gaccccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 52
<211> 1155
<212> DNA
<213> Homo sapiens

<400> 52
atggattgca gtaacggatc ggcagagtgt accggagaag gaggatcaaa agaggtggtg 60

gggactttta aggctaaaga cctaatagtc acaccagcta ccattttaaa ggaaaaacca 120

gaccccaata atctggtttt tggaactgtg ttcacggatc atatgctgac ggtggagtgg 180

tcctcagagt ttggatggga gaaacctcat atcaagcctc ttcagaacct gtcattgcac 240

cctggctcat cagctttgca ctatgcagtg gaattatttg aaggattgaa ggcatttcga 300

ggagtagata ataaaattcg actgtttcag ccaaacctca acatggatag aatgtatcgc 360

tctgctgtga gggcaactct gccggtattt gacaaagaag agctcttaga gtgtattcaa 420

cagcttgtga aattggatca agaatgggtc ccatattcaa catctgctag tctgtatatt 480

cgtcctgcat tcattggaac tgagccttct cttggagtca agaagcctac caaagccctg 540

ctctttgtac tcttgagccc agtgggacct tatttttcaa gtggaacctt taatccagtg 600

tccctgtggg ccaatcccaa gtatgtaaga gcctggaaag gtggaactgg ggactgcaag 660

atgggaggga attacggctc atctcttttt gcccaatgtg aagacgtaga taatgggtgt 720

cagcaggtcc tgtggctcta tggcagagac catcagatca ctgaagtggg aactatgaat 780

ctttttcttt actggataaa tgaagatgga gaagaagaac tggcaactcc tccactagat 840

ggcatcattc ttccaggagt gacaaggcgg tgcattctgg acctggcaca tcagtggggt 900

gaatttaagg tgtcagagag atacctcacc atggatgact tgacaacagc cctggagggg 960

aacagagtga gagagatgtt tagctctggt acagcctgtg ttgtttgccc agtttctgat 1020

atactgtaca aaggcgagac aatacacatt ccaactatgg agaatggtcc taagctggca 1080

agccgcatct tgagcaaatt aactgatatc cagtatggaa gagaagagag cgactggaca 1140

attgtgctat cctga 1155

<210> 53
<211> 2511

<212> DNA
<213> Homo sapiens

<400> 53
cttttcacac tggccttaaa gaggatatat tagaagttga agtaggaagg gagccagaga 60
ggccgatggc gcaaaggtac gacgatctac cccattacgg gggcatggat ggagtaggca 120
tcccctccac gatgtatggg gacccgcatg cagccaggtc catgcagccg gtccaccacc 180
tgaaccacgg gcctcctctg cactcgcatc agtacccgca cacagctcat accaacgcca 240
tggcccccag catgggctcc tctgtcaatg acgctttaaa gagagataaa gatgccattt 300
atggacaccc cctcttccct ctcttagcac tgatttttga gaaatgtgaa ttagctactt 360
gtacccccg cgagccgggg gtggcgggcg gggacgtctg ctcgtcagag tcattcaatg 420
aagatatagc cgtgttcgcc aaacagattc gcgcagaaaa acctctattt tcttctaatc 480
cagaactgga taacttgatg attcaagcca tacaagtatt aaggtttcat ctattggaat 540
tagagaaggt acacgaatta tgtgacaatt tctgccaccg gtatattagc tgtttgaaag 600
ggaaaatgcc tatcgatttg gtgatagacg atagagaagg aggatcaaaa tcagacagtg 660
aagatataac aagatcagca aatctaactg accagccctc ttggaacaga gatcatgatg 720
acacggcatc tactcgttca ggaggaaccc caggcccttc cagcggtggc cacacgtcac 780
acagtgggga caacagcagt gagcaaggtg atggcttgga caacagtgta gcttccccca 840
gcacaggtga cgatgatgac cctgataagg acaaaaagcg tcacaaaaag cgtggcatct 900
ttcccaaagt agccacaaat atcatgaggg cgtggctgtt ccagcatcta acacaccctt 960
acccttctga agaacagaaa aagcagttgg cacaagacac gggactcacc atccttcaag 1020
tgaacaattg gtttattaat gcccggagaa gaatagtgca gcccatgata gaccagtcca 1080
accgagcagt aagtcaagga acaccttata atcctgatgg acagcccatg ggaggtttcg 1140
taatggacgg tcagcaacat atgggaatta gagcaccagg acctatgagt ggaatgggca 1200
tgaatatggg catggagggg cagtggcact acatgtaacc ttcatctagt taaccaatcg 1260
caaagcaagg gggaaggctg caaagtatgc caggggagta tgtagcccgg ggtggtccaa 1320
tgggtgtgag tatgggacag ccaagttata cccaacccca gatgccccc catcctgctc 1380
agctgcgtca tgggccccc atgcatacgt acattcctgg acaccctcac cacccaacag 1440
tgatgatgca tggaggaccg ccccaccctg gaatgccaat gtcagcatca agccccacag 1500
ttcttaatac aggagaccca acaatgagtg gacaagtcat ggacattcat gctcagtagc 1560
ttaagggaat atgcattgtc tgcaatggtg actgatttca aatcatgttt tttctgcaat 1620
gactgtggag ttccattctt ggcatctact ctggaccaag gagcatccct aattcttcat 1680
agggaccttt aaaaagcagg aaataccaac tgaagtcaat ttgggggaca tgctaaataa 1740

ctatataaga cattaagaga acaaagagtg aaatattgta aatgctatta tactgttatc 1800

catattacgt tgtttcttat agattttttta aaaaaaatgt gaaatttttc cacactatgt 1860

gtgttgtttc catagctctt cacttcctcc agaagcctcc ttacattaaa aagccttaca 1920

gttatcctgc aagggacagg aaggtctgat ttgcaggatt tttagagcat taaaataact 1980

atcaggcaga agaatctttc ttctcgccta ggatttcagc catgcgcgcg ctctctctct 2040

ttctctctct tttcctctct ctccctcttt ctagcctggg gcttgaattt gcatgtctaa 2100

ttcatttact caccatattt gaattggcct gaacagatgt aaatcgggaa ggatgggaaa 2160

aactgcagtc atcaacaatg attaatcagc tgttgcaggc agtgtcttaa ggagactggt 2220

aggaggaggc atggaaacca aaaggccgtg tgtttagaag cctaattgtc acatcaagca 2280

tcattgtccc catgcaacaa ccaccacctt atacatcact tcctgtttta agcagctcta 2340

aaacatagac tgaagattta tttttaatat gttgacttta tttctgagca aagcatcggt 2400

catgtgtgta ttttttcata gtcccacctt ggagcattta tgtagacatt gtaaataaat 2460

tttgtgcaaa aaggactgga aaaatgaact gtattattgc aatttttttt t 2511

<210> 54
<211> 1887
<212> DNA
<213> Homo sapiens

<400> 54
tcgcccaatt ccgggctcag acactgggct cccagctggg gactgctcca tggccatgga 60

gatagacagc aggcctgggg ggctccccgg cagtagctgc aacctaggtg cagcccgaga 120

acacatgcag gcggtcaccc gaaactacat cacccacccc cgtgtcacct acaggactgt 180

gtgcagcgtg aacgggcccc tggtggtgct ggaccgggtc aagtttgccc agtatgcgga 240

gatcgtccac ttcaccctcc cagatgggac tcagaggagc gggcaggtgc ttgaggtggc 300

tggcaccaag gcgattgttc aggtgtttga agggacatca gggatcgatg ccaggaagac 360

cacttgcgaa tttacagggg acatcctacg aactccggtg tcagaggaca tgctgggtcg 420

ggttttcaat ggctccggca agcccattga caaggggcca gtggtcatgg cggaggactt 480

tctggatatc aatggccagc ccatcaaccc gcactcccgc atctaccccg aggagatgat 540

tcagacgggc atttctccta ttgacgtcat gaacagcatt gcccgcggcc agaagatccc 600

catcttctca gcagccgggc tcccccacaa tgagattgcc gctcagatct gccgccaggc 660

ggggctggtg aagaagtcca aggctgtgct ggattaccat gacgacaact tcgccatcgt 720

ctttgcagcc atgggggtga acatggagac agccagattc ttcaagtctg actttgagca 780

gaatggaacc atggggaacg tctgcctctt cctgaacttg gccaatgacc ccacgatcga 840

gcggatcatc accccgcgcc tggcgctgac cactgctgaa ttccttgcct accagtgtga 900

gaagcatgtg ctggtcatac tgacggacat gagttcctat gcagaggcct tgcgggaggt 960

ctctgctgct agagaggagg tgcctgggcg ccgagggttt cctggatata tgtacacaga 1020

cctggccacc atctacgagc gggcgggccg tgtggagggt cggggaggat ccatcacaca 1080

gatccccatc ctcaccatgc ccaacgacga tatcacccac cctatcccag acttgacggg 1140

cttcatcaca gagggacaga tctacgtgga cagacagctt cacaacagac agatctaccc 1200

ccccatcaac gtgctccctt ccctgtcgcg gctgatgaag tcagccattg gggaaggcat 1260

gacaagaaag gaccatggag atgtctccaa ccagctgtac gcctgctatg ccatcgggaa 1320

ggacgtgcag gccatgaagg cagtagttgg ggaggaggcg ctcacctctg aggacctgct 1380

ctacctggaa ttcctgcaga agtttgagaa gaacttcatc aatcagggcc cctacgagaa 1440

ccgctcgatg ttcgagtcgc tggaccttag ctggaagctg ctgcgcatct tccccaagga 1500

gatgctgaag cgcattccgc aggccgtgat cgacgagttc tattcccgcg aggggcggct 1560

gcaggacctc gcgcctgaca ctgcgctcta gccccgcgcg ccgtggcacc ccaacaccgg 1620

caggaaccta ccctcggctc ccgggtctcc ccgtccctcg ccacccctaa ccagcggctt 1680

tcgcgccgcc ctccgccctc cgtggctccg aggtggtggg ggcgccgca gtcatccctt 1740

tcctcgctcg attccttttc ccgcgctcca tgcctccccc tcagctcccg gtgctgcgga 1800

agaactgaag gttcatgcct actctgacgg gagcatctgt attttttatg ttaaaagccc 1860

acaaaataaa aataaaaatg aactgag 1887

<210> 55
<211> 1506
<212> DNA
<213> Homo sapiens

<400> 55
aggcggacaa agcccgattg ttcctgggcc ctttccccat cgcgcctggg cctgctcccc 60

agcccggggc aggggcgggg gccagtgtgg tgacacacgc tgtagctgtc tccccggctg 120

gctggctcgc tctctcctgg ggacacagag gtcggcaggc agcacacaga gggacctacg 180

ggcagctgtt ccttcccccg actcaagaat ccccggaggc ccggaggcct gcagcaggag 240

cggccatgaa gaagctgatg gtggtgctga gtctgattgc tgcagcctgg gcagaggagc 300

agaataagtt ggtgcatggc ggaccctgcg acaagacatc tcacccctac caagctgccc 360

tctacacctc gggccacttg ctctgtggtg gggtccttat ccatccactg tgggtcctca 420

cagctgccca ctgcaaaaaa ccgaatcttc aggtcttcct ggggaagcat aaccttcggc 480

aaagggagag ttcccaggag cagagttctg ttgtccgggc tgtgatccac cctgactatg 540

```
atgccgccag ccatgaccag gacatcatgc tgttgcgcct ggcacgccca gccaaactct     600
ctgaactcat ccagcccctt cccctggaga gggactgctc agccaacacc accagctgcc     660
acatcctggg ctggggcaag acagcagatg gtgatttccc tgacaccatc cagtgtgcat     720
acatccacct ggtgtcccgt gaggagtgtg agcatgccta ccctggccag atcacccaga     780
acatgttgtg tgctggggat gagaagtacg ggaaggattc ctgccagggt gattctgggg     840
gtccgctggt atgtggagac cacctccgag gccttgtgtc atggggtaac atcccctgtg     900
gatcaaagga gaagccagga gtctacacca acgtctgcag atacacgaac tggatccaaa     960
aaaccattca ggccaagtga ccctgacatg tgacatctac ctcccgacct accaccccac    1020
tggctggttc cagaacgtct ctcacctaga ccttgcctcc cctcctctcc tgcccagctc    1080
tgaccctgat gcttaataaa cgcagcgacg tgagggtcct gattctccct ggttttaccc    1140
cagctccatc cttgcatcac tggggaggac gtgatgagtg aggacttggg tcctcggtct    1200
taccccccacc actaagagaa tacaggaaaa tcccttctag gcatctcctc tccccaaccc   1260
ttccacacgt ttgatttctt cctgcagagg cccagccacg tgtctggaat cccagctccg    1320
ctgcttactg tcggtgtccc cttgggatgt acctttcttc actgcagatt tctcacctgt    1380
aagatgaaga taaggatgat acagtctcca tcaggcagtg gctgttggaa agatttaaga    1440
tttcacacct atgacataca tgggatagca cctgggccgc catgcactca ataaagaatg    1500
tatttt                                                               1506

<210>  56
<211>  2907
<212>  DNA
<213>  Homo sapiens

<400>  56
gccatctggg cccaggcccc atgccccgag gaggggtggt ctgaagccca ccagagcccc      60
ctgccagact gtctgcctcc cttctgactg tggccgcttg gcatggccag caacagcagc     120
tcctgcccga cacctggggg cgggcacctc aatgggtacc cggtgcctcc ctacgccttc     180
ttcttccccc ctatgctggg tggactctcc ccgccaggcg ctctgaccac tctccagcac     240
cagcttccag ttagtggata tagcacacca tccccagcca ccattgagac ccagagcagc     300
agttctgaag agatagtgcc cagccctccc tcgccacccc ctctaccccg catctacaag     360
ccttgctttg tctgtcagga caagtcctca ggctaccact atggggtcag cgcctgtgag     420
ggctgcaagg gcttcttccg ccgcagcatc cagaagaaca tggtgtacac gtgtcaccgg     480
gacaagaact gcatcatcaa caaggtgacc cggaaccgct gccagtactg ccgactgcag     540
aagtgctttg aagtgggcat gtccaaggag tctgtgagaa acgaccgaaa caagaagaag     600
```

aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg ccccctcacc 1020
gacctggtct ttgccttcgc caaccagctg ctgcccctgg agatggatga tgcggagacg 1080
gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca agggggctga gcgggtgatc acgctgaaga tggagatccc gggctccatg 1320
ccgcctctca tccaggaaat gttggagaac tcagagggcc tggacactct gagcggacag 1380
ccgggggggtg gggggcggga cgggggtggc ctggcccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680
gcctgctccc acagcctggg ctgacgtcag aggccgaggc caggaactga gtgaggcccc 1740
tggtcctggg tctcaggatg ggtcctgggg gcctcgtgtt catcaagaca cccctctgcc 1800
cagctcacca catcttcatc accagcaaac gccaggactt ggctccccca tcctcagaac 1860
tcacaagcca ttgctcccca gctggggaac ctcaacctcc cccctgcctc ggttggtgac 1920
agaggggggtg ggacaggggc gggggggttcc ccctgtacat accctgccat accaacccca 1980
ggtattaatt ctcgctggtt ttgtttttat tttaattttt ttgttttgat ttttttaata 2040
agaattttca ttttaagcac atttatactg aaggaatttg tgctgtgtat tgggggggagc 2100
tggatccaga gctggagggg gtgggtccgg gggagggagt ggctcggaag gggcccccac 2160
tctcctttca tgtccctgtg ccccccagtt ctcctcctca gccttttcct cctcagtttt 2220
ctctttaaaa ctgtgaagta ctaactttcc aaggcctgcc ttcccctccc tcccactgga 2280
gaagccgcca gcccctttct ccctctgcct gaccactggg tgtggacggt gtggggcagc 2340
cctgaaagga caggctcctg gccttggcac ttgcctgcac ccaccatgag gcatggagca 2400

gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg ccccctcctt accccgcagg acgggcctac agggggggtct cccctcaccc 2760

ctgcaccccc agctggggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 57
<211> 2907
<212> DNA
<213> Homo sapiens

<400> 57
gccatctggg cccaggcccc atgccccgag gaggggtggt ctgaagccca ccagagcccc 60

ctgccagact gtctgcctcc cttctgactg tggccgcttg gcatggccag caacagcagc 120

tcctgcccga cacctggggg cgggcacctc aatgggtacc cggtgcctcc ctacgccttc 180

ttcttccccc ctatgctggg tggactctcc ccgccaggcg ctctgaccac tctccagcac 240

cagcttccag ttagtggata tagcacacca tccccagcca ccattgagac ccagagcagc 300

agttctgaag agatagtgcc cagccctccc tcgccacccc ctctaccccg catctacaag 360

ccttgctttg tctgtcagga caagtcctca ggctaccact atggggtcag cgcctgtgag 420

ggctgcaagg gcttcttccg ccgcagcatc cagaagaaca tggtgtacac gtgtcaccgg 480

gacaagaact gcatcatcaa caaggtgacc cggaaccgct gccagtactg ccgactgcag 540

aagtgctttg aagtgggcat gtccaaggag tctgtgagaa acgaccgaaa caagaagaag 600

aaggaggtgc ccaagcccga gtgctctgag agctacacgc tgacgccgga ggtgggggag 660

ctcattgaga aggtgcgcaa agcgcaccag gaaaccttcc ctgccctctg ccagctgggc 720

aaatacacta cgaacaacag ctcagaacaa cgtgtctctc tggacattga cctctgggac 780

aagttcagtg aactctccac caagtgcatc attaagactg tggagttcgc caagcagctg 840

cccggcttca ccaccctcac catcgccgac cagatcaccc tcctcaaggc tgcctgcctg 900

gacatcctga tcctgcggat ctgcacgcgg tacacgcccg agcaggacac catgaccttc 960

tcggacgggc tgaccctgaa ccggacccag atgcacaacg ctggcttcgg cccccctcacc 1020

gacctggtct ttgccttcgc caaccagctg ctgcccctgg agatggatga tgcggagacg 1080

gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca agggggctga gcgggtgatc acgctgaaga tggagatccc gggctccatg 1320
ccgcctctca tccaggaaat gttggagaac tcagagggcc tggacactct gagcggacag 1380
ccgggggggtg gggggcggga cgggggtggc ctggcccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680
gcctgctccc acagcctggg ctgacgtcag aggccgaggc caggaactga gtgaggcccc 1740
tggtcctggg tctcaggatg ggtcctgggg gcctcgtgtt catcaagaca cccctctgcc 1800
cagctcacca catcttcatc accagcaaac gccaggactt ggctccccca tcctcagaac 1860
tcacaagcca ttgctcccca gctggggaac ctcaacctcc cccctgcctc ggttggtgac 1920
agagggggtg ggacaggggc gggggggttcc ccctgtacat accctgccat accaacccca 1980
ggtattaatt ctcgctggtt ttgtttttat tttaattttt ttgttttgat ttttttaata 2040
agaattttca ttttaagcac atttatactg aaggaatttg tgctgtgtat tgggggggagc 2100
tggatccaga gctggagggg gtgggtccgg gggagggagt ggctcggaag gggcccccac 2160
tctcctttca tgtccctgtg ccccccagtt ctcctcctca gccttttcct cctcagtttt 2220
ctctttaaaa ctgtgaagta ctaactttcc aaggcctgcc ttcccctccc tcccactgga 2280
gaagccgcca gcccctttct ccctctgcct gaccactggg tgtggacggt gtggggcagc 2340
cctgaaagga caggctcctg gccttggcac ttgcctgcac ccaccatgag gcatggagca 2400
gggcagagca agggccccgg gacagagttt tcccagacct ggctcctcgg cagagctgcc 2460
tcccgtcagg gcccacatca tctaggctcc ccagccccca ctgtgaaggg gctggccagg 2520
ggcccgagct gcccccaccc ccggcctcag ccaccagcac ccccataggg cccccagaca 2580
ccacacacat gcgcgtgcgc acacacacaa acacacacac actggacagt agatgggccg 2640
acacacactt ggcccgagtt cctccatttc cctggcctgc cccccacccc caacctgtcc 2700
caccccccgtg ccccctcctt accccgcagg acgggcctac agggggggtct cccctcaccc 2760
ctgcaccccc agctggggga gctggctctg ccccgacctc cttcaccagg ggttggggcc 2820
ccttcccctg gagcccgtgg gtgcacctgt tactgttggg ctttccactg agatctactg 2880
gataaagaat aaagttctat ttattct 2907

<210> 58
<211> 5026
<212> DNA
<213> Homo sapiens

<400> 58
agaggaggaa attgttcctc gtctgataag acaacagtgg agaaaggacg catgctgttt 60

cttagggaca cggctgactt ccagatatga ccatgtattt gtggcttaaa ctcttggcat 120

ttggctttgc ctttctggac acagaagtat ttgtgacagg gcaaagccca acaccttccc 180

ccactggatt gactacagca aagatgccca gtgttccact ttcaagtgac cccttaccta 240

ctcacaccac tgcattctca cccgcaagca cctttgaaag agaaaatgac ttctcagaga 300

ccacaacttc tcttagtcca gacaatactt ccacccaagt atccccggac tctttggata 360

atgctagtgc ttttaatacc acaggtgttt catcagtaca gacgcctcac cttcccacgc 420

acgcagactc gcagacgccc tctgctggaa ctgacacgca gacattcagc ggctccgccg 480

ccaatgcaaa actcaaccct accccaggca gcaatgctat ctcagatgtc ccaggagaga 540

ggagtacagc cagcaccttt cctacagacc cagtttcccc attgacaacc accctcagcc 600

ttgcacacca cagctctgct gccttacctg cacgcacctc caacaccacc atcacagcga 660

acacctcaga tgcctacctt aatgcctctg aaacaaccac tctgagccct tctggaagcg 720

ctgtcatttc aaccacaaca atagctacta ctccatctaa gccaacatgt gatgaaaaat 780

atgcaaacat cactgtggat tacttatata acaaggaaac taaattattt acagcaaagc 840

taaatgttaa tgagaatgtg gaatgtggaa acaatacttg cacaaacaat gaggtgcata 900

accttacaga atgtaaaaat gcgtctgttt ccatatctca taattcatgt actgctcctg 960

ataagacatt aatattagat gtgccaccag ggttgaaaa gtttcagtta catgattgta 1020

cacaagttga aaaagcagat actactattt gtttaaaatg gaaaaatatt gaaaccttta 1080

cttgtgatac acagaatatt acctacagat ttcagtgtgg taatatgata tttgataata 1140

aagaaattaa attagaaaac cttgaacccg aacatgagta taagtgtgac tcagaaatac 1200

tctataataa ccacaagttt actaacgcaa gtaaaattat taaaacagat tttgggagtc 1260

caggagagcc tcagattatt ttttgtagaa gtgaagctgc acatcaagga gtaattacct 1320

ggaatccccc tcaaagatca tttcataatt ttaccctctg ttatataaaa gagacagaaa 1380

aagattgcct caatctggat aaaaacctga tcaaatatga tttgcaaaat ttaaaacctt 1440

atacgaaata tgttttatca ttacatgcct acatcattgc aaaagtgcaa cgtaatggaa 1500

gtgctgcaat gtgtcatttc acaactaaaa gtgctcctcc aagccaggtc tggaacatga 1560

ctgtctccat gacatcagat aatagtatgc atgtcaagtg taggcctccc agggaccgta 1620

atggcccccca tgaacgttac catttggaag ttgaagctgg aaatactctg gttagaaatg 1680

agtcgcataa gaattgcgat ttccgtgtaa aagatcttca atattcaaca gactacactt 1740

ttaaggccta ttttcacaat ggagactatc ctggagaacc ctttatttta catcattcaa 1800

catcttataa ttctaaggca ctgatagcat ttctggcatt tctgattatt gtgacatcaa 1860

tagccctgct tgttgttctc tacaaaatct atgatctaca taagaaaaga tcctgcaatt 1920

tagatgaaca gcaggagctt gttgaaaggg atgatgaaaa acaactgatg aatgtggagc 1980

caatccatgc agatattttg ttggaaactt ataagaggaa gattgctgat gaaggaagac 2040

tttttctggc tgaatttcag agcatcccgc gggtgttcag caagtttcct ataaaggaag 2100

ctcgaaagcc ctttaaccag aataaaaacc gttatgttga cattcttcct tatgattata 2160

accgtgttga actctctgag ataaacggag atgcagggtc aaactacata aatgccagct 2220

atattgatgg tttcaaagaa cccaggaaat acattgctgc acaaggtccc agggatgaaa 2280

ctgttgatga tttctggagg atgatttggg aacagaaagc cacagttatt gtcatggtca 2340

ctcgatgtga agaaggaaac aggaacaagt gtgcagaata ctggccgtca atggaagagg 2400

gcactcgggc ttttggagat gttgttgtaa agatcaacca gcacaaaaga tgtccagatt 2460

acatcattca gaaattgaac attgtaaata aaaaagaaaa agcaactgga agagaggtga 2520

ctcacattca gttcaccagc tggccagacc acggggtgcc tgaggatcct cacttgctcc 2580

tcaaactgag aaggagagtg aatgccttca gcaatttctt cagtggtccc attgtggtgc 2640

actgcagtgc tggtgttggg cgcacaggaa cctatatcgg aattgatgcc atgctagaag 2700

gcctggaagc cgagaacaaa gtggatgttt atggttatgt tgtcaagcta aggcgacaga 2760

gatgcctgat ggttcaagta gaggcccagt acatcttgat ccatcaggct ttggtggaat 2820

acaatcagtt tggagaaaca gaagtgaatt tgtctgaatt acatccatat ctacataaca 2880

tgaagaaaag ggatccaccc agtgagccgt ctccactaga ggctgaattc cagagacttc 2940

cttcatatag gagctggagg acacagcaca ttggaaatca agaagaaaat aaaagtaaaa 3000

acaggaattc taatgtcatc ccatatgact ataacagagt gccacttaaa catgagctgg 3060

aaatgagtaa agagagtgag catgattcag atgaatcctc tgatgatgac agtgattcag 3120

aggaaccaag caaatacatc aatgcatctt ttataatgag ctactggaaa cctgaagtga 3180

tgattgctgc tcagggacca ctgaaggaga ccattggtga cttttggcag atgatcttcc 3240

aaagaaaagt caaagttatt gttatgctga cagaactgaa acatggagac caggaaatct 3300

gtgctcagta ctggggagaa ggaaagcaaa catatggaga tattgaagtt gacctgaaag 3360

acacagacaa atcttcaact tataccttc gtgtctttga actgagacat tccaagagga 3420

```
aagactctcg aactgtgtac cagtaccaat atacaaactg gagtgtggag cagcttcctg   3480
cagaacccaa ggaattaatc tctatgattc aggtcgtcaa acaaaaactt ccccagaaga   3540
attcctctga agggaacaag catcacaaga gtacacctct actcattcac tgcagggatg   3600
gatctcagca aacgggaata ttttgtgctt tgttaaatct cttagaaagt gcggaaacag   3660
aagaggtagt ggatattttt caagtggtaa aagctctacg caaagctagg ccaggcatgg   3720
tttccacatt cgagcaatat caattcctat atgacgtcat tgccagcacc taccctgctc   3780
agaatggaca agtaaagaaa aacaaccatc aagaagataa aattgaattt gataatgaag   3840
tggacaaagt aaagcaggat gctaattgtg ttaatccact tggtgcccca gaaaagctcc   3900
ctgaagcaaa ggaacaggct gaaggttctg aacccacgag tggcactgag ggccagaac    3960
attctgtcaa tggtcctgca agtccagctt taaatcaagg ttcataggaa aagacataaa   4020
tgaggaaact ccaaacctcc tgttagctgt tatttctatt tttgtagaag taggaagtga   4080
aaataggtat acagtggatt aattaaatgc agcgaaccaa tatttgtaga agggttatat   4140
tttactactg tggaaaaata tttaagatag ttttgccaga acagtttgta cagacgtatg   4200
cttattttaa aattttatct cttattcagt aaaaaacaac ttctttgtaa tcgttatgtg   4260
tgtatatgta tgtgtgtatg ggtgtgtgtt tgtgtgagag acagagaaag agagagaatt   4320
ctttcaagtg aatctaaaag cttttgcttt tcctttgttt ttatgaagaa aaaatacatt   4380
ttatattaga agtgttaact tagcttgaag gatctgtttt taaaaatcat aaactgtgtg   4440
cagactcaat aaaatcatgt acatttctga aatgacctca agatgtcctc cttgttctac   4500
tcatatatat ctatcttata tacttactat tttacttcta gagatagtac ataaaggtgg   4560
tatgtgtgtg tatgctacta caaaaaagtt gttaactaaa ttaacattgg gaaatcttat   4620
attccatata ttagcattta gtccaatgtc tttttaagct tatttaatta aaaaatttcc   4680
agtgagctta tcatgctgtc tttacatggg gttttcaatt ttgcatgctc gattattccc   4740
tgtacaatat ttaaaattta ttgcttgata cttttgacaa caaattaggt tttgtacaat   4800
tgaacttaaa taaatgtcat taaaataaat aaatgcaata tgtattaata ttcattgtat   4860
aaaaatagaa gaatacaaac atatttgtta aatatttaca tatgaaattt aatatagcta   4920
tttttatgga atttttcatt gatatgaaaa atatgatatt gcatatgcat agttcccatg   4980
ttaaatccca ttcataactt tcattaaagc atttactttg aatttc                  5026
```

<210> 59
<211> 625
<212> DNA
<213> Homo sapiens

<400> 59

aggcgggccg ctcccacttc ggcacgaggg gcacgaggta aatcttttct gcttactgaa 60

aaggaagagt ctgatgatta gttactgatc ctctttgcat ttgtaaagct ttggagatat 120

tgaatcatgt taccatttct gttttttttcc accctgtttt cttccatatt tactgaagct 180

cagaagcagt attgggtctg caactcatcc gatgcaagta tttcatacac ctactgtgat 240

aaaatgcaat acccaatttc aattaatgtt aacccctgta tagaattgaa aggatccaaa 300

ggattattgc acattttcta cattccaagg agagatttaa agcaattata tttcaatctc 360

tatataactg tcaacaccat gaatcttcca aagcgcaaag aagttatttg ccgaggatct 420

gatgacgatt actctttttg cagagctctg aagggagaga ctgtgaatac aacaatatca 480

ttctccttca agggaataaa attttctaag ggaaaataca aatgtgttgt tgaagctatt 540

tctgggagcc cagaagaaat gctcttttgc ttggagtttg tcatcctaca ccaacctaat 600

tcaaattaga ataaattgag tattt 625

<210> 60
<211> 2088
<212> DNA
<213> Homo sapiens

<400> 60
gaattcggca cgagcgcgcg gcgaatctca acgctgcgcc gtctgcgggc gcttccgggc 60

caccagtttc tctgctttcc accctggcgc cccccagccc tggctcccca gctgcgctgc 120

cccgggcgtc cacgccctgc gggcttagcg ggttcagtgg gctcaatctg cgcagcgcca 180

cctccatgtt gaccaagcct ctacaggggc ctcccgcgcc ccccgggacc cccacgccgc 240

cgccaggagg caaggatcgg gaagcgttcg aggccgagta tcgactcggc cccctcctgg 300

gtaagggggg ctttggcacc gtcttcgcag gacaccgcct cacagatcga ctccaggtgg 360

ccatcaaagt gattccccgg aatcgtgtgc tgggctggtc cccccttgtca gactcagtca 420

catgcccact cgaagtcgca ctgctatgga aagtgggtgc aggtggtggg caccctggcg 480

tgatccgcct gcttgactgg tttgagacac aggaaggctt catgctggtc ctcgagcggc 540

ctttgcccgc ccaggatctc tttgactata tcacagagaa gggcccactg ggtgaaggcc 600

caagccgctg cttctttggc caagtagtgg cagccatcca gcactgccat tcccgtggag 660

ttgtccatcg tgacatcaag gatgagaaca tcctgataga cctacgccgt ggctgtgcca 720

aactcattga ttttggttct ggtgccctgc ttcatgatga accctacact gactttgatg 780

ggacaagggt gtacagcccc ccagagtgga tctctcgaca ccagtaccat gcactcccgg 840

ccactgtctg gtcactgggc atcctcctct atgacatggt gtgtggggac attccctttg 900

agagggacca ggagattctg gaagctgagc tccacttccc agcccatgtc tccccagact 960

gctgtgccct aatccgccgg tgcctggccc ccaaaccttc ttcccgaccc tcactggaag 1020

agatcctgct ggacccctgg atgcaaacac cagccgagga tgttacccct caacccctcc 1080

aaaggaggcc ctgcccccttt ggcctggtcc ttgctaccct aagcctggcc tggcctggcc 1140

tggcccccaa tggtcagaag agccatccca tggccatgtc acagggatag atggacattt 1200

gttgacttgg ttttacaggt cattaccagt cattaaagtc cagtattact aaggtaaggg 1260

attgaggatc aggggttaga agacataaac caagtttgcc cagttccctt cccaatccta 1320

caaaggagcc ttcctcccag aacctgtggt ccctgatttt ggagggggaa cttcttgctt 1380

ctcattttgc taaggaagtt tattttggtg aagttgttcc cattttgagc cccgggactc 1440

ttattttgat gatgtgtcac cccacattgg cacctcctac taccaccaca caaacttagt 1500

tcatatgctt ttacttgggc aagggtgctt tccttccaat accccagtag cttttatttt 1560

agtaaaggga ccctttcccc tagcctaggg tcccatattg ggtcaagctg cttacctgcc 1620

tcagcccagg attttttatt ttgggggagg taatgccctg ttgttacccc aaggcttctt 1680

tttttttttt ttttttttg ggtgagggga ccctactttg ttatcccaag tgctcttatt 1740

ctggtgagaa gaaccttaat tccataattt gggaaggaat ggaagatgga caccaccgga 1800

caccaccaga caataggatg ggatggatgg tttttttgggg gatgggctag gggaaataag 1860

gcttgctgtt tgttttcctg gggcgctccc tccaattttg cagatttttg caacctcctc 1920

ctgagccggg attgtccaat tactaaaatg taaataatca cgtattgtgg ggaggggagt 1980

tccaagtgtg ccctcctttt ttttcctgcc tggattattt aaaaagccat gtgtggaaac 2040

ccactattta ataaaagtaa tagaatcaga aaaaaaaaaa aaaaaaaa 2088

<210> 61
<211> 2270
<212> DNA
<213> Homo sapiens

<400> 61
ctcctccagc ctctcacact ctcctcagct ctctcatctc ctggaaccat ggccagcaca 60

tccaccacca tcaggagcca cagcagcagc cgccggggtt tcagtgccaa ctcagccagg 120

ctccctgggg tcagccgctc tggcttcagc agcgtctccg tgtcccgctc caggggcagt 180

ggtggcctgg gtggtgcatg tggaggagct ggctttggca gccgcagtct gtatggcctg 240

gggggctcca agaggatctc cattggaggg ggcagctgtg ccatcagtgg cggctatggc 300

agcagagccg gaggcagcta tggctttggt ggcgccggga gtggatttgg tttcggtggt 360

ggagccggca ttggctttgg tctgggtggt ggagccggcc ttgctggtgg ctttgggggc 420

cctggcttcc ctgtgtgccc ccctggaggc atccaagagg tcaccgtcaa ccagagtctc 480

ctgactcccc tcaacctgca aatcgatccc accatccagc gggtgcgggc tgaggagcgt 540
gaacagatca agaccctcaa caacaagttt gcctccttca tcgacaaggt gcggttcctg 600
gagcagcaga acaaggttct ggaaacaaag tggaccctgc tgcaggagca gggcaccaag 660
actgtgaggc agaacctgga gccgttgttc gagcagtaca tcaacaacct caggaggcag 720
ctggacagca ttgtcgggga acggggccgc ctggactcag agctcagagg catgcaggac 780
ctggtggagg acttcaagaa caaatatgag gatgaaatca acaagcgcac agcagcagag 840
aatgaatttg tgactctgaa gaaggatgtg gatgctgcct acatgaacaa ggttgaactg 900
caagccaagg cagacactct cacagacgag atcaacttcc tgagagcctt gtatgatgca 960
gagctgtccc agatgcagac ccacatctca gacacatctg tggtgctgtc catggacaac 1020
aaccgcaacc tggacctgga cagcatcatc gctgaggtca aggcccaata tgaggagatt 1080
gctcagagaa gccgggctga ggctgagtcc tggtaccaga ccaagtacga ggagctgcag 1140
gtcacagcag gcagacatgg ggacgacctg cgcaacacca agcaggagat tgctgagatc 1200
aaccgcatga tccagaggct gagatctgag atcgaccacg tcaagaagca gtgcgccaac 1260
ctgcaggccg ccattgctga tgctgagcag cgtggggaga tggccctcaa ggatgccaag 1320
aacaagctgg aagggctgga ggatgccctg cagaaggcca agcaggacct ggcccggctg 1380
ctgaaggagt accaggagct gatgaatgtc aagctggccc tggacgtgga gatcgccacc 1440
taccgcaagc tgctggaggg tgaggagtgc aggctgaatg gcgaaggcgt tggacaagtc 1500
aacatctctg tggtgcagtc caccgtctcc agtggctatg gcggtgccag tggtgtcggc 1560
agtggcttag gcctgggtgg aggaagcagc tactcctatg gcagtggtct tggcgttgga 1620
ggtggcttca gttccagcag tggcagagcc attgggggtg gcctcagctc tgttggaggc 1680
ggcagttcca ccatcaagta caccaccacc tcctcctcca gcaggaagag ctataagcac 1740
taaagtgcgt ctgctagctc tcggtcccac agtcctcagg cccctctctg gctgcagagc 1800
cctctcctca ggttgcctgt cctctcctgg cctccagtct cccctgctgt cccaggtaga 1860
gctggggatg aatgcttagt gccctcactt cttctctctc tctctatacc atctgagcac 1920
ccattgctca ccatcagatc aacctctgat tttacatcat gatgtaatca ccactggagc 1980
ttcactgtta ctaaattatt aatttcttgc ctccagtgtt ctatctctga ggctgagcat 2040
tataagaaaa tgacctctgc tccttttcat tgcagaaaat tgccaggggc ttatttcaga 2100
acaacttcca cttactttcc actggctctc aaactctcta acttataagt gttgtgaacc 2160
cccacccagg cagtatccat gaaagcacaa gtgactagtc ctatgatgta caaagcctgt 2220
atctctgtga tgatttctgt gctcttcact gtttgcaatt gctaaataaa 2270

<210> 62
<211> 2048
<212> DNA
<213> Homo sapiens

<400> 62
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tattttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 63
<211> 2048
<212> DNA
<213> Homo sapiens

<400> 63
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tattttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 64
<211> 2816
<212> DNA
<213> Homo sapiens

<400> 64
tcgttgatat caaagacagt tgaaggaaat gaattttgaa acttcacggt gtgccaccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420

agtccctata acacagacca cgcgcagaac agcgtcacgg cgccctcgcc ctacgcacag 480

cccagctcca ccttcgatgc tctctctcca tcacccgcca tcccctccaa caccgactac 540

ccaggcccgc acagtttcga cgtgtccttc cagcagtcga gcaccgccaa gtcggccacc 600

tggacgtatt ccactgaact gaagaaactc tactgccaaa ttgcaaagac atgccccatc 660

cagatcaagg tgatgacccc acctcctcag ggagctgtta tccgcgccat gcctgtctac 720

aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt tttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460
ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 65
<211> 2816
<212> DNA
<213> Homo sapiens

<400> 65
tcgttgatat caaagacagt tgaaggaaat gaattttgaa acttcacggt gtgccacccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420

agtccctata acacagacca cgcgcagaac agcgtcacgg cgccctcgcc ctacgcacag 480

cccagctcca ccttcgatgc tctctctcca tcacccgcca tcccctccaa caccgactac 540

ccaggcccgc acagtttcga cgtgtccttc cagcagtcga gcaccgccaa gtcggccacc 600

tggacgtatt ccactgaact gaagaaactc tactgccaaa ttgcaaagac atgccccatc 660

cagatcaagg tgatgacccc acctcctcag ggagctgtta tccgcgccat gcctgtctac 720

aaaaaagctg agcacgtcac ggaggtggtg aagcggtgcc ccaaccatga gctgagccgt 780

gaattcaacg agggacagat tgcccctcct agtcatttga ttcgagtaga ggggaacagc 840

catgcccagt atgtagaaga tcccatcaca ggaagacaga gtgtgctggt accttatgag 900

ccacccccagg ttggcactga attcacgaca gtcttgtaca atttcatgtg taacagcagt 960

tgtgttggag ggatgaaccg ccgtccaatt ttaatcattg ttactctgga aaccagagat 1020

gggcaagtcc tgggccgacg ctgctttgag gcccggatct gtgcttgccc aggaagagac 1080

aggaaggcgg atgaagatag catcagaaag cagcaagttt cggacagtac aaagaacggt 1140

gatggtacga agcgcccgtt tcgtcagaac acacatggta tccagatgac atccatcaag 1200

aaacgaagat ccccagatga tgaactgtta tacttaccag tgaggggccg tgagacttat 1260

gaaatgctgt tgaagatcaa agagtccctg gaactcatgc agtaccttcc tcagcacaca 1320

attgaaacgt acaggcaaca gcaacagcag cagcaccagc acttacttca gaaacatctc 1380

ctttcagcct gcttcaggaa tgagcttgtg gagccccgga gagaaactcc aaaacaatct 1440

gacgtcttct ttagacattc caagccccca aaccgatcag tgtacccata gagccctatc 1500

tctatatttt aagtgtgtgt gttgtatttc catgtgtata tgtgagtgtg tgtgtgtgta 1560

tgtgtgtgcg tgtgtatcta gccctcataa acaggacttg aagacacttt ggctcagaga 1620

cccaactgct caaaggcaca aagccactag tgagagaatc ttttgaaggg actcaaacct 1680

ttacaagaaa ggatgttttc tgcagatttt gtatccttag accggccatt ggtgggtgag 1740

gaaccactgt gtttgtctgt gagctttctg ttgtttcctg ggagggaggg gtcaggtggg 1800

gaaaggggca ttaagatgtt tattggaacc cttttctgtc ttcttctgtt gtttttctaa 1860

aattcacagg gaagcttttg agcaggtctc aaacttaaga tgtcttttta agaaaaggag 1920

aaaaaagttg ttattgtctg tgcataagta agttgtaggt gactgagaga ctcagtcaga 1980

cccttttaat gctggtcatg taataatatt gcaagtagta agaaacgaag gtgtcaagtg 2040

tactgctggg cagcgaggtg atcattacca aaagtaatca actttgtggg tggagagttc 2100

tttgtgagaa cttgcattat ttgtgtcctc ccctcatgtg taggtagaac atttcttaat 2160

gctgtgtacc tgcctctgcc actgtatgtt ggcatctgtt atgctaaagt ttttcttgta 2220

catgaaaccc tggaagacct actacaaaaa aactgttgtt tggcccccat agcaggtgaa 2280

ctcattttgt gcttttaata gaaagacaaa tccaccccag taatattgcc cttacgtagt 2340

tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 66
<211> 5838
<212> DNA
<213> Homo sapiens

<400> 66
ccgggcaggt ggctcatgct cgggagcgtg gttgagcggc tggcgcggtt gtcctggagc 60

aggggcgcag gaattctgat gtgaaactaa cagtctgtga gccctggaac ctccgctcag 120

agaagatgaa ggatatcgac ataggaaaag agtatatcat ccccagtcct gggtatagaa 180
gtgtgaggga gagaaccagc acttctggga cgcacagaga ccgtgaagat tccaagttca 240
ggagaactcg accgttggaa tgccaagatg ccttggaaac agcagcccga gccgagggcc 300
tctctcttga tgcctccatg cattctcagc tcagaatcct ggatgaggag catcccaagg 360
gaaagtacca tcatggcttg agtgctctga agcccatccg gactacttcc aaacaccagc 420
acccagtgga caatgctggg ctttttcct gtatgacttt ttcgtggctt tcttctctgg 480
cccgtgtggc ccacaagaag gggagctct caatggaaga cgtgtggtct ctgtccaagc 540
acgagtcttc tgacgtgaac tgcagaagac tagagagact gtggcaagaa gagctgaatg 600
aagttgggcc agacgctgct tccctgcgaa gggttgtgtg gatcttctgc cgcaccaggc 660
tcatcctgtc catcgtgtgc ctgatgatca cgcagctggc tggcttcagt ggaccagcct 720
tcatggtgaa acacctcttg gagtataccc aggcaacaga gtctaacctg cagtacagct 780
tgttgttagt gctgggcctc ctcctgacgg aaatcgtgcg gtcttggtcg cttgcactga 840
cttgggcatt gaattaccga accggtgtcc gcttgcgggg ggccatccta accatggcat 900
ttaagaagat ccttaagtta aagaacatta aagagaaatc cctgggtgag ctcatcaaca 960
tttgctccaa cgatgggcag agaatgtttg aggcagcagc cgttggcagc ctgctggctg 1020
gaggacccgt tgttgccatc ttaggcatga tttataatgt aattattctg ggaccaacag 1080
gcttcctggg atcagctgtt tttatcctct tttacccagc aatgatgttt gcatcacggc 1140
tcacagcata tttcaggaga aaatgcgtgg ccgccacgga tgaacgtgtc cagaagatga 1200
atgaagttct tacttacatt aaatttatca aaatgtatgc ctgggtcaaa gcattttctc 1260
agagtgttca aaaaatccgc gaggaggagc gtcggatatt ggaaaaagcc gggtacttcc 1320
agggtatcac tgtgggtgtg gctcccattg tggtggtgat tgccagcgtg gtgaccttct 1380
ctgttcatat gaccctgggc ttcgatctga cagcagcaca ggctttcaca gtggtgacag 1440
tcttcaattc catgactttt gctttgaaag taacaccgtt ttcagtaaag tccctctcag 1500
aagcctcagt ggctgttgac agatttaaga gtttgtttct aatggaagag gttcacatga 1560
taaagaacaa accagccagt cctcacatca agatagagat gaaaaatgcc accttggcat 1620
gggactcctc ccactccagt atccagaact cgcccaagct gacccccaaa atgaaaaaag 1680
acaagagggc ttccaggggc aagaaagaga aggtgaggca gctgcagcgc actgagcatc 1740
aggcggtgct ggcagagcag aaaggccacc tcctcctgga cagtgacgag cggcccagtc 1800
ccgaagagga agaaggcaag cacatccacc tgggccacct gcgcttacag aggacactgc 1860
acagcatcga tctggagatc caagagggta aactggttgg aatctgcggc agtgtgggaa 1920
gtggaaaaac ctctctcatt tcagccattt taggccagat gacgcttcta gagggcagca 1980

ttgcaatcag tggaaccttc gcttatgtgg cccagcaggc ctggatcctc aatgctactc 2040
tgagagacaa catcctgttt gggaaggaat atgatgaaga aagatacaac tctgtgctga 2100
acagctgctg cctgaggcct gacctggcca ttcttcccag cagcgacctg acggagattg 2160
gagagcgagg agccaacctg agcggtgggc agcgccagag gatcagcctt gcccgggcct 2220
tgtatagtga caggagcatc tacatcctgg acgacccct cagtgcctta gatgcccatg 2280
tgggcaacca catcttcaat agtgctatcc ggaaacatct caagtccaag acagttctgt 2340
ttgttaccca ccagttacag tacctggttg actgtgatga agtgatcttc atgaaagagg 2400
gctgtattac ggaaagaggc acccatgagg aactgatgaa tttaaatggt gactatgcta 2460
ccatttttaa taacctgttg ctgggagaga caccgccagt tgagatcaat tcaaaaaagg 2520
aaaccagtgg ttcacagaag aagtcacaag acaagggtcc taaaacagga tcagtaaaga 2580
aggaaaaagc agtaaagcca gaggaagggc agcttgtgca gctggaagag aaagggcagg 2640
gttcagtgcc ctggtcagta tatggtgtct acatccaggc tgctgggggc cccttggcat 2700
tcctggttat tatggccctt ttcatgctga atgtaggcag caccgccttc agcacctggt 2760
ggttgagtta ctggatcaag caaggaagcg ggaacaccac tgtgactcga gggaacgaga 2820
cctcggtgag tgacagcatg aaggacaatc ctcatatgca gtactatgcc agcatctacg 2880
ccctctccat ggcagtcatg ctgatcctga aagccattcg aggagttgtc tttgtcaagg 2940
gcacgctgcg agcttcctcc cggctgcatg acgagctttt ccgaaggatc cttcgaagcc 3000
ctatgaagtt ttttgacacg acccccacag ggaggattct caacaggttt tccaaagaca 3060
tggatgaagt tgacgtgcgg ctgccgttcc aggccgagat gttcatccag aacgttatcc 3120
tggtgttctt ctgtgtggga atgatcgcag gagtcttccc gtggttcctt gtggcagtgg 3180
ggcccttgt catcctcttt tcagtcctgc acattgtctc cagggtcctg attcgggagc 3240
tgaagcgtct ggacaatatc acgcagtcac ctttcctctc ccacatcacg tccagcatac 3300
agggccttgc caccatccac gcctacaata aagggcagga gtttctgcac agataccagg 3360
agctgctgga tgacaaccaa gctccttttt ttttgtttac gtgtgcgatg cggtggctgg 3420
ctgtgcggct ggacctcatc agcatcgccc tcatcaccac cacggggctg atgatcgttc 3480
ttatgcacgg gcagattccc ccagcctatg cgggtctcgc catctcttat gctgtccagt 3540
taacggggct gttccagttt acggtcagac tggcatctga gacagaagct cgattcacct 3600
cggtggagag gatcaatcac tacattaaga ctctgtcctt ggaagcacct gccagaatta 3660
agaacaaggc tccctcccct gactggcccc aggagggaga ggtgaccttt gagaacgcag 3720
agatgaggta ccgagaaaac ctccctcttg tcctaaagaa agtatccttc acgatcaaac 3780

ctaaagagaa gattggcatt gtggggcgga caggatcagg gaagtcctcg ctggggatgg 3840
ccctcttccg tctggtggag ttatctggag gctgcatcaa gattgatgga gtgagaatca 3900
gtgatattgg ccttgccgac ctccgaagca aactctctat cattcctcaa gagccggtgc 3960
tgttcagtgg cactgtcaga tcaaatttgg acccctcaa ccagtacact gaagaccaga 4020
tttgggatgc cctggagagg acacacatga aagaatgtat tgctcagcta cctctgaaac 4080
ttgaatctga agtgatggag aatggggata acttctcagt gggggaacgg cagctcttgt 4140
gcatagctag agccctgctc cgccactgta agattctgat tttagatgaa gccacagctg 4200
ccatggacac agagacagac ttattgattc aagagaccat ccgagaagca tttgcagact 4260
gtaccatgct gaccattgcc catcgcctgc acacggttct aggctccgat aggattatgg 4320
tgctggccca gggacaggtg gtggagtttg acaccccatc ggtccttctg tccaacgaca 4380
gttcccgatt ctatgccatg tttgctgctg cagagaacaa ggtcgctgtc aagggctgac 4440
tcctccctgt tgacgaagtc tcttttcttt agagcattgc cattccctgc ctggggcggg 4500
cccctcatcg cgtcctccta ccgaaacctt gcctttctcg attttatctt tcgcacagca 4560
gttccggatt ggcttgtgtg tttcactttt agggagagtc atattttgat tattgtattt 4620
attccatatt catgtaaaca aaatttagtt tttgttctta attgcactct aaaaggttca 4680
gggaaccgtt attataattg tatcagaggc ctataatgaa gctttatacg tgtagctata 4740
tctatatata attctgtaca tagcctatat ttacagtgaa aatgtaagct gtttatttta 4800
tattaaaata agcactgtgc taataacagt gcatattcct ttctatcatt tttgtacagt 4860
ttgctgtact agagatctgg ttttgctatt agactgtagg aagagtagca tttcattctt 4920
ctctagctgg tggtttcacg gtgccaggtt ttctgggtgt ccaaaggaag acgtgtggca 4980
atagtgggcc ctccgacagc cccctctgcc gcctccccac agccgctcca ggggtggctg 5040
gagacgggtg ggcggctgga gaccatgcag agcgccgtga gttctcaggg ctcctgcctt 5100
ctgtcctggt gtcacttact gtttctgtca ggagagcagc ggggcgaagc ccaggcccct 5160
tttcactccc tccatcaaga atggggatca cagagacatt cctccgagcc ggggagtttc 5220
tttcctgcct tcttcttttt gctgttgttt ctaaacaaga atcagtctat ccacagagag 5280
tcccactgcc tcaggttcct atggctggcc actgcacaga gctctccagc tccaagacct 5340
gttggttcca agccctggag ccaactgctg ctttttgagg tggcactttt tcatttgcct 5400
attcccacac ctccacagtt cagtggcagg gctcaggatt tcgtgggtct gttttccttt 5460
ctcaccgcag tcgtcgcaca gtctctctct ctctctcccc tcaaagtctg caactttaag 5520
cagctcttgc taatcagtgt ctcacactgg cgtagaagtt tttgtactgt aaagagacct 5580
acctcaggtt gctggttgct gtgtggtttg gtgtgttccc gcaaaccccc tttgtgctgt 5640

```
ggggctggta gctcaggtgg gcgtggtcac tgctgtcatc agttgaatgg tcagcgttgc   5700

atgtcgtgac caactagaca ttctgtcgcc ttagcatgtt tgctgaacac cttgtggaag   5760

caaaaatctg aaaatgtgaa taaaattatt ttggattttg taaaaaaaaa aaaaaaaaaa   5820

aaaaaaaaaa aaaaaaaa                                                 5838
```

<210> 67
<211> 1181
<212> DNA
<213> Homo sapiens

<400> 67

```
ggcacgaggc ccgggccccc caaagtcccg gccgggccga gggtcggcgg ccgccggcgg     60

gccgggcccg cgcacagcgc ccgcatgtac aacatgatgg agacggagct gaagccgccg    120

ggcccgcagc aaacttcggg gggcggcggc ggcaactcca ccgcggcggc ggccggcggc    180

aaccagaaaa acagcccgga ccgcgtcaag cggcccatga atgccttcat ggtgtggtcc    240

cgcgggcagc ggcgcaagat ggcccaggag aaccccaaga tgcacaactc ggagatcagc    300

aagcgcctgg gcgccgagtg gaaacttttg tcggagacgg agaagcggcc gttcatcgac    360

gaggctaagc ggctgcgagc gctgcacatg aaggagcacc cggattataa ataccggccc    420

cggcggaaaa ccaagacgct catgaagaag gataagtaca cgctgcccgg cgggctgctg    480

gcccccggcg gcaatagcat ggcgagcggg gtcggggtgg gcgccggcct gggcgcgggc    540

gtgaaccagc gcatggacag ttacgcgcac atgaacggct ggagcaacgg cagctacagc    600

atgatgcagg accagctggg ctacccgcag cacccgggcc tcaatgcgca cggcgcagcg    660

cagatgcagc ccatgcaccg ctacgacgtg agcgccctgc agtacaactc catgaccagc    720

tcgcagacct acatgaacgg ctcgcccacc tacagcatgt cctactcgca gcagggcacc    780

cctggcatgg ctcttggctc catgggttcg gtggtcaagt ccgaggccag ctccagcccc    840

cctgtggtta cctcttcctc ccactccagg gcgccctgcc aggccgggga cctccgggac    900

atgatcagca tgtatctccc cggcgccgag gtgccggaac ccgccgcccc cagcagactt    960

cacatgtccc agcactacca gagcggcccg gtgcccggca cggccattaa cggcacactg   1020

cccctctcac acatgtgagg gccggacagc gaactggagg ggggagaaat tttcaaagaa   1080

aaacgaggga aatgggaggg gtgcaaaaga ggagagtaag aaacagcatg gagaaaaccc   1140

ggtacgctca aaaaaaaaaa aaaaaaaaaa aaaaaaaaaa a                       1181
```

<210> 68
<211> 4755
<212> DNA
<213> Homo sapiens

<400> 68
gctgctctcg ttcgcttggc tcagctcagc tcagctcagc gcagctccgc ggccgccaag 60
ccgaggcggg cacggtctcc gagtcgcgga cgccagctcc gagctccctc tctccgccgc 120
gcctccgcca ggtcgcgcct tcgtcgggac cacttcgggc aggagtcgcg tggcgaaggc 180
ctgcggccgc ggcacaaagt tgggggccgc gaagatgagg ctgtccccgg cgcccctgaa 240
gctgagccgg actccggcac tgctggccct ggcgctgccc ctggccgcgg cgctggcctt 300
ctccgacgag accctggaca aagtgcccaa gtcagagggc tactgcagcc gtatcctgcg 360
cgcccagggc acgcggcgcg agggctacac cgagttcagc ctccgcgtgg agggcgaccc 420
cgacttctac aagccgggaa ccagctaccg cgtaacactt tcagctgctc ctccctccta 480
cttcagagga ttcacattaa ttgccctcag agagaacaga gagggtgata aggaagaaga 540
ccatgctggg accttccaga tcatagacga agaagaaact cagtttatga gcaattgccc 600
tgttgcagtc actgaaagca ctccacggag gaggacccgg atccaggtgt tttggatagc 660
accaccagcg ggaacaggct gcgtgattct gaaggccagc atcgtacaaa aacgcattat 720
ttattttcaa gatgagggct ctctgaccaa gaaactttgt gaacaagatt ccacatttga 780
tggggtgact gacaaaccca tcttagactg ctgtgcctgc ggaactgcca agtacagact 840
cacattttat gggaattggt ccgagaagac acacccaaag gattaccctc gtcgggccaa 900
ccactggtct gcgatcatcg gaggatccca ctccaagaat tatgtactgt gggaatatgg 960
aggatatgcc agcgaaggcg tcaaacaagt tgcagaattg ggctcacccg tgaaaatgga 1020
ggaagaaatt cgacaacaga gtgatgaggt cctcaccgtc atcaaagcca aagcccagtg 1080
gccagcctgg cagcctctca acgtgagagc agcaccttca gctgaatttt ccgtggacag 1140
aacgcgccat ttaatgtcct tcctgaccat gatgggccct agtcccgact ggaacgtagg 1200
cttatctgca gaagatctgt gcaccaagga atgtggctgg gtccagaagg tggtgcaaga 1260
cctgattccc tgggacgctg gcaccgacag cggggtgacc tatgagtcac ccaacaaacc 1320
caccattccc caggagaaaa tccggcccct gaccagcctg gaccatcctc agagtccttt 1380
ctatgaccca gagggtgggt ccatcactca agtagccaga gttgtcatcg agagaatcgc 1440
acggaagggt gaacaatgca atattgtacc tgacaatgtc gatgatattg tagctgacct 1500
ggctccagaa gagaaagatg aagatgacac ccctgaaacc tgcatctact ccaactggtc 1560
cccatggtcc gcctgcagct cctccacctg tgacaaaggc aagaggatgc gacagcgcat 1620
gctgaaagca cagctggacc tcagcgtccc ctgccctgac acccaggact tccagccctg 1680
catgggccct ggctgcagtg acgaagacgg ccccacctgc accatgtccg agtggatcac 1740
ctggtcgccc tgcagcatct cctgcggcat gggcatgagg tcccgggaga ggtatgtgaa 1800

gcagttcccg gaggacggct ccgtgtgcac gctgcccact gaggaaacgg agaagtgcac 1860
ggtcaacgag gagtgctctc ccagcagctg cctgatgacc gagtggggcg agtgggacga 1920
gtacagcgcc acctgcggca tgggcatgaa gaagcggcac cgcatgatca agatgaaccc 1980
cgcagatggc tccatgtgca aagccgagac atcacaggca gagaagcgca tgatgccaga 2040
gtgccacacc atcccatgct tgctgtcccc atggtccgag tggagtgact gcagcgtgac 2100
ctgcgggaag ggcatgcgaa cccgacagcg gatgctcaag tctctggcag aacttggaga 2160
ctgcaatgag gatctggagc aggtggagaa gtgcatgctc cctgaatgcc ccattgactg 2220
tgagctcacc gagtggtccc agtggtcgga atgtaacaag tcatgtggga aaggccacgt 2280
gattcgaacc cggatgatcc aaatggagcc tcagtttgga ggtgcaccct gcccagagac 2340
tgtgcagcga aaaaagtgcc gcatccgaaa atgccttcga aatccatcca tccaaaagct 2400
acgctggagg gaggcccgag agagccggcg gagtgagcag ctgaaggaag agtctgaagg 2460
ggagcagttc ccaggttgta ggatgcgccc atggacggcc tggtcagaat gcaccaaact 2520
gtgcggaggt ggaattcagg aacgttacat gactgtaaag aagagattca aaagctccca 2580
gtttaccagc tgcaaagaca agaaggagat cagagcatgc aatgttcatc cttgttagca 2640
agggtacgag ttccccaggg ctgcactcta gattccagag tcaccaatgg ctggattatt 2700
tgcttgttta agacaattta aattgtgtac gctagttttc atttttgcag tgtggttcgc 2760
ccagtagtct tgtggatgcc agagacatcc tttctgaata cttcttgatg ggtacaggct 2820
gagtggggcg ccctcacctc cagccagcct cttcctgcag aggagtagtg tcagccacct 2880
tgtactaagc tgaaacatgt ccctctggag cttccacctg gccagggagg acggagactt 2940
tgacctactc cacatggaga ggcaaccatg tctggaagtg actatgcctg agtcccaggg 3000
tgcggcaggt aggaaacatt cacagatgaa gacagcagat tccccacatt ctcatctttg 3060
gcctgttcaa tgaaaccatt gtttgcccat ctcttcttag tggaacttta ggtctctttt 3120
caagtctcct cagtcatcaa tagttcctgg ggaaaaacag agctggtaga cttgaagagg 3180
agcattgatg ttgggtggct tttgttcttt cactgagaaa ttcggaatac atttgtctca 3240
cccctgatat tggttcctga tgcccccccca acaaaaataa ataaataaat tatggctgct 3300
ttatttaaat ataaggtagc tagtttttac acctgagata aataataagc ttagagtgta 3360
tttttccctt gcttttgggg gttcagagga gtatgtacaa ttcttctggg aagccagcct 3420
tctgaacttt ttggtactaa atccttattg gaaccaagac aaaggaagca aaattggtct 3480
ctttagagac caatttgcct aaattttaaa atcttcctac acacatctag acgttcaagt 3540
ttgcaaatca gtttttagca agaaaacatt tttgctatac aaacattttg ctaagtctgc 3600

ccaaagcccc cccaatgcat tccttcaaca aaatacaatc tctgtacttt aaagttattt 3660

tagtcatgaa attttatatg cagagagaaa aagttaccga gacagaaaac aaatctaagg 3720

gaaaggaata ttatgggatt aagctgagca agcaattctg gtggaaagtc aaacctgtca 3780

gtgctccaca ccagggctgt ggtcctccca gacatgcata ggaatggcca caggtttaca 3840

ctgccttccc agcaattata agcacaccag attcagggag actgaccacc aagggatagt 3900

gtaaaaggac attttctcag ttgggtccat cagcagtttt tcttcctgca tttattgttg 3960

aaaactattg tttcatttct tcttttatag gccttattac tgcttaatcc aaatgtgtac 4020

cattggtgag acacatacaa tgctctgaat acactacgaa tttgtattaa acacatcaga 4080

atatttccaa atacaacata gtatagtcct gaatatgtac ttttaacaca agagagacta 4140

ttcaataaaa actcactggg tctttcatgt ctttaagcta agtaagtgtt cagaaggttc 4200

tttttttatat tgtcctccac ctccatcatt ttcaataaaa gatagggctt ttgctccctt 4260

gttcttggag ggaccattat tacatctctg aactaccttt gtatccaaca tgttttaaat 4320

ccttaaatga attgctttct cccaaaaaaa gcacagtata aagaaacaca agatttaatt 4380

atttttctac ttggggggaa aaaagtcctc atgtagaagc acccactttt gcaatgttgt 4440

tctaagctat ctatctaact ctcagcccat gataaagttc cttaagctgg tgattcctaa 4500

tcaaggacaa gccaccctag tgtctcatgt ttgtatttgg tcccagttgg gtacatttta 4560

aaatcctgat tttggagact taaaaccagg ttaatggcta agaatgggta acatgactct 4620

tgttggattg ttattttttg tttgcaatgg ggaatttata agaagcatca agtctctttc 4680

ttaccaaagt cttgttaggt ggtttatagt tcttttggct aacaaatcat tttggaaata 4740

aagatttttt actac 4755

<210> 69
<211> 6841
<212> DNA
<213> Homo sapiens

<400> 69
gccggagggc gcccgagggg ccccgggccg cggcgctcag ggcccgggcg gccggcggcg 60

gccccggggc tgggggggagt ccagcccgga tattgagtgc agccattgag aaaagccaaa 120

ctcttgtgtg tgcgcgtctc gatagccccc aagatggccg ccaatgtggg atcgatgttt 180

caatattgga agcgatttga tctacggcga ctccagaagg agcttaattc cgtcgcttct 240

gagctgtctg cacggcagga ggagagtgaa cattctcata aacatttaat tgaactccgc 300

cgggaattta agaaaaatgt acctgaggaa atcagagaga tggtggctcc tgtattaaaa 360

agcttccaag ccgaggtggt ggcccttagt aagagaagtc aggaggcgga ggctgctttt 420

ctgagtgttt acaagcaatt aattgaagca ccagaccccg tgcctgtgtt tgaggcggca 480
cgcagcctag acgacagact gcagcccccc agctttgacc ccagtgggca gccccggcga 540
gacctccaca cttcgtggaa gaggaacccc gagctcctca gccccaaaga gcagagagag 600
gggacgtcgc ctgccgggcc cacgctgacc gagggaagcc gcctcccagg cattcccggg 660
aaagccctcc tgacagaaac cttgctgcag agaaatgagg cggaaaaaca aaagggcctt 720
caagaagtac agatcacttt ggcggccaga ctgggggagg cagaggagaa aatcaaagtc 780
ctacattcag cgctaaaggc tacgcaggca gagctgctag agctgcggcg gaagtacgac 840
gaggaggcag catccaaggc agatgaagtc ggcctgatca tgaccaacct ggagaaagct 900
aatcagcgag ctgaggctgc ccagcgggag gtggaaagtc tccgggaaca gctggcctct 960
gtcaacagct ccatccgcct ggcttgctgc tctccccagg ggcccagtgg ggataaggtg 1020
aacttcactc tgtgctcggg ccctcggctg gaggccgcgc tggcctccaa ggacagggag 1080
atcctgcggc tgctgaagga cgtgcagcac ctccagagct cactgcagga gctggaggag 1140
gcatccgcca accagatcgc cgacctggag cggcagctca cggccaagtc cgaggccata 1200
gaaaagctgg aagagaagct ccaggcccag tctgactatg aggaaattaa aacggagctg 1260
agcatcctga aagccatgaa gctggcctcc agcacctgca gcctccccca gggcatggcc 1320
aagcctgaag actcactgct tattgcaaag gaggccttct tccccacgca gaaattcctt 1380
ctggagaagc ccagcctcct ggccagccct gaggaagacc catcagagga cgattccatc 1440
aaggattcac tgggcacgga gcagtcctac ccctcccctc agcagctccc acctccacca 1500
gggccagaag accccctgtc tcccagcccc gggcagcccc tgctgggccc cagcttgggg 1560
cctgacggca ctcggacttt ctcgctgtcc cccttcccca gcctggcatc aggggagaga 1620
ctgatgatgc ccccagccgc cttcaaggga gaggcgggcg gcctgctggt gttcccccca 1680
gccttctatg gcgccaagcc ccccacagcc cctgccaccc cggcccctgg ccctgagcca 1740
ctgggcggtc ctgagcccgc ggatggtggt gggggcggag cggcggggcc cggggcagag 1800
gaggagcagc tggacacggc agagatcgcc ttccaggtga aggagcagct gctgaaacac 1860
aacatcgggc agcgggtgtt tgggcattac gtgctggggc tgtcgcaggg ctcggtcagc 1920
gagatcctag cccggcccaa gccctggcgc aagctcacgg tgaagggcaa ggagcccttc 1980
atcaagatga agcagttcct gtcggatgag cagaatgtac tggcgctcag gaccatccaa 2040
gtgcggcagc gaggcagcat caccccgaga atccgcacgc ctgagacagg ctcagacgac 2100
gccatcaaga gcattctaga gcaggccaag aaggagatcg agtcgcagaa gggcggcgag 2160
cccaagacct cggtggcccc gctgagcatc gccaacggca cgacccccgc cagcacctcg 2220
gaggacgcca tcaagagcat cctggagcag gcacgccgtg agatgcaggc gcaacagcag 2280

gcgctgctgg agatggaggt ggcgcccagg ggccgctcgg tgcccccctc gcccccggag 2340

cggccatcac tggccaccgc gagccagaac ggggccccgg ccttggtgaa gcaggaggag 2400

ggcagcgggg gccccgcgca ggcgccgctc ccggtcctgt cccccgccgc cttcgtgcag 2460

agcatcatcc gcaaggtcaa gtccgagatc ggcgacgccg gctacttcga ccaccactgg 2520

gcctccgacc gcggcctgct cagccgcccc tacgcctccg tgtcgccctc gctgtcctcc 2580

tcctcctcct ctggctactc tggccagccc aacggccgcg cctggccccg cggggacgag 2640

gcccctgtgc cccccgagga cgaggcggcg gcaggggcgg aggacgaacc ccccaggacg 2700

ggcgagctca aggctgaggg cgcgacggcc gaggcgggcg cgcggctgcc ctactacccg 2760

gcctacgtgc cgcgcaccct gaagcccacc gtgccgccgc tgacccccga gcagtacgag 2820

ctgtacatgt accgtgaggt agacacgctg gagctcaccc gccaggtcaa ggagaagctg 2880

gccaagaacg gcatctgcca gaggatcttc ggggagaagg tgctgggcct gtcacagggc 2940

agcgtgagcg acatgctgtc ccggccgaag ccatggagca agctgacgca gaaggggcgg 3000

gagcccttca tccgcatgca gctgtggctc tctgaccagc tcggccaggc agtgggccag 3060

cagcctggtg cctcccaggc cagtcccaca gaaccaaggt cctcaccatc cccacccccc 3120

agccccacag agcctgagaa gagctcccag gagccgttga gcctgtccct ggagagcagc 3180

aaggagaacc agcagccaga gggccgctcc agctcctcgt tgagcgggaa gatgtactca 3240

ggcagccagg ccccaggggg catccaggag atcgtggcca tgtcccccga gctggacacg 3300

tactccatca ccaagagggt gaaggaggtc ctcacagaca acaatctagg gcagcggctg 3360

tttggggaaa gcatcctggg tctgacacag ggctccgtgt ctgacctgct gtcccggccc 3420

aaaccctggc acaagctgag cctgaagggg cgggagcctt ttgtccgcat gcagctgtgg 3480

ctcaatgacc cccataacgt ggagaagctg agggatatga agaagctgga gaagaaagcc 3540

tacctgaaac gtcgctatgg cctcatcagc accggctcag acagtgagtc cccggccacc 3600

cgctcagagt gccccagccc ctgcctgcag ccccaggacc tgagcctcct gcagatcaag 3660

aagccccggg tggtgctggc acccgaggag aaggaggcac tgcggaaggc ctatcagctg 3720

gaaccctacc cctcgcagca gaccatcgag ctcctctcct tccagctcaa cctcaagacc 3780

aacaccgtca tcaactggtt ccacaactac aggtcccgga tgcgccggga gatgttggtg 3840

gaggggaccc aggatgagcc agaccttgat ccaagcgggg gtcctggaat cctaccgcca 3900

ggccactccc acccagaccc caccccgcag agccctgact ctgagactga ggaccagaag 3960

ccaaccgtga aggaactgga gcttcaggag ggccctgagg agaacagcac acccctgacc 4020

acccaggaca aggcccaagt gaggatcaag caggaacaga tggaggagga tgctgaggaa 4080

gaggcaggca gccagcccca ggactcaggg gagctggaca aaggccaagg tccccccaaa 4140
gaggagcatc ccgaccctcc gggtaatgat ggactcccaa aagtggctcc cgggcccctc 4200
cttccaggtg gatccacccc agactgtccc tcacttcatc cccaacagga gagtgaggcc 4260
ggggagcgac ttcacccgga ccctttaagt tttaagtcag cctcagagtc ctcacgctgc 4320
agcctggagg tgtcactgaa ctcgccctcg gccgcctcct caccaggcct catgatgtct 4380
gtgtcacctg tcccctcctc ctcagctccc atctccccat ccccacctgg cgccccccct 4440
gccaaagtgc cgagtgccag ccccactgct gacatggctg gagccttgca ccccagtgcc 4500
aaggtgaacc ccaacttgca gcggcggcat gagaagatgg ccaatctgaa caacatcatt 4560
taccgactag agcgggctgc caatcgggag gaggccctgg agtgggagtt ctgaaggcag 4620
ggtgaggggg caagggacat accctggtaa ctaccttcct tctcgcactt actctcctca 4680
acaggatggg gtaagggagg gaggaactca accatcaaaa tgtggacagc aatgttatgc 4740
cgtttacgtt ttttgttgta atcctagttc tatgaagctg tgtgagcagg tgggtcaaat 4800
gccattgcct ccacttttct gcaccccccct gctcctcttc accctgaccc ctctgcagga 4860
ggcagaagca aaatggcacc acatattcac ctgaaaactc caaactcttt tagaaaaata 4920
aataaatatt tatagacctc ttttagatat tttaataaag gatcctttgg aatttatccc 4980
agctgatgct gttttgatat tacagagagt tataaaatca ggatgctgtc acaactgttg 5040
cgaagtatac actgaagttg tgtcgttttt gccactagat gagattaaaa gaagacaatt 5100
attcaaagcc atcacaaaac actataagac tgaccaaaat ttagataacc tttgaaccac 5160
gattttttttc cacatctgtc tgtgagacac agcgcaatgc tactgccctt ccagaaactg 5220
tgctaaaaag agaaagtcca aaagactcta aacaaaaacc tcgacgccgt tgaggatgtg 5280
tttcattctg gtggtctgtt ttgcaagctt gataacagaa tgtccgtgcc attgtaaatg 5340
ttgtagagat gtgggccgtg gcccaaccgt cctatatgag atgtagcatg gtacagaaca 5400
aactgcttac acaggtctca ctagttagaa acctgtgggc catggaggtc agacatccat 5460
cttgtccatc tataggcaag aagtgtttcc agatcctttg gaaaggtggg catggggcag 5520
gtgcttggag agtggcgttt gagccagagc gaccccattt cccgtgtgaa ccataggcac 5580
aacccaggaa gtttccccac ttgtaggagt gtgggtattc cagagcaaga ctgtggccac 5640
catcttcccc tcttggtgtt ttccgaaagt gacagtgttg gtcatcccat gaccactgaa 5700
gcttagtaac cagcgccaaa aagtagattc atcaaactag agaccccagc tccccttctc 5760
gccatcttct ttctcaagtt gaccgtggtg ctgtttctgg aaggcatctg caactccaag 5820
tccatgcaga actctggaag gccaagttca tcgcagcatg ttcaccatat cccagcctcc 5880
aaatctatcc tcctaccttc caacgcatga cctgttgggg agcagagact taacccccaa 5940

ctcagaggaa cccttcctcc agcgtctttg gcatggtttc tagggtgaga gttcccaatt 6000

tggatagaac ggccaccata ttggttactg aatctctctc ccttgttttt attacgtttc 6060

ctttttcaaa ctgtccatgg gaaggctgaa ttgagtgact ccccagaatg aagatgagaa 6120

ggtgaatata atcaatgcca atgtaatgcc agcgggtgag atggccgatg gaggtttcaa 6180

agatgtagct agcattttga aaccatatgg gcaaaacccg gcaaccagaa ggggacagat 6240

aaggaccgtt ccagaaatcc caactctcac acccagccca ggctgcagtc tccacaccaa 6300

acagtcaaca aaacacaaac cctgaaggaa aaccttttcc atacacccag gctatgcatt 6360

gaagagtttt ccactgtata catttttatc cagatgaagg tatttttata ttttgacaat 6420

aggaaacagt gaccattttc agagtaatca aatctggaac aaatgaaaca tcttttagcc 6480

accaccaccc tgttgcaatt aagacaaccg tgggggaaca caccactttt tactgttgaa 6540

accaacacaa cgttgaaatc caggcttata cgcagactcc gattcctaga gaactaaatt 6600

tggctttagt gtgacgggat ttgattaagc acttagtata gtcttttgaa cacggaaatc 6660

ctgttgtact taaagctagc ggacccgtga acaactttgt caggttcacg tcctataacg 6720

gttaaaaaac acacacacac atacacaaac cgtttctatg agagattgat gaactttgtt 6780

taaaatttta aaaaaaggaa cacgttctgt aaacgagtcg ctaaatacag aattgtataa 6840

t 6841

<210> 70
<211> 443
<212> DNA
<213> Homo sapiens

<400> 70
tttttttttt tttttccatt aacccaacac aagtttattt actaagtcaa gtcaaactga 60

ggagtatttg tttctttggt agttggtaga caaagaatac atatacatat tctatttccc 120

attaagcatt cgatcatgtt acaaaacaat ggcctagaga actatcattg aagatttacc 180

aaatctgcct gaagcaaaat gtgataaact gcagaaatgg tggagaatga cactggaagt 240

cataaagttg attctcaaac acgggtttga actgcatagg accgcttata tgcatatttt 300

gataaatata ttgaaaattt ttttggaaat ttgcaacaat ttaaaaaact tgcagatgca 360

ccatgtagct tagaaatact gaaaaactaa gaaaaaggtg tgtcatgaat tcatataata 420

tatgtagaca ctagtctatt tta 443

<210> 71
<211> 2432
<212> DNA
<213> Homo sapiens

<400> 71

```
ggcgagtggc gagtggcgag tgtcaggggg gcggccggcg ggggcggggc ggccggagga      60
ggcgttggca gcgggctcgg acccacgcgg cgccgcggcc cgcctggcct gcagcgctcc     120
cacccccggc ggcggcacga tgccctttga cttcaggagg tttgacatct acaggaaggt     180
gcccaaggac cttacgcagc caacgtacac cggggccatt atctccatct gctgctgcct     240
cttcatcctc ttcctcttcc tctcggagct caccggattt ataacgacag aagttgtgaa     300
cgagctctat gtcgatgacc cagacaagga cagcggtggc aagatcgacg tcagtctgaa     360
catcagttta cccaatctgc actgcgagtt ggttgggctt gacattcagg atgagatggg     420
caggcacgaa gtgggccaca tcgacaactc catgaagatc ccgctgaaca atggggcagg     480
ctgccgcttc gaggggcagt tcagcatcaa caaggtcccc ggcaacttcc acgtgtccac     540
acacagtgcc acagcccagc cacagaaccc agacatgacg catgtcatcc acaagctctc     600
ctttggggac acgctacagg tccagaacat ccacggagct ttcaatgctc tcgggggagc     660
agacagactc acctccaacc ccctggcctc ccacgactac atcctgaaga ttgtgcccac     720
ggtttatgag gacaagagtg gcaagcagcg gtactcctac cagtacacgg tggccaacaa     780
ggaatacgtc gcctacagcc acacgggccg catcatccct gcaatctggt tccgctacga     840
cctcagcccc atcacggtca agtacacaga gagacggcag ccgctgtaca gattcatcac     900
cacgatctgt gccatcattg gcgggacctt caccgtcgcc ggcatcctgg actcatgcat     960
cttcacagcc tctgaggcct ggaagaagat ccagctgggc aagatgcatt gacgccacac    1020
ccagcctaat ggccgaggac cctgggcatc gccagccttg cctccagtgc cctgtctcct    1080
ttggccctca atctggtccc aaatctggct gtgtcccaaa gggtgtgtgg gaagtggggg    1140
gaaagtagag gatggctcga tgttttgcag ctacctcttt tccccgtgtt tctttttaga    1200
caaattacac tgcctgaagt tgcagttccc ctttccctgg ggagccccaa gaacagagtc    1260
aggcaagggg tggggagtcc agggatcttg ggacccctc ctaggagagc tgcagtctct     1320
tccctcaggg gaacatccca gaatgcatat cgatcagctc tcagccaggc ttcgacaatc    1380
tcgcagcccc cactaggtgg acacattaat gatttggttt ctcccctggg cagccaacct    1440
gccccagagg caccagacct gggctttcag ctttgggacc aggctgccca aaggtactcc    1500
tttatacacc cggcaccttc cacgaaagat ggtacttccc aagcaagccc ctatgatttg    1560
tcactataga tggaaccctg acttctgccc catcccttcc tgcccaacct agaacccagg    1620
cctcaagtct ttaccccacc cctttcttgt tcttccaaga agcagatgcc cagttgctca    1680
gcagcagcgg tagagacttg aatctgccca ccagtcacaa ggcgggtcac agattcctct    1740
tcctctcttc tcctcgttcc tctgaaccct ccaccaatgt gcctcagcct gtgtgctgtg    1800
```

tggcaacagc attctggttc ccactgccaa gatctcccac cactctgctg ggatctgcag 1860

tggcagggag tgggggttgt gtaaagggga agtcatcttt tgagatccag atagacatgg 1920

tttgtgcact tacgtccaga tgggaagcat ccttcctgca accctaaaat aatcatgcag 1980

cctctcagac ggacgccatc ggtcccaagg ccttaggtgg aggaagcaaa gcaggccagg 2040

cctgtcctgt ccgtggacct ctaccttctg gactccctac gggtgcagag cacttgggtt 2100

tctctacagc catcgtggcc cacttgacac tgtgctcctc catcagctgg tcacatgcca 2160

acacgttccc agcccctgag gcagctccag ggtgccccac ctgctcctga ggtgggtccc 2220

taccctgctg ctcctcttca tcctttccct tttgtcctga aagggaggag caatggtcca 2280

ggcattaatt ccacccaggg aattttagct atgccctcat gtcccaggga gagagccaca 2340

cgcctgtttt ccatttatag caagattgtt tgcatacttt tgtaatgaag gggagtgtcc 2400

agtggaagga tttttaaaat tatcttatgg at 2432

<210> 72
<211> 2782
<212> DNA
<213> Homo sapiens

<400> 72
gcggccgcgg cggggcggcg cgggaaccgg gccccggggg gagtcggccg ggctgctgct 60

gctgctgctc caggtgctgc cgcccggggc tacggcaggg ccgggacgcc ggggcacgcg 120

gggcgctggc ggcggcggcg tctgctggcc cggcgcggcc ccagcctttc cccgggacgc 180

gcggctgctg ctgctcctgc cgccgctgcc gccactgtcg ccgccgccgc cgagctccgc 240

gcccgcagcc tccgcctccc ggatggacgc tctgccccgc agcgggctga acctgaagga 300

ggagccgctg ctgcccgccg gcctgggctc agtgcgctcc tggatgcagg gcgcgggcat 360

cctggacgcc agcaccgcgg cgcagagtgg cgtgggtctg gcacgagcac attttgagaa 420

gcagcctccc tccaacctca ggaaatccaa cttcttccac ttcgtgctgg ccatgtacga 480

ccggcagggg cagcccgtgg aggtggagcg cacagccttc atcgacttcg tggaaaagga 540

ccgagagccc ggggcggaaa agactaacaa tgggatccat taccgcctcc ggctggtgta 600

taacaatgga ctgcggacag agcaagacct ctacgtgcgt ctcatcgact ccatgtccaa 660

acaggccatc atctatgagg ggcaggacaa gaaccccgaa atgtgccgag tgctgctcac 720

ccatgagatc atgtgcagcc ggtgctgtga ccggaagagc tgtggcaacc ggaatgagac 780

gccctcagac cccgtcatca ttgacaggtt cttcctcaag ttcttcctca aatgcaacca 840

gaactgcctg aagaatgcgg ggaatcccag agacatgcgc cgcttccagg tggtggtgtc 900

cacgacggtg agcgtggacg gacacgtgct ggccgtgtcc gacaacatgt ttgtgcacaa 960

caactccaag catggccgca gggcgcgccg cctggacccc tccgaagctg ccaccccctg 1020
catcaaggcc atcagccccg gggagggctg gaccacgggc ggcgccaccg tcattgtcat 1080
cggcgacaac ttcttcgacg ggttgcaggt cgtgttcgga aacgtgctcg tgtggagcga 1140
gctcatcacg ccccacgcca tccgggtgca gacgcccccg cggcacatcc ccggggtggt 1200
ggaggtgacc ctctcctaca agtccaagca gttttgcaag ggatgccccg gccgctttgt 1260
ctacacagct ctgaacgagc ccaccattga ctacggattc cagaggctac agaaagtcat 1320
tcccagacac ccccggagacc ccgagaggct gcccaaggaa gtgctgctga agcgggcggc 1380
cgacttggca gaagccctgt acggagtgcc cggcagtaac caggagctgc tcctgaagcg 1440
cgcggcggac gtggccgagg ctctgtacag caccccccgc gcacccgggc cgctcgcacc 1500
cctggccccg agccacccac actccgccgt cgtgggcatc aacgccttca gcagcccgct 1560
ggccatcgcc gtcggggacg ccaccccggg gcccgagccg ggctacgcgc gcagctgcag 1620
cagcgcgtcc ccccgcgggt tcgcgcccag ccccggctcg cagcagagcg gctacggcgg 1680
cggcctcgga gctggcctgg gcggctacgg cgcgccgggc gtggccggcc tcggcgtgcc 1740
tgggtccccc agcttcctca atggctccac cgccacctcg cccttcgcca aggagcgcct 1800
tcgcccccgt gctgcgcccc ccaagctccc caccccaggc ctgccccaga gcccacggag 1860
aggggcttcc agaccagtct tttgaggatt ctgacaagtt ccactctcca gcccgggggc 1920
ttcagggcct ggcatactcc taattacggt ctgcagctgt tcccatggag cccggactgg 1980
aggtccctct gggattcaca gccacacccc ggatggtggc acagacagat gcagggccag 2040
ggccatgggc ggacctcaac ccgtgagctg aacggggaga ggccttcacc ccatgctcaa 2100
gcctccccgc tagcagcccc acaggcttct ctcgcctccc tgtcttgggg tagtcagaag 2160
ccccagcact gtgcagatgc tcttggcagg acagcatcgc agggaggtgc tgggattctg 2220
ggcctcactg tctgggtctt ggttcctctg aaagagatgg atcttgtgca gaccagggtt 2280
gttgagtgag gggagcgtgg gatggggacc gtgggaaaga ggacagctca gggagaagtg 2340
acctggaaag gtcctgtttg catctgaccc atctcaactg gcccagcatc ccaacttctc 2400
tgcagcgaaa gggtggcgcc ccgcagcctc gggaggcctg cccaggctcc cgtggagctt 2460
ccaacagctg cttggccccg cagctgcccc cacttccttt gagacctgca ctctcatgct 2520
tgccgcatca tgcctccctg tgggggcttt gggcatggag gaggcagaag aggggtgcc 2580
aggcctcctg tatttggggt cttcccccag tggatgtctc atggactctg gccccacaca 2640
ctcacaatga ctctggctgg ccccacgcag cgggcccagc cgccccccag gtggcctcac 2700
attctgctct gctaagtttg gagaaaacag aacaataaac cagatgcagg tggtgcccgc 2760

ccggcctctc acctgcctcc tt 2782

<210> 73
<211> 1722
<212> DNA
<213> Homo sapiens

<400> 73
ggggaaaaga gctaggaaag agctgcaaag cagtgtgggc tttttccctt tttttgctcc 60

ttttcattac ccctcctccg ttttcaccct tctccggact tcgcgtagaa cctgcgaatt 120

tcgaagagga ggtggcaaag tgggagaaaa gaggtgttag ggtttggggt ttttttgttt 180

ttgtttttgt tttttaattt cttgatttca acattttctc ccaccctctc ggctgcagcc 240

aacgcctctt acctgttctg cggcgccgcg caccgctggc agctgagggt tagaaagcgg 300

ggtgtatttt agattttaag caaaaatttt aaagataaat ccatttttct ctcccacccc 360

caacgccatc tccactgcat ccgatctcat tatttcggtg gttgcttggg ggtgaacaat 420

tttgtggctt tttttcccct ataattctga cccgctcagg cttgagggtt tctccggcct 480

ccgctcactg cgtgcacctg gcgctgccct gcttccccca acctgttgca aggctttaat 540

tcttgcaact gggacctgct cgcaggcacc ccagccctcc acctctctct acatttttgc 600

aagtgtctgg gggagggcac ctgctctacc tgccagaaat tttaaaacaa aaacaaaaac 660

aaaaaaatct ccgggggccc tcttggcccc tttatccctg cactctcgct ctcctgcccc 720

accccgaggt aaagggggcg actaagagaa gatggtgttg ctcaccgcgg tcctcctgct 780

gctggccgcc tatgcggggc cggcccagag cctgggctcc ttcgtgcact gcgagccctg 840

cgacgagaaa gccctctcca tgtgcccccc cagcccccctg ggctgcgagc tggtcaagga 900

gccgggctgc ggctgctgca tgacctgcgc cctggccgag gggcagtcgt gcggcgtcta 960

caccgagcgc tgcgcccagg ggctgcgctg cctcccccgg caggacgagg agaagccgct 1020

gcacgccctg ctgcacggcc gcggggtttg cctcaacgaa aagagctacc gcgagcaagt 1080

caagatcgag agagactccc gtgagcacga ggagcccacc acctctgaga tggccgagga 1140

gacctactcc cccaagatct tccggcccaa acacacccgc atctccgagc tgaaggctga 1200

agcagtgaag aaggaccgca gaaagaagct gacccagtcc aagtttgtcg gggagccgga 1260

gaacactgcc cacccccgga tcatctctgc acctgagatg agacaggagt ctgagcaggg 1320

cccctgccgc agacacatgg aggcttccct gcaggagctc aaagccagcc cacgcatggt 1380

gccccgtgct gtgtacctgc ccaattgtga ccgcaaagga ttctacaaga gaaagcagtg 1440

caaaccttcc cgtggccgca agcgtggcat ctgctggtgc gtggacaagt acgggatgaa 1500

gctgccaggc atggagtacg ttgacgggga ctttcagtgc cacaccttcg acagcagcaa 1560

cgttgagtga tgcgtccccc cccaaccttt ccctcacccc ctcccacccc cagccccgac 1620

tccagccagc gcctccctcc accccaggac gccactcatt tcatctcatt taagggaaaa 1680

atatatatct atctatttga ggaaaaaaaa aaaaaaaaaa aa 1722

<210> 74
<211> 2626
<212> DNA
<213> Homo sapiens

<400> 74
gggtacggct gcgagaagac gacagaaggg gatgtcacct gctttatttc tggctttggc 60

ctgtggtctg tgatacccat cctgcttgat gttctgcaga atggcacttg actgctgggc 120

atgcatgaag ttaagggcaa gaaacagtat gccatgtgtt ctgtaccatc atgtgtctct 180

tcttgcttct gggcccttct actggtgaac tttcatcaag atctgcgcca tgccgtgtca 240

ctatcaagcc attaagtttt gtctgggttg ctgtcagccc cagttggctt cctggtcaac 300

aaggacctca agaactgcct gtggaccgag gccccctacc agtgcatgag acacacacct 360

accctcccca gctttccagg aaccctactg gctgccagac tgatgggcgg gctggtatgt 420

gtggacatgt gttcactgtc attatgctgt ggctccaggt gagggtgagg actgggccta 480

tatagaatcc agataccatt gtcaacttcc cttattcccg tctaagatgt gagcagagtg 540

ccatagtagg ggttctggga agaggtattt ctgatttgtg ggcctctgct tgcttgactt 600

caggtcactt atacttctta ttttgcttgc ctgccttcat ccctcatttc ctccctctca 660

ttcttctttc ctccctccct ttcctggtag cctcctttcc tccccttctg ccttcccctt 720

ccttctttcc ttattctttt ttattttgtt taaatagtac cacagagaaa acaactgaaa 780

aaccacattt ttctacatac agctggggag gtagctgaga acttggcact gcgcacacat 840

actaggttga aagagagttg aggaaaccag aaggccaagt ggatctgctg gcaaaccctg 900

aacctgtctc ctgcgcttgc tctacagttc tgaagttgaa aatcgttttc atgcctagca 960

tctgcttgag ttataaaccc caaggcagcc atgtcataga ctagtgttta ctcttgtttt 1020

gactttgttt taatgcttcc taagacccaa gtgcctcctg ctgtttcctc ctttgtggta 1080

gcctctggcc atctggacct caatccccag ctttcccact ttcagcagtc ctttgctctc 1140

tttgcttcta cctcaaatag ccccaggagt gggctttagt ctccaatatg gagcatctca 1200

agcttctcct gggggatggg gattgggatg ggcggaatct gttttggatc tccgggttat 1260

ttccagtggg tgtaaaagca gagctgggcc tttccctctc ttatccctga gggtgggtaa 1320

gaaggactgt atctacacct gttcttccct accttctctt ttgttaggga ggcctcattc 1380

taagttcctc aagagagtcc ttggcttaaa gctgtagcaa gggtgtgcta ggtgggggat 1440

ttggagcaaa accgtcgagt aggcatgata ctggtatgga gtgggcctgc aaaatcagac 1500

agaaatggct tgagaagccg cagggggagc atgcctgtct ctcagtgata gagtatggga 1560

gggacctccc tagcttggaa aatgagaatt gaaggggtta tgaacaaata ggatgcctag 1620

ttgaggatgt tcccaaagtt ttgtccaatc ttatcattag tagattttat aagccacaga 1680

gacaaaccag aaacggaata atgttacttt ggatgcttta ttttttgtt ctaggtgtgg 1740

ctttgtacat gcagaagaat gctatatgct gcacattttg cctttaaagt cttacgactt 1800

tccccatttt agtctaatgg gaagatacag atgtgcaagt ctgcttttt gtttttgtt 1860

attattttt tttttgctct gtgttatgga cattttcaga catgcacaga agtggagagg 1920

atggtccttg gaccccatgt gtccatcacc tagctgcatc acttatcagc tatggtcaac 1980

ctggtttcat ctgtatctct ctcttttcac ctgtattgtt tattgaaaat ccaagacact 2040

atgccaatgc aaccgtgact actttgggag attggtagtc tcttttgatg gtgatagtga 2100

tggggtgcac tatcataatc acatcaggtc tgctttttgc ttttaatgtt aactaatgaa 2160

gttccagaga tgggccttag aaatgtgttt taagaattaa caaggagtct caaaaagaaa 2220

tgagagggat gcttcctttc ccttgcatct acaaaacaag agagagactg ttctgttgta 2280

aaactctttc aaaaattctg atatggtaag gtacttgaga cccttcacca gaatgtcaat 2340

ctttttttct gtgtaacatg gaaacttgtg tgaccattag cattgttatc agcttgtact 2400

ggtctcataa ctctggtttt ggaagaataa tttggaaatt gttgctgtgt tctgtgaaaa 2460

taacctcccc aaaataatta gtaactggtt gttctacttg gtaatttgac accctgttaa 2520

taacgcaatt atttctgtgt tcttaaacag tataaatagt tgtaagtttg catgcatgat 2580

ggaaaaataa aaacctgtat ctctgtcaaa aaaaaaaaaa aaaaaa 2626

<210> 75
<211> 3337
<212> DNA
<213> Homo sapiens

<400> 75
gtcgagcctc tagcccgccc gggtttcctt cgcagtcgcg caccgacgct caaacgcgcg 60

ctccaacccg cagcctcctc ctgcctcacc gcccgaagat ggcggctctc aaactcctct 120

cctccgggct tcggctctgc gcctctgccc gcggatctgg ggcaacctgg tacaagggat 180

gtgtttgttc cttttccacc agtgctcatc gccataccaa gttttataca gatccagtag 240

aagctgtaaa agacatccct gatggtgcca cggttttggt tggtggtttt gggctatgtg 300

gaattccaga gaatcttata gatgctttac tgaaaactgg agtaaaagga ctaactgcag 360

tcagcaacaa tgcaggggtt gacaattttg gtttggggct tttgcttcgg tcgaagcaga 420

taaaacgcat ggtctcttca tatgtgggag aaaatgcaga atttgaacga cagtacttat 480
ctggtgaatt agaagtggag ctgacaccac agggcacact tgcagagagg atccgtgcag 540
gcggggctgg agttcctgca ttttacaccc caacagggta tgggaccctg gtacaagaag 600
gaggatcgcc catcaaatac aacaaagatg gcagtgttgc cattgccagt aagccaagag 660
aggtgaggga gttcaatggt cagcacttta ttttggagga agcaattaca ggggattttg 720
ctttggtgaa agcctggaag gcggaccgag caggaaacgt gattttcagg aaaagtgcaa 780
ggaatttcaa cttgccaatg tgcaaagctg cagaaaccac agtggtagag gttgaagaaa 840
ttgtggatat tggagcattt gctccagaag acatccatat tcctcagatt tatgtacatc 900
gccttataaa gggagaaaaa tatgagaaaa gaattgagcg tttatcaatc cggaaagagg 960
gagatgggga agccaaatct gctaaacctg gagatgacgt aagggaacga atcatcaaga 1020
gggccgctct tgagtttgag gatggcatgt atgctaattt gggcatagga atccctctcc 1080
tggccagcaa ttttatcagc ccaaatataa ctgttcatct tcaaagtgaa aatggagttc 1140
tgggtttggg tccatatcca cgacaacatg aagctgatgc agatctcatc aatgcaggca 1200
aggaaacagt tactattctt ccaggagcct ctttttctc cagcgatgaa tcatttgcaa 1260
tgattagagg tggacacgtc gatctgacaa tgctaggagc gatgcaggtt tccaaatatg 1320
gtgacctggc taactggatg atacctggga agatggtgaa aggaatggga ggtgctatgg 1380
atttagtgtc cagtgcgaaa accaaagtgg tggtcaccat ggagcattct gcaaagggaa 1440
atgcacataa aatcatggag aaatgtacat taccattgac tggaaagcaa tgtgtcaacc 1500
gcattattac tgaaaaggct gtgtttgatg tggacaagaa gaaagggttg actctgattg 1560
agctctggga aggcctgaca gtggatgacg tacaaaagag tactgggtgt gattttgcag 1620
tttcaccaaa actcatgcca atgcagcaga tcgcaaattg aaatatggat atttgtacca 1680
ggctgcgtgt ttttcatttt aaacacacaa gatttaattg aaaggacatc aataatcata 1740
attgtgtatt taacaggtgg ttttttatta gttttcttgt gtttcagact ttatgcagcc 1800
atataaactg ttctctaggc atgctgtgac attttaataa aaagcaaaag gagcatttat 1860
aattatctca tttgttaagg ctgagaaggt tgtttttata ataggtaatt atattgaatg 1920
cattttcact gaatatggta tgtatgctaa attatatgaa cctttcccca agaagggccc 1980
tagaaattga tgtggctttc ctcttaaata ttaattatta gtcctgaaag aaagataaca 2040
tatgtgattt ttgtggttag gagagttgct gtcatgattg ttttttcttc agcctcctct 2100
gacttttctt ttggggcttc agattttatg attacatctt gtccccctag aacatccccc 2160
ttcctcccat actgctttta aacagatgcc caagaaggca agcaggaatg cctcttgtgg 2220
gggagggcag ggagaaataa ctagttcaaa ccaactatct atctatgctt tgcaaagact 2280

```
aaggcgtatt ataggaagag ggctagaaac ctaactgatt cttctcagtt ttctcatttt   2340

aaaacagccc agtattcctt tgtatcctca agggtccttg agaatacttc tgttattgaa   2400

accctgtggg ctacttgtac tgtacctcct ctcaagccaa gaagggctgt gggataattt   2460

accatgaatc cttagtagca atgacagcag agttaaaaaa taaaaggtgt tttactttca   2520

ggctcttgtt ttggttcaga ggagatttta aatattgaat gacacttcta cagaacaacg   2580

gtttttcttc tgccaaggct acttccttta acgaagtgcc tttaattcag ccttatccaa   2640

ctagggaaaa taatgttgga caagtctagg atttgaagag tcagtgaact tttagtgtca   2700

gggaataaac atggtgggta gattaggttt gaaaaaaact tccttagagg tatttattct   2760

caatacctga caggggccca tgggaatgac ttcagaagca tcccggataa tagatgggta   2820

aaaagtctag gcaccctgaa gaacaggtga gacagctggc ctctggacag aggtaggcat   2880

agtacagtac gatatatcat tcctctggtc ctaaatatac aaacttattc atgtttttag   2940

gtgatgatgg tcattgaaac tcacttcttt tcaggtgtag ctacaattgt gtaatgtaca   3000

atattagaga aaggacaggc tttttatgag taacacacac catatataaa acagcctttc   3060

tggctgacca catggttaaa tgcatacctt cccagtactg gggggaaaat gacccttctt   3120

agaatgtgca agttccatag agtaatatat tgatatgatt ttgaaaagaa ttgttgatag   3180

ttacatcttc aaacttatca ttccagtatg catctttaag ataatgtgat tctaagtaga   3240

tgactttata ttcttgatta aagagtgcta tacatgttaa gaaatgcatt aaggaataca   3300

ataaatattc taaactgatg aaaaaaaaaa aaaaaaa                            3337


<210>  76
<211>  2460
<212>  DNA
<213>  Homo sapiens

<400>  76
aaagtcaaac cccgacaccg cggcgggccg gtgagctcac tagctgaccc ggcaggtcag     60

gatctggctt agcggcgccg cgagctccag tgcgcgcacc cgtggccgcc tcccagccct    120

ctttgccgga cgagctctgg gccgccacaa gactaaggaa tggccacccc gcccaagaga    180

agctgcccgt ctttctcagc cagctctgag ggacccgca  tcaagaaaat ctccatcgaa    240

gggaacatcg ctgcagggaa gtcaacattt gtgaatatcc ttaaacaatt gtgtgaagat    300

tgggaagtgg ttcctgaacc tgttgccaga tggtgcaatg ttcaaagtac tcaagatgaa    360

tttgaggaac ttacaatgtc tcagaaaaat ggtgggaatg ttcttcagat gatgtatgag    420

aaacctgaac gatggtcttt taccttccaa acatatgcct gtctcagtcg aataagagct    480

cagcttgcct ctctgaatgg caagctcaaa gatgcagaga aacctgtatt atttttgaa     540
```

cgatctgtgt atagtgacag gtatattttt gcatctaatt tgtatgaatc tgaatgcatg 600
aatgagacag agtggacaat ttatcaagac tggcatgact ggatgaataa ccaatttggc 660
caaagccttg aattggatgg aatcatttat cttcaagcca ctccagagac atgcttacat 720
agaatatatt tacggggaag aaatgaagag caaggcattc ctcttgaata tttagagaag 780
cttcattata aacatgaaag ctggctcctg cataggacac tgaaaaccaa cttcgattat 840
cttcaagagg tgcctatctt aacactggat gttaatgaag actttaaaga caaatatgaa 900
agtctggttg aaaaggtcaa agagtttttg agtactttgt gatcttgctg aagactacag 960
gcagccaaat ggttccagat acttcagctt tgtgtatctt cgtaacttca tattaatata 1020
agtttcttta gaaaacccaa gtttttaatc gtttttgttt taaggaaaaa agatttttaa 1080
aatgaatctt atgcaaaact tttgatcag tttcttttct tttgtttttt ttttaaaaaa 1140
gacatttaaa gacaaagaca ttatttctca tagcaggaaa tgtagaggta gatggttcca 1200
gtatcagcat agtgactaaa ctacattata aaagatccag cttccttctg tcattcccct 1260
cttttgtctt cctcagcagg ttggcttttt tccctggtgc ctctcacttc gttggtgacc 1320
agtttcttaa actgaaagct ttaatgttac atagtaaatg gtagtgtgtc ctgtgtaaat 1380
tagtgtacct attaaaagtt gcaaagtgga attaaaggaa tccctagaat aaggattctg 1440
aagttttatt ttaaattatt atcttcttaa cagtttagtc ccacctctta cttcctgcct 1500
cagtctgctt tctctactgt ctggattaat taggcagcct gctataaagt taaagtcaca 1560
catttctatt ttgcaaacac tgtgattact ctttgctttg tagtttgctt tgctttgtag 1620
ggttctgctt ttaagttttt ctctttttca gacaaattac tgataaaaat gatattgctc 1680
tatatgtaat atatcctgaa agcattattt tttgttgaat aggaaataaa attaatgaag 1740
acagaggcta gaaagcatcc attaattaat gagacacact taactactta tctctaaacc 1800
atctatgtga atatttgtaa aaataatgaa tggactcatc ttagttctgt atataaatat 1860
attttctttc tagtttgttt agttaaggtg tgcagtgttt ttcctgtgta ttaaaccttt 1920
ccattttacg ttttagaaaa ttttatgtat tttaaaataa ggggaagagt cattttcacc 1980
tttaaactac tatttttctt tccaagtcat ttttgttttt ggtttcttat tcaaagatga 2040
taatttagtg gattaaccag tccagacgca ctgatctttg caaaggagac ttaatttcaa 2100
atctgtaatt accatacata aactgtctca ttatacgtat gcattttttt agtttgtttt 2160
tgtttggtat aaattaattt gttaattaaa tatttcttaa gtataaacct tatgaactac 2220
agtggagcta cactcattga aatgtaattt cagttctaaa aagatgtaat aatcatttta 2280
gaattaaaat ttattctact tttaaataaa ttatgaatat taaaggtgaa aattgtataa 2340

attactttga ttccatttta agtggagaca tatttcagtg atttttagta acctttaaaa 2400

atgtataatg acttttaaaa tttgtagaat tgaaaagacg ctaataaaaa tttattattt 2460

<210> 77
<211> 7680
<212> DNA
<213> Homo sapiens

<400> 77
gcggacactc ctctcggctc ctccccggca gcggcggcgg ctcggagcgg gctccggggc 60

tcgggtgcag cggccagcgg gcctggcggc gaggattacc cggggaagtg gttgtctcct 120

ggctggagcc gcgagacggg cgctcagggc gcggggccgg cggcggcgaa cgagaggacg 180

gactctggcg gccgggtcgt tggccggggg agcgcgggca ccgggcgagc aggccgcgtc 240

gcgctcacca tggtcagcta ctgggacacc ggggtcctgc tgtgcgcgct gctcagctgt 300

ctgcttctca caggatctag ttcaggttca aaattaaaag atcctgaact gagtttaaaa 360

ggcacccagc acatcatgca agcaggccag acactgcatc tccaatgcag gggggaagca 420

gcccataaat ggtctttgcc tgaaatggtg agtaaggaaa gcgaaaggct gagcataact 480

aaatctgcct gtggaagaaa tggcaaacaa ttctgcagta ctttaacctt gaacacagct 540

caagcaaacc acactggctt ctacagctgc aaatatctag ctgtacctac ttcaaagaag 600

aaggaaacag aatctgcaat ctatatattt attagtgata caggtagacc tttcgtagag 660

atgtacagtg aaatccccga aattatacac atgactgaag gaagggagct cgtcattccc 720

tgccgggtta cgtcacctaa catcactgtt actttaaaaa agtttccact tgacactttg 780

atccctgatg gaaaacgcat aatctgggac agtagaaagg gcttcatcat atcaaatgca 840

acgtacaaag aaatagggct tctgacctgt gaagcaacag tcaatgggca tttgtataag 900

acaaactatc tcacacatcg acaaaccaat acaatcatag atgtccaaat aagcacacca 960

cgcccagtca aattacttag aggccatact cttgtcctca attgtactgc taccactccc 1020

ttgaacacga gagttcaaat gacctggagt taccctgatg aaaaaaataa gagagcttcc 1080

gtaaggcgac gaattgacca aagcaattcc catgccaaca tattctacag tgttcttact 1140

attgacaaaa tgcagaacaa agacaaagga ctttatactt gtcgtgtaag gagtggacca 1200

tcattcaaat ctgttaacac ctcagtgcat atatatgata aagcattcat cactgtgaaa 1260

catcgaaaac agcaggtgct tgaaaccgta gctggcaagc ggtcttaccg gctctctatg 1320

aaagtgaagg catttccctc gccggaagtt gtatggttaa aagatgggtt acctgcgact 1380

gagaaatctg ctcgctattt gactcgtggc tactcgttaa ttatcaagga cgtaactgaa 1440

gaggatgcag ggaattatac aatcttgctg agcataaaac agtcaaatgt gtttaaaaac 1500

ctcactgcca ctctaattgt caatgtgaaa ccccagattt acgaaaaggc cgtgtcatcg 1560
tttccagacc cggctctcta cccactgggc agcagacaaa tcctgacttg taccgcatat 1620
ggtatccctc aacctacaat caagtggttc tggcacccct gtaaccataa tcattccgaa 1680
gcaaggtgtg acttttgttc caataatgaa gagtccttta tcctggatgc tgacagcaac 1740
atgggaaaca gaattgagag catcactcag cgcatggcaa taatagaagg aaagaataag 1800
atggctagca ccttggttgt ggctgactct agaatttctg gaatctacat ttgcatagct 1860
tccaataaag ttgggactgt gggaagaaac ataagctttt atatcacaga tgtgccaaat 1920
gggtttcatg ttaacttgga aaaaatgccg acggaaggag aggacctgaa actgtcttgc 1980
acagttaaca agttcttata cagagacgtt acttggattt tactgcggac agttaataac 2040
agaacaatgc actacagtat tagcaagcaa aaaatggcca tcactaagga gcactccatc 2100
actcttaatc ttaccatcat gaatgtttcc ctgcaagatt caggcaccta tgcctgcaga 2160
gccaggaatg tatacacagg ggaagaaatc ctccagaaga aagaaattac aatcagagat 2220
caggaagcac catacctcct gcgaaacctc agtgatcaca cagtggccat cagcagttcc 2280
accactttag actgtcatgc taatggtgtc cccgagcctc agatcacttg gtttaaaaac 2340
aaccacaaaa tacaacaaga gcctggaatt attttaggac caggaagcag cacgctgttt 2400
attgaaagag tcacagaaga ggatgaaggt gtctatcact gcaaagccac caaccagaag 2460
ggctctgtgg aaagttcagc atacctcact gttcaaggaa cctcggacaa gtctaatctg 2520
gagctgatca ctctaacatg cacctgtgtg gctgcgactc tcttctggct cctattaacc 2580
ctccttatcc gaaaaatgaa aaggtcttct tctgaaataa agactgacta cctatcaatt 2640
ataatggacc cagatgaagt tcctttggat gagcagtgtg agcggctccc ttatgatgcc 2700
agcaagtggg agtttgcccg ggagagactt aaactgggca aatcacttgg aagaggggct 2760
tttggaaaag tggttcaagc atcagcattt ggcattaaga aatcacctac gtgccggact 2820
gtggctgtga aaatgctgaa agaggggggcc acggccagcg agtacaaagc tctgatgact 2880
gagctaaaaa tcttgaccca cattggccac catctgaacg tggttaacct gctgggagcc 2940
tgcaccaagc aaggagggcc tctgatggtg attgttgaat actgcaaata tggaaatctc 3000
tccaactacc tcaagagcaa acgtgactta ttttttctca acaaggatgc agcactacac 3060
atggagccta agaaagaaaa aatggagcca ggcctggaac aaggcaagaa accaagacta 3120
gatagcgtca ccagcagcga aagctttgcg agctccggct ttcaggaaga taaaagtctg 3180
agtgatgttg aggaagagga ggattctgac ggtttctaca aggagcccat cactatggaa 3240
gatctgattt cttacagttt tcaagtggcc agaggcatgg agttcctgtc ttccagaaag 3300
tgcattcatc gggacctggc agcgagaaac attcttttat ctgagaacaa cgtggtgaag 3360

atttgtgatt ttggccttgc ccgggatatt tataagaacc ccgattatgt gagaaaagga 3420
gatactcgac ttcctctgaa atggatggct cccgaatcta tctttgacaa aatctacagc 3480
accaagagcg acgtgtggtc ttacggagta ttgctgtggg aaatcttctc cttaggtggg 3540
tctccatacc caggagtaca aatggatgag gacttttgca gtcgcctgag ggaaggcatg 3600
aggatgagag ctcctgagta ctctactcct gaaatctatc agatcatgct ggactgctgg 3660
cacagagacc caaaagaaag gccaagattt gcagaacttg tggaaaaact aggtgatttg 3720
cttcaagcaa atgtacaaca ggatggtaaa gactacatcc caatcaatgc catactgaca 3780
ggaaatagtg ggtttacata ctcaactcct gccttctctg aggacttctt caaggaaagt 3840
atttcagctc cgaagtttaa ttcaggaagc tctgatgatg tcagatatgt aaatgctttc 3900
aagttcatga gcctggaaag aatcaaaacc tttgaagaac ttttaccgaa tgccacctcc 3960
atgtttgatg actaccaggg cgacagcagc actctgttgg cctctcccat gctgaagcgc 4020
ttcacctgga ctgacagcaa acccaaggcc tcgctcaaga ttgacttgag agtaaccagt 4080
aaaagtaagg agtcggggct gtctgatgtc agcaggccca gtttctgcca ttccagctgt 4140
gggcacgtca gcgaaggcaa gcgcaggttc acctacgacc acgctgagct ggaaaggaaa 4200
atcgcgtgct gctccccgcc cccagactac aactcggtgg tcctgtactc caccccaccc 4260
atctagagtt tgacacgaag ccttatttct agaagcacat gtgtatttat acccccagga 4320
aactagcttt tgccagtatt atgcatatat aagtttacac ctttatcttt ccatgggagc 4380
cagctgcttt ttgtgatttt tttaatagtg cttttttttt ttgactaaca agaatgtaac 4440
tccagataga gaaatagtga caagtgaaga acactactgc taaatcctca tgttactcag 4500
tgttagagaa atccttccta aacccaatga cttccctgct ccaacccccg ccacctcagg 4560
gcacgcagga ccagtttgat tgaggagctg cactgatcac ccaatgcatc acgtacccca 4620
ctgggccagc cctgcagccc aaaacccagg gcaacaagcc cgttagcccc aggggatcac 4680
tggctggcct gagcaacatc tcgggagtcc tctagcaggc ctaagacatg tgaggaggaa 4740
aaggaaaaaa agcaaaaagc aagggagaaa agagaaaccg ggagaaggca tgagaaagaa 4800
tttgagacgc accatgtggg cacggagggg gacggggctc agcaatgcca tttcagtggc 4860
ttcccagctc tgacccttct acatttgagg gcccagccag gagcagatgg acagcgatga 4920
ggggacattt tctggattct gggaggcaag aaaaggacaa atatcttttt tggaactaaa 4980
gcaaatttta gacctttacc tatggaagtg gttctatgtc cattctcatt cgtggcatgt 5040
tttgatttgt agcactgagg gtggcactca actctgagcc catacttttg gctcctctag 5100
taagatgcac tgaaaactta gccagagtta ggttgtctcc aggccatgat ggccttacac 5160

tgaaaatgtc acattctatt ttgggtatta atatatagtc cagacactta actcaatttc 5220
ttggtattat tctgttttgc acagttagtt gtgaaagaaa gctgagaaga atgaaaatgc 5280
agtcctgagg agagttttct ccatatcaaa acgagggctg atggaggaaa aaggtcaata 5340
aggtcaaggg aagaccccgt ctctatacca accaaaccaa ttcaccaaca cagttgggac 5400
ccaaaacaca ggaagtcagt cacgtttcct tttcatttaa tggggattcc actatctcac 5460
actaatctga aaggatgtgg aagagcatta gctggcgcat attaagcact ttaagctcct 5520
tgagtaaaaa ggtggtatgt aatttatgca aggtatttct ccagttggga ctcaggatat 5580
tagttaatga gccatcacta gaagaaaagc ccattttcaa ctgctttgaa acttgcctgg 5640
ggtctgagca tgatgggaat agggagacag ggtaggaaag ggcgcctact cttcagggtc 5700
taaagatcaa gtgggccttg gatcgctaag ctggctctgt ttgatgctat ttatgcaagt 5760
tagggtctat gtatttagga tgcgcctact cttcagggtc taaagatcaa gtgggccttg 5820
gatcgctaag ctggctctgt ttgatgctat ttatgcaagt tagggtctat gtatttagga 5880
tgtctgcacc ttctgcagcc agtcagaagc tggagaggca acagtggatt gctgcttctt 5940
ggggagaaga gtatgcttcc ttttatccat gtaatttaac tgtagaacct gagctctaag 6000
taaccgaaga atgtatgcct ctgttcttat gtgccacatc cttgtttaaa ggctctctgt 6060
atgaagagat gggaccgtca tcagcacatt ccctagtgag cctactggct cctggcagcg 6120
gcttttgtgg aagactcact agccagaaga gaggagtggg acagtcctct ccaccaagat 6180
ctaaatccaa acaaaagcag gctagagcca gaagagagga caaatctttg ttgttcctct 6240
tctttacaca tacgcaaacc acctgtgaca gctggcaatt ttataaatca ggtaactgga 6300
aggaggttaa actcagaaaa aagaagacct cagtcaattc tctacttttt tttttttttt 6360
tccaaatcag ataatagccc agcaaatagt gataacaaat aaaaccttag ctgttcatgt 6420
cttgatttca ataattaatt cttaatcatt aagagaccat aataaatact ccttttcaag 6480
agaaaagcaa aaccattaga attgttactc agctccttca aactcaggtt tgtagcatac 6540
atgagtccat ccatcagtca aagaatggtt ccatctggag tcttaatgta gaaagaaaaa 6600
tggagacttg taataatgag ctagttacaa agtgcttgtt cattaaaata gcactgaaaa 6660
ttgaaacatg aattaactga taatattcca atcatttgcc atttatgaca aaaatggttg 6720
gcactaacaa agaacgagca cttcctttca gagtttctga gataatgtac gtggaacagt 6780
ctgggtggaa tggggctgaa accatgtgca agtctgtgtc ttgtcagtcc aagaagtgac 6840
accgagatgt taattttagg gacccgtgcc ttgtttccta gcccacaaga atgcaaacat 6900
caaacagata ctcgctagcc tcatttaaat tgattaaagg aggagtgcat ctttggccga 6960
cagtggtgta actgtgtgtg tgtgtgtgtg tgtgtgtgtg tgtgtgtgtg tgtgggtgtg 7020

ggtgtatgtg tgttttgtgc ataactattt aaggaaactg gaattttaaa gttactttta 7080

tacaaaccaa gaatatatgc tacagatata agacagacat ggtttggtcc tatatttcta 7140

gtcatgatga atgtattttg tataccatct tcatataata tacttaaaaa tatttcttaa 7200

ttgggatttg taatcgtacc aacttaattg ataaacttgg caactgcttt tatgttctgt 7260

ctccttccat aaatttttca aaatactaat tcaacaaaga aaaagctctt ttttttccta 7320

aaataaactc aaatttatcc ttgtttagag cagagaaaaa ttaagaaaaa ctttgaaatg 7380

gtctcaaaaa attgctaaat attttcaatg gaaaactaaa tgttagttta gctgattgta 7440

tggggttttc gaacctttca ctttttgttt gttttaccta tttcacaact gtgtaaattg 7500

ccaataattc ctgtccatga aaatgcaaat tatccagtgt agatatattt gaccatcacc 7560

ctatggatat tggctagttt tgcctttatt aagcaaattc atttcagcct gaatgtctgc 7620

ctatatattc tctgctcttt gtattctcct ttgaacccgt taaaacatcc tgtggcactc 7680

<210> 78
<211> 3160
<212> DNA
<213> Homo sapiens

<400> 78
attctcgccg cgcccgggcg gacgatccag cgaacagccc cgcttctaac ccgagatgct 60

gctgccggcg cccgcgctcc gccgcgccct gctgtcccgc ccctggaccg gggccggcct 120

gcggtggaag cacacctcct ccctgaaggt ggccaacgag cccgtcttag ccttcacgca 180

gggcagccct gagcgagatg ccctgcaaaa ggccttgaag gacctgaagg gccggatgga 240

agccatccca tgcgtggtgg gggatgagga ggtgtggacg tcggacgtgc agtaccaagt 300

gtcgcctttt aaccatggac ataaggtggc caagttctgt tatgcagaca agagcctgct 360

caacaaagcc attgaggctg ccctggctgc ccggaaagag tgggacctga agcctattgc 420

agaccgggcc cagatcttcc tgaaggcggc agacatgctg agtgggccgc gcagggctga 480

gatcctcgcc aagaccatgg tgggacaggg taagaccgtg atccaagcgg agattgacgc 540

tgcagcggaa ctcatcgact tcttccggtt caatgccaag tatgcggtgg agctggaggg 600

gcagcagccc atcagcgtgc cccgagcac caacagcacg gtgtaccggg gtctggaggg 660

cttcgtggcg gccatctcgc cctttaactt cactgcaatc ggcggcaacc tggcgggggc 720

accggccctg atgggcaacg tggtcctatg gaagcccagt gacactgcca tgctggccag 780

ctatgctgtc taccgcatcc ttcgggaggc tggcctgccc cccaacatca tccagtttgt 840

gccagctgat gggcccctat ttggggacac tgtcaccagc tcagagcacc tctgtggcat 900

caacttcaca ggcagtgtgc ccaccttcaa acacctgtgg aagcaggtgg cccagaacct 960

ggaccggttc cacaccttcc cacgcctggc tggagagtgc ggcggaaaga acttccactt 1020
cgtgcaccgc tcggccgacg tggagagcgt ggtgagcggg accctccgct cagccttcga 1080
gtacggtggc cagaagtgtt ccgcctgctc gcgtctctac gtgccgcact cgctgtggcc 1140
gcagatcaaa gggcggctgc tggaggagca cagtcggatc aaagtgggcg accctgcaga 1200
ggattttggg accttcttct ctgcagtgat tgatgccaag tcctttgccc gtatcaagaa 1260
gtggctggag cacgcgcgct cctcgcccag cctcaccatc ctggctgggg gcaagtgtga 1320
tgactccgtg ggctactttg tggagccctg catcgtggag agcaaggacc ctcaggagcc 1380
catcatgaag gaggagatct tcgggcctgt actgtctgtg tacgtctacc cggacgacaa 1440
gtacaaggag acgctgcagc tggttgacag caccaccagc tatggcctca cgggggcagt 1500
gttctcccag gataaggacg tcgtgcagga ggccacaaag gtgctgagga atgctgccgg 1560
caacttctac atcaacgaca agtccactgg ctcgatagtg ggccagcagc cctttggggg 1620
ggcccgagcc tctggaacca atgacaagcc aggggggccca cactacatcc tgcgctggac 1680
gtcgccgcag gtcatcaagg agacacataa gcccctgggg gactggagct acgcgtacat 1740
gcagtgagcc cctctcgggc tccaccgtcc agctgtctgt ccgtccaggt ggccgacctc 1800
actgcacaga ccccactcca gcccctccac cccttcttca tgcacagctg cctttctata 1860
atccgggctt gactcccttc ttaccactgt attctggcct ctcccatgcc tcaggctctg 1920
gtttgagatc gtgctgggga ggaacatggc cactacccct tatcccatcg gccatgtggg 1980
aggtatgacc ctggtgcctg gcaggttctc cctctgccct ccactgggcc cagtggctca 2040
gggacctggg gaaaggagat ggagcagctc ttgggatcct ttggggaaaa ggaggccatt 2100
ctgggcccct tggcaaacct caccactcac agaggctcct ggccttgatc cctgcccctc 2160
caggtgtcca gggtaaagtg taactcagac tgacctgtgg ggcacagggg gcaccagctg 2220
gccttgccct ctctggtctg ggctgtctac cttcctcact gtatctttgc ccagacccac 2280
ctgggccagt aggcccctgt ccccagccac acaccttaga tgctggcatg ccttactcca 2340
ggtgcctgtg tttggccgag gcctgtgtga ttcccggtct gcaccacatg gcggggttgg 2400
ggggccgctg gaggccacct gccaaggcgt gggatgggat ggtcctgccg gtttaggccg 2460
tgattctgga aaaccttgga tgggccttcg tcctatgtca gccttccctt tgatcctcag 2520
gccctacctg tagagacctc cactcctaga gccagtctca gggtctggga tttccctgca 2580
ggagctcagc caccactgtg ccatggtgac acaggccaag gcagacattg gccctccctt 2640
ctcccagccc ccagaggcct ggccttgggt tcgtcagcat gggccgagga cgttgcctgt 2700
agaatcctcc tctgcctggg agtggctctg tgtggaccag tccctcactg gcccattctt 2760

tttttgacgc agccaatctg tgaccacgat tcctcccaca gatgcctcct gcttggattc 2820

tgagtggtca gagatctgta aagcatgact ttcaaggatg gttcttaggg gactgtgaaa 2880

gtgttgggtc ttcctccagg atgcctgcat gggaccccac ccggagctgg tgtggccatt 2940

ccccaagtgc cactggccca tggatggggg tgggtgctgg tgccagctgg gctgggtgtg 3000

ggttctgtgt ccttccagga tatgtgtcat ttcccatgag ggccggggc aggtggctgg 3060

gtgggggcac aggctggagt attcttagtt ctactggttc tacactgtga ggtggcaatg 3120

ggatttgctc agatgccacc caataaaatg cctgttactt 3160

<210> 79
<211> 1546
<212> DNA
<213> Homo sapiens

<400> 79
cacggccgga gagacgcgga ggaggagaca tgagccggcg ggcgcccaga cggagcggcc 60

gtgacgcttt cgcgctgcag ccgcgcgccc cgaccccgga gcgctgaccc ctggccccac 120

gcagctccgc gcccgggccg gagagcgcaa ctcggcttcc agacccgccg cgcatgctgt 180

ccccggactg agccgggcag ccagcctccc acggacgccc ggacggccgg ccggccagca 240

gtgagcgagc ttccccgcac cggccaggcg cctcctgcac agcggctgcc gccccgcagc 300

ccctgcgcca gcccggaggg cgcagcgctc gggaggagcc gcgcggggcg ctgatgccgc 360

agggcgcgcc gcggagcgcc ccggagcagc agagtctgca gcagcagcag ccggcgagga 420

gggagcagca gcagcggcgg cggcggcggc ggcggcggcg gaggcgcccg gtcccggccg 480

cgcggagcgg acatgtgcag gctgggctag gagccgccgc ctccctcccg cccagcgatg 540

tattcagcgc cctccgcctg cacttgcctg tgtttacact tcctgctgct gtgcttccag 600

gtacaggtgc tggttgccga ggagaacgtg gacttccgca tccacgtgga gaaccagacg 660

cgggctcggg acgatgtgag ccgtaagcag ctgcggctgt accagctcta cagccggacc 720

agtgggaaac acatccaggt cctgggccgc aggatcagtg cccgcggcga ggatggggac 780

aagtatgccc agctcctagt ggagacagac accttcggta gtcaagtccg gatcaagggc 840

aaggagacgg aattctacct gtgcatgaac cgcaaaggca agctcgtggg gaagcccgat 900

ggcaccagca aggagtgtgt gttcatcgag aaggttctgg agaacaacta cacggccctg 960

atgtcggcta agtactccgg ctggtacgtg ggcttcacca agaaggggcg gccgcggaag 1020

ggccccaaga cccgggagaa ccagcaggac gtgcatttca tgaagcgcta ccccaagggg 1080

cagccggagc ttcagaagcc cttcaagtac acgacggtga ccaagaggtc ccgtcggatc 1140

cggcccacac accctgccta ggccaccccg ccgcggcccc tcaggtcgcc ctggccacac 1200

tcacactccc agaaaactgc atcagaggaa tatttttaca tgaaaaataa ggaagaagct 1260

ctatttttgt acattgtgtt taaaagaaga caaaaactga accaaaactc ttgggggag 1320

gggtgataag gattttattg ttgacttgaa acccccgatg acaaaagact cacgcaaagg 1380

gactgtagtc aacccacagg tgcttgtctc tctctaggaa cagacaactc taaactcgtc 1440

cccagaggag gacttgaatg aggaaaccaa cactttgaga aaccaaagtc ctttttccca 1500

aaggttctga aaggaaaaaa aaaaaaaaac aaaaaaaaaa aaaaaa 1546

<210> 80
<211> 2819
<212> DNA
<213> Homo sapiens

<400> 80
ctgggcccag ctcccccgag aggtggtcgg atcctctggg ctgctcggtc gatgcctgtg 60

ccactgacgt ccaggcatga ggtggttcct gccctggacg ctggcagcag tgacagcagc 120

agccgccagc accgtcctgg ccacggccct ctctccagcc cctacgacca tggactttac 180

cccagctcca ctggaggaca cctcctcacg cccccaattc tgcaagtggc catgtgagtg 240

cccgccatcc ccaccccgct gcccgctggg ggtcagcctc atcacagatg gctgtgagtg 300

ctgtaagatg tgcgctcagc agcttgggga caactgcacg gaggctgcca tctgtgaccc 360

ccaccggggc ctctactgtg actacagcgg ggaccgcccg aggtacgcaa taggagtgtg 420

tgcacaggtg gtcggtgtgg gctgcgtcct ggatggggtg cgctacaaca acggccagtc 480

cttccagcct aactgcaagt acaactgcac gtgcatcgac ggcgcggtgg gctgcacacc 540

actgtgcctc cgagtgcgcc ccccgcgtct ctggtgcccc cacccgcggc gcgtgagcat 600

acctggccac tgctgtgagc agtgggtatg tgaggacgac gccaagaggc cacgcaagac 660

cgcacccgt gacacaggag ccttcgatgc tgtgggtgag gtggaggcat ggcacaggaa 720

ctgcatagcc tacacaagcc cctggagccc ttgctccacc agctgcggcc tgggggtctc 780

cactcggatc tccaatgtta acgcccagtg ctggcctgag caagagagcc gcctctgcaa 840

cttgcggcca tgcgatgtgg acatccatac actcattaag gcagggaaga agtgtctggc 900

tgtgtaccag ccagaggcat ccatgaactt cacacttgcg ggctgcatca gcacacgctc 960

ctatcaaccc aagtactgtg gagtttgcat ggacaatagg tgctgcatcc cctacaagtc 1020

taagactatc gacgtgtcct tccagtgtcc tgatgggctt ggcttctccc gccaggtcct 1080

atggattaat gcctgcttct gtaacctgag ctgtaggaat cccaatgaca tctttgctga 1140

cttggaatcc taccctgact tctcagaaat tgccaactag gcaggcacaa atcttgggtc 1200

ttggggacta acccaatgcc tgtgaagcag tcagccctta tggccaataa cttttcacca 1260

atgagcctta gttaccctga tctggaccct tggcctccat ttctgtctct aaccattcaa 1320
atgacgcctg atggtgctgc tcaggcccat gctatgagtt ttctccttga tatcattcag 1380
catctactct aaagaaaaat gcctgtctct agctgttctg gactacaccc aagcctgatc 1440
cagcctttcc aagtcactag aagtcctgct ggatcttgcc taaatcccaa gaaatggaat 1500
caggtagact tttaatatca ctaatttctt ctttagatgc caaaccacaa gactctttgg 1560
gtccattcag atgaatagat ggaatttgga acaatagaat aatctattat ttggagcctg 1620
ccaagaggta ctgtaatggg taattctgac gtcagcgcac caaaactatc ctgattccaa 1680
atatgtatgc acctcaaggt catcaaacat ttgccaagtg agttgaatag ttgcttaatt 1740
ttgattttta atggaaagtt gtatccatta acctgggcat tgttgaggtt aagtttctct 1800
tcacccctac actgtgaagg gtacagatta ggtttgtccc agtcagaaat aaaatttgat 1860
aaacattcct gttgatggga aaagccccca gttaatactc cagagacagg gaaaggtcag 1920
cccgtttcag aaggaccaat tgactctcac actgaatcag ctgctgactg gcagggcttt 1980
gggcagttgg ccaggctctt ccttgaatct tctcccttgt cctgcttggg gttcatagga 2040
attggtaagg cctctggact ggcctgtctg gcccctgaga gtggtgccct ggaacactcc 2100
tctactctta cagagccttg agagacccag ctgcagacca tgccagaccc actgaaatga 2160
ccaagacagg ttcaggtagg ggtgtgggtc aaaccaagaa gtgggtgccc ttggtagcag 2220
cctggggtga cctctagagc tggaggctgt gggactccag gggcccccgt gttcaggaca 2280
catctattgc agagactcat ttcacagcct ttcgttctgc tgaccaaatg gccagttttc 2340
tggtaggaag atggaggttt accggttgtt tagaaacaga aatagactta ataaaggttt 2400
aaagctgaag aggttgaagc taaaaggaaa aggttgttgt taatgaatat caggctatta 2460
tttattgtat taggaaaata taatatttac tgttagaatt cttttattta gggccttttc 2520
tgtgccagac attgctctca gtgctttgca tgtattagct cactgaatct tcacgacaat 2580
gttgagaagt tcccattatt atttctgttc ttacaaatgt gaaacggaag ctcatagagg 2640
tgagaaaact caaccagagt cacccagttg gtgactggga aagttaggat tcagatcgaa 2700
attggactgt ctttataacc catattttcc ccctgttttt agagcttcca aatgtgtcag 2760
aataggaaaa cattgcaata aatggcttga ttttttaaaa aaaaaaaaaa aaaaaaaaa 2819

<210> 81
<211> 2584
<212> DNA
<213> Homo sapiens

<400> 81
tggcggcggc ggcggcggtt gtcccggctg tgccggttgg tgtggcccgt cagcccgcgt 60

accacagcgc ccgggccgcg tcgagcccag tacagccaag ccgctgcggc cgggtccggc 120
gcgggcggcg cgcgcagacg gagggcggcg gccgcggcca gggcggcccg tgggaccgcg 180
ggcccccggc gcagcgctgc ccggctcccg gccctgccgg cctcctccct tggcgccgcg 240
gccatggcgg ccagcgcgaa gcggaagcag gaggagaagc acctgaagat gctgcgggac 300
atgaccggcc tcccgcgcaa ccgaaagtgc ttcgactgcg accagcgcgg ccccacctac 360
gttaacatga cggtcggctc cttcgtgtgt acctcctgct ccggcagcct gcgaggatta 420
aatccaccac acagggtgaa atctatctcc atgacaacat tcacacaaca ggaaattgaa 480
ttcttacaaa aacatggaaa tgaagtctgt aaacagattt ggctaggatt atttgatgat 540
agatcttcag caattccaga cttcagggat ccacaaaaag tgaaagagtt tctacaagaa 600
aagtatgaaa agaaaagatg gtatgtcccg ccagaacaag ccaaagtcgt ggcatcagtt 660
catgcatcta tttcagggtc ctctgccagt agcacaagca gcacacctga ggtcaaacca 720
ctgaaatctc ttttagggga ttctgcacca acactgcact taaataaggg cacacctagt 780
cagtccccag ttgtaggtcg ttctcaaggg cagcagcagg agaagaagca atttgacctt 840
ttaagtgatc tcggctcaga catctttgct gctccagctc ctcagtcaac agctacagcc 900
aattttgcta actttgcaca tttcaacagt catgcagctc agaattctgc aaatgcagat 960
tttgcaaact ttgatgcatt tggacagtct agtggttcga gtaattttgg aggtttcccc 1020
acagcaagtc actctccttt tcagccccaa actacaggtg gaagtgctgc atcagtaaat 1080
gctaattttg ctcattttga taacttcccc aaatcctcca gtgctgattt tggaaccttc 1140
aatacttccc agagtcatca aacagcatca gctgttagta aagtttcaac gaacaaagct 1200
ggtttacaga ctgcagacaa atatgcagca cttgctaatt tagacaatat cttcagtgcc 1260
gggcaaggtg gtgatcaggg aagtggcttt gggaccacag gtaaagctcc tgttggttct 1320
gtggtttcag ttcccagtca gtcaagtgca tcttcagaca agtatgcagc tctggcagaa 1380
ctagacagcg ttttcagttc tgcagccacc tccagtaatg cgtatacttc cacaagtaat 1440
gctagcagca atgtttttgg aacagtgcca gtggttgctt ctgcacagac acagcctgct 1500
tcatcaagtg tgcctgctcc atttggacgt acgccttcca caaatccatt tgttgctgct 1560
gctggtcctt ctgtggcatc ttctacaaac ccatttcaga ccaatgccag aggagcaaca 1620
gcggcaacct ttggcactgc atccatgagc atgcccacgg gattcggcac tcctgctccc 1680
tacagtcttc ccaccagctt tagtggcagc tttcagcagc ctgcctttcc agcccaagca 1740
gctttccctc aacagacagc tttttctcaa cagcccaatg gtgcaggttt tgcagcattt 1800
ggacaaacaa agccagtagt aacccctttt ggtcaagttg cagctgctgg agtatctagt 1860
aatcctttta tgactggtgc accaacagga caatttccaa caggaagctc atcaaccaat 1920

cctttcttat agccttatat agacaattta ctggaacgaa cttttatgtg gtcacattac 1980

atctctccac ctcttgcact gttgtcttgt ttcactgatc ttagctttaa acacaagaga 2040

agtctttaaa aagcctgcat tgtgtattaa acaccaggta atatgtgcaa aaccgagggc 2100

tccagtaaca ccttctaacc tgtgaattgg cagaaaaggg tagcggtatc atgtatatta 2160

aaattggcta atattaagtt attgcagata ccacattcat tatgctgcag tactgtacat 2220

atttttctta gaaattagct atttgtgcat atcagtattt gtaactttaa cacattgtta 2280

tgtgagaaat gttactgggg aaatagatca gccactttta aggtgctgtc atatatcttg 2340

gaatgaatga cctaaaatca ttttaaccat tgctactgga aagtaacaga gtcaaaattg 2400

gaaggtttta ttcattcttg aatttttcct ttctaaagag ctcttctatt tatacatgcc 2460

taaattcttt taaaatgtag agggatacct gtctgcataa taaagctgat catgttttgc 2520

tacagtttgc aggtgaaaaa aaataaatat tataaaataa aaaaaaaaaa aaagaaaaaa 2580

aaaa 2584

<210> 82
<211> 2115
<212> DNA
<213> Homo sapiens

<400> 82
gaaatgaacc tctcttattg atttttattg gcctagagcc aggagtactg cattcagttg 60

actttcaggg taaaaagaaa acagtcctgg ttgttgtcat cataaacata tggaccagtg 120

tgatggtgaa atgagatgag gctccgcaat ggaactgtag ccactgcttt agcatttatc 180

acttccttcc ttactttgtc ttggtatact acatggcaaa atgggaaaga aaaactgatt 240

gcttatcaac gagaattcct tgctttgaaa gaacgtcttc gaatagctga acacagaatc 300

tcacagcgct cttctgaatt aaatacgatt gtgcaacagt tcaagcgtgt aggagcagaa 360

acaaatggaa gtaaggatgc gttgaataag ttttcagata ataccctaaa gctgttaaag 420

gagttaacaa gcaaaaaatc tcttcaagtg ccaagtattt attatcattt gcctcattta 480

ttgaaaaatg aaggaagtct tcaacctgct gtacagattg gcaacggaag aacaggagtt 540

tcaatagtca tgggcattcc cacagtgaag agagaagtta aatcttacct catagaaact 600

cttcattccc ttattgataa cctgtatcct gaagagaagt tggactgtgt tatagtagtc 660

ttcataggag agacagatat tgattatgta catggtgttg tagccaacct ggagaaagaa 720

ttttctaaag aaatcagttc tggcttggtg gaagtcatat cacccccctga aagctattat 780

cctgacttga caaacctaaa ggagacattt ggagactcca aagaaagagt aagatggaga 840

acaaagcaaa acctagatta ctgttttcta atgatgtatg ctcaagaaaa gggcatatat 900

tacattcagc ttgaagatga tattattgtc aaacaaaatt attttaatac cataaaaaat 960

tttgcacttc aactttcttc tgaggaatgg atgattctag agttttccca gctgggcttc 1020

attggtaaaa tgtttcaagc gccggatctt actctgattg tagaattcat attcatgttt 1080

tacaaggaga aacccattga ttggctcctg gaccatattc tctgggtgaa agtctgcaac 1140

cctgaaaaag atgcaaaaca ttgtgataga cagaaagcaa atctgcgaat tcgcttcaga 1200

ccttcccttt tccaacatgt tggtctgcac tcatcactat caggaaaaat ccaaaaactc 1260

acggataaag attatatgaa accattactt cttaaaatcc atgtaaaccc acctgcggag 1320

gtatctactt ccttgaaggt ctaccaaggg catacgctgg agaaaactta catgggagag 1380

gatttcttct gggctatcac accgatagct ggagactaca tcttgtttaa atttgataaa 1440

ccagtcaatg tagaaagtta tttgttccat agcggcaacc aagaacatcc tggagatatt 1500

ctgctaaaca caactgtgga agttttgcct tttaagagtg aaggtttgga aataagcaaa 1560

gaaaccaaag acaaacgatt agaagatggc tatttcagaa taggaaaatt tgagaatggt 1620

gttgcagaag gaatggtgga tccaagtctc aatcccattt cagcctttcg actttcagtt 1680

attcagaatt ctgctgtttg ggccattctt aatgagattc atattaaaaa agccaccaac 1740

tgatcatctg agaaaccaac acatttttc ctgtgaattt gttaattaaa gatagttaag 1800

catgtatctt tttttattt ctacttgaac actacctctt gtgaagtcta ctgtagataa 1860

gacgattgtc atttccactt ggaaagtgaa tctcccataa taattgtatt tgtttgaaac 1920

taagctgtcc tcagatttta acttgactca aacatttttc aattatgaca gcctgttaat 1980

atgacttgta ctattttggt attatactaa tacataagag ttgtacatat tgttacattc 2040

tttaaatttg agaaaaacta atgttacata cattttatga aggggggtact tttgaggttc 2100

acttatttta ctatt 2115

<210> 83
<211> 1635
<212> DNA
<213> Homo sapiens

<400> 83
gggggtggcg gggacgcgag tggcggccgc ggggccccgg acaagggtcc gcagagctgc 60

agccttcgag ggccagccct ctccgagtcc ggggctgggt cccaccagtg acaaggcggc 120

agccccgcgc acaccaaaga gaaagcggct gtggcggcag cggcagcccc agccatgctg 180

tgttatgtga cgaggccgga cgcggtgctg atggaggtgg aggtggaggc gaaagccaac 240

ggcgaggact gcctcaacca ggtgtgcagg cgactgggaa tcatagaagt tgactatttt 300

ggactgcaat ttacgggtag caaaggtgaa agtttatggc taaacctgag aaaccggatc 360

tcccagcaga tggatgggct agccccttac aggcttaaac ttagagtcaa gttcttcgtg 420

gagcctcatc tcatcttaca ggagcagact aggcatatct ttttcttgca catcaaggag 480

gccctcttgg caggccacct cttgtgttcc ccagagcagg cagtggaact cagtgccctc 540

ctggcccaga ccaagtttgg agactacaac cagaacactg ccaagtataa ctatgaggag 600

ctctgtgcca aggagctctc ctctgccacc ttgaacagca ttgttgcaaa acataaggag 660

ttggagggga ccagccaggc ttcagctgaa taccaagttt tgcagattgt gtcggcaatg 720

gaaaactatg gcatagaatg gcattctgtg cgggatagcg aagggcagag actgctcatt 780

ggggttggac ctgaaggaat ctcaatttgt aaagatgact ttagcccaat taataggata 840

gcttatcctg tggtgcagat ggccacccag tcaggaaaga atgtatattt gacggtcacc 900

aaggaatctg ggaacagcat cgtgctcttg tttaaaatga tcagcaccag ggcggccagc 960

gggctctacc gagcgataac agagacgcac gcattctaca ggtgtgacac agtgaccagc 1020

gccgtgatga tgcagtatag ccgtgacttg aagggccact tggcatctct gtttctgaat 1080

gaaaacatta accttggcaa gaaatatgtc tttgatatta aaagaacatc aaaggaggtg 1140

tatgaccatg ccaggagggc tctgtacaat gctggcgttg tggacctcgt ttcaagaagc 1200

aaccagagcc cttcacactc gcctctgaag tcctcagaaa gcagcatgaa ctgcagcagc 1260

tgcgagggcc tcagctgcca gcagacccgg gtgctgcagg agaagctacg caagctgaag 1320

gaagccatgc tgtgcatggt gtgctgcgag gaggagatca actccacctt ctgtccctgt 1380

ggccacactg tgtgctgtga gagctgcgcc gcccagctac agtcatgtcc cgtctgcagg 1440

tcgcgtgtgg agcatgtcca gcacgtctat ctgccaacgc acaccagtct tctcaatctg 1500

actgtaatct aatctgttgt gcttttgttg gacttggcat gtttccatga actgcactat 1560

tataaactat taaaatgata gatgttggag aaagtaatta ttccaacacc catctgccca 1620

tgcgatgtta aaaaa 1635

<210> 84
<211> 2118
<212> DNA
<213> Homo sapiens

<400> 84
ctgtgaagat ggcgctctcc agggtgtgct gggctcggtc ggctgtgtgg ggctcggcag 60

tcacccctgg acattttgtc acccggaggc tgcaacttgg tcgctctggc ctggcttggg 120

gggcccctcg gtcttcaaag cttcaccttt ctccaaaggc agatgtgaag aacttgatgt 180

cttatgtggt aaccaagaca aaagcgatta atgggaaata ccatcgtttc ttgggtcgtc 240

atttcccccg cttctatatc ctgtacacaa tcttcatgaa aggattgcag atgttatggg 300

```
ctgatgccaa aaaggctaga agaataaaga caaatatgtg gaagcacaat ataaagtttc     360
atcaacttcc ataccgggag atggagcatt tgagacagtt ccgccaagac gtcaccaagt     420
gtcttttcct aggtattatt tccattccac cttttgccaa ctacctggtc ttcttgctaa     480
tgtacctgtt tcccaggcaa ctactgatca ggcatttctg gaccccaaaa caacaaactg     540
atttcttaga tatctatcat gctttccgga agcagtccca cccagaaatt attagttatt     600
tagaaaaggt catccctctc atttctgatg caggactccg gtggcgtctg acagatctgt     660
gcaccaagat acagcgtggt acccacccag caatacatga tatcttggct ctgagagagt     720
gtttctctaa ccatcctctg ggcatgaacc aactccaggc tttgcacgtg aaagccttga     780
gccgggccat gcttctcaca tcttacctgc ctcctccctt gttgagacat cgtttgaaga     840
ctcatacaac tgtgattcac caactggaca aggctttggc aaagctgggg attggccagc     900
tgactgctca ggaagtaaaa tcggcttgtt atctccgtgg cctgaattct acgcatattg     960
gtgaagatag gtgtcgaact tggctgggag aatggctgca gatttcctgc agcctgaaag    1020
aagctgagct gtctctcttg ctgcacaacg tggtcctgct ctccaccaac taccttggga    1080
caaggcgctg aatgaaccat ggagcggatg gcattgtcct gcagtcgtat agtatagcag    1140
tgcaggaaca aacagcactt gccagcaaag tctgtgtgta ctgttaagtg tgtgggaggc    1200
agagagagga gcaggggcca tgggcttcac agcatggcac acctgtggga actgcagaca    1260
ttcctctcac agctagaact gaaacaaacc ctcttgctag ggtggtccg tgtgaggtgt     1320
catcctgtcc ccctcataat tactaatagc tggaactggc agcagcctct actgggcttt    1380
tactgtgatg tgttcagttc atgtcctagg aagtcagctt ttgccccagg tgggaatcct    1440
tatttggctt aggactgatc cacttccatg ttacttacat ctgtgggttt ttgttgttgc    1500
tgttagaaaa tttttggctg gtgaaaacag cactcctttg gctggagcac ttgtgtccat    1560
gcatgtactt gggtgtttcc ctccatcctt tctgatatga ccaaaaatca agttgttttg    1620
ttttttgtca ccttcactgg catgggctaa ccacttcttt ttcaaaccct ctgaacacct    1680
ttttctgatg ggtaacttgc aggaatattc tattggaaaa gataacagga agtacaagtg    1740
cttcttgacc ccttcctcaa tgtttctagc cttcactctc cattgtcttt tctgggctgt    1800
attacagccc tctgtggatc ttcaactctg ctgcctccac tgtgatgcag cagtccaact    1860
gtaactgaca gtggctgcct tctctgggcc atggatcaca cctgtaaggt actaattact    1920
gcccagcctg ggagatcag gagaggtctg catagttagt aagttgggtt tagcttttgt     1980
gtgtgcatca gtgacttaga gttctgtaat aacttattgt aaatgcatga agcactgttt    2040
ttaaacccaa gtaaagactg cttgaaacct gttgatggaa aaaaaaaaaa aaaaaaaaaa    2100
```

aaaaaaaaaa aaaaaaaa 2118

<210> 85
<211> 4221
<212> DNA
<213> Homo sapiens

<400> 85
cgataacgat ttgtgttgtg agaggcgcaa gctgcgattt ctgctgaact tggaggcatt 60

tctacgactt ttctctcagc tgaggctttt cctccgaccc tgatgctctt caattcggtg 120

ctccgccagc cccagcttgg cgtcctgaga aatggatggt cttcacaata ccctcttcaa 180

tcccttctga ctggttatca gtgcagtggt aatgatgaac acacttctta tggagaaaca 240

ggagtcccag ttcctccttt tggatgtacc ttctcttctg ctcccaatat ggaacatgta 300

ctagcagttg ccaatgaaga aggctttgtt cgattgtata acacagaatc acaaagtttc 360

agaaagaagt gcttcaaaga atggatggct cactggaatg ccgtctttga cctggcctgg 420

gttcctggtg aacttaaact tgttacagca gcaggtgatc aaacagccaa attttgggac 480

gtaaaagctg gtgagctgat tggaacatgc aaaggtcatc aatgcagcct caagtcagtt 540

gccttttcta agtttgagaa agctgtattc tgtacgggtg gaagagatgg caacattatg 600

gtctgggata ccaggtgcaa caaaaaagat gggttttata ggcaagtgaa tcaaatcagt 660

ggagctcaca atacctcaga caagcaaacc ccttcaaaac ccaagaagaa acagaattca 720

aaaggacttg ctccttctgt ggatttccag caaagtgtta ctgtggtcct ctttcaagac 780

gagaatacct tagtctcagc aggagctgtg gatgggataa tcaaagtatg ggatttacgt 840

aagaattata ctgcttatcg acaagaaccc atagcatcca agtctttcct gtacccaggt 900

agcagcactc gaaaacttgg atattcaagt ctgattttgg attccactgg ctctacttta 960

tttgctaatt gcacagacga taacatctac atgtttaata tgactgggtt gaagacttct 1020

ccagtggcta ttttcaatgg acaccagaac tctacctttt atgtaaaatc cagccttagt 1080

ccagatgacc agtttttagt cagtggctca agtgatgaag ctgcctacat atggaaggtc 1140

tccacaccct ggcaacctcc tactgtgctc ctgggtcatt ctcaagaggt cacgtctgtg 1200

tgctggtgtc catctgactt cacaaagatt gctacctgtt ctgatgacaa tacactaaaa 1260

atctggcgct tgaatagagg cttagaggag aaaccaggag gtgataaact ttccacggtg 1320

ggttgggcct ctcagaagaa aaaagagtca agacctggcc tagtaacagt aacgagtagc 1380

cagagtactc ctgccaaagc cccaagggta aagtgcaatc catccaattc ttccccgtca 1440

tccgcagctt gtgccccaag ctgtgctgga gacctccctc ttccttcaaa tactcctacg 1500

ttctctatta aaacctctcc tgccaaggcc cggtctccca tcaacagaag aggctctgtc 1560

tcctccgtct ctcccaagcc accttcatct ttcaagatgt cgattagaaa ctgggtgacc 1620
cgaacacctt cctcatcacc acccatcact ccacctgctt cggagaccaa gatcatgtct 1680
ccgagaaaag cccttattcc tgtgagccag aagtcatccc aagcagaggc ttgctctgag 1740
tctagaaata gagtaaagag gaggctagac tcaagctgtc tggagagtgt gaaacaaaag 1800
tgtgtgaaga gttgtaactg tgtgactgag cttgatggcc aagttgaaaa tcttcatttg 1860
gatctgtgct gccttgctgg taaccaggaa gaccttagta aggactctct aggtcctacc 1920
aaatcaagca aaattgaagg agctggtacc agtatctcag agcctccgtc tcctatcagt 1980
ccgtatgctt cagaaagctg tggaacgcta cctcttcctt tgagaccttg tggagaaggg 2040
tctgaaatgg taggcaaaga gaatagttcc ccagagaata aaaactggtt gttggccatg 2100
gcagccaaac ggaaggctga gaatccatct ccacgaagtc cgtcatccca gacacccaat 2160
tccaggagac agagcggaaa gacattgcca agcccggtca ccatcacgcc cagctccatg 2220
aggaaaatct gcacatactt ccatagaaag tcccaggagg acttctgtgg tcctgaacac 2280
tcaacagaat tatagattct aatctgagtg agttactgag ctttggtcca ctaaaacaag 2340
ctgagctttg gtccactaaa acaagatgaa aaatacaaga gtgactctat aactctggtc 2400
tttaagaaag ctgccttttc atttttagac aaaatctttt caacgctgaa atgtacctaa 2460
tctggttcta ctaccataat gtatatgcag cttcccgagg atgaatgctg tgtttaaatt 2520
tcataaagta aatttgtcac tctagcattt tgaatgaata gtcttcactt tttaaattat 2580
tcatcttctc tataataatg acatcccagt tcatggaggc aaaaaacaag tttcttgtta 2640
tcctgaaact ttctatgctc agtggaaagt atctgccagc cacagcatga ggcctgtgaa 2700
ggctgactga gaaatcctct gctgaagacc cctggttctg ttctgcctcc aacatgtata 2760
attttatttg aaatacataa tcttttcact atgcttttgt ggggtttttt ttaagtatgt 2820
gtaaaaatgt gatgctcaga taagtacatt tatatcagtt cagtgttaaa atgcagtctc 2880
ttgagttaaa gtcatcttta ttttaaatgc agtgataaat gtcaactctt cggagaaact 2940
aggagaacaa caacagaaag ctgtgtttgt ctttttttctc tcaaatatat ctcccgtatg 3000
agatttcagg tccccatgtt ttcaccaagc aatctgctat gtcagccaac ccaacatcac 3060
tttctacagg aggttatgat ttttgccatt tactagagga agatgtttta tgaaatcaat 3120
ttggggtttg aattcaggtg cagtcatcag ttctttaggg gctgcaatgt tttaaaaaaa 3180
ataagtcatc agattttaag aaaaaagtga tgatttctta ttgatatttt tgtaacagaa 3240
tatagctctt aactgaaaat ccagaaccag aaacataaat cttgagtttc ttttcatgta 3300
cataaaaagc aatagccttt tagtatagat agccctgagc caaaaagtaa tagaattttc 3360
tctagatatt taatacagag agtgtataga ctgactctaa gttaataatg tgcaaaatat 3420

cttaaacatc cctcccctta ttcaacaatt atgtatcagt gatcttgaac cattgtttta 3480

tatttttcac ctttgtaacc tcatggaaag aggctttaca tactttctat gtactattta 3540

cttagaaggg agccccccttc cagtcatgaa acttcatttg ttttatccat atccctgagg 3600

actgtgtaga ctttatgtca gttctgtgta gactttatgt cagtttttgt cattatttga 3660

aaatctattc tgacaacttt ttaattcctt tgatcttata agttaaagct gtaacaactg 3720

aaattgcatg gatcaagtaa gcatagtttt atccagggag aaaaataaaa ggaagccata 3780

gaattgctct ggtcaaaacc aagcacacca tagccttaac tgaatattta ggaaatctgc 3840

ctaatctgct tatatttggt gtttgttttt tgactgttgg gctttgggaa gatgttattt 3900

atgaccaata tctgccagta acgctgttta tctcacttgc tttgaaagcc aatgggggaa 3960

aaaaatccat gaaaaaaaaa agattgataa agtagatgat tttgtttgta tccctaccca 4020

tctcctggca gccctactga gtgaaattgg gatacatttg gctgtcagaa attataccga 4080

gtctactggg tataacatgt ctcacttgga aagctagtac ttttaaatgg gtgccaaagg 4140

tcaactgtaa tgagataatt atccctgcct gtgtccatgt cagactttga gctgatcctg 4200

aataataaag ccttttacct t 4221

<210> 86
<211> 867
<212> DNA
<213> Homo sapiens

<400> 86
cgtttcagcg tggcggcgct ggtgctggcg ttggccctgg aggacggccc cgagtgatgg 60

ctggcgcctg cctcccgggt gtctcccggg tacagatgga gtcgtcccgc ggccgccggc 120

ggcaaggtcg gcagctgcga ggccaagaga gaccccagga cacacacagc tgcctcccgg 180

tgcgagaaga agaccccggc ttgagagtga gatggcgttt aatgattgct tcagtttgaa 240

ctaccctggc aacccctgcc caggggactt gatcgaagtg ttccgtcctg gctatcagca 300

ctgggccctg tacttgggtg atggttacgt tatcaacata gcacctgtag atggcattcc 360

tgcgtccttt acaagcgcca agtctgtatt cagcagtaag gccctggtga aaatgcagct 420

cttgaaggat gttgtgggaa atgacacata cagaataaac aataaatacg atgaaacgta 480

ccccccctctc cctgtggaag aaatcataaa gcggtcagag tttgtaattg gacaggaggt 540

ggcctataac ttacttgtca acaactgtga acattttgtg acattgcttc gctatggaga 600

aggagtttca gagcaggcca accgagcgat aagtaccgtt gagtttgtga cagctgctgt 660

tggtgtcttc tcattcctgg gcttgtttcc aaaaggacaa agagcaaaat actattaaca 720

atttaccaaa gagatattga tattgaagga atttgggagg aggaaaagaa acctggggtg 780

aatacttatt ttcagtgcat cattactgtt ccagattcct atgatggatg gcagactctt 840

taataaattg cttactgata ttatctt 867

<210> 87
<211> 561
<212> DNA
<213> Homo sapiens

<220>
<221> misc_feature
<222> (534)..(534)
<223> any kind of base

<400> 87
tttttttttc aaattttatt ttttgtactt ttattgaaaa ggtacattta aaaaaataca 60

cagacatttt accatttaca ggttgcagat atagatgctc taaaagagtc cactctattt 120

tgttgttcta tgataactct tgcccctgat atcacaaaca ttccagtctt gttgatatcg 180

gcttaaaaag gggggcatgg gagcatgacc tgcaatatat tcagcgaaca gaaagacaaa 240

ttgttcaatt ataaattttt ttatcttctg tacattattg cattagggag ccacaaaatt 300

atgtagcatc attacaaatg aaaacaggtt aaaaatgaag aagatactta tatagaaata 360

catggattca ttgtcttctt gcagaatgca caagaggtgc aaaaatgtgc aatttaggaa 420

gctctttttc tgtttgtata cgtttgctta gcaacacaaa ccagtgagga agctacaaaa 480

taagttaaac aaaaatagca aacaggtagt aattatagct atgttatatg gctntctatt 540

tcatttaaat atctccaaat a 561

<210> 88
<211> 471
<212> DNA
<213> Homo sapiens

<400> 88
tttcatgaat aattttattt ttattttggt catgactttt taaaattaat ggaaagtctc 60

agtctgctca aatgataaac caaaaaatgg ggtcatgaag caaattcagt ctttgcattt 120

cttcaacaac caactcacat ttctctgctc cttgactcag aggctggaca tgtgctaaca 180

gctttttgc ttctgtatat ccttaatagg atggcagaat cccggtgtta aaagaatctt 240

aaagtcagcc gtgtcaccat gggacctcga ttagcaaaca gatgtagaca ttccttccaa 300

attcagtcaa agaaaaaact ctaataccag cagccatgtt cagggtgtgc acgacatgct 360

gagcgcttgg gatacatccc cttgtttaat tctcacagaa actttgtgag acaagtacta 420

taatccctgt ttaacagatg ggcaaactga ggtttggaaa aacactcatt t 471

<210> 89
<211> 727
<212> DNA
<213> Homo sapiens

<400> 89
taaaatatta ccttttattt tctcaggcac acaagtgatt tggataccaa ttatcaagac 60

attttactga tttcccttta gaatgatcta ttttaaatct tcagccacct tagaaaaagt 120

ttgcagcaat cactttgcaa ttcataagta tcaggttaga atttagttgt ggaataagtt 180

aacagtttat gttcaatatg tgaggttatt tgaactcctg agttttaaat atcctgtgga 240

acagtgattc ctctcccttc taatggcttt tcagattaaa gcaatgactt taaaaagatt 300

acatcctaaa tacttgatta caacagaaat cgaccaatct aaaaatcaga tagtgttata 360

ctgaacatca ttctgatata atgagtagcc tctggctgaa acaaaattcc accaccaagg 420

ccatcaacca ggttagtact gtttttcctg ggtctatgt aaactctcct tttctctgca 480

aatgctgctt ggctgtgaac agcatggatt tacctgcacc aatgtggcac acacctagca 540

actttctcaa gcattctaaa gatatcccca gagctacaat attgacatat gcacagcact 600

ttctctagac agtccaatca gcgtgcacat cacacacaca gaatgctggg atatgctata 660

ctgcacactt agtacacagg tggaatagag tacaatgact aaagctcaca gaaaatgttt 720

tagtctt 727

<210> 90
<211> 460
<212> DNA
<213> Homo sapiens

<400> 90
tttttgatat aaagggcttt tattttcttt acagttttct gtattttcca aatttctaca 60

taaacataca cataaaagtc ataaaaatgt ccctcaaaat gacactccct ccaatgcagg 120

gagtgaggag gtgtgtgctg ggacgccaca gggtggctgc tgcagctaat ccctgtgcag 180

gtgcagccac tgctacactc cagcgtctga ggcccctctg cactggcagt gttagaatgg 240

ctgtctgaga gccaaggctg ttcacccgag cagagagtca tggagctggt acaggcagga 300

gccaaggtaa gcactaggct ggtgtgtgcc ctccaggggg caccctcctt gaagcaggcc 360

ctccaaggta ctcgccccct gaggcagccg ataacagccg gaagccttcc cctgggggga 420

cctggccatt tgtggagcag ggaaaggctc cactgccagt 460

<210> 91
<211> 392
<212> DNA
<213> Homo sapiens

<400> 91
tttttttttt tttttttgag ttgctgaaaa gtttactttt gagttttaaa ctgtactttg 60

aaaactatat tgtgaactgt gttgacatga ggaaaggctg ggctccctga aaacatccag 120

ttccacagca gggcgggccc aagcccagcc aatccccagg caatgcaggg cagggatccc 180

acctggtata agtacgggca gagggcagag ccaggctgta tcgaggggcg gcgctgggac 240

cctcctcgcc cagaattcct actcatcccc agcacagaag tgctctgtag ggccagctga 300

ggacaccccg gttcactgag ggtggcccac agtaagtcgc cgtctggcag taagttagct 360

ctgcaggtgt ggaccccaag accacacacg gc 392

<210> 92
<211> 496
<212> DNA
<213> Homo sapiens

<400> 92
tttttgtat ttttctcaat ataatgttgc aagatgtcat tatattctca ttacccataa 60

cttcccatcc aaatcttaat ggcacatttg atattttttc aaatggcttt gagatataat 120

tcacatacca cacaattcat gtatctaaag tatacagttc tgtagttctg tggttgttgg 180

ttacatccac cagactgagg gctccctgag ggttggtgcc tcagctttcc cttcacttac 240

tcattcacca aacattcagc gcctactgag tactggggtt atgatggcca acaggagaga 300

cagtctctgc ctactgtttg gtgggggtgg agtacttagt aaccgtcgtc attattgagt 360

gcttacctgt gctgggcaca gtgctgctac tgggtgactg tgtccccagg gctgtcccag 420

gctgggggtc tggaggagta gttatcagtt gaactgagtt aatccacagt ggaaggtaac 480

ccactccctg ccctag 496

<210> 93
<211> 472
<212> DNA
<213> Homo sapiens

<400> 93
ttaacacaaa agcttttact tggaaactgg caaatactgg actagaatac tgacatgctc 60

acgctctggc gggagctcgg atgcagaagc tattgcacaa agcccctctg attgccttgc 120

tcctctttgg catgtatcag cagagcccca agggccaatt gcccacaggt ggggactgtt 180

ctccatcaag gtatggggac ccctacttcc ttgttttgtt aaaaagtgca ggtaggcgaa 240

gaaagcccag gcagttgacc cagtctttga aacagctgac tccccagagc tggggccagg 300

ggagcctggt cttgaggggt aagggctgca gggccaggct gatggcctgt gtcatggcat 360

tggccatctc ctctccagct tctcctcagc catcgcccgt ccgtcatggt ggtgtcggct 420

gcacctggac cttcctgcct ctccgctcag ggcagcagca gtgagtgcag ca 472

<210> 94
<211> 585
<212> DNA
<213> Homo sapiens

<400> 94
tttttatatt atagtaaatt tatttggtat aattacatat taacattatt tacaatgcta 60

atttttttat ttataaagta tctttatatg gacaaaaaga tacaaactgt tatcatttta 120

agtacaaagt atttctagaa atacattata agccattaaa aaaaaaaacg atatttcaag 180

attggttctt acatgctatg accaactcat atgaaagagc aagttgctcc cccttcattc 240

cttcccccaa ctccaaaggg aaaggaattt gatatttagg ctttaaaaaa tttcctacta 300

cctttatctt ttaaaaaacc ctactcaaaa caactatctc ttataaggga aaatatcata 360

gataagattt tcctttagaa aatgacatta aaagtggcat gagccctaga atgatatgtg 420

tattagaggc acttaaaaaa aatcagaagg gatccatagg aaggaattta attcagcaaa 480

tactgagtgt ccactgcatg caaggtaccc tgccagaaat ctcaaatgag taagttgtcc 540

ttagggatag aaggtgctaa gcatcatgca aaagatatga actga 585

<210> 95
<211> 400
<212> DNA
<213> Homo sapiens

<400> 95
ttattgtttt ttaaaaatac aattttgaaa tttattgttg aaaatggaca catggaacaa 60

accaaacctt gttttatcat gtaattttca gaaaatatgt gatccataaa gattaaaaga 120

aagttgtatt aagtctggca gctttagtat taacttgaaa taaaatatgg caagctttcc 180

acgtcctcct ttatttccac aatccatatg tacgagctag attccagtca gaacttccac 240

aaatacttca ctctttggta gcagcggtta taaattacgc ctttgctaat ttgcgttgtt 300

cccaaccagg agaaacatta ccacaaaaaa agtcagtttc atcctgcagt gttcccgcag 360

caaccatatt aaagctgaag aataaagctc ctttgtagta 400

<210> 96
<211> 461
<212> DNA
<213> Homo sapiens

<400> 96
ttatgccatg aattcctttt tactgaaata ccgtaggact cactaccaca ataagtactt 60

aagctgaaag agtttctaat gggagccaag taaattcagc tctccactgt gcaaagcatc 120

ttgtcatttc tataataaaa gtctttccat gttcagtcca ctttggctct ggaacctgga 180

tgagtcatgc ttggggccca gggtgcctgt gaggatgctg catgagaatt tcagctgtgg 240

tggcagtggc tgggagtccc actgactcag gggaggcca ggcgagatga gctggaactt 300

ttaggggaga gctggcactt agggacatca ttgattgtgc ttttcttagc ctagttctgt 360

cctgcaattt atttttctta tcatgtgact gtcggctgaa gtctggggtt atttggtttc 420

tttttcttct tccttgctgg acagtctcca tggggtcacg g 461

<210> 97
<211> 542
<212> DNA
<213> Homo sapiens

<400> 97
taaatccagt aaagcatagt actagattca tcatacttgt acaatacaac gggcgacatg 60

aaaatggcaa aggctctcct ttttgtgaac aatttaatac aactggtggt ccaataactc 120

tacaatcagc cttgtagagg tcattaaaga cagaatcctg aaagtccgtg actacaaata 180

cattttcaaa ttccggagaa tccaaacctt caaattcttc cactgactcc atctttacaa 240

agcccacttt aatgtccttt aaggctttta taagttcttc ttgttttcca gcttcttgaa 300

ccaatatcac tcttgtttca atctgaggca tctcttcttc tacatatgaa gtagatccaa 360

taagtaagtt ttccttggaa atctcagtaa ctttagaatc aaaaatggaa gagtctgcca 420

agctagtcct cccagtagtg gatgttaata cactattttc agccatgatt tgtattcttc 480

taaatcagca ctctcaaaaa agccctagga gttccacctc ttcaaacgcc gactcctctc 540

ac 542

<210> 98
<211> 1017
<212> DNA
<213> Homo sapiens

<400> 98
atgggcacct ggcttctggc ctgcacctgc gtctgcacct gtgtctgctc gggagtctct 60

gtctcagggg atggacgagg gccaagggct ggaacctcca cctgcctcac caacaacatt 120

ctcaggattg attgccactg gtctgcccca gagctgggtc agggctccag ccccgggctc 180

cccttcatca gccccgtggt gctgacacat gcccttttca gcaaccaggc tgctggtggc 240

acacagaagt gcatctggca gggcagtgag tgcactgtag tgttgccgcc caaggcagca 300

ctcctgccat ctgacaattt catcatcact ttctaccact gcatgtccgg gagggatcag 360

agcacgtcag ctcgccactg catcctgacc tggagcctca gtcctgcctt ggagtcaatg 420

accacacttc tcagctatga gctggacttc aagaggcagg aagaggcctg ggagcgggcc 480

cagcacaggg atcacattgt cggggtgacc tggctcatac ttgaagcctt tgagctggac 540

cctggcttta tccttgaggc caggctgcgt gtccagacgg ccatgctggg ggatgacggg 600

gcacaggagg agcgagggga gccagcccat gggaagagtg aggcccagga gtgtggttca 660

cacaaggtcc ttcagcaggt gacacaaacc tccaaggccc atcacaaggt ccttcagcag 720

atgacacaaa cctccaaggc tcatcacaaa ccttccactt tggcccaggg cactaaaggg 780

cgcacttttg ccagccctgg gcccttcctg cccacggacc ctctgatccc accctggggg 840

tggccaggca acacctttgt tgctgtgtcc atctttctcc tgctgactgg cccgacctac 900

ctcctgttca agctgtcgcc cagactcctc actttgggca aaggacaaga agcaactcgg 960

atgggggccc acagggctgg tgtgctgctg agccaggact gtgctggcac ccgatga 1017

<210> 99
<211> 1099
<212> DNA
<213> Homo sapiens

<400> 99
agccccaagc ttaccacctg cacccggaga gctgtgtcac catgtgggtc ccggttgtct 60

tcctcaccct gtccgtgacg tggattggtg agaggggcca tggttggggg gatgcaggag 120

agggagccag ccctgactgt caagctgagg ctctttcccc cccaacccag caccccagcc 180

cagacaggga gctgggctct tttctgtctc tcccagcccc actccaagcc catacccccca 240

gcccctccat attgcaacag tcctcactcc cacaccaggt ccccgctccc tcccacttac 300

cccagaactt tctccccatt gcccagccag ctccctgctc ccagctgctt tactaaaggg 360

gaagttcctg ggcatctccg tgtttctctt tgtggggctc aaaacctcca aggacctctc 420

tcaatgccat tggttccttg gaccgtatca ctggtccacc tcctgagccc ctcaatccta 480

tcacagtcta ctgacttttc ccattcagct gtgagtgccc aaccctatcc cagagacctt 540

gatgcttggc ctcccaatct tgccctagga tacccagatg ccaaccagac acctccttct 600

tcctagccag gctatctggc ctgagacaac aaatgggtcc ctcagtctgg caatgggact 660

ctgagaactc ctcattccct gactcttagc cccagactct tcattcagtg gcccacattt 720

tccttaggaa aaacatgagc atccccagcc acaactgcca gctctctgat tccccaaatc 780

tgcatccttt tcaaaaccta aaaacaaaaa gaaaaacaaa taaaacaaaa ccaactcaga 840

ccagaactgt tttctcaacc tgggacttcc taaactttcc aaaaccttcc tcttccagca 900

actgaacctc gccataaggc acttatccct ggttcctagc acccccttatc ccctcagaat 960

ccacaacttg taccaagttt cccttctccc agtccaagac cccaaatcac cacaaaggac 1020

ccaatcccca gactcaagat atggtctggg cgctgtcttg tgtctcctac cctgatccct 1080

gggttcaact ctgctccca 1099

<210> 100
<211> 1095
<212> DNA
<213> Homo sapiens

<400> 100
ccaagcttac cacctgcacc cggagagctg tgtcaccatg tgggtcccgg ttgtcttcct 60

caccctgtcc gtgacgtgga ttggtgagag gggccatggt tggggggatg caggagaggg 120

agccagccct gactgtcaag ctgaggctct ttcccccca acccagcacc ccagcccaga 180

cagggagctg ggctcttttc tgtctctccc agccccactt caagcccata cccccagccc 240

ctccatattg caacagtcct cactcccaca ccaggtcccc gctccctccc acttacccca 300

gaactttctc cccattgccc agccagctcc ctgctcccag ctgctttact aaaggggaag 360

ttcctgggca tctccgtgtt tctctttgtg ggctcaaaa cctccaagga cctctctcaa 420

tgccattggt tccttggacc gtatcactgg tccatctcct gagcccctca atcctatcac 480

agtctactga cttttcccat tcagctgtga gtgtccaacc ctatcccaga gaccttgatg 540

cttggcctcc caatcttgcc ctaggatacc cagatgccaa ccagacacct ccttcttcct 600

agccaggcta tctggcctga gacaacaaat gggtccctca gtctggcaat gggactctga 660

gaactcctca ttccctgact cttagcccca gactcttcat tcagtggccc acattttcct 720

taggaaaaac atgagcatcc ccagccacaa ctgccagctc tctgagtccc caaatctgca 780

tcctttttcaa aacctaaaaa caaaaagaaa aacaaataaa acaaaaccaa ctcagaccag 840

aactgttttc tcaacctggg acttcctaaa ctttccaaaa ccttcctctt ccagcaactg 900

aacctcgcca taaggcactt atccctggtt cctagcaccc cttatcccct cagaatccac 960

aacttgtacc aagtttccct tctcccagtc caagacccca aatcaccaca aaggacccaa 1020

tccccagact caagatatgg tctgggcgct gtcttgtgtc tcctaccctg atccctgggt 1080

tcaactctgc tccca 1095

SEQUENCE LISTING

<110>  WANG, YIXIN

<120>  CANCER DIAGNOSTIC PANEL

<130>  P033681EP

<150>  US 60/368,667
<151>  2002-03-29

<160>  100

<170>  PatentIn version 3.1

<210>  1
<211>  1466
<212>  DNA
<213>  human

<400>  1
agccccaagc ttaccacctg cacccggaga gctgtgtgtc accatgtggg tcccggttgt      60

cttcctcacc ctgtccgtga cgtggattgg tgctgcaccc ctcatcctgt ctcggattgt     120

gggaggctgg gagtgcgaga agcattccca accctggcag gtgcttgtgg cctctcgtgg     180

cagggcagtc tgcggcggtg ttctggtgca cccccagtgg gtcctcacag ctgcccactg     240

catcaggaac aaaagcgtga tcttgctggg tcggcacagc ctgtttcatc ctgaagacac     300

aggccaggta tttcaggtca gccacagctt cccacacccg ctctacgata tgagcctcct     360

gaagaatcga ttcctcaggc caggtgatga ctccagccac gacctcatgc tgctccgcct     420

gtcagagcct gccgagctca cggatgctgt gaaggtcatg gacctgccca cccaggagcc     480

agcactgggg accacctgct acgcctcagg ctggggcagc attgaaccag aggagttctt     540

gaccccaaag aaacttcagt gtgtggacct ccatgttatt tccaatgacg tgtgtgcgca     600

agttcaccct cagaaggtga ccaagttcat gctgtgtgct ggacgctgga caggggggcaa     660

aagcacctgc tcgggtgatt ctgggggccc acttgtctgt aatggtgtgc ttcaaggtat     720

cacgtcatgg ggcagtgaac catgtgccct gcccgaaagg ccttccctgt acaccaaggt     780

ggtgcattac cggaagtgga tcaaggacac catcgtggcc aacccctgag cacccctatc     840

aaccccctat tgtagtaaac ttggaacctt ggaaatgacc aggccaagac tcaagcctcc     900

ccagttctac tgacctttgt ccttaggtgt gaggtccagg gttgctagga aaagaaatca     960

gcagacacag gtgtagacca gagtgtttct taaatggtgt aattttgtcc tctctgtgtc    1020

ctggggaata ctggccatgc ctggagacat atcactcaat ttctctgagg acacagatag    1080

```
gatggggtgt ctgtgttatt tgtggggtac agagatgaaa gaggggtggg atccacactg   1140
agagagtgga gagtgacatg tgctggacac tgtccatgaa gcactgagca gaagctggag   1200
gcacaacgca ccagacactc acagcaagga tggagctgaa aacataaccc actctgtcct   1260
ggaggcactg ggaagcctag agaaggctgt gagccaagga gggagggtct tcctttggca   1320
tgggatgggg atgaagtaag gagagggact ggacccccctg gaagctgatt cactatgggg   1380
ggaggtgtat tgaagtcctc cagacaaccc tcagatttga tgatttccta gtagaactca   1440
cagaaataaa gagctgttat actgtg                                         1466
```

<210> 2
<211> 786
<212> DNA
<213> human

<400> 2

```
atgtgggacc tggttctctc catcgccttg tctgtggggt gcactggtgc cgtgcccctc     60
atccagtctc ggattgtggg aggctgggag tgtgagaagc attcccaacc ctggcaggtg    120
gctgtgtaca gtcatggatg ggcacactgt gggggtgtcc tggtgcaccc ccagtgggtg    180
ctcacagctg cccattgcct aaagaagaat agccaggtct ggctgggtcg gcacaacctg    240
tttgagcctg aagacacagg ccagagggtc cctgtcagcc acagcttccc acacccgctc    300
tacaatatga gccttctgaa gcatcaaagc cttagaccag atgaagactc cagccatgac    360
ctcatgctgc tccgcctgtc agagcctgcc aagatcacag atgttgtgaa ggtcctgggc    420
ctgcccaccc aggagccagc actggggacc acctgctacg cctcaggctg gggcagcatc    480
gaaccagagg agttcttgcg ccccaggagt cttcagtgtg tgagcctcca tctcctgtcc    540
aatgacatgt gtgctagagc ttactctgag aaggtgacag agttcatgtt gtgtgctggg    600
ctctggacag gtggtaaaga cacttgtggg ggtgattctg gggtccact tgtctgtaat     660
ggggtgcttc aaggtatcac atcatggggc cctgagccat gtgccctgcc tgaaaagcct    720
gctgtgtaca ccaaggtggt gcattaccgg aagtggatca aggacaccat cgcagccaac    780
ccctga                                                               786
```

<210> 3
<211> 503
<212> DNA
<213> human

<400> 3

```
gacagcggct tccttgatcc ttgccacccg cgactgaaca ccgacagcag cagcctcacc     60
```

```
atgaagttgc tgatggtcct catgctggcg gccctctccc agcactgcta cgcaggctct    120
ggctgcccct tattggagaa tgtgatttcc aagacaatca atccacaagt gtctaagact    180
gaatacaaag aacttcttca agagttcata gacgacaatg ccactacaaa tgccatagat    240
gaattgaagg aatgtttcct taaccaaacg gatgaaactc tgagcaatgt tgaggtgttt    300
atgcaattaa tatatgacag cagtctttgt gatttatttt aactttctgc aagacctttg    360
gctcacagaa ctgcagggta tggtgagaaa ccaactacgg attgctgcaa accacacctt    420
ctctttctta tgtcttttta ctacaaacta caagacaatt gttgaaacct gctatacatg    480
tttattttaa taaattgatg gca                                            503
```

<210> 4
<211> 2560
<212> DNA
<213> human

<400> 4

```
gaattccggc cagaagaaat ctggcctcgg aacacgccat tctccgcgcc gcttccaata     60
accactaaca tccctaacga gcatccgagc cgagggctct gctcggaaat cgtcctggcc    120
caactcggcc cttcgagctc tcgaagatta ccgcatctat tttttttttc tttttttct     180
tttcctagcg cagataaagt gagcccggaa agggaaggag gggcggggga caccattgcc    240
ctgaaagaat aaataagtaa ataaacaaac tggctcctcg ccgcagctgg acgcggtcgg    300
ttgagtccag gttgggtcgg acctgaaccc ctaaaagcgg aaccgcctcc cgccctcgcc    360
atcccggagc tgagtcgccg gcggcggtgg ctgctgccag acccggagtt tcctctttca    420
ctggatggag ctgaactttg ggcggccaga gcagcacagc tgtccgggga tcgctgcacg    480
ctgagctccc tcggcaagac ccagcggcgg ctcgggattt ttttgggggg gcggggacca    540
gccccgcgcc ggcaccatgt tcctggcgac cctgtacttc gcgctgccgc tcttggactt    600
gctcctgtcg gccgaagtga gcggcggaga ccgcctggat tgcgtgaaag ccagtgatca    660
gtgcctgaag gagcagagct gcagcaccaa gtaccgcacg ctaaggcagt gcgtggcggg    720
caaggagacc aacttcagcc tggcatccgg cctggaggcc aaggatgagt gccgcagcgc    780
catggaggcc ctgaagcaga agtcgctcta caactgccgc tgcaagcggg gtatgaagaa    840
ggagaagaac tgcctgcgca tttactggag catgtaccag agcctgcagg gaaatgatct    900
gctggaggat tccccatatg aaccagttaa cagcagattg tcagatatat tccgggtggt    960
cccattcata tcagatgttt ttcagcaagt ggagcacatt cccaaaggga acaactgcct   1020
```

ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260
tgaagagagg gagaagccca actgtttgaa tttgcaggac tcctgcaaga cgaattacat 1320
ctgcagatct cgccttgcgg atttttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg ggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160
aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 5
<211> 2560
<212> DNA

<213>  human

<400>  5
gaattccggc cagaagaaat ctggcctcgg aacacgccat tctccgcgcc gcttccaata 60
accactaaca tccctaacga gcatccgagc cgagggctct gctcggaaat cgtcctggcc 120
caactcggcc cttcgagctc tcgaagatta ccgcatctat tttttttttc tttttttttct 180
tttcctagcg cagataaagt gagcccggaa agggaaggag gggcgggga caccattgcc 240
ctgaaagaat aaataagtaa ataaacaaac tggctcctcg ccgcagctgg acgcggtcgg 300
ttgagtccag gttgggtcgg acctgaaccc ctaaaagcgg aaccgcctcc cgccctcgcc 360
atcccggagc tgagtcgccg gcggcggtgg ctgctgccag acccggagtt tcctctttca 420
ctggatggag ctgaactttg ggcggccaga gcagcacagc tgtccgggga tcgctgcacg 480
ctgagctccc tcggcaagac ccagcggcgg ctcgggattt ttttgggggg gcggggacca 540
gccccgcgcc ggcaccatgt tcctggcgac cctgtacttc gcgctgccgc tcttggactt 600
gctcctgtcg gccgaagtga gcggcggaga ccgcctggat tgcgtgaaag ccagtgatca 660
gtgcctgaag gagcagagct gcagcaccaa gtaccgcacg ctaaggcagt gcgtggcggg 720
caaggagacc aacttcagcc tggcatccgg cctggaggcc aaggatgagt gccgcagcgc 780
catggaggcc ctgaagcaga agtcgctcta caactgccgc tgcaagcggg gtatgaagaa 840
ggagaagaac tgcctgcgca tttactggag catgtaccag agcctgcagg gaaatgatct 900
gctggaggat tccccatatg aaccagttaa cagcagattg tcagatatat tccgggtggt 960
cccattcata tcagatgttt ttcagcaagt ggagcacatt cccaaaggga acaactgcct 1020
ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260
tgaagagagg gagaagccca actgtttgaa tttgcaggac tcctgcaaga cgaattacat 1320
ctgcagatct cgccttgcgg atttttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620

gccagccttc ccagtacaga ccaccactgc cactaccacc actgccctcc gggttaagaa 1680

caagcccctg gggccagcag ggtctgagaa tgaaattccc actcatgttt tgccaccgtg 1740

tgcaaattta caggcacaga agctgaaatc caatgtgtcg ggcaatacac acctctgtat 1800

ttccaatggt aattatgaaa aagaaggtct cggtgcttcc agccacataa ccacaaaatc 1860

aatggctgct cctccaagct gtggtctgag cccactgctg gtcctggtgg taaccgctct 1920

gtccacccta ttatctttaa cagaaacatc atagctgcat taaaaaaata caatatggac 1980

atgtaaaaag acaaaaacca agttatctgt ttcctgttct cttgtatagc tgaaattcca 2040

gtttaggagc tcagttgaga aacagttcca ttcaactgga acattttttt ttttcctttt 2100

aagaaagctt cttgtgatcc ttcggggctt ctgtgaaaaa cctgatgcag tgctccatcc 2160

aaactcagaa ggctttggga tatgctgtat tttaaaggga cagtttgtaa cttgggctgt 2220

aaagcaaact ggggctgtgt tttcgatgat gatgatcatc atgatcatga tgattttaac 2280

agttttactt ctggcctttc ctagctagag aaggagttaa tatttctaag gtaactccca 2340

tatctccttt aatgacattg atttctaatg atataaattt cagcctacat tgatgccaag 2400

ctttttttgcc acaaagaaga ttcttaccaa gagtgggctt tgtggaaaca gctggtactg 2460

atgttcacct ttatatatgt actagcattt tccacgctga tgtttatgta ctgtaaacag 2520

ttctgcactc ttgtacaaaa gaaaaaacca cccggaattc 2560

<210> 6
<211> 2439
<212> DNA
<213> human

<400> 6
cagcacccag ctccccgcca ccgccatggt ccccgacacc gcctgcgttc ttctgctcac 60

cctggctgcc ctcggcgcgt ccggacaggg ccagagcccg ttgggctcag acctgggccc 120

gcagatgctt cgggaactgc aggaaaccaa cgcggcgctg caggacgtgc gggactggct 180

gcggcagcag gtcagggaga tcacgttcct gaaaaacacg gtgatggagt gtgacgcgtg 240

cgggatgcag cagtcagtac gcaccggcct acccagcgtg cggcccctgc tccactgcgc 300

gcccggcttc tgcttccccg gcgtggcctg catccagacg gagagcggcg gccgctgcgg 360

cccctgcccc gcgggcttca cgggcaacgg ctcgcactgc accgacgtca acgagtgcaa 420

cgcccacccc tgcttccccc gagtccgctg tatcaacacc agcccggggt tccgctgcga 480

ggcttgcccg ccggggtaca gcggccccac ccaccagggc gtggggctgg ctttcgccaa 540

ggccaacaag caggtttgca cggacatcaa cgagtgtgag accgggcaac ataactgcgt 600
ccccaactcc gtgtgcatca acacccgggg ctccttccag tgcggcccgt gccagcccgg 660
cttcgtgggc gaccaggcgt ccggctgcca gcgcggcgca cagcgcttct gccccgacgg 720
ctcgcccagc gagtgccacg agcatgcaga ctgcgtccta gagcgcgatg gctcgcggtc 780
gtgcgtgtgt cgcgttggct gggccggcaa cgggatcctc tgtggtcgcg acactgacct 840
agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440
cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920
gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct gggggtcttc tgcttctccc aggagaacat 2220
catctgggcc aacctgcgtt accgctgcaa tgacaccatc ccagaggact atgagaccca 2280

tcagctgcgg caagcctagg gaccagggtg aggacccgcc ggatgacagc caccctcacc 2340

gcggctggat gggggctctg cacccagccc aaggggtggc cgtcctgagg gggaagtgag 2400

aagggctcag agaggacaaa ataaagtgtg tgtgcaggg 2439

<210> 7
<211> 2439
<212> DNA
<213> human

<400> 7
cagcacccag ctccccgcca ccgccatggt ccccgacacc gcctgcgttc ttctgctcac 60

cctggctgcc ctcggcgcgt ccggacaggg ccagagcccg ttgggctcag acctgggccc 120

gcagatgctt cgggaactgc aggaaaccaa cgcggcgctg caggacgtgc gggactggct 180

gcggcagcag gtcagggaga tcacgttcct gaaaaacacg gtgatggagt gtgacgcgtg 240

cgggatgcag cagtcagtac gcaccggcct acccagcgtg cggcccctgc tccactgcgc 300

gcccggcttc tgcttccccg gcgtggcctg catccagacg gagagcggcg gccgctgcgg 360

cccctgcccc gcgggcttca cgggcaacgg ctcgcactgc accgacgtca acgagtgcaa 420

cgcccacccc tgcttccccc gagtccgctg tatcaacacc agcccggggt tccgctgcga 480

ggcttgcccg ccggggtaca gcggccccac ccaccagggc gtggggctgg ctttcgccaa 540

ggccaacaag caggtttgca cggacatcaa cgagtgtgag accgggcaac ataactgcgt 600

ccccaactcc gtgtgcatca acacccgggg ctccttccag tgcggcccgt gccagcccgg 660

cttcgtgggc gaccaggcgt ccggctgcca gcgcggcgca cagcgcttct gccccgacgg 720

ctcgcccagc gagtgccacg agcatgcaga ctgcgtccta gagcgcgatg gctcgcggtc 780

gtgcgtgtgt cgcgttggct gggccggcaa cgggatcctc tgtggtcgcg acactgacct 840

agacggcttc ccggacgaga agctgcgctg cccggagccg cagtgccgta aggacaactg 900

cgtgactgtg cccaactcag ggcaggagga tgtggaccgc gatggcatcg gagacgcctg 960

cgatccggat gccgacgggg acggggtccc caatgaaaag gacaactgcc cgctggtgcg 1020

gaacccagac cagcgcaaca cggacgagga caagtggggc gatgcgtgcg acaactgccg 1080

gtcccagaag aacgacgacc aaaaggacac agaccaggac ggccggggcg atgcgtgcga 1140

cgacgacatc gacggcgacc ggatccgcaa ccaggccgac aactgcccta gggtacccaa 1200

ctcagaccag aaggacagtg atggcgatgg tataggggat gcctgtgaca actgtcccca 1260

gaagagcaac ccggatcagg cggatgtgga ccacgacttt gtgggagatg cttgtgacag 1320

```
cgatcaagac caggatggag acggacatca ggactctcgg gacaactgtc ccacggtgcc    1380
taacagtgcc caggaggact cagaccacga tggccagggt gatgcctgcg acgacgacga    1440
cgacaatgac ggagtccctg acagtcggga caactgccgc ctggtgccta accccggcca    1500
ggaggacgcg gacagggacg gcgtgggcga cgtgtgccag gacgactttg atgcagacaa    1560
ggtggtagac aagatcgacg tgtgtccgga gaacgctgaa gtcacgctca ccgacttcag    1620
ggccttccag acagtcgtgc tggacccgga gggtgacgcg cagattgacc ccaactgggt    1680
ggtgctcaac cagggaaggg agatcgtgca gacaatgaac agcgacccag gcctggctgt    1740
gggttacact gccttcaatg gcgtggactt cgagggcacg ttccatgtga acacggtcac    1800
ggatgacgac tatgcgggct tcatctttgg ctaccaggac agctccagct tctacgtggt    1860
catgtggaag cagatggagc aaacgtattg gcaggcgaac cccttccgtg ctgtggccga    1920
gcctggcatc caactcaagg ctgtgaagtc ttccacaggc cccggggaac agctgcggaa    1980
cgctctgtgg catacaggag acacagagtc ccaggtgcgg ctgctgtgga aggacccgcg    2040
aaacgtgggt tggaaggaca agaagtccta tcgttggttc ctgcagcacc ggccccaagt    2100
gggctacatc agggtgcgat tctatgaggg ccctgagctg gtggccgaca gcaacgtggt    2160
cttggacaca accatgcggg gtggccgcct gggggtcttc tgcttctccc aggagaacat    2220
catctgggcc aacctgcgtt accgctgcaa tgacaccatc ccagaggact atgagaccca    2280
tcagctgcgg caagcctagg gaccagggtg aggacccgcc ggatgacagc caccctcacc    2340
gcggctggat gggggctctg cacccagccc aaggggtggc cgtcctgagg gggaagtgag    2400
aagggctcag agaggacaaa ataaagtgtg tgtgcaggg                           2439
```

```
<210>  8
<211>  1700
<212>  DNA
<213>  human

<400>  8
haggtgagcg gttgctcgtc gtcggggcgg ccggcagcgg cggctccagg gcccagcatg      60
cgcgggggac cccgcggcca ccatgtatgt gggctatgtg ctggacaagg attcgcccgt     120
gtaccccggc ccagccaggc cagccagcct cggcctgggc ccggcaaact acggcccccc     180
ggccccgccc ccggcgcccc cgcagtaccc cgacttctcc agctactctc acgtggagcc     240
ggcccccgcg cccccgacgg cctgggggc gcccttccct gcgcccaagg acgactgggc     300
cgccgcctac ggcccgggcc ccgcggcccc tgccgccagc ccagcttcgc tggcattcgg     360
```

gccccctcca gactttagcc cggtgccggc gccccctggg cccggcccgg gcctcctggc 420

gcagcccctc gggggcccgg gcacaccgtc ctcgcccgga gcgcagaggc cgacgcccta 480

cgagtggatg cggcgcagcg tggcggccgg aggcggcggt ggcagcggta agactcggac 540

caaggacaag taccgcgtgg tctacaccga ccaccaacgc ctggagctgg agaaggagtt 600

tcattacagc cgttacatca caatccggcg gaaatcagag ctggctgcca atctggggct 660

cactgaacgg caggtgaaga tctggttcca aaaccggcgg gcaaaggagc gcaaagtgaa 720

caagaagaaa cagcagcagc aacagccccc acagccgccg atggcccacg acatcacggc 780

caccccagcc gggccatccc tgggggggcct gtgtcccagc aacaccagcc tcctggccac 840

ctcctctcca atgcctgtga aagaggagtt tctgccatag ccccatgccc agcctgtgcg 900

ccgggggacc tggggactcg ggtgctggga gtgtggctcc tgtgggccca ggaggtctgg 960

tccgagtctc agccctgacc ttctgggaca tggtggacag tcacctatcc accctctgca 1020

tccccttggc ccattgtgtg cagtaagcct gttggataaa gaccttccag ctcctgtgtt 1080

ctagacctct gggggataag ggagtccagg gtggatgatc tcaatctccc gtgggcatct 1140

caagccccaa atggttgggg gaggggccta gacaaggctc caggccccac ctcctcctcc 1200

atacgttcag aggtgcagct ggaggcctgt gtggggacca cactgatcct ggagaaaagg 1260

gatggagctg aaaaagatgg aatgcttgca gagcatgacc tgaggaggga ggaacgtggt 1320

caactcacac ctgcctcttc tgcagcctca cctctacctg cccccatcat aagggcactg 1380

agcccttccc aggctggata ctaagcacaa agcccatagc actgggctct gatggctgct 1440

ccactgggtt acagaatcac agccctcatg atcattctca gtgagggctc tggattgaga 1500

gggaggccct gggaggagag aagggggcag agtcttccct accaggtttc tacacccccg 1560

ccaggctgcc catcagggcc cagggagccc ccagaggact ttattcggac caagcagagc 1620

tcacagctgg acaggtgttg tatatagagt ggaatctctt ggatgcagct tcaagaataa 1680

atttttcttc tcttttcaaa 1700

<210> 9
<211> 4098
<212> DNA
<213> human

<400> 9
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agctatggag ccgcggccca 180
cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300
ctgcgcccgc gagccgggac ggcggcgggg tccgcgatga gggccccgcg gcggccgggg 360
acgggctggg cagacccttg ggcccaccc cgagccagag ccgtttccag gtggacctgg 420
tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540
gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atcttttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800

gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct ttttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540

tgaaagaaga tgaaccatgg cgaataacag ataatgagct tgaactttat aagaccaaga 3600

cataccggca gatcaggtta aatgagttat taaaggaaca ttcaagcaca gctaatatta 3660

ttgtcatgag tctcccagtt gcacgaaaag gtgctgtgtc tagtgctctc tacatggcat 3720

ggttagaagc tctatctaag gacctaccac caatcctcct agttcgtggg aatcatcaga 3780

gtgtccttac cttctattca taaatgttct atacagtgga cagccctcca gaatggtact 3840

tcagtgccta gtgtagtaac ctgaaatctt caatgacaca ttaacatcac aatggcgaat 3900

ggtgactttt ctttcacgat ttcattaatt tgaaagcaca caggaaagct tgctccattg 3960

ataacgtgta tggagacttc ggttttagtc aattccatat ctcaatctta atggtgattc 4020

ttctctgttg aactgaagtt tgtgagagta gttttccttt gctacttgaa tagcaataaa 4080

agcgtgttaa ctttttgg 4098

<210> 10
<211> 4098
<212> DNA
<213> human

<400> 10
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agctatggag ccgcggccca 180

cggcgccctc ctccggcgcc ccgggactgg ccggggtcgg ggagacgccg tcagccgctg 240

cgctggccgc agccagggtg gaactgcccg gcacggctgt gccctcggtg ccggaggatg 300

ctgcgcccgc gagccgggac ggcggcgggg tccgcgatga gggccccgcg gcggccgggg 360

acgggctggg cagacccttg ggcccaccc cgagccagag ccgtttccag gtggacctgg 420

tttccgagaa cgccgggcgg gccgctgctg cggcggcggc ggcggcggcg gcagcggcgg 480

cggctggtgc tggggcgggg gccaagcaga cccccgcgga cggggaagcc agcggcgaga 540

gcgagccagc taaaggcagc gaggaagcca agggccgctt ccgcgtgaac ttcgtggacc 600

cagctgcctc ctcgtcggct gaagacagcc tgtcagatgc tgccggggtc ggagtcgacg 660

ggcccaacgt gagcttccag aacggcgggg acacggtgct gagcgagggc agcagcctgc 720

actccggcgg cggcggcggc agtgggcacc accagcacta ctattatgat acccacacca 780

acacctacta cctgcgcacc ttcggccaca acaccatgga cgctgtgccc aggatcgatc 840

actaccggca cacagccgcg cagctgggcg agaagctgct ccggcctagc ctggcggagc 900

tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atcttttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580

atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct tttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840
tcagtgccta gtgtagtaac ctgaaatctt caatgacaca ttaacatcac aatggcgaat 3900
ggtgactttt ctttcacgat ttcattaatt tgaaagcaca caggaaagct tgctccattg 3960
ataacgtgta tggagacttc ggttttagtc aattccatat ctcaatctta atggtgattc 4020
ttctctgttg aactgaagtt tgtgagagta gttttccttt gctacttgaa tagcaataaa 4080
agcgtgttaa ctttttgg 4098

<210> 11
<211> 3311
<212> DNA
<213> human

<400> 11

```
tgctaatgct tttggtacaa atggatgtgg aatataattg aatattttct tgtttaaggg      60
gagcatgaag aggtgttgag gttatgtcaa gcatctggca cagctgaagg cagatggaaa     120
tatttacaag tacgcaattt gagactaaga tattgttatc attctcctat tgaagacaag     180
agcaatagta aaacacatca ggtcaggggg ttaaagacct gtgataaacc acttccgata     240
agttggaaac gtgtgtctat attttcatat ctgtatatat ataatggtaa agaaagacac     300
cttcgtaacc cgcattttcc aaagagagga atcacaggga gatgtacagc aatggggcca     360
tttaagagtt ctgtgttcat cttgattctt caccttctag aaggggccct gagtaattca     420
ctcattcagc tgaacaacaa tggctatgaa ggcattgtcg ttgcaatcga ccccaatgtg     480
ccagaagatg aaacactcat tcaacaaata aaggacatgg tgacccaggc atctctgtat     540
ctgtttgaag ctacaggaaa gcgattttat ttcaaaaatg ttgccatttt gattcctgaa     600
acatggaaga caaaggctga ctatgtgaga ccaaaacttg agacctacaa aaatgctgat     660
gttctggttg ctgagtctac tcctccaggt aatgatgaac cctacactga gcagatgggc     720
aactgtggag agaagggtga aaggatccac ctcactcctg atttcattgc aggaaaaaag     780
ttagctgaat atggaccaca aggtaaggca tttgtccatg agtgggctca tctacgatgg     840
ggagtatttg acgagtacaa taatgatgag aaattctact tatccaatgg aagaatacaa     900
gcagtaagat gttcagcagg tattactggt acaaatgtag taaagaagtg tcagggaggc     960
agctgttaca ccaaaagatg cacattcaat aaagttacag gactctatga aaaaggatgt    1020
gagtttgttc tccaatcccg ccagacggag aaggcttcta taatgtttgc acaacatgtt    1080
gattctatag ttgaattctg tacagaacaa aaccacaaca aagaagctcc aaacaagcaa    1140
aatcaaaaat gcaatctccg aagcacatgg gaagtgatcc gtgattctga ggactttaag    1200
aaaaccactc ctatgacaac acagccacca aatcccacct tctcattgct gcagattgga    1260
caaagaattg tgtgtttagt ccttgacaaa tctggaagca tggcgactgg taaccgcctc    1320
aatcgactga atcaagcagg ccagcttttc ctgctgcaga cagttgagct ggggtcctgg    1380
gttgggatgg tgacatttga cagtgctgcc catgtacaaa gtgaactcat acagataaac    1440
agtggcagtg acagggacac actcgccaaa agattacctg cagcagcttc aggagggacg    1500
tccatctgca gcgggcttcg atcggcattt actgtgatta ggaagaaata tccaactgat    1560
ggatctgaaa ttgtgctgct gacggatggg gaagacaaca ctataagtgg gtgctttaac    1620
gaggtcaaac aaagtggtgc catcatccac acagtcgctt tggggccctc tgcagctcaa    1680
```

gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520
aagcaagtgt gtttcagcag aacatcctcg ggaggctcat ttgtggcttc tgatgtccca 2580
aatgctccca tacctgatct cttcccacct ggccaaatca ccgacctgaa ggcggaaatt 2640
cacgggggca gtctcattaa tctgacttgg acagctcctg gggatgatta tgaccatgga 2700
acagctcaca agtatatcat tcgaataagt acaagtattc ttgatctcag agacaagttc 2760
aatgaatctc ttcaagtgaa tactactgct ctcatcccaa aggaagccaa ctctgaggaa 2820
gtctttttgt ttaaaccaga aaacattact tttgaaaatg gcacagatct tttcattgct 2880
attcaggctg ttgataaggt cgatctgaaa tcagaaatat ccaacattgc acgagtatct 2940
ttgtttattc ctccacagac tccgccagag acacctagtc ctgatgaaac gtctgctcct 3000
tgtcctaata ttcatatcaa cagcaccatt cctggcattc acattttaaa aattatgtgg 3060
aagtggatag gagaactgca gctgtcaata gcctagggct gaatttttgt cagataaata 3120
aaataaatca ttcatccttt ttttgattat aaaattttct aaaatgtatt ttagacttcc 3180
tgtagggggc gatatactaa atgtatatag tacatttata ctaaatgtat tcctgtaggg 3240
ggcgatatac taaatgtatt ttagacttcc tgtagggggc gataaaataa aatgctaaac 3300
aactgggtaa a 3311

<210> 12
<211> 3311
<212> DNA
<213> human

<400> 12
tgctaatgct tttggtacaa atggatgtgg aatataattg aatattttct tgtttaaggg 60
gagcatgaag aggtgttgag gttatgtcaa gcatctggca cagctgaagg cagatggaaa 120
tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500

tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980
gtggacaaaa acaccaaaat ggcctacctc caaatcccag gcattgctaa ggttggcact 2040
tggaaataca gtctgcaagc aagctcacaa accttgaccc tgactgtcac gtcccgtgcg 2100
tccaatgcta ccctgcctcc aattacagtg acttccaaaa cgaacaagga caccagcaaa 2160
ttccccagcc ctctggtagt ttatgcaaat attcgccaag gagcctcccc aattctcagg 2220
gccagtgtca cagccctgat tgaatcagtg aatggaaaaa cagttacctt ggaactactg 2280
gataatggag caggtgctga tgctactaag gatgacggtg tctactcaag gtatttcaca 2340
acttatgaca cgaatggtag atacagtgta aaagtgcggg ctctgggagg agttaacgca 2400
gccagacgga gagtgatacc ccagcagagt ggagcactgt acatacctgg ctggattgag 2460
aatgatgaaa tacaatggaa tccaccaaga cctgaaatta ataaggatga tgttcaacac 2520
aagcaagtgt gtttcagcag aacatcctcg ggaggctcat ttgtggcttc tgatgtccca 2580
aatgctccca tacctgatct cttcccacct ggccaaatca ccgacctgaa ggcggaaatt 2640
cacgggggca gtctcattaa tctgacttgg acagctcctg gggatgatta tgaccatgga 2700
acagctcaca agtatatcat tcgaataagt acaagtattc ttgatctcag agacaagttc 2760
aatgaatctc ttcaagtgaa tactactgct ctcatcccaa aggaagccaa ctctgaggaa 2820
gtctttttgt ttaaaccaga aaacattact tttgaaaatg gcacagatct tttcattgct 2880
attcaggctg ttgataaggt cgatctgaaa tcagaaatat ccaacattgc acgagtatct 2940
ttgtttattc ctccacagac tccgccagag acacctagtc ctgatgaaac gtctgctcct 3000
tgtcctaata ttcatatcaa cagcaccatt cctggcattc acattttaaa aattatgtgg 3060
aagtggatag gagaactgca gctgtcaata gcctagggct gaatttttgt cagataaata 3120
aaataaatca ttcatccttt ttttgattat aaaattttct aaaatgtatt ttagacttcc 3180
tgtagggggc gatatactaa atgtatatag tacatttata ctaaatgtat tcctgtaggg 3240

ggcgatatac taaatgtatt ttagacttcc tgtagggggc gataaaataa aatgctaaac 3300

aactgggtaa a 3311

<210> 13
<211> 2609
<212> DNA
<213> human

<220>
<221> misc_feature
<222> (2025)..(2025)
<223> any kind of base

<220>
<221> misc_feature
<222> (2036)..(2036)
<223> any kind of base

<220>
<221> misc_feature
<222> (2164)..(2164)
<223> any kind of base

<220>
<221> misc_feature
<222> (2264)..(2264)
<223> any kind of base

<400> 13
gctgatagca cagttctgtc cagagaagga aggcggaata aacttattca ttcccaggaa 60

ctcttggggt aggtgtgtgt ttttcacatc ttaaaggctc acagaccctg cgctggacaa 120

atgttccatt cctgaaggac ctctccagaa tccggattgc tgaatcttcc ctgttgccta 180

gaagggctcc aaaccacctc ttgacaatgg gaaactgggt ggttaaccac tggttttcag 240

ttttgtttct ggttgtttgg ttagggctga atgttttcct gtttgtggat gccttcctga 300

aatatgagaa ggccgacaaa tactactaca caagaaaaat ccttgggtca acattggcct 360

gtgcccgagc gtctgctctc tgcttgaatt ttaacagcac gctgatcctg cttcctgtgt 420

gtcgcaatct gctgtccttc ctgaggggca cctgctcatt ttgcagccgc acactgagaa 480

agcaattgga tcacaacctc accttccaca agctggtggc ctatatgatc tgcctacata 540

cagctattca catcattgca cacctgttta actttgactg ctatagcaga agccgacagg 600

ccacagatgg ctcccttgcc tccattctct ccagcctatc tcatgatgag aaaaaggggg 660

```
gttcttggct aaatcccatc cagtcccgaa acacgacagt ggagtatgtg acattcacca     720
gcgttgctgg tctcactgga gtgatcatga caatagcctt gattctcatg gtaacttcag     780
ctactgagtt catccggagg agttattttg aagtcttctg gtatactcac caccttttta     840
tcttctatat ccttggctta gggattcacg gcattggtgg aattgtccgg ggtcaaacag     900
aggagagcat gaatgagagt catcctcgca agtgtgcaga gtcttttgag atgtgggatg     960
atcgtgactc ccactgtagg cgccctaagt ttgaagggca tccccctgag tcttggaagt    1020
ggatccttgc accggtcatt ctttatatct gtgaaaggat cctccggttt taccgctccc    1080
agcagaaggt tgtgattacc aaggttgtta tgcacccatc caaagttttg gaattgcaga    1140
tgaacaagcg tggcttcagc atggaagtgg ggcagtatat ctttgttaat tgcccctcaa    1200
tctctctcct ggaatggcat ccttttactt tgacctctgc tccagaggaa gatttcttct    1260
ccattcatat ccgagcagca ggggactgga cagaaaatct cataagggct ttcgaacaac    1320
aatattcacc aattcccagg attgaagtgg atggtccctt tggcacagcc agtgaggatg    1380
ttttccagta tgaagtggct gtgctggttg gagcaggaat tggggtcacc ccctttgctt    1440
ctatcttgaa atccatctgg tacaaattcc agtgtgcaga ccacaacctc aaaacaaaaa    1500
agatctattt ctactggatc tgcagggaga caggtgcctt ttcctggttc aacaacctgt    1560
tgacttccct ggaacaggag atggaggaat taggcaaagt gggttttcta aactaccgtc    1620
tcttcctcac cggatgggac agcaatattg ttggtcatgc agcattaaac tttgacaagg    1680
ccactgacat cgtgacaggt ctgaaacaga aaacctcctt tgggagacca atgtgggaca    1740
atgagttttc tacaatagct acctcccacc ccaagtctgt agtgggagtt ttcttatgtg    1800
gccctcggac tttggcaaag agcctgcgca aatgctgtca ccgatattcc agtctggatc    1860
ctagaaaggt tcaattctac ttcaacaaag aaaatttttg agttatagga ataaggacgg    1920
taatctgcat tttgtctctt tgtatcttca gtaattgagt tataggaata aggacggtaa    1980
tctgcatttt gtctctttgt atcttcagta atttacttgg tctcntcagg tttgancagt    2040
cactttagga taagaatgtg cctctcaagc cttgactccc tggtattctt tttttgattg    2100
cattcaactt cgttacttga gcttcagcaa cttaagaact tctgaagttc ttaaagttct    2160
gaanttctta aagcccatgg atcctttctc agaaaaataa ctgtaaatct ttctggacag    2220
ccatgactgt agcaaggctt gatagcagaa gtttggtggt tcanaattat acaactaatc    2280
ccaggtgatt ttatcaattc cagtgttacc atctcctgag ttttggtttg taatcttttg    2340
```

tccctcccac ccccacagaa gattttaagt agggtgactt tttaaataaa aatttattga 2400

ataattaatg ataaaacata ataataaaca taaataataa acaaaattac cgagaacccc 2460

atccccatat aacaccaaca gtgtacatgt ttactgtcac ttttgatatg gtttatccag 2520

tgtgaacagc aatttattat ttttgctcat caaaaaataa aggatttttt ttcacttgaa 2580

aaaaaaaaaa aaaaaaaaaa aaaaaaaaa 2609

<210> 14
<211> 15720
<212> DNA
<213> human

<400> 14
caacccacac cgcccctgcc agccaccatg gggctgccac tagcccgcct ggcggctgtg 60

tgcctggccc tgtctttggc aggggctcg gagctccaga cagagggcag aacccgatac 120

cacggccgca acgtctgcag cacctggggc aacttccact acaagacctt cgacggggac 180

gtcttccgct tccccggcct ctgcgactac aacttcgcct ccgactgccg aggctcctac 240

aaggaatttg ctgtgcacct gaagcggggt ccgggccagg ctgaggcccc cgccggggtg 300

gagtccatcc tgctgaccat caaggatgac accatctacc tcacccgcca cctggctgtg 360

cttaacgggg ccgtggtcag caccccgcac tacagccccg ggctgctcat tgagaagagc 420

gatgcctaca ccaaagtcta ctcccgcgcc ggcctcaccc tcatgtggaa ccgggaggat 480

gcactcatgc tggagctgga cactaagttc cggaaccaca cctgtggcct ctgcggggac 540

tacaacggcc tgcagagcta ttcagaattc ctctctgacg gcgtgctctt cagtcccctg 600

gagtttggga acatgcagaa gatcaaccag cccgatgtgg tgtgtgagga tcccgaggag 660

gaggtggccc ccgcatcctg ctccgagcac cgcgccgagt gtgagaggct gctgaccgcc 720

gaggccttcg cggactgtca ggacctggtg ccgctggagc cgtatctgcg cgcctgccag 780

caggaccgct gccggtgccc gggcggtgac acctgcgtct gcagcaccgt ggccgagttc 840

tcccgccagt gctcccacgc cggcggccgg cccgggaact ggaggaccgc cacgctctgc 900

cccaagacct gccccgggaa cctggtgtac ctggagagcg gctcgccctg catggacacc 960

tgctcacacc tggaggtgag cagcctgtgc gaggagcacc gcatggacgg ctgtttctgc 1020

ccagaaggca ccgtatatga cgacatcggg gacagtggct gcgttcctgt gagccagtgc 1080

cactgcaggc tgcacggaca cctgtacaca ccgggccagg agatcaccaa tgactgcgag 1140

cagtgtgtct gtaacgctgg ccgctgggtg tgcaaagacc tgccctgccc cggcacctgt 1200

gccctggaag gcggctccca catcaccacc ttcgatggga agacgtacac cttccacggg 1260
gactgctact atgtcctggc caagggtgac cacaacgatt cctacgctct cctgggcgag 1320
ctggccccct gtggctccac agacaagcag acctgcctga agacggtggt gctgctggct 1380
gacaagaaga agaatgcggt ggtcttcaag tccgatggca gtgtactgct caaccagctg 1440
caggtgaacc tgccccacgt gaccgcgagc ttctctgtct tccgcccgtc ttcctaccac 1500
atcatggtga gcatggccat tggcgtccgg ctgcaggtgc agctggcccc agtcatgcaa 1560
ctctttgtga cactggacca ggcctcccag gggcaggtgc agggcctctg cgggaacttc 1620
aacggcctgg aaggtgacga cttcaagacg gccagcgggc tggtggaggc cacgggggcc 1680
ggctttgcca acacctggaa ggcacagtca acctgccatg acaagctgga ctggttggac 1740
gatccctgct ccctgaacat cgagagcgcc aactacgccg agcactggtg ctccctcctg 1800
aagaagacag agaccccctt tggcaggtgc cactcggctg tggaccctgc tgagtattac 1860
aagaggtgca aatatgacac gtgtaactgt cagaacaatg aggactgcct gtgcgccgcc 1920
ctgtcctcct acgcgcgcgc ctgcaccgcc aagggcgtca tgctgtgggg ctggcgggag 1980
catgtctgca acaaggatgt gggctcctgc cccaactcgc aggtcttcct gtacaacctg 2040
accacctgcc agcagacctg ccgctccctc tccgaggccg acagccactg tctcgagggc 2100
tttgcgcctg tggacggctg cggctgccct gaccacacct tcctggacga gaagggccgc 2160
tgcgtacccc tggccaagtg ctcctgttac caccgcggtc tctacctgga ggcgggggat 2220
gtggtcgtca ggcaggaaga acgatgtgtg tgccgggatg ggcggctgca ctgtaggcag 2280
atccggctga tcggccagag ctgcacggcc ccaaagatcc acatggactg cagcaacctg 2340
actgcactgg ccacctcgaa gccccgagcc ctcagctgcc agacgctggc cgccggctat 2400
taccacacag agtgtgtcag tggctgtgtg tgccccgacg ggctgatgga tgacggccgg 2460
ggtggctgcg tggtggagaa ggaatgccct tgcgtccata acaacgacct gtattcttcc 2520
ggcgccaaga tcaaggtgga ctgcaatacc tgcacctgca agagaggacg ctgggtgtgc 2580
acccaggctg tgtgccatgg cacctgctcc atttacggga gtggccacta catcaccttt 2640
gatgggaagt actacgactt tgacggacac tgctcctacg tggctgttca ggactactgc 2700
ggccagaact cctcactggg ctcattcagc atcatcaccg agaacgtccc ctgtggcact 2760
acgggcgtca cctgctccaa ggccatcaag atcttcatgg ggaggacgga gctgaagttg 2820
gaagacaagc accgtgtggt gatccagcgt gatgagggtc accacgtggc ctacaccacg 2880
cgggaggtgg gccagtacct ggtggtggag tccagcacgg gcatcatcgt catctgggac 2940

aagaggacca ccgtgttcat caagctggct ccctcctaca agggcaccgt gtgtggcctg 3000
tgtgggaact ttgaccaccg ctccaacaac gacttcacca cgcgggacca catggtggtg 3060
agcagcgagc tggacttcgg gaacagctgg aaggaggccc ccacctgccc agatgtgagc 3120
accaaccccg agccctgcag cctgaacccg caccgccgct cctgggccga gaagcagtgc 3180
agcatcctca aaagcagcgt gttcagcatc tgccacagca aggtggaccc caagcccttc 3240
tacgaggcct gtgtgcacga ctcgtgctcc tgtgacacgg gtggggactg tgagtgcttc 3300
tgctctgccg tggcctccta cgcccaggag tgtaccaaag aggggggcctg cgtgttctgg 3360
aggacgccgg acctgtgccc catattctgc gactactaca accctccgca tgagtgtgag 3420
tggcactatg agccatgtgg gaaccggagc ttcgagacct gcaggaccat caacggcatc 3480
cactccaaca tctccgtgtc ctacctggag ggctgctacc cccggtgccc caaggacagg 3540
cccatctatg aggaggatct gaagaagtgt gtcactgcag acaagtgtgg ctgctatgtc 3600
gaggacaccc actacccacc tggagcatcg gttcccaccg aggagacctg caagtcctgc 3660
gtgtgtacca actcctccca agtcgtctgc aggccggagg aaggaaagat tcttaaccag 3720
acccaggatg gcgccttctg ctactgggag atctgtggcc ccaacgggac ggtggagaag 3780
cacttcaaca tctgttccat tacgacacgc ccgtccaccc tgaccacctt caccaccatc 3840
accctcccca ccacccccac ctccttcacc actaccacca ccaccaccac cccgacctcc 3900
agcacagttt tatcaacaac tccgaagctg tgctgcctct ggtctgactg gatcaatgag 3960
gaccacccca gcagtggcag cgacgacggt gaccgagaac catttgatgg ggtctgcggg 4020
gcccctgagg acatcgagtg caggtcggtc aaggatcccc acctcagctt ggagcagcat 4080
ggccagaagg tgcagtgtga tgtctctgtt gggttcattt gcaagaatga agaccagttt 4140
ggaaatggac catttggact gtgttacgac tacaagatac gtgtcaattg ttgctggccc 4200
atggataagt gtatcaccac tcccagccct ccaactacca ctcccagccc tccaccaacc 4260
acgacgacca cccttccacc aaccaccacc cccagccctc caaccaccac cacaaccacc 4320
cctccaccaa ccaccacccc cagccctcca ataaccacca cgaccacccc tctaccaacc 4380
accactccca gccctccaat aagcaccaca accacccctc caccaaccac cactcccagc 4440
cctccaacca ccactcccag ccctccaacc accactccca gccctccaac aaccaccaca 4500
accacccctc caccaaccac cactcccagc cctccaatga ctacgcccat cactccacca 4560
gccagcacta ccacccttcc accaaccacc actcccagcc ctccaacaac caccacaacc 4620

acccctccac caaccaccac tcccagtcct ccaacgacta cgcccatcac tccaccaacc 4680

agcactacta cccttccacc aaccaccact cccagccctc caccaaccac cacaaccacc 4740

cctccaccaa ccaccactcc cagccctcca acaaccacca ctcccagtcc tccaacaatc 4800

accacaacca cccctccacc aaccaccact cccagccctc caacaacgac cacaaccacc 4860

cctccaccaa ccaccactcc cagccctcca acgactacac ccatcactcc accaaccagc 4920

actaccaccc ttccaccaac caccactccc agccctccac caaccaccac aaccacccct 4980

ccaccaacca ccactcccag ccctccaaca accaccactc ccagccctcc aataaccacc 5040

acaaccaccc ctccaccaac caccactccc agctctccaa taaccaccac tcccagccct 5100

ccaacaacca ccatgaccac cccttcacca accaccaccc ccagctctcc aataaccacc 5160

acaaccaccc cttcctcaac taccactccc agccctccac caaccaccat gaccacccct 5220

tcaccaacca ccactcccag ccctccaaca accaccatga ccacccttcc accaaccacc 5280

acttccagcc ctctaacaac tactcctcta cctccatcaa taactcctcc tacattttca 5340

ccattctcaa cgacaacccc tactacccca tgcgtgcctc tctgcaattg gactggctgg 5400

ctggattctg gaaaacccaa ctttcacaaa ccaggtggag acacagaatt gattggagac 5460

gtctgtggac caggctgggc agctaacatc tcttgcagag ccaccatgta tcctgatgtt 5520

cccattggac agcttggaca aacagtggtg tgtgatgtct ctgtggggct gatatgcaaa 5580

aatgaagacc aaaagccagg tggggtcatc cctatggcct tctgcctcaa ctacgagatc 5640

aacgttcagt gctgtgagtg tgtcacccaa cccaccacca tgacaaccac caccacagag 5700

aacccaactc cgccaaccac gacacccatc accaccacca ctacggtgac ccccaacccca 5760

acacccaccg gcacacagac ccccaaccacg acacccatca ccaccaccac tacggtgacc 5820

ccaaccccaa cacccaccgg cacacagacc ccaaccacga cacccatcac caccaccact 5880

acggtgaccc caaccccaac acccaccggc acacagaccc caaccacgac acccatcacc 5940

accaccacta cggtgacccc aaccccaaca cccaccggca cacagacccc aaccacgaca 6000

cccatcacca ccaccactac ggtgacccca accccaacac ccaccggcac acagacccca 6060

accacgacac ccatcaccac caccactacg gtgaccccaa ccccaacacc caccggcaca 6120

cagaccccaa ccacgacacc catcaccacc accactacgg tgaccccaac cccaacaccc 6180

accggcacac agaccccaac cacgacaccc atcaccacca ccactacggt gaccccaacc 6240

ccaacaccca ccggcacaca gaccccaacc acgacaccca tcaccaccac cactacggtg 6300

accccaaccc caacacccac cggcacacag accccaacca cgacacccat caccaccacc 6360

actacggtga ccccaacccc aacacccacc ggcacacaga ccccaaccac gacacccatc 6420
accaccacca ctacggtgac cccaacccca acacccaccg gcacacagac cccaaccacg 6480
acacccatca ccaccaccac tacggtgacc ccaaccccaa cacccaccgg cacacagacc 6540
ccaaccacga cacccatcac caccaccact acggtgaccc caaccccaac acccaccggc 6600
acacagaccc caaccacgac acccatcacc accaccacta cggtgacccc aaccccaaca 6660
cccaccggca cacagacccc aaccacgaca cccatcacca ccaccactac ggtgacccca 6720
accccaacac ccaccggcac acagacccca accacgacac ccatcaccac caccactacg 6780
gtgaccccaa ccccaacacc caccggcaca cagaccccaa ccacgacacc catcaccacc 6840
accactacgg tgaccccaac cccaacaccc accggcacac agaccccaac cacgacaccc 6900
atcaccacca ccactacggt gaccccaacc ccaacaccca ccggcacaca gaccccaacc 6960
acgacaccca tcaccaccac cactacggtg accccaaccc caacacccac cggcacacag 7020
accccaacca cgacacccat caccaccacc actacggtga ccccaacccc aacacccacc 7080
ggcacacaga ccccaaccac gacacccatc accaccacca ctacggtgac cccaacccca 7140
acacccaccg gcacacagac cccaaccacg acacccatca ccaccaccac tacggtgacc 7200
ccaaccccaa cacccaccgg cacacagacc ccaaccacga cacccatcac caccaccact 7260
acggtgaccc caaccccaac acccaccggc acacagaccc caaccacgac acccatcacc 7320
accaccacta cggtgacccc aaccccaaca cccaccggca cacagacccc aaccacgaca 7380
cccatcacca ccaccactac ggtgacccca accccaacac ccaccggcac acagacccca 7440
accacgacac ccatcaccac caccactacg gtgaccccaa ccccaacacc caccggcaca 7500
cagaccccaa ccacgacacc catcaccacc accactacgg tgaccccaac cccaacaccc 7560
accggcacac agaccccaac cacgacaccc atcaccacca ccactacggt gaccccaacc 7620
ccaacaccca ccggcacaca gaccccaacc acgacaccca tcaccaccac cactacggtg 7680
accccaaccc caacacccac cggcacacag accccaacca cgacacccat caccaccacc 7740
actacggtga ccccaacccc aacacccacc ggcacacaga ccccaaccac gacacccatc 7800
accaccacca ctacggtgac cccaacccca acacccaccg gcacacagac cccaaccacg 7860
acacccatca ccaccaccac tacggtgacc ccaaccccaa cacccaccgg cacacagacc 7920
ccaaccacga cacccatcac caccaccact acggtgaccc caaccccaac acccaccggc 7980
acacagaccc caaccacgac acccatcacc accaccacta cggtgacccc aaccccaaca 8040

cccaccggca cacagacccc aaccacgaca cccatcacca ccaccactac ggtgacccca 8100
accccaacac ccaccggcac acagacccca accacgacac ccatcaccac caccactacg 8160
gtgaccccaa ccccaacacc caccggcaca cagaccccaa ccacgacacc catcaccacc 8220
accactacgg tgaccccaac cccaacaccc accggcacac agaccccaac cacgacaccc 8280
atcaccacca ccactacggt gaccccaacc ccaacaccca ccggcacaca gaccccaacc 8340
acgacaccca tcaccaccac cactacggtg accccaaccc caacacccac cggcacacag 8400
accccaacca cgacacccat caccaccacc actacggtga ccccaacccc aacacccacc 8460
ggcacacaga ccccaaccac gacacccatc accaccacca ctacggtgac cccaacccca 8520
acacccaccg gcacacagac cccaaccacg acacccatca ccaccaccac tacggtgacc 8580
ccaaccccaa cacccaccgg cacacagacc ccaaccacga cacccatcac caccaccact 8640
acggtgaccc caaccccaac acccaccggc acacagaccc caaccacgac acccatcacc 8700
accaccacta cggtgacccc aaccccaaca cccaccggca cacagacccc aaccacgaca 8760
cccatcacca ccaccactac ggtgacccca accccaacac ccaccggcac acagacccca 8820
accacgacac ccatcaccac caccactacg gtgaccccaa ccccaacacc caccggcaca 8880
cagaccccaa ccacgacacc catcaccacc accactacgg tgaccccaac cccaacaccc 8940
accggcacac agaccccaac cacgacaccc atcaccacca ccactacggt gaccccaacc 9000
ccaacaccca ccggcacaca gaccccaacc acgacaccca tcaccaccac cactacggtg 9060
accccaaccc caacacccac cggcacacag accccaacca cgacacccat caccaccacc 9120
actacggtga ccccaacccc aacacccacc ggcacacaga ccccaaccac gacacccatc 9180
accaccacca ctacggtgac cccaacccca acacccaccg gcacacagac cccaaccacg 9240
acacccatca ccaccaccac tacggtgacc ccaaccccaa cacccaccgg cacacagacc 9300
ccaaccacga cacccatcac caccaccact acggtgaccc caaccccaac acccaccggc 9360
acacagaccc caaccacgac acccatcacc accaccacta cggtgacccc aaccccaaca 9420
cccaccggca cacagacccc aaccacgaca cccatcacca ccaccactac ggtgacccca 9480
accccaacac ccaccggcac acagacccca accacgacac ccatcaccac caccactacg 9540
gtgaccccaa ccccaacacc caccggcaca cagaccccaa ccacgacacc catcaccacc 9600
accactacgg tgaccccaac cccaacaccc accggcacac agaccccaac cacgacaccc 9660
atcaccacca ccactacggt gaccccaacc ccaacaccca ccggcacaca gaccccaacc 9720
acgacaccca tcaccaccac cactacggtg accccaaccc caacacccac cggcacacag 9780

accccaacca cgacacccat caccaccacc actacggtga ccccaacccc aacacccacc 9840
ggcacacaga ccccaaccac gacacccatc accaccacca ctacggtgac cccaacccca 9900
acacccaccg gcacacagac cccaaccacg acacccatca ccaccaccac tacggtgacc 9960
ccaaccccaa cacccaccgg cacacagacc ccaaccacga cacccatcac caccaccact 10020
acggtgaccc caaccccaac acccaccggc acacagaccc caaccacgac acccatcacc 10080
accaccacta cggtgacccc aaccccaaca cccaccggca cacagacccc aaccacgaca 10140
cccatcacca ccaccactac ggtgacccca accccaacac ccaccggcac acagacccca 10200
accacgacac ccatcaccac caccactacg gtgaccccaa ccccaacacc caccggcaca 10260
cagaccccaa ccacgacacc catcaccacc accactacgg tgaccccaac cccaacaccc 10320
accggcacac agaccccaac cacgacaccc atcaccacca ccactacggt gaccccaacc 10380
ccaacaccca ccggcacaca gaccccaacc acgacaccca tcaccaccac cactacggtg 10440
accccaaccc caacacccac cggcacacag accccaacca cgacacccat caccaccacc 10500
actacggtga ccccaacccc aacacccacc ggcacacaga ccccaaccac gacacccatc 10560
accaccacca ctacggtgac cccaacccca acacccaccg gcacacagac cccaaccacg 10620
acacccatca ccaccaccac tacggtgacc ccaaccccaa cacccaccgg cacacagacc 10680
ccaaccacga cacccatcac caccaccact acggtgaccc caaccccaac acccaccggc 10740
acacagaccc caaccacgac acccatcacc accaccacta cggtgacccc aaccccaaca 10800
cccaccggca cacagacccc aaccacgaca cccatcacca ccaccactac ggtgacccca 10860
accccaacac ccaccggcac acagacccca accacgacac ccatcaccac caccactacg 10920
gtgaccccaa ccccaacacc caccggcaca cagaccccaa ccacgacacc catcaccacc 10980
accactacgg tgaccccaac cccaacaccc accggcacac agaccccaac cacgacaccc 11040
atcaccacca ccactacggt gaccccaacc ccaacaccca ccggcacaca gaccccaacc 11100
acgacaccca tcaccaccac cactacggtg accccaaccc caacacccac cggcacacag 11160
accccaacca cgacacccat caccaccacc actacggtga ccccaacccc aacacccacc 11220
ggcacacaga ccccaaccac gacacccatc accaccacca ctacggtgac cccaacccca 11280
acacccaccg gcacacagac cccaaccacg acacccatca ccaccaccac tacggtgacc 11340
ccaaccccaa cacccaccgg cacacagacc ccaaccacga cacccatcac caccaccact 11400
acggtgaccc caaccccaac acccaccggc acacagaccc caaccacgac acccatcacc 11460

accaccacta cggtgacccc aaccccaaca cccaccggca cacagacccc aaccacgaca 11520
cccatcacca ccaccactac ggtgacccca accccaacac ccaccggcac acagacccca 11580
accacgacac ccatcaccac caccactacg gtgaccccaa ccccaacacc caccggcaca 11640
cagaccccaa ccacgacacc catcaccacc accactacgg tgaccccaac cccaacaccc 11700
accggcacac agaccccaac cacgacaccc atcaccacca ccactacggt gaccccaacc 11760
ccaacaccca ccggcacaca gaccccaacc acgacaccca tcaccaccac cactacggtg 11820
accccaaccc caacacccac cggcacacag accccaacca cgacacccat caccaccacc 11880
actacggtga ccccaacccc aacacccacc ggcacacaga ccccaaccac gacacccatc 11940
accaccacca ctacggtgac cccaacccca acacccaccg gcacacagac cccaaccacg 12000
acacccatca ccaccaccac tacggtgacc ccaaccccaa cacccaccgg cacacagacc 12060
ccaaccacga cacccatcac caccaccact acggtgaccc caaccccaac acccaccggc 12120
acacagaccc caaccacgac acccatcacc accaccacta cggtgacccc aaccccaaca 12180
cccaccggca cacagacccc aaccacgaca cccatcacca ccaccactac ggtgacccca 12240
accccaacac ccaccggcac acagacccca accacgacac ccatcaccac caccactacg 12300
gtgaccccaa ccccaacacc caccggcaca cagaccccaa ccacgacacc catcaccacc 12360
accactacgg tgaccccaac cccaacaccc accggcacac agaccccaac cacgacaccc 12420
atcaccacca ccactacggt gaccccaacc ccaacaccca ccggcacaca gaccccaacc 12480
acgacaccca tcaccaccac cactacggtg accccaaccc caacacccac cggcacacag 12540
accccaacca cgacacccat caccaccacc actacggtga ccccaacccc aacacccacc 12600
ggcacacaga ccgggccccc cacccacaca agcacagcac cgattgctga gttgaccaca 12660
tccaatcctc cgcctgagtc ctcaacccct cagacctctc ggtccacctc ttcccctctc 12720
acggagtcaa ccacccttct gagtacccta ccacctgcca ttgagatgac cagcacggcc 12780
ccaccctcca cacccacggc acccacgacc acgagcggag gccacacact gtctccaccg 12840
cccagcacca ccacgtcccc tccaggcacc cccactcgcg gtaccacgac cgggtcatct 12900
tcagccccca cccccagcac tgtgcagacg accaccacca gtgcctggac cccaacgccg 12960
accccactct ccacacccag catcatcagg accacaggcc tgaggcccta cccttcctct 13020
gtgcttatct gctgtgtcct gaacgacacc tactacgcac caggtgagga ggtgtacaac 13080
ggcacatacg gagacacctg ttatttcgtc aactgctcac tgagctgtac gttggagttc 13140
tataactggt cctgcccatc cacgccctcc ccaacaccca cgccctccaa gtcgacgccc 13200

acgccttcca agccatcgtc cacgccctcc aagccgacgc ccggcaccaa gcccccgag 13260
tgcccagact ttgatcctcc cagacaggag aacgagactt ggtggctgtg cgactgcttc 13320
atggccacgt gcaagtacaa caacacggtg gagatcgtga aggtggagtg tgagccgccg 13380
cccatgccca cctgctccaa cggcctccaa cccgtgcgcg tcgaggaccc cgacggctgc 13440
tgctggcact gggagtgcga ctgctactgc acgggctggg gcgacccgca ctatgtcacc 13500
ttcgacggac tctactacag ctaccagggc aactgcacct acgtgctggt ggaggagatc 13560
agcccctccg tggacaactt cggagtttac atcgacaact accactgcga tcccaacgac 13620
aaggtgtcct gtccccgcac cctcatcgtg cgccacgaga cccaggaggt gctgatcaag 13680
accgtgcata tgatgcccat gcaggtgcag gtgcaggtga acaggcaggc ggtggcactg 13740
ccctacaaga agtacgggct ggaggtgtac cagtctggca tcaactacgt ggtggacatc 13800
cccgagctgg gtgtcctcgt ctcctacaat ggcctgtcct tctccgtcag gctgccctac 13860
caccggtttg gcaacaacac caagggccag tgtggcacct gcaccaacac cacctccgac 13920
gactgcattc tgcccagcgg ggagatcgtc tccaactgtg aggctgcggc tgaccagtgg 13980
ctggtgaacg acccctccaa gccacactgc cccacagca gctccacgac caagcgcccg 14040
gccgtcactg tgcccggggg cggtaaaacg accccacaca aggactgcac cccatctccc 14100
ctctgccagc tcatcaagga cagcctgttt gcccagtgcc acgcactggt gcccccgcag 14160
cactactacg atgcctgcgt gttcgacagc tgcttcatgc cgggctcgag cctggagtgc 14220
gccagtctgc aggcctacgc agccctctgt gcccagcaga acatctgcct cgactggcgg 14280
aaccacacgc atggggcctg cttggtggag tgcccatctc acagggagta ccaggcctgt 14340
ggccctgcag aagagcccac gtgcaaatcc agctcctccc agcagaacaa cacagtcctg 14400
gtggaaggct gcttctgtcc tgagggcacc atgaactacg ctcctggctt tgatgtctgc 14460
gtgaagacct gcggctgtgt gggacctgac aatgtgccca gagagtttgg ggagcacttc 14520
gagttcgact gcaagaactg tgtctgcctg gagggtggaa gtggcatcat ctgccaaccc 14580
aagaggtgca gccagaagcc cgttacccac tgcgtggaag acggcaccta cctcgccacg 14640
gaggtcaacc ctgccgacac ctgctgcaac attaccgtct gcaagtgcaa caccagcctg 14700
tgcaaagaga agccctccgt gtgcccgctg ggattcgaag tgaagagcaa gatggtgcct 14760
ggaaggtgct gtcccttcta ctggtgtgag tccaaggggg tgtgtgttca cgggaatgct 14820
gagtaccagc ccggttctcc agtttattcc tccaagtgcc aggactgcgt gtgcacggac 14880

aaggtggaca acaacaccct gctcaacgtc atcgcctgca cccacgtgcc ctgcaacacc 14940

tcctgcagcc ctggcttcga actcatggag gcccccgggg agtgctgtaa gaagtgtgaa 15000

cagacgcact gtatcatcaa acggcccgac aaccagcacg tcatcctgaa gcccggggac 15060

ttcaagagcg acccgaagaa caactgcaca ttcttcagct gcgtgaagat ccacaaccag 15120

ctcatctcgt ccgtctccaa catcacctgc cccaactttg atgccagcat ttgcatcccg 15180

ggctccatca cattcatgcc caatggatgc tgcaagacct gcacccctcg caatgagacc 15240

agggtgccct gctccaccgt ccccgtcacc acggaggttt cgtacgccgg ctgcaccaag 15300

accgtcctca tgaatcattg ctccgggtcc tgcgggacat ttgtcatgta ctcggccaag 15360

gcccaggccc tggaccacag ctgctcctgc tgcaaagagg agaaaaccag ccagcgtgag 15420

gtggtcctga gctgccccaa tggcggctcg ctgacacaca cctacaccca catcgagagc 15480

tgccagtgcc aggacaccgt ctgcgggctc ccaccggca cctcccgccg ggcccggcgc 15540

tcccctaggc atctggggag cgggtgagcg gggtgggcac agccccttc actgccctcg 15600

acagctttac ctcccccgga ccctctgagc ctcctaagct cggcttcctc tcttcagata 15660

tttattgtct gagtctttgt tcagtccttg ctttccaata ataaactcag gggacatgc 15720

<210> 15
<211> 3697
<212> DNA
<213> human

<400> 15
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420

gctaatggaa ttatagtgga gggtccagtc cctatcacca tagaagtgaa ggacatcaac 480

gacaatcgac ccacgtttct ccagtcaaag tacgaaggct cagtaaggca gaactctcgc 540

ccaggaaagc ccttcttgta tgtcaatgcc acagacctgg atgatccggc cactcccaat 600

ggccagcttt attaccagat tgtcatccag cttcccatga tcaacaatgt catgtacttt 660

cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag accctttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccaccct tggaaggcat tgtttcttta 2400

ccagttacat tctgcagttg tgtggaagga agttgtttcc ggccagcagg tcaccagact 2460

gggataccca ctgtgggcat ggcagttggt atactgctga ccacccttct ggtgattggt 2520

ataattttag cagttgtgtt tatccgcata aagaaggata aaggcaaaga taatgttgaa 2580

agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700

tgcattataa ttttttaaac agatattccc tcttgtcctt taatatttgc taaatatttc 2760

tttttgagg tggagtcttg ctctgtcgcc caggctggag tacagtggtg tgatcccagc 2820

tcactgcaac ctccgcctcc tgggttcaca tgattctcct gcctcagctt cctaagtagc 2880

tgggtttaca ggcacccacc accatgccca gctaattttt gtatttttaa tagagacggg 2940

gtttcgccat ttggccaggc tggtcttgaa ctcctgacgt caagtgatct gcctgccttg 3000

gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tcccttttgg ggcaagacag actcattaaa tattctgtac atttttttctt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 16
<211> 3697
<212> DNA
<213> human

<400> 16
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240
ggccaagaac cgagtcaaat tatattccag tttaaggcca atcctcctgc tgtgactttt 300
gaactaactg gggagacaga caacatattt gtgatagaac gggagggact tctgtattac 360
aacagagcct tggacaggga aacaagatct actcacaatc tccaggttgc agccctggac 420
gctaatggaa ttatagtgga gggtccagtc cctatcacca tagaagtgaa ggacatcaac 480
gacaatcgac ccacgtttct ccagtcaaag tacgaaggct cagtaaggca gaactctcgc 540
ccaggaaagc ccttcttgta tgtcaatgcc acagacctgg atgatccggc cactcccaat 600
ggccagcttt attaccagat tgtcatccag cttcccatga tcaacaatgt catgtacttt 660
cagatcaaca acaaaacggg agccatctct cttacccgag agggatctca ggaattgaat 720
cctgctaaga atccttccta taatctggtg atctcagtga aggacatggg aggccagagt 780
gagaattcct tcagtgatac cacatctgtg gatatcatag tgacagagaa tatttggaaa 840
gcaccaaaac ctgtggagat ggtggaaaac tcaactgatc ctcaccccat caaaatcact 900
caggtgcggt ggaatgatcc cggtgcacaa tattccttag ttgacaaaga gaagctgcca 960
agattcccat tttcaattga ccaggaagga gatatttacg tgactcagcc cttggaccga 1020
gaagaaaagg atgcatatgt tttttatgca gttgcaaagg atgagtacgg aaaaccactt 1080
tcatatccgc tggaaattca tgtaaaagtt aaagatatta atgataatcc acctacatgt 1140
ccgtcaccag taaccgtatt tgaggtccag gagaatgaac gactgggtaa cagtatcggg 1200
acccttactg cacatgacag ggatgaagaa aatactgcca acagtttttct aaactacagg 1260
attgtggagc aaactcccaa acttcccatg gatggactct tcctaatcca aacctatgct 1320
ggaatgttac agttagctaa acagtccttg aagaagcaag atactcctca gtacaactta 1380
acgatagagg tgtctgacaa agatttcaag acccttttgtt ttgtgcaaat caacgttatt 1440
gatatcaatg atcagatccc catctttgaa aaatcagatt atggaaacct gactcttgct 1500
gaagacacaa acattgggtc caccatctta accatccagg ccactgatgc tgatgagcca 1560
tttactggga gttctaaaat tctgtatcat atcataaagg gagacagtga gggacgcctg 1620
ggggttgaca cagatcccca taccaacacc ggatatgtca taattaaaaa gcctcttgat 1680
tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860

ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatgggggt cggccaccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa ttttttaaac agatattccc tcttgtcctt taatatttgc taaatatttc 2760
tttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120
cttgtgtttt tcccttttgg ggcaagacag actcattaaa tattctgtac atttttttctt 3180
tatcaaggag atatatcagt gttgtctcat agaactgcct ggattccatt tatgtttttt 3240
ctgattccat cctgtgtccc cttcatcctt gactcctttg gtatttcact gaatttcaaa 3300
catttgtcag agaagaaaaa cgtgaggact caggaaaaat aaataaataa aagaacagcc 3360
ttttccctta gtattaacag aaatgtttct gtgtcattaa ccatctttaa tcaatgtgac 3420
atgttgctct ttggctgaaa ttcttcaact tggaaatgac acagacccac agaaggtgtt 3480
caaacacaac ctactctgca aaccttggta aaggaaccag tcagctggcc agatttcctc 3540
actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 17
<211> 1597
<212> DNA
<213> human

<400> 17
aacaaactgc acccactgaa ctccgcagct agcatccaaa tcagcccttg agatttgagg 60

ccttggagac tcaggagttt tgagagcaaa atgacaacac ccagaaattc agtaaatggg 120

actttcctgg cagagccaat gaaaggccct attgctatgc aatctggtcc aaaaccactc 180

ttcaggagga tgtcttcact ggtgggcccc acgcaaagct tcttcatgag ggaatctaag 240

actttggggg ctgtccagat tatgaatggg ctcttccaca ttgccctggg gggtcttctg 300

atgatcccag cagggatcta tgcacccatc tgtgtgactg tgtggtaccc tctctgggga 360

ggcattatgt atattatttc cggatcactc ctggcagcaa cggagaaaaa ctccaggaag 420

tgtttggtca aaggaaaaat gataatgaat tcattgagcc tctttgctgc catttctgga 480

atgattcttt caatcatgga catacttaat attaaaattt cccatttttt aaaaatggag 540

agtctgaatt ttattagagc tcacacacca tatattaaca tatacaactg tgaaccagct 600

aatccctctg agaaaaactc cccatctacc caatactgtt acagcataca atctctgttc 660

ttgggcattt tgtcagtgat gctgatcttt gccttcttcc aggaacttgt aatagctggc 720

atcgttgaga atgaatggaa aagaacgtgc tccagaccca aatctaacat agttctcctg 780

tcagcagaag aaaaaaaaga acagactatt gaaataaaag aagaagtggt tgggctaact 840

gaaacatctt cccaaccaaa gaatgaagaa gacattgaaa ttattccaat ccaagaagag 900

gaagaagaag aaacagagac gaactttcca gaacctcccc aagatcagga atcctcacca 960

atagaaaatg acagctctcc ttaagtgatt tcttctgttt tctgtttcct tttttaaaca 1020

ttagtgttca tagcttccaa gagacatgct gactttcatt tcttgaggta ctctgcacat 1080

acgcaccaca tctctatctg gcctttgcat ggagtgacca tagctccttc tctcttacat 1140

tgaatgtaga gaatgtagcc attgtagcag cttgtgttgt cacgcttctt cttttgagca 1200

actttcttac actgaagaaa ggcagaatga gtgcttcaga atgtgatttc ctactaacct 1260

gttccttgga taggcttttt agtatagtat tttttttgt cattttctcc atcagcaacc 1320

agggagactg cacctgatgg aaaagatata tgactgcttc atgacattcc taaactatct 1380

tttttttatt ccacatctac gtttttggtg gagtcccttt tgcatcattg ttttaaggat 1440

gataaaaaaa aaataacaac tagggacaat acagaaccca ttccatttat ctttctacag 1500

ggctgacatt gtggcacatt cttagagtta ccacacccca tgagggaagc tctaaatagc 1560

caacacccat ctgtttttttg taaaaacagc atagctt 1597

<210> 18
<211> 2907
<212> DNA
<213> human

<400> 18
gccatctggg cccaggcccc atgccccgag gaggggtggt ctgaagccca ccagagcccc 60

ctgccagact gtctgcctcc cttctgactg tggccgcttg gcatggccag caacagcagc 120

tcctgcccga cacctggggg cgggcacctc aatgggtacc cggtgcctcc ctacgccttc 180

ttcttccccc ctatgctggg tggactctcc ccgccaggcg ctctgaccac tctccagcac 240

cagcttccag ttagtggata tagcacacca tccccagcca ccattgagac ccagagcagc 300

agttctgaag agatagtgcc cagccctccc tcgccacccc ctctaccccg catctacaag 360

ccttgctttg tctgtcagga caagtcctca ggctaccact atggggtcag cgcctgtgag 420

ggctgcaagg gcttcttccg ccgcagcatc cagaagaaca tggtgtacac gtgtcaccgg 480

gacaagaact gcatcatcaa caaggtgacc cggaaccgct gccagtactg ccgactgcag 540

aagtgctttg aagtgggcat gtccaaggag tctgtgagaa acgaccgaaa caagaagaag 600

aaggaggtgc ccaagcccga gtgctctgag agctacacgc tgacgccgga ggtgggggag 660

ctcattgaga aggtgcgcaa agcgcaccag gaaaccttcc ctgccctctg ccagctgggc 720

aaatacacta cgaacaacag ctcagaacaa cgtgtctctc tggacattga cctctgggac 780

aagttcagtg aactctccac caagtgcatc attaagactg tggagttcgc caagcagctg 840

cccggcttca ccaccctcac catcgccgac cagatcaccc tcctcaaggc tgcctgcctg 900

gacatcctga tcctgcggat ctgcacgcgg tacacgcccg agcaggacac catgaccttc 960

tcggacgggc tgaccctgaa ccggacccag atgcacaacg ctggcttcgg ccccctcacc 1020

gacctggtct ttgccttcgc caaccagctg ctgcccctgg agatggatga tgcggagacg 1080

gggctgctca gcgccatctg cctcatctgc ggagaccgcc aggacctgga gcagccggac 1140

cgggtggaca tgctgcagga gccgctgctg gaggcgctaa aggtctacgt gcggaagcgg 1200

aggcccagcc gcccccacat gttccccaag atgctaatga agattactga cctgcgaagc 1260

atcagcgcca aggggggctga gcgggtgatc acgctgaaga tggagatccc gggctccatg 1320
ccgcctctca tccaggaaat gttggagaac tcagagggcc tggacactct gagcggacag 1380
ccgggggtg gggggcggga cgggggtggc ctggccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680
gcctgctccc acagcctggg ctgacgtcag aggccgaggc caggaactga gtgaggcccc 1740
tggtcctggg tctcaggatg ggtcctgggg gcctcgtgtt catcaagaca cccctctgcc 1800
cagctcacca catcttcatc accagcaaac gccaggactt ggctccccca tcctcagaac 1860
tcacaagcca ttgctcccca gctggggaac ctcaacctcc cccctgcctc ggttggtgac 1920
agagggggtg ggacaggggc gggggttcc ccctgtacat accctgccat accaacccca 1980
ggtattaatt ctcgctggtt ttgtttttat tttaattttt ttgttttgat ttttttaata 2040
agaattttca ttttaagcac atttatactg aaggaatttg tgctgtgtat tggggggagc 2100
tggatccaga gctggagggg gtgggtccgg gggagggagt ggctcggaag gggcccccac 2160
tctcctttca tgtccctgtg ccccccagtt ctcctcctca gccttttcct cctcagtttt 2220
ctctttaaaa ctgtgaagta ctaactttcc aaggcctgcc ttcccctccc tcccactgga 2280
gaagccgcca gccccttttct ccctctgcct gaccactggg tgtggacggt gtggggcagc 2340
cctgaaagga caggctcctg gccttggcac ttgcctgcac ccaccatgag gcatggagca 2400
gggcagagca agggccccgg gacagagttt tcccagacct ggctcctcgg cagagctgcc 2460
tcccgtcagg gcccacatca tctaggctcc ccagccccca ctgtgaaggg gctggccagg 2520
ggcccgagct gcccccaccc ccggcctcag ccaccagcac ccccataggg cccccagaca 2580
ccacacacat gcgcgtgcgc acacacacaa acacacacac actggacagt agatgggccg 2640
acacacactt ggcccgagtt cctccatttc cctggcctgc cccccacccc caacctgtcc 2700
caccccccgtg ccccctcctt accccgcagg acgggcctac agggggggtct cccctcaccc 2760
ctgcaccccc agctggggga gctggctctg ccccgacctc cttcaccagg ggttggggcc 2820
ccttcccctg gagcccgtgg gtgcacctgt tactgttggg ctttccactg agatctactg 2880
gataaagaat aaagttctat ttattct 2907

<210> 19
<211> 2907
<212> DNA
<213> human

<400> 19
gccatctggg cccaggcccc atgccccgag gaggggtggt ctgaagccca ccagagcccc 60

ctgccagact gtctgcctcc cttctgactg tggccgcttg gcatggccag caacagcagc 120

tcctgcccga cacctggggg cgggcacctc aatgggtacc cggtgcctcc ctacgccttc 180

ttcttccccc ctatgctggg tggactctcc ccgccaggcg ctctgaccac tctccagcac 240

cagcttccag ttagtggata tagcacacca tccccagcca ccattgagac ccagagcagc 300

agttctgaag agatagtgcc cagccctccc tcgccacccc ctctaccccg catctacaag 360

ccttgctttg tctgtcagga caagtcctca ggctaccact atggggtcag cgcctgtgag 420

ggctgcaagg gcttcttccg ccgcagcatc cagaagaaca tggtgtacac gtgtcaccgg 480

gacaagaact gcatcatcaa caaggtgacc cggaaccgct gccagtactg ccgactgcag 540

aagtgctttg aagtgggcat gtccaaggag tctgtgagaa acgaccgaaa caagaagaag 600

aaggaggtgc ccaagcccga gtgctctgag agctacacgc tgacgccgga ggtgggggag 660

ctcattgaga aggtgcgcaa agcgcaccag gaaaccttcc ctgccctctg ccagctgggc 720

aaatacacta cgaacaacag ctcagaacaa cgtgtctctc tggacattga cctctgggac 780

aagttcagtg aactctccac caagtgcatc attaagactg tggagttcgc caagcagctg 840

cccggcttca ccaccctcac catcgccgac cagatcaccc tcctcaaggc tgcctgcctg 900

gacatcctga tcctgcggat ctgcacgcgg tacacgcccg agcaggacac catgaccttc 960

tcggacgggc tgaccctgaa ccggacccag atgcacaacg ctggcttcgg ccccctcacc 1020

gacctggtct ttgccttcgc caaccagctg ctgcccctgg agatggatga tgcggagacg 1080

gggctgctca gcgccatctg cctcatctgc ggagaccgcc aggacctgga gcagccggac 1140

cgggtggaca tgctgcagga gccgctgctg gaggcgctaa aggtctacgt gcggaagcgg 1200

aggcccagcc gcccccacat gttccccaag atgctaatga agattactga cctgcgaagc 1260

atcagcgcca agggggctga gcgggtgatc acgctgaaga tggagatccc gggctccatg 1320

ccgcctctca tccaggaaat gttggagaac tcagagggcc tggacactct gagcggacag 1380

ccgggggggtg gggggcggga cgggggtggc ctggcccccc cgccaggcag ctgtagcccc 1440

agcctcagcc ccagctccaa cagaagcagc ccggccaccc actccccgtg accgcccacg 1500

ccacatggac acagccctcg ccctccgccc cggcttttct ctgcctttct accgaccatg 1560

tgaccccgca ccagccctgc ccccacctgc cctcccgggc agtactgggg accttccctg 1620

ggggacgggg agggaggagg cagcgactcc ttggacagag gcctgggccc tcagtggact 1680

gcctgctccc acagcctggg ctgacgtcag aggccgaggc caggaactga gtgaggcccc 1740

tggtcctggg tctcaggatg ggtcctgggg gcctcgtgtt catcaagaca cccctctgcc 1800

cagctcacca catcttcatc accagcaaac gccaggactt ggctccccca tcctcagaac 1860

tcacaagcca ttgctcccca gctggggaac ctcaacctcc cccctgcctc ggttggtgac 1920

agaggggggtg ggacaggggc gggggggttcc ccctgtacat accctgccat accaacccca 1980

ggtattaatt ctcgctggtt ttgtttttat tttaattttt ttgttttgat ttttttaata 2040

agaattttca ttttaagcac atttatactg aaggaatttg tgctgtgtat tgggggggagc 2100

tggatccaga gctggagggg gtgggtccgg gggagggagt ggctcggaag gggcccccac 2160

tctcctttca tgtccctgtg ccccccagtt ctcctcctca gccttttcct cctcagtttt 2220

ctctttaaaa ctgtgaagta ctaactttcc aaggcctgcc ttcccctccc tcccactgga 2280

gaagccgcca gccccttttct ccctctgcct gaccactggg tgtggacggt gtggggcagc 2340

cctgaaagga caggctcctg gccttggcac ttgcctgcac ccaccatgag gcatggagca 2400

gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg ccccctcctt accccgcagg acgggcctac agggggggtct cccctcaccc 2760

ctgcaccccc agctggggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 20
<211> 1347
<212> DNA
<213> human

<400> 20
atggatccca aatatttcat cttaattttg ttttgtggac acctgaacaa tacatttttt 60

tcaaagacag agacaattac aacagagaag cagtcacagc ctaccttata cacatcatca 120

atgtcacagg tattggctaa ttctcaaaac acaacaggga atcctttggg tcaaccaaca 180

caattcagcg acactttttc tggacaatca atatcacctg ccaaagtcac tgctggacaa 240

ccaacaccag ctgtctatac ctcttctgaa aaaccagaag cacatacttc tgctggacaa 300

ccacttgcct acaacaccaa acaaccaaca ccaatagcca acacctcctc ccagcaagcc 360

gtgttcacct ctgccagaca actaccatct gcccgtactt ctaccacaca accaccaaag 420

tcatttgtct atacttttac tcaacaatca tcatctgtcc agatcccttc tagaaaacaa 480

ataactgttc ataatccatc cacacaacca acatcaactg tcaaaaattc acctaggagt 540

acaccaggat ttatcttaga tactaccagt aacaaacaaa ccccacaaaa aaacaattat 600

aattcaatag ctgccatact aattggtgta cttctgactt ctatgttggt agctataatc 660

atcattgtac tttggaaatg cttaaggaaa ccagttttaa atgatcaaaa ttgggcaggt 720

agatctccat ttgctgatgg agaaacccct gacatttgta tggataacat cagagaaaat 780

gaaatatcca caaaacgtac atcaatcatt tcacttacac cctggaaacc aagcaaaagc 840

acacttttag cagatgactt agaaattaag ttgtttgaat caagtgaaaa cattgaagac 900

tccaacaacc ccaaaacaga gaaaataaaa gatcaagtaa atggtacatc agaagatagt 960

gctgatggtt caacagttgg aactgctgtt tcttcttcag atgatgcaga tctgcctcca 1020

ccacctcccc ttctggattt ggaaggacag gaaagtaacc aatctgacaa acccacaatg 1080

acaattgtat ctcctcttcc aaatgattct actagtctcc ctccatctct ggactgtctc 1140

aatcaagact gtggagatca taaatctgag ataatacaat catttccacc gcttgactca 1200

cttaacttgc ccctgccacc agtagatttt atgaaaaacc aagaagattc caaccttgag 1260

atccagtgtc aggagttctc tattcctccc aactctgatc aagatcttaa tgaatccctg 1320

ccacctccac ctgcagaact gttataa 1347

<210> 21
<211> 2170
<212> DNA
<213> human

<400> 21
ctgagggctc atccctctgc agagcgcggg gtcaccggga ggagacgcca tgacgcccgc 60

cctcacagcc ctgctctgcc ttgggctgag tctgggcccc aggacccgcg tgcaggcagg 120

gcccttcccc aaacccaccc tctgggctga gccaggctct gtgatcagct gggggagccc 180

cgtgaccatc tggtgtcagg ggagcctgga ggcccaggag taccgactgg ataaagaggg 240
aagcccagag cccttggaca gaaataaccc actggaaccc aagaacaagg ccagattctc 300
catcccatcc atgacagagc accatgcggg gagataccgc tgccactatt acagctctgc 360
aggctggtca gagcccagcg accccctgga gctggtgatg acaggattct acaacaaacc 420
caccctctca gccctgccca gccctgtggt ggcctcaggg gggaatatga ccctccgatg 480
tggctcacag aagggatatc accattttgt tctgatgaag gaaggagaac accagctccc 540
ccggaccctg gactcacagc agctccacag tgggggggttc caggccctgt tccctgtggg 600
ccccgtgaac cccagccaca ggtggaggtt cacatgctat tactattata tgaacacccc 660
ccaggtgtgg tcccacccca gtgacccccct ggagattctg ccctcaggcg tgtctaggaa 720
gccctccctc ctgaccctgc agggccctgt cctggcccct gggcagagcc tgaccctcca 780
gtgtggctct gatgtcggct acgacagatt tgttctgtat aaggaggggg aacgtgactt 840
cctccagcgc cctggccagc agccccaggc tgggctctcc caggccaact tcaccctggg 900
ccctgtgagc ccctcccacg gggggccagta caggtgctat ggtgcacaca acctctcctc 960
cgagtggtcg gcccccagcg accccctgaa catcctgatg gcaggacaga tctatgacac 1020
cgtctccctg tcagcacagc cgggccccac agtggcctca ggagagaacg tgaccctgct 1080
gtgtcagtca tggtggcagt ttgacacttt ccttctgacc aaagaagggg cagcccatcc 1140
cccactgcgt ctgagatcaa tgtacggagc tcataagtac caggctgaat tccccatgag 1200
tcctgtgacc tcagcccacg cggggaccta caggtgctac ggctcataca gctccaaccc 1260
ccacctgctg tctttcccca gtgagcccct ggaactcatg gtctcaggac actctggagg 1320
ctccagcctc ccacccacag ggccgccctc cacacctggt ctgggaagat acctggaggt 1380
tttgattggg gtctcggtgg ccttcgtcct gctgctcttc ctcctcctct tcctcctcct 1440
ccgacgtcag cgtcacagca aacacaggac atctgaccag agaaagactg atttccagcg 1500
tcctgcaggg gctgcggaga cagagcccaa ggacaggggc ctgctgagga ggtccagccc 1560
agctgctgac gtccaggaag aaaacctcta tgctgccgtg aaggacacac agtctgagga 1620
cagggtggag ctggacagtc agagcccaca cgatgaagac cccccaggcag tgacgtatgc 1680
cccggtgaaa cactccagtc ctaggagaga aatggcctct cctccctcct cactgtctgg 1740
ggaattcctg gacacaaagg acagacaggt ggaagaggac aggcagatgg acactgaggc 1800
tgctgcatct gaagcctccc aggatgtgac ctacgcccag ctgcacagct tgacccttag 1860
acggaaggca actgagcctc ctccatccca ggaaggggaa cctccagctg agcccagcat 1920

ctacgccact ctggccatcc actagcccgg ggggtacgca gaccccacac tcagcagaag 1980

gagactcagg actgctgaag gcacgggagc tgcccccagt ggacaccagt gaaccccagt 2040

cagcctggac ccctaacaca gaccatgagg agacgctggg aacttgtggg actcacctga 2100

ctcaaagatg actaatatcg tcccattttg gaaataaagc aacagacttc tcaacaatca 2160

atgagttaat 2170

<210> 22
<211> 1453
<212> DNA
<213> human

<400> 22
gggagtgcat ccgccccaac ccttttcccc ctcgtctcct gtgagaattc cccgtcggat 60

acgagcagcg tggccgttgg ctgcctcgca caggacttcc ttcccgactc catcactttc 120

tcctggaaat acaagaacaa ctctgacatc agcagcaccc ggggcttccc atcagtcctg 180

agagggggca agtacgcagc cacctcacag gtgctgctgc cttccaagga cgtcatgcag 240

ggcacagacg aacacgtggt gtgcaaagtc cagcacccca acggcaacaa agaaaagaac 300

gtgcctcttc cagtgattgc cgagctgcct cccaaagtga gcgtcttcgt cccaccccgc 360

gacggcttct tcggcaaccc ccgcaagtcc aagctcatct gccaggccac gggtttcagt 420

ccccggcaga ttcaggtgtc ctggctgcgc gaggggaagc aggtggggtc tggcgtcacc 480

acggaccagg tgcaggctga ggccaaagag tctgggccca cgacctacaa ggtgaccagc 540

acactgacca tcaaagagag cgactggctc agccagagca tgttcacctg ccgcgtggat 600

cacaggggcc tgaccttcca gcagaatgcg tcctccatgt gtgtccccga tcaagacaca 660

gccatccggg tcttcgccat ccccccatcc tttgccagca tcttcctcac caagtccacc 720

aagttgacct gcctggtcac agacctgacc acctatgaca gcgtgaccat ctcctggacc 780

cgccagaatg gcgaagctgt gaaaacccac accaacatct ccgagagcca ccccaatgcc 840

actttcagcg ccgtgggtga ggccagcatc tgcgaggatg actggaattc cggggagagg 900

ttcacgtgca ccgtgaccca cacagacctg ccctcgccac tgaagcagac catctcccgg 960

cccaaggggg tggccctgca caggcccgat gtctacttgc tgccaccagc ccgggagcag 1020

ctgaacctgc gggagtcggc caccatcacg tgcctggtga cgggcttctc tcccgcggac 1080

gtcttcgtgc agtggatgca gaggggcag ccccttgtccc cggagaagta tgtgaccagc 1140

gccccaatgc ctgagcccca ggccccaggc cggtacttcg cccacagcat cctgaccgtg 1200

tccgaagagg aatggaacac gggggagacc tacacctgcg tggtggccca tgaggccctg 1260
cccaacaggg tcaccgagag gaccgtggac aagtccaccg gtaaacccac cctgtacaac 1320
gtgtccctgg tcatgtccga cacagctggc acctgctact gaccctgctg gcctgcccac 1380
aggctcgggg cggctggccg ctctgtgtgt gcatgcaaac taacccgtgt caacggggtg 1440
agatgttgca tct 1453

<210> 23
<211> 1192
<212> DNA
<213> human

<400> 23
cagatccatc aggtccaagc tgtgttgact accactgctt ttcccttcgt ctcaattatg 60
tcttggaaga aggctttgcg gatccctgga ggccttcggg tagcaactgt gaccttgatg 120
ctggcgatgc tgagcacccc ggtggctgag ggcagagact ctcccgagga tttcgtgtac 180
cagtttaagg gcatgtgcta cttcaccaac gggacggagc gcgtgcgtct tgtgaccaga 240
tacatctata accgagagga gtacgcacgc ttcgacagcg acgtgggggt gtatcgggcg 300
gtgacgccgc tggggccgcc tgacgccgag tactggaaca gccagaagga agtcctggag 360
aggacccggg cggagttgga cacggtgtgc agacacaact accagttgga gctccgcacg 420
accttgcagc ggcgagtgga gcccacagtg accatctccc catccaggac agaggccctc 480
aaccaccaca acctgctggt ctgctcagtg acagatttct atccagccca gatcaaagtc 540
cggtggtttc ggaatgacca ggaggagaca actggcgttg tgtccacccc ccttattagg 600
aacggtgact ggaccttcca gatcctggtg atgctggaaa tgactcccca gcgtggagac 660
gtctacacct gccacgtgga gcaccccagc ctccagaacc ccatcatcgt ggagtggcgg 720
gctcagtctg aatctgccca gagcaagatg ctgagtggca ttggaggctt cgtgctgggg 780
ctgatcttcc tcgggctggg ccttattatc catcacagga gtcagaaagg gctcctgcac 840
tgactcctga gactatttta actgggattg gttatcactt ttctgtaacg cctgcttgtc 900
cctgcccaga attcccagct gcctgtgtca gcctgtcccc cgagatcaga gtcctaccgt 960
ggctgtcacg cagccaccag gtcatctcct ttcatcccca cctcaaggct gatggctgtg 1020
accctgcttc ctgcactgac ccagagcctc tgcctgtgca cggccagctg cgtctactga 1080
ggccccaagg ggtttctgtt tctattctct cctcagactg ctcaagagaa gcacatgaaa 1140
accattacct gactttagag ctttttttaca taattaaaca tgatcctgag tt 1192

<210> 24
<211> 2048
<212> DNA
<213> human

<400> 24
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

```
tatgttgaaa tggagatttt taagagtttt aaccagctgc tgcagatata taactcaaaa   1560
cagatatagc gtataaagat atagtaaatg catctcccag agtaatattc acttaacaca   1620
ttgaaactat tatttttag atttgaatat aaatgtattt tttaaacact tgttatgagt   1680
taacttggat tacattttga aatcagttca ttccatgatg catattactg gattagatta   1740
agaaagacag aaaagattaa gggacgggca catttttcaa cgattaagaa tcatcattac   1800
ataacttggt gaaactgaaa aagtatatca tatgggtaca caaggctatt tgccagcata   1860
tattaatatt ttagaaaata ttccttttgt aatactgaat ataaacatag agctagagtc   1920
atattatcat acttatcata atgttcaatt tgatacagta gaattgcaag tccctaagtc   1980
cctattcact gtgcttagta gtgactccat ttaataaaaa gtgtttttag tttttaacaa   2040
ctaaaccg                                                            2048
```

<210> 25
<211> 2048
<212> DNA
<213> human

<400> 25

```
atgtgaaggc acaagctgct gttatataca acagagtgaa ctgagcatca gtcagaaaaa     60
gtctatgttt gcagaaatac agatccaaga caaagacagg atgggcactg ctggaaaagt    120
tattaaatgc aaagcagctg tgctttggga gcagaagcaa cccttctcca ttgaggaaat    180
agaagttgcc ccaccaaaga ctaaagaagt tcgcattaag attttggcca caggaatctg    240
tcgcacagat gaccatgtga taaaaggaac aatggtgtcc aagtttccag tgattgtggg    300
acatgaggca actgggattg tagagagcat tggagaagga gtgactacag tgaaaccagg    360
tgacaaagtc atccctctct ttctgccaca atgtagagaa tgcaatgctt gtcgcaaccc    420
agatggcaac ctttgcatta ggagcgatat tactggtcgt ggagtactgg ctgatggcac    480
caccagattt acatgcaagg gcaaaccagt acaccacttc atgaacacca gtacatttac    540
cgagtacaca gtggtggatg aatcttctgt tgctaagatt gatgatgcag ctcctcctga    600
gaaagtctgt ttaattggct gtgggttttc cactggatat ggcgctgctg ttaaaactgg    660
caaggtcaaa cctggttcca cttgcgtcgt ctttggcctg ggaggagttg gcctgtcagt    720
catcatgggc tgtaagtcag ctggtgcatc taggatcatt gggattgacc tcaacaaaga    780
caaatttgag aaggccatgg ctgtaggtgc cactgagtgt atcagtccca aggactctac    840
caaacccatc agtgaggtgc tgtcagaaat gacaggcaac aacgtgggat acacctttga    900
```

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tattttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 26
<211> 2816
<212> DNA
<213> human

<400> 26
tcgttgatat caaagacagt tgaaggaaat gaattttgaa acttcacggt gtgccaccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980

ccccttttaat gctggtcatg taataatatt gcaagtagta agaaacgaag gtgtcaagtg 2040

tactgctggg cagcgaggtg atcattacca aaagtaatca actttgtggg tggagagttc 2100

tttgtgagaa cttgcattat ttgtgtcctc ccctcatgtg taggtagaac atttcttaat 2160

gctgtgtacc tgcctctgcc actgtatgtt ggcatctgtt atgctaaagt ttttcttgta 2220

catgaaaccc tggaagacct actacaaaaa aactgttgtt tggcccccat agcaggtgaa 2280

ctcattttgt gcttttaata gaaagacaaa tccaccccag taatattgcc cttacgtagt 2340

tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 27
<211> 2816
<212> DNA
<213> human

<400> 27
tcgttgatat caaagacagt tgaaggaaat gaattttgaa acttcacggt gtgccaccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420

agtccctata acacagacca cgcgcagaac agcgtcacgg cgccctcgcc ctacgcacag 480

cccagctcca ccttcgatgc tctctctcca tcacccgcca tcccctccaa caccgactac 540

ccaggcccgc acagtttcga cgtgtccttc cagcagtcga gcaccgccaa gtcggccacc 600

tggacgtatt ccactgaact gaagaaactc tactgccaaa ttgcaaagac atgccccatc 660

cagatcaagg tgatgacccc acctcctcag ggagctgtta tccgcgccat gcctgtctac 720

aaaaaagctg agcacgtcac ggaggtggtg aagcggtgcc ccaaccatga gctgagccgt 780

gaattcaacg agggacagat tgcccctcct agtcatttga ttcgagtaga ggggaacagc 840

catgcccagt atgtagaaga tcccatcaca ggaagacaga gtgtgctggt accttatgag 900

ccaccccagg ttggcactga attcacgaca gtcttgtaca atttcatgtg taacagcagt 960

tgtgttggag ggatgaaccg ccgtccaatt ttaatcattg ttactctgga aaccagagat 1020

gggcaagtcc tgggccgacg ctgctttgag gcccggatct gtgcttgccc aggaagagac 1080

aggaaggcgg atgaagatag catcagaaag cagcaagttt cggacagtac aaagaacggt 1140

gatggtacga agcgcccgtt tcgtcagaac acacatggta tccagatgac atccatcaag 1200

aaacgaagat ccccagatga tgaactgtta tacttaccag tgaggggccg tgagacttat 1260

gaaatgctgt tgaagatcaa agagtccctg gaactcatgc agtaccttcc tcagcacaca 1320

attgaaacgt acaggcaaca gcaacagcag cagcaccagc acttacttca gaaacatctc 1380

ctttcagcct gcttcaggaa tgagcttgtg gagccccgga gagaaactcc aaaacaatct 1440

gacgtcttct ttagacattc caagcccccca aaccgatcag tgtacccata gagccctatc 1500

tctatatttt aagtgtgtgt gttgtatttc catgtgtata tgtgagtgtg tgtgtgtgta 1560

tgtgtgtgcg tgtgtatcta gccctcataa acaggacttg aagacacttt ggctcagaga 1620

cccaactgct caaaggcaca aagccactag tgagagaatc ttttgaaggg actcaaacct 1680

ttacaagaaa ggatgttttc tgcagatttt gtatccttag accggccatt ggtgggtgag 1740

gaaccactgt gtttgtctgt gagctttctg ttgtttcctg ggagggaggg gtcaggtggg 1800

gaaaggggca ttaagatgtt tattggaacc cttttctgtc ttcttctgtt gtttttctaa 1860

aattcacagg gaagcttttg agcaggtctc aaacttaaga tgtcttttta agaaaaggag 1920

aaaaaagttg ttattgtctg tgcataagta agttgtaggt gactgagaga ctcagtcaga 1980

cccttttaat gctggtcatg taataatatt gcaagtagta agaaacgaag gtgtcaagtg 2040

tactgctggg cagcgaggtg atcattacca aaagtaatca actttgtggg tggagagttc 2100

tttgtgagaa cttgcattat ttgtgtcctc ccctcatgtg taggtagaac atttcttaat 2160

gctgtgtacc tgcctctgcc actgtatgtt ggcatctgtt atgctaaagt tttcttgta 2220

catgaaaccc tggaagacct actacaaaaa aactgttgtt tggcccccat agcaggtgaa 2280

ctcattttgt gcttttaata gaaagacaaa tccaccccag taatattgcc cttacgtagt 2340

tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 28
<211> 3803
<212> DNA
<213> human

<400> 28
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300

aaacgcgctg ggcgtctgct ccgacaccgc agcgcccctg gccgcggtgg acctcaagtg 360

ggagcacaac cccgctgtgt tcctgggccc cggctgcgtg tacgccgccg ccccagtggg 420

gcgcttcacc gcgcactggc gggtcccgct gctgaccgcc ggcgccccgg cgctgggctt 480

cggtgtcaag gacgagtatg cgctgaccac ccgcgcgggg cccagctacg ccaagctggg 540

ggacttcgtg gcggcgctgc accgacggct gggctgggag cgccaagcgc tcatgctcta 600

cgcctaccgg ccgggtgacg aagagcactg cttcttcctc gtggaggggc tgttcatgcg 660

ggtccgcgac cgcctcaata ttacggtgga ccacctggag ttcgccgagg acgacctcag 720

ccactacacc aggctgctgc ggaccatgcc gcgcaaaggc cgagttatct acatctgcag 780

ctcccctgat gccttcagaa ccctcatgct cctggccctg gaagctggct tgtgtgggga 840

ggactacgtt ttcttccacc tggatatctt tgggcaaagc ctgcaaggtg gacagggccc 900

tgctccccgc aggccctggg agagagggga tgggcaggat gtcagtgccc gccaggcctt 960

tcaggctgcc aaaatcatta catataaaga cccagataat cccgagtact tggaattcct 1020

gaagcagtta aaacacctgg cctatgagca gttcaacttc accatggagg atggcctggt 1080

gaacaccatc ccagcatcct tccacgacgg gctcctgctc tatatccagg cagtgacgga 1140

gactctggca catgggggaa ctgttactga tggggagaac atcactcagc ggatgtggaa 1200

ccgaagcttt caaggtgtga caggatacct gaaaattgat agcagtggcg atcgggaaac 1260

agacttctcc ctctgggata tggatcccga gaatggtgcc ttcagggttg tactgaacta 1320

caatgggact tcccaagagc tggtggctgt gtcggggcgc aaactgaact ggcccctggg 1380

gtaccctcct cctgacatcc ccaaatgtgg ctttgacaac gaagacccag catgcaacca 1440

agatcacctt tccaccctgg aggtgctggc tttggtgggc agcctctcct tgctcggcat 1500

tctgattgtc tccttcttca tatacaggaa gatgcagctg gagaaggaac tggcctcgga 1560

gctgtggcgg gtgcgctggg aggacgttga gcccagtagc cttgagaggc acctgcggag 1620

tgcaggcagc cggctgaccc tgagcgggag aggctccaat tacggctccc tgctaaccac 1680

agagggccag ttccaagtct ttgccaagac agcatattat aagggcaacc tcgtggctgt 1740

gaaacgtgtg aaccgtaaac gcattgagct gacacgaaaa gtcctgtttg aactgaagca 1800

tatgcgggat gtgcagaatg aacacctgac caggtttgtg ggagcctgca ccgacccccc 1860

caatatctgc atcctcacag agtactgtcc ccgtgggagc ctgcaggaca ttctggagaa 1920

tgagagcatc accctggact ggatgttccg gtactcactc accaatgaca tcgtcaaggg 1980

catgctgttt ctacacaatg gggctatctg ttcccatggg aacctcaagt catccaactg 2040

cgtggtagat gggcgctttg tgctcaagat caccgactat gggctggaga gcttcaggga 2100

cctggaccca gagcaaggac acaccgttta tgccaaaaag ctgtggacgg cccctgagct 2160

cctgcgaatg gcttcacccc ctgtgcgggg ctcccaggct ggtgacgtat acagctttgg 2220

gatcatcctt caggagattg ccctgaggag tggggtcttc cacgtggaag gtttggacct 2280

gagccccaaa gagatcatcg agcgggtgac tcggggtgag cagcccccct tccggccctc 2340

cctggccctg cagagtcacc tggaggagtt ggggctgctc atgcagcggt gctgggctga 2400

ggacccacag gagaggccac cattccagca gatccgcctg acgttgcgca aatttaacag 2460

ggagaacagc agcaacatcc tggacaacct gctgtcccgc atggagcagt acgcgaacaa 2520

tctggaggaa ctggtggagg agcggaccca ggcatacctg gaggagaagc gcaaggctga 2580

ggccctgctc taccagatcc tgcctcactc agtggctgag cagctgaagc gtggggagac 2640

ggtgcaggcc gaagcctttg acagtgttac catctacttc agtgacattg tgggtttcac 2700

agcgctgtcg gcggagagca cacccatgca ggtggtgacc ctgctcaatg acctgtacac 2760

ttgctttgat gctgtcatag acaactttga tgtgtacaag gtggagacaa ttggcgatgc 2820

ctacatggtg gtgtcagggc tccctgtgcg gaacgggcgg ctacacgcct gcgaggtagc 2880

ccgcatggcc ctggcactgc tggatgctgt gcgctccttc cgaatccgcc accggcccca 2940

ggagcagctg cgcttgcgca ttggcatcca cacaggacct gtgtgtgctg gagtggtggg 3000

actgaagatg ccccgttact gtctctttgg ggatacagtc aacacagcct caagaatgga 3060

gtctaatggg gaagccctga agatccactt gtcttctgag accaaggctg tcctggagga 3120

gtttggtggt ttcgagctgg agcttcgagg ggatgtagaa atgaagggca aaggcaaggt 3180

tcggacctac tggctccttg ggagaggggg gagtagcacc cgaggctgac ctgcctcctc 3240

tcctatccct ccacacctcc cctaccctgt gccagaagca acagaggtgc caggcctcag 3300

cctcacccac agcagcccca tcgccaaagg atggaagtaa tttgaatagc tcaggtgtgc 3360

tgaccccagt gaagacacca gataggacct ctgagagggg actggcatgg ggggatctca 3420

gagcttacag gctgagccaa gcccacggcc atgcacaggg acactcacac aggcacacgc 3480

acctgctctc cacctggact caggccgggc tgggctgtgg atccttgatc ccctcccctc 3540

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gacccccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 29
<211> 3802
<212> DNA
<213> human

<400> 29
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300

aaacgcgctg ggcgtctgct ccgacaccgc agcgcccctg gccgcggtgg acctcaagtg 360

ggagcacaac cccgctgtgt tcctgggccc cggctgcgtg tacgccgccg ccccagtggg 420

gcgcttcacc gcgcactggc gggtcccgct gctgaccgcc ggcgccccgg cgctgggctt 480
cggtgtcaag gacgagtatg cgctgaccac ccgcgcgggg cccagctacg ccaagctggg 540
ggacttcgtg gcggcgctgc accgacggct gggctgggag cgccaagcgc tcatgctcta 600
cgcctaccgg ccgggtgacg aagagcactg cttcttcctc gtggaggggc tgttcatgcg 660
ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380
gtaccctcct cctgacatcc ccaaatgtgg ctttgacaac gaagacccag catgcaacca 1440
agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg ggctatctg ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160

```
cctgcgaatg gcttcacccc ctgtgcgggg ctcccaggct ggtgacgtat acagctttgg   2220
gatcatcctt caggagattg ccctgaggag tggggtcttc cacgtggaag gtttggacct   2280
gagccccaaa gagatcatcg agcgggtgac tcggggtgag cagcccccct tccggccctc   2340
cctggccctg cagagtcacc tggaggagtt ggggctgctc atgcagcggt gctgggctga   2400
ggacccacag gagaggccac cattccagca gatccgcctg acgttgcgca aatttaacag   2460
ggagaacagc agcaacatcc tggacaacct gctgtcccgc atggagcagt acgcgaacaa   2520
tctggaggaa ctggtggagg agcggaccca ggcatacctg gaggagaagc gcaaggctga   2580
ggccctgctc taccagatcc tgcctcactc agtggctgag cagctgaagc gtggggagac   2640
ggtgcaggcc gaagcctttg acagtgttac catctacttc agtgacattg tgggtttcac   2700
agcgctgtcg gcggagagca cacccatcag gtggtgaccc tgctcaatga cctgtacact   2760
tgctttgatg ctgtcataga caactttgat gtgtacaagg tggagacaat tggcgatgcc   2820
tacatggtgg tgtcagggct ccctgtgcgg aacgggcggc tacacgcctg cgaggtagcc   2880
cgcatggccc tggcactgct ggatgctgtg cgctccttcc gaatccgcca ccggccccag   2940
gagcagctgc gcttgcgcat tggcatccac acaggacctg tgtgtgctgg agtggtggga   3000
ctgaagatgc cccgttactg tctctttggg gatacagtca acacagcctc aagaatggag   3060
tctaatgggg aagccctgaa gatccacttg tcttctgaga ccaaggctgt cctggaggag   3120
tttggtggtt tcgagctgga gcttcgaggg gatgtagaaa tgaagggcaa aggcaaggtt   3180
cggacctact ggctccttgg ggagaggggg agtagcaccc gaggctgacc tgcctcctct   3240
cctatccctc cacacctccc ctaccctgtg ccagaagcaa cagaggtgcc aggcctcagc   3300
ctcacccaca gcagccccat cgccaaagga tggaagtaat ttgaatagct caggtgtgct   3360
gaccccagtg aagacaccag ataggacctc tgagagggga ctggcatggg gggatctcag   3420
agcttacagg ctgagccaag cccacggcca tgcacaggga cactcacaca ggcacacgca   3480
cctgctctcc acctggactc aggccgggct gggctgtgga tccttgatcc cctcccctcc   3540
ccatgctctc ctccctcagc cttgctaccc tgtgacttac tgggaggaga gtcacctgaa   3600
ggggaacatg aaaagagact aggtgaagag agggcagggg agcccacatc tggggctggc   3660
ccacaatacc tgctcccccg accccctcca cccagcagta gacacagtgc acaggggaga   3720
agaggggtgg cgcagaaggg ttgggggcct gtatgccttg cttctaccat gagcagagac   3780
aattaaaatc tttattccag tg                                            3802
```

<210> 30
<211> 2970
<212> DNA
<213> human

<400> 30
```
ggggtaagga gttcaaggca gcgcccacac ccgggggctc tccgcaaccc gaccgcctgt      60

ccgctccccc acttcccgcc ctccctccca cctactcatt cacccaccca cccacccaga     120

gccgggacgg cagcccaggc gcccgggccc cgccgtctcc tcgccgcgat cctggacttc     180

ctcttgctgc aggacccggc ttccacgtgt gtcccggagc cggcgtctca gcacacgctc     240

cgctccgggc ctgggtgcct acagcagcca gagcagcagg gagtccggga cccgggcggc     300

atctgggcca agttaggcgc cgccgaggcc agcgctgaac gtctccaggg ccggaggagc     360

cgcggggcgt ccgggtctga gcctcagcaa atgggctccg acgtgcggga cctgaacgcg     420

ctgctgcccg ccgtcccctc cctgggtggc ggcggcggct gtgccctgcc tgtgagcggc     480

gcggcgcagt gggcgccggt gctggacttt gcgcccccgg gcgcttcggc ttacgggtcg     540

ttgggcggcc ccgcgccgcc accggctccg ccgccacccc cgccgccgcc gcctcactcc     600

ttcatcaaac aggagccgag ctggggcggc gcggagccgc acgaggagca gtgcctgagc     660

gccttcactg tccacttttc cggccagttc actggcacag ccggagcctg tcgctacggg     720

cccttcggtc ctcctccgcc cagccaggcg tcatccggcc aggccaggat gtttcctaac     780

gcgccctacc tgcccagctg cctcgagagc cagcccgcta ttcgcaatca gggttacagc     840

acggtcacct tcgacgggac gcccagctac ggtcacacgc cctcgcacca tgcggcgcag     900

ttccccaacc actcattcaa gcatgaggat cccatgggcc agcagggctc gctgggtgag     960

cagcagtact cggtgccgcc cccggtctat ggctgccaca cccccaccga cagctgcacc    1020

ggcagccagg ctttgctgct gaggacgccc tacagcagtg acaatttata ccaaatgaca    1080

tcccagcttg aatgcatgac ctggaatcag atgaacttag gagccacctt aaagggccac    1140

agcacagggt acgagagcga taaccacaca acgcccatcc tctgcggagc ccaatacaga    1200

atacacacgc acggtgtctt cagaggcatt caggatgtgc gacgtgtgcc tggagtagcc    1260

ccgactcttg tacggtcggc atctgagacc agtgagaaac gccccttcat gtgtgcttac    1320

ccaggctgca ataagagata ttttaagctg tcccacttac agatgcacag caggaagcac    1380

actggtgaga aaccatacca gtgtgacttc aaggactgtg aacgaaggtt ttctcgttca    1440

gaccagctca aaagacacca aaggagacat acaggtgtga aaccattcca gtgtaaagct    1500
```

tgtcagcgaa agttctcccg gtccgaccac ctgaagaccc acaccaggac tcatacaggt 1560
gaaaagccct tcagctgtcg gtggccaagt tgtcagaaaa agtttgcccg gtcagatgaa 1620
ttagtccgcc atcacaacat gcatcagaga aacatgacca aactccagct ggcgctttga 1680
ggggtctccc tcggggaccg ttcagtgtcc caggcagcac agtgtgtgaa ctgctttcaa 1740
gtctgactct ccactcctcc tcactaaaaa ggaaacttca gttgatcttc ttcatccaac 1800
ttccaagaca agataccggt gcttctggaa actaccaggt gtgcctggaa gagttggtct 1860
ctgccctgcc tacttttagt tgactcacag gccctggaga agcagctaac aatgtctggt 1920
tagttaaaag cccattgcca tttggtctgg attttctact gtaagaagag ccatagctga 1980
tcatgtcccc ctgacccttc ccttcttttt ttatgctcgt tttcgctggg gatggaatta 2040
ttgtaccatt ttctatcatg gaatatttat aggccagggc atgtgtatgt gtctgctaat 2100
gtaaactttg tcatggtttc catttactaa cagcaacagc aagaaataaa tcagagagca 2160
aggcatcggg ggtgaatctt gtctaacatt cccgaggtca gccaggctgc taacctggaa 2220
agcaggatgt agttctgcca ggcaactttt aaagctcatg catttcaagc agctgaagaa 2280
agaatcagaa ctaaccagta cctctgtata gaaatctaaa agaattttac cattcagtta 2340
attcaatgtg aacactggca cactgctctt aagaaactat gaagatctga gattttttg 2400
tgtatgtttt tgactctttt gagtggtaat catatgtgtc tttatagatg tacatacctc 2460
cttgcacaaa tggaggggaa ttcattttca tcactgggag tgtccttagt gtataaaaac 2520
catgctggta tatggcttca agttgtaaaa atgaaagtga ctttaaaaga aaatagggga 2580
tggtccagga tctccactga taagactgtt tttaagtaac ttaaggacct ttgggtctac 2640
aagtatatgt gaaaaaaatg agacttactg ggtgaggaaa tccattgttt aaagatggtc 2700
gtgtgtgtgt gtgtgtgtgt gtgtgtgttg tgttgtgttt tgttttttaa gggagggaat 2760
ttattattta ccgttgcttg aaattactgt gtaaatatat gtctgataat gatttgctct 2820
ttgacaacta aaattaggac tgtataagta ctagatgcat cactgggtgt tgatcttaca 2880
agatattgat gataacactt aaaattgtaa cctgcatttt tcactttgct ctcaattaaa 2940
gtctattcaa aaggaaaaaa aaaaaaaaaa 2970

<210> 31
<211> 2422
<212> DNA
<213> human

<400> 31

gtcagcctcc cttccaccgc catattgggc cactaaaaaa agggggctcg tcttttcggg 60

gtgtttttct ccccctcccc tgtccccgct tgctcacggc tctgcgactc cgacgccggc 120

aaggtttgga gagcggctgg gttcgcggga cccgcgggct tgcacccgcc cagactcgga 180

cgggctttgc caccctctcc gcttgcctgg tcccctctcc tctccgccct cccgctcgcc 240

agtccatttg atcagcggag actcggcggc cgggccgggg cttccccgca gcccctgcgc 300

gctcctagag ctcgggccgt ggctcgtcgg ggtctgtgtc ttttggctcc gagggcagtc 360

gctgggcttc cgagaggggt tcgggccgcg taggggcgct ttgttttgtt cggttttgtt 420

tttttgagag tgcgagagag gcggtcgtgc agacccggga gaaagatgtc aaacgtgcga 480

gtgtctaacg ggagccctag cctggagcgg atggacgcca ggcaggcgga gcaccccaag 540

ccctcggcct gcaggaacct cttcggcccg gtggaccacg aagagttaac ccgggacttg 600

gagaagcact gcagagacat ggaagaggcg agccagcgca agtggaattt cgattttcag 660

aatcacaaac ccctagaggg caagtacgag tggcaagagg tggagaaggg cagcttgccc 720

gagttctact acagaccccc gcggcccccc aaaggtgcct gcaaggtgcc ggcgcaggag 780

agccaggatg tcagcgggag ccgcccggcg gcgcctttaa ttggggctcc ggctaactct 840

gaggacacgc atttggtgga cccaaagact gatccgtcgg acagccagac ggggttagcg 900

gagcaatgcg caggaataag gaagcgacct gcaaccgacg attcttctac tcaaaacaaa 960

agagccaaca gaacagaaga aaatgtttca gacggttccc caaatgccgg ttctgtggag 1020

cagacgccca agaagcctgg cctcagaaga cgtcaaacgt aaacagctcg aattaagaat 1080

atgtttcctt gtttatcaga tacatcactg cttgatgaag caaggaagat atacatgaaa 1140

attttaaaaa tacatatcgc tgacttcatg gaatggacat cctgtataag cactgaaaaa 1200

caacaacaca ataacactaa aattttaggc actcttaaat gatctgcctc taaaagcgtt 1260

ggatgtagca ttatgcaatt aggtttttcc ttatttgctt cattgtacta cctgtgtata 1320

tagtttttac cttttatgta gcacataaac tttggggaag ggagggcagg gtggggctga 1380

ggaactgacg tggagcgggg tatgaagagc ttgctttgat ttacagcaag tagataaata 1440

tttgacttgc atgaagagaa gcaattttgg ggaagggttt gaattgtttt ctttaaagat 1500

gtaatgtccc tttcagagac agctgatact tcatttaaaa aaatcacaaa aatttgaaca 1560

ctggctaaag ataattgcta tttattttta caagaagttt attctcattt gggagatctg 1620

gtgatctccc aagctatcta aagtttgtta gatagctgca tgtggctttt ttaaaaaagc 1680

aacagaaacc tatcctcact gccctcccca gtctctctta aagttggaat ttaccagtta 1740

attactcagc agaatggtga tcactccagg tagtttgggg caaaaatccg aggtgcttgg 1800

gagttttgaa tgttaagaat tgaccatctg cttttattaa atttgttgac aaaatttct 1860

cattttcttt tcacttcggg ctgtgtaaac acagtcaaaa taattctaaa tccctcgata 1920

tttttaaaga tctgtaagta acttcacatt aaaaaatgaa atattttta atttaaagct 1980

tactctgtcc atttatccac aggaaagtgt tatttttaaa ggaaggttca tgtagagaaa 2040

agcacacttg taggataagt gaaatggata ctacatcttt aaacagtatt tcattgcctg 2100

tgtatggaaa aaccatttga agtgtacctg tgtacataac tctgtaaaaa cactgaaaaa 2160

ttatactaac ttatttatgt taaaagattt tttttaatct agacaatata caagccaaag 2220

tggcatgttt tgtgcatttg taaatgctgt gttgggtaga ataggttttc ccctcttttg 2280

ttaaataata tggctatgct taaaaggttg catactgagc caagtataat tttttgtaat 2340

gtgtgaaaaa gatgccaatt attgttacac attaagtaat caataaagaa aacttccata 2400

gctaaaaaaa aaaaaaaaaa aa 2422

<210> 32
<211> 1181
<212> DNA
<213> human

<400> 32
ggcacgaggc ccgggccccc caaagtcccg gccgggccga gggtcggcgg ccgccggcgg 60

gccgggcccg cgcacagcgc ccgcatgtac aacatgatgg agacggagct gaagccgccg 120

ggcccgcagc aaacttcggg gggcggcggc ggcaactcca ccgcggcggc ggccggcggc 180

aaccagaaaa acagcccgga ccgcgtcaag cggcccatga atgccttcat ggtgtggtcc 240

cgcgggcagc ggcgcaagat ggcccaggag aaccccaaga tgcacaactc ggagatcagc 300

aagcgcctgg gcgccgagtg gaaacttttg tcggagacgg agaagcggcc gttcatcgac 360

gaggctaagc ggctgcgagc gctgcacatg aaggagcacc cggattataa ataccggccc 420

cggcggaaaa ccaagacgct catgaagaag gataagtaca cgctgcccgg cgggctgctg 480

gcccccggcg gcaatagcat ggcgagcggg gtcggggtgg gcgccggcct gggcgcgggc 540

gtgaaccagc gcatggacag ttacgcgcac atgaacggct ggagcaacgg cagctacagc 600

atgatgcagg accagctggg ctacccgcag cacccgggcc tcaatgcgca cggcgcagcg 660

cagatgcagc ccatgcaccg ctacgacgtg agcgccctgc agtacaactc catgaccagc 720

tcgcagacct acatgaacgg ctcgcccacc tacagcatgt cctactcgca gcagggcacc 780

cctggcatgg ctcttggctc catgggttcg gtggtcaagt ccgaggccag ctccagcccc 840
cctgtggtta cctcttcctc ccactccagg gcgccctgcc aggccgggga cctccgggac 900
atgatcagca tgtatctccc cggcgccgag gtgccggaac ccgccgcccc cagcagactt 960
cacatgtccc agcactacca gagcggcccg gtgcccggca cggccattaa cggcacactg 1020
cccctctcac acatgtgagg gccggacagc gaactggagg ggggagaaat tttcaaagaa 1080
aaacgaggga aatgggaggg gtgcaaaaga ggagagtaag aaacagcatg gagaaaaccc 1140
ggtacgctca aaaaaaaaaa aaaaaaaaaa aaaaaaaaaa a 1181

<210> 33
<211> 1305
<212> DNA
<213> human

<400> 33
ggggccccgc gccggcccgc gccctgccca gtgcggcctc cttccacccg ccgctgcctg 60
gcccgcgccg tccggccgag ctgcccggcg ggctggtccc cgcgcccgag ccgcccggcc 120
gggaccccga acaaggccga gatgacttcc aaggaggacg gcaaggcggc gccgggggag 180
gagcggcgcc gcagcccgct ggaccacctg cctccgcctg ccaactccaa caagccagac 240
gccgttcagc atcgaggaca tcctcaacaa gccgtctgtg cggagaagtt actcgctgcg 300
tggggcggcg cacctgctgg ccgccgcgga caagcacgcg cagggcggct tgccctggcg 360
ggccgcgcgc tgctctcgaa gacctcgccg ctgtgcgcgc tggaggagct cgccagcaag 420
acgtttaagg ggctggaggt cagcgttctg caggcagccg aaggccgcga cggtatgacc 480
atctttgggc agcggcagac ccctaagaag cggcgaaagt cgcgcacggc cttcaccaac 540
caccagatct atgaattgga aaagcgcttt ctataccaga agtacctgtc ccccgccgat 600
cgcgaccaaa tcgcgcagca gctgggcctc accaacgcgc aagtcatcac ctggttccag 660
aatcggcgcg ctaagctcaa gcgggaactg gaggagatga aggccgacgt ggagtccccc 720
aagaaactgg gccccagcgg gcagatggac atcgtggcgc tggccgaact cgagcagaac 780
tcggaggcca cagccggcgg tggcggcggc tgcggcaggg ccaagtcgag gcccggctct 840
ccggtcctcc ccccaggcgc cccgaaggcc cccgggcgct gcgccctgca gctctcgcct 900
gcctctccgc tcacggacca gccggccagc agccaggact gctcggagga cgaggaagac 960
gaagagatcg acgtggacga ttgagcggcg ccccgggtct tccgccgccc tgggctccta 1020
gcgctcgaaa gcccaacgcc tcccggaccg gaccgccgag gggagctggg acctcctctg 1080

ccactcccgc ctcctcccct gtccccggac tcggctcctg gcagccgcct cttccctctc 1140

gaagcaataa acccaggctg gccggccggg ccggccgcca ccagcggcct ccgccgcccc 1200

ggaagccctc gccgagcaat tctgtatggc ttctatataa atatttaaac ctatatagcg 1260

ggttctcccc aaaaaaaaaa aaaaaaaaaa aaaaaaaaaa aaaaa 1305

<210> 34
<211> 1926
<212> DNA
<213> human

<400> 34
gctggagcat cccgctctgg tgccgctgca gccggcagag atggttgagc tcatgttccc 60

gctgttgctc ctccttctgc ccttccttct gtatatggct gcgccccaaa tcaggaaaat 120

gctgtccagt ggggtgtgta catcaactgt tcagcttcct gggaaagtag ttgtggtcac 180

aggagctaat acaggtatcg ggaaggagac agccaaagag ctggctcaga gaggagctcg 240

agtatattta gcttgccggg atgtggaaaa gggggaattg gtggccaaag agatccagac 300

cacgacaggg aaccagcagg tgttggtgcg gaaactggac ctgtctgata ctaagtctat 360

tcgagctttt gctaagggct tcttagctga ggaaaagcac ctccacgttt tgatcaacaa 420

tgcaggagtg atgatgtgtc cgtactcgaa gacagcagat ggctttgaga tgcacatagg 480

agtcaaccac ttgggtcact tcctcctaac ccatctgctg ctagagaaac taaaggaatc 540

agccccatca aggatagtaa atgtgtcttc cctcgcacat cacctgggaa ggatccactt 600

ccataacctg cagggcgaga aattctacaa tgcaggcctg gcctactgtc acagcaagct 660

agccaacatc ctcttcaccc aggaactggc ccggagacta aaaggctctg gcgttacgac 720

gtattctgta caccctggca cagtccaatc tgaactggtt cggcactcat ctttcatgag 780

atggatgtgg tggcttttct cctttttcat caagactcct cagcagggag cccagaccag 840

cctgcactgt gccttaacag aaggtcttga gattctaagt gggaatcatt tcagtgactg 900

tcatgtggca tgggtctctg cccaagctcg taatgagact atagcaaggc ggctgtggga 960

cgtcagttgt gacctgctgg gcctcccaat agactaacag gcagtgcagt tggacccaag 1020

agaagactgc agcagactac acagtacttc ttgtcaaaat gattctcctt caaggttttc 1080

aaaaccttta gcacaaagag agcaaaacct tccagccttg cctgcttggt gtccagttaa 1140

aactcagtgt actgccagat tcgtctaaat gtctgtcatg tccagattta ctttgcttct 1200

gttactgcca gagttactag agatatcata ataggataag aagaccctca tatgacctgc 1260

acagctcatt ttccttctga aagaaactac tacctaggag aatctaagct atagcaggga 1320

tgatttatgc aaatttgaac tagcttcttt gttcacaatt cagttcctcc caaccaacca 1380

gtcttcactt caagagggcc acactgcaac ctcagcttaa catgaataac aaagactggc 1440

tcaggagcag ggcttgccca ggcatggtgg atcaccggag tcagtagttc aagaccagcc 1500

tggccaacat ggtgaaaccc cacctctact aaaaattgtg tatatctttg tgtgtcttcc 1560

tgtttatgtg tgccaaggga gtattttcac aaagttcaaa acagccacaa taatcagaga 1620

tggagcaaac cagtgccatc cagtctttat gcaaatgaaa tgctgcaaag ggaagcagat 1680

tctgtatatg ttggtaacta cccaccaaga gcacatgggt agcagggaag aagtaaaaaa 1740

agagaaggag aatactggaa gataatgcac aaaatgaagg gactagttaa ggattaacta 1800

gccctttaag gattaactag ttaaggatta atagcaaaag acattaaata tgctaacata 1860

gctatggagg aattgagggc aagcacccag gactgatgag gtcttaacaa aaaccagtgt 1920

ggcaaa 1926

<210> 35
<211> 1195
<212> DNA
<213> human

<400> 35
ccgagactca cggtcaagct aaggcgaaga gtgggtggct gaagccatac tattttatag 60

aattaatgga aagcagaaaa gacatcacaa accaagaaga actttggaaa atgaagccta 120

ggagaaattt agaagaagac gattatttgc ataaggacac gggagagacc agcatgctaa 180

aaagacctgt gcttttgcat ttgcaccaaa cagcccatgc tgatgaattt gactgccctt 240

cagaacttca gcacacacag gaactctttc cacagtggca cttgccaatt aaaatagctg 300

ctattatagc atctctgact tttctttaca ctcttctgag ggaagtaatt caccctttag 360

caacttccca tcaacaatat ttttataaaa ttccaatcct ggtcatcaac aaagtcttgc 420

caatggtttc catcactctc ttggcattgg tttacctgcc aggtgtgata gcagcaattg 480

tccaacttca taatggaacc aagtataaga agtttccaca ttggttggat aagtggatgt 540

taacaagaaa gcagtttggg cttctcagtt tctttttgc tgtactgcat gcaatttata 600

gtctgtctta cccaatgagg cgatcctaca gatacaagtt gctaaactgg gcatatcaac 660

aggtccaaca aaataaagaa gatgcctgga ttgagcatga tgtttggaga atggagattt 720

atgtgtctct gggaattgtg ggattggcaa tactggctct gttggctgtg acatctattc 780

catctgtgag tgactctttg acatggagag aatttcacta tattcagagc aagctaggaa 840

ttgtttccct tctactgggc acaatacacg cattgatttt tgcctggaat aagtggatag 900

atataaaaca atttgtatgg tatacacctc caacttttat gatagctgtt ttccttccaa 960

ttgttgtcct gatatttaaa agcatactat tcctgccatg cttgaggaag aagatactga 1020

agattagaca tggttgggaa gacgtcacca aaattaacaa aactgagata tgttcccagt 1080

tgtagaatta ctgtttacac acatttttgt tcaatattga tatattttat caccaacatt 1140

tcaagtttgt atttgttaat aaaatgatta ttcaaggaaa aaaaaaaaaa aaaaa 1195

<210> 36
<211> 3414
<212> DNA
<213> human

<400> 36
gcttacacag tatggccggc gacattagct agcgctcgct ctactctctc taacgggaaa 60

gcagcggaat acaagagact gaactgtatc tgcctctatt tccaaaagac tcacgttcaa 120

ctttcgctca cacaaagccg ggaaaatttt attagtcctt tttttaaaaa aagttaatat 180

aaaattatag caaaaaaaaa aaggaacctg aactttagta acacagctgg aacaatcgca 240

gcggcggcgg cagcggcggg agaagaggtt taatttagtt gattttctgt ggttgttggt 300

tgttcgctag tctcacggtg atggaagctg cacatttttt cgaagggacc gagaagctgc 360

tggaggtttg gttctcccgg cagcagcccg acgcaaacca aggatctggg gatcttcgca 420

ctatcccaag atctgagtgg gacatacttt tgaaggatgt gcaatgttca atcataagtg 480

tgacaaaaac tgacaagcag gaagcttatg tactcagtga gagtagcatg tttgtctcca 540

agagacgttt cattttgaag acatgtggta ccaccctctt gctgaaagca ctggttcccc 600

tgttgaagct tgctagggat tacagtgggt ttgactcaat tcaaagcttc ttttattctc 660

gtaagaattt catgaagcct tctcaccaag ggtacccaca ccggaatttc caggaagaaa 720

tagagtttct taatgcaatt ttcccaaatg gagcaggata ttgtatggga cgtatgaatt 780

ctgactgttg gtacttatat actctggatt tcccagagag tcgggtaatc agtcagccag 840

atcaaacctt ggaaattctg atgagtgagc ttgacccagc agttatggac cagttctaca 900

tgaaagatgg tgttactgca aaggatgtca ctcgtgagag tggaattcgt gacctgatac 960

caggttctgt cattgatgcc acaatgttca atccttgtgg gtattcgatg aatggaatga 1020

aatcggatgg aacttattgg actattcaca tcactccaga accagaattt tcttatgtta 1080

gctttgaaac aaacttaagt cagacctcct atgatgacct gatcaggaaa gttgtagaag 1140
tcttcaagcc aggaaaattt gtgaccacct tgtttgttaa tcagagttct aaatgtcgca 1200
cagtgcttgc ttcgccccag aagattgaag gttttaagcg tcttgattgc cagagtgcta 1260
tgttcaatga ttacaatttt gtttttacca gttttgctaa gaagcagcaa caacagcaga 1320
gttgattaag aaaaatgaag aaaaaacgca aaaagagaac acatgtagaa ggtggtggat 1380
gctttctaga tgtcgatgct gggggcagtg ctttccataa ccaccactgt gtagttgcag 1440
aaagccctag atgtaatgat agtgtaatca ttttgaattg tatgcattat tatatcaagg 1500
agttagatat cttgcatgaa tgctctcttc tgtgtttagg tattctctgc cactcttgct 1560
gtgaaattga agtggatgta gaaaaaacct tttactatat gaaactttac aacacttgtg 1620
aaagcaactc aatttggttt atgcacagtg taatatttct ccaagtatca tccaaaattc 1680
cccacagaca aggctttcgt cctcattagg tgttggcctc agcctaaccc tctaggactg 1740
ttctattaaa ttgctgccag aattttacat ccagttacct ccactttcta gaacatattc 1800
tttactaatg ttattgaaac caatttctac ttcatactga tgtttttgga aacagcaatt 1860
aaagtttttc ttccatagtt gagtccttag aaaatgattc cagttactca ttttgcatat 1920
tgctatttaa cattattgga ccctgcattt atagtccttt gatttcttcc ctctccctgg 1980
tgtctccccc aagaccccaa ataaagcaat accctgttaa cactgtgggt ttatatacta 2040
attctatacc ccagatgggg aattaggggg gagatggtcc ctgggcttaa tattctttaa 2100
agggcatggg aatttagcct ctcttttatt gtaatgtgct cttttggaaa atagttggtt 2160
agcagggaga ccagagttgt agattgagat tgggtgtact ggctgttctg tggaaaacat 2220
acattctgtg ttcctcgaat aagtgaaatt gagcttctaa tgagatgcac ccctttacta 2280
acttgatgat gatataaaat tcatttttat ttagttaatt accagagaga tttagcataa 2340
ttttgcttct ggattcagta aatcaagtca gcttggatca ttcaccttaa cttttccttt 2400
agcagccctt tccactagtt tcccattaag tagtgttcta taaactttga tccaaagcag 2460
aatcaatgtc ttttccatct cgtgacttaa agttctgtga ctgtgatgca tgtgagtgtt 2520
ccgacttcat ctgttcctct taactacggt gtttccctta ccgatcggca ttcataggat 2580
gaaatgaatg actgtcccag aatgagaatt tgtccagatt attcagataa acatcataaa 2640
gagaataaca ttataaataa gtagaatatg aataaataga ataataaaat tccaaaatac 2700
tcaatgggaa atgactagta atataggctt tcaagagttg gtacctttac gtatatttgc 2760

agattctctg ggattttaag gaactgagaa aacagaaaag ttgactaaat tttatatttc 2820

ttgtcctcta aatattttga taatttctgg attgatgcag tgatgttttt cgttccttgt 2880

atttataaat gaaaaccttt ttttggtgtt tctaaaccta aaatctactt ggtttgaaat 2940

caagtggttg gaacactgtt tgacttttat ttgaagcatg ttgttgattg aaaatttcat 3000

tgaggaagtt ttcaatcagt gtgatcagtt tgattctgta atgagcacag cacctaatat 3060

tttgaggagc tctgttttga ggaccaatgc ttaaggtgga ccttgttgct aaacaatatc 3120

ccaatagatt tgttgacttg aggtctggtt tggttttgtt tttgttttgt tttggttttg 3180

ttttgtttcc caatagaatt aagaattcta atgttgaaaa actgtataaa tttttatggg 3240

acaaagccta gaaaagagaa atgtagtttg aatcataatc taaatcatcg tatgatagga 3300

aggaaaagtt ttggtgccat aatttctcct ttcactggtg ttggacttaa atcagttgaa 3360

atgtatttct gtaccacaat ttacgcttca ataaaagttt aattgtctag tgag 3414

<210> 37
<211> 3287
<212> DNA
<213> human

<400> 37
agactgaggc ggaggcagcc ccgcgccgcg ccggacccga gcatatttca ttttctgtca 60

ttggactttg agccattaga accatgagca actacagtgt gtcactggtt ggcccagctc 120

cttggggttt ccggctgcag ggcggtaagg atttcaacat gcctctgaca atctctagtc 180

taaaagatgg cggcaaggca gcccaggcaa atgtaagaat aggcgatgtg gttctcagca 240

ttgatggaat aaatgcacaa ggaatgactc atcttgaagc ccagaataag attaagggtt 300

gtacaggctc tttgaatatg actctgcaaa gagcatctgc tgcacccaag cctgagccgg 360

ttcctgttca aaagggagaa cctaaagaag tagttaaacc tgtgcccatt acatctcctg 420

ctgtgtccaa agtcacttcc acaaacaaca tggcctacaa taaggcacca cggccttttg 480

gttctgtgtc ttcaccaaaa gtcacatcca tcccatcacc atcgtctgcc ttcaccccag 540

cccatgcgac cacctcatca catgcttccc cttcacccgt ggctgccgtc actcctcccc 600

tgttcgctgc atctggactg catgctaatg ccaatcttag tgctgaccag tctccatctg 660

cactgagcgc tggtaaaact gcagttaatg tcccacggca gcccacagtc accagcgtgt 720

gttccgagac ttctcaggag ctagcagagg gacagagaag aggatcccag ggtgacagta 780

aacagcaaaa tggcccacca agaaaacaca ttgtggagcg ctatacagag ttttatcatg 840

tacccactca cagtgatgcc agcaagaaga gactgattga ggatactgaa gactggcgtc 900
caagaactgg aacaactcag tctcgctctt tccgaatcct tgcccagatc actgggactg 960
aacatttgaa agaatctgaa gccgataata caaagaaggc aaataactct caggagcctt 1020
ctccgcagtt ggcttccttg gtagcttcca cacggagcat gcccgagagc ctggacagcc 1080
caacctctgg cagaccaggg gttaccagcc tcacaactgc agctgccttc aagcctgtag 1140
gatccactgg cgtcatcaag tcaccaagct ggcaacggcc aaaccaagga gtaccttcca 1200
ctggaagaat ctcaaacagc gctacttact caggatcagt ggcaccagcc aactcagctt 1260
tgggacaaac ccagccaagt gaccaggaca ctttagtgca aagagctgag cacattccag 1320
cagggaaacg aactccgatg tgcgcccatt gtaaccaggt catcagagga ccattcttag 1380
tggcactggg gaaatcttgg cacccagaag aattcaactg cgctcactgc aaaaatacaa 1440
tggcctacat tggatttgta gaggagaaag gagccctgta ttgtgagctg tgctatgaga 1500
aattctttgc ccctgaatgt ggtcgatgcc aaaggaagat ccttggagaa gtcatcaatg 1560
cgttgaaaca aacttggcat gtttcctgtt ttgtgtgtgt agcctgtgga aagcccattc 1620
ggaacaatgt ttttcacttg gaggatggtg aaccctactg tgagactgat tattatgccc 1680
tctttggtac tatatgccat ggatgtgaat ttcccataga agctggtgac atgttcctgg 1740
aagctctggg ctacacctgg catgacactt gctttgtatg ctcagtgtgt tgtgaaagtt 1800
tggaaggtca gacctttttc tccaagaagg acaagcccct gtgtaagaaa catgctcatt 1860
ctgtgaattt ttgaaagtca acagttcagg agaagagaag gaatttgaag agaaaaagga 1920
aaattaaaat tactaattaa tttttagatt caatatttat atggagtttt gaaaaataat 1980
agtggccctg aaggaataaa ttccagcttt aaaaaccaag tctgaggaaa tatttggctt 2040
cataaagtaa agagacggtt tggcatttat tattactttt tcctgtattt tatgcccata 2100
aaataagctt tataaaaacc aatttcctga tggactatta aattcatctt agaataaatt 2160
agtgaagaat ttaattttag aataaataat ccaatctgaa ataattatac cttctttcct 2220
tgttaggtag ttatgagtaa atctgcaaaa ggcaatgaaa atgccttaaa ttttatcaat 2280
aacagaatta ttgtatttaa aaaaaaacta atacttatct ttaaaatagt aaataggatt 2340
ttaaacagag aattttatca gtaataggtg tcagttttta aaaaattgct tgtaggctga 2400
gcgcggtggc tcacgcctgt aatcccagca ctttgggagg ccaaggtggg tggaccacat 2460
gaggtcagga gtttgagatc agcctggcca acatggtgaa accccatctc tactaaaaat 2520
acaaaaatta gccggacgca gtggcacgcg cctgtaatcc cagctactca agaggctgag 2580

gcacgagaat cacttgaacc cgggagggag aggttgcagt gagccaagat cgtaccactg 2640
cactccagcc tgggtgacag agtgagactc cgtctccaaa aaaaaacttt gcttgtatat 2700
tatttttgcc ttacagtgga tcattctagt aggaaaggac aataagattt tttatcaaaa 2760
tgtgtcatgc cagtaagaga tgttatattc ttttcttatt tcttccccac ccaaaaataa 2820
gctaccatat agcttataag tctcaaattt ttgcctttta ctaaaatgtg attgtttctg 2880
ttcattgtgt atgcttcatc acctatatta ggcaaattcc atttttcccc ttgcgctaag 2940
gtaaagattt aattaaataa ttttggcctc tcatagtttt ctctctcttt aaagagaata 3000
aatagagggc caggtgtggt ggctcacgcc tgtgatccca gcactttggg aggccaagac 3060
gggcggatca tgaggtcaag agatcaagat catcctggcc aacatggtga aaccctgtct 3120
ctactaaaaa tacaaaaatg agctgggcat ggtggggcgt gcctgtagtc ccatgtactt 3180
gggaggctga ggcaggaaaa ttcttgaacc caggagacgg aagttgcagt gagctgagat 3240
cacaccactg cactccagcc tggtgacaga gcaagactcc ggctctt 3287

<210> 38
<211> 1254
<212> DNA
<213> human

<400> 38
atggcagtgg agaccacggt ccacactcac ctctctgcgt ctccaccgca gggctctccc 60
tacgaccaca cacccggcat ggcgggctcc ttggggtacc atccttacgc ggcgcccctg 120
ggatcgtacc cttacgggga cccagcgtac cggaagaacg ccacaaggga cgccacggct 180
accctcaagg cctggctcaa cgagcaccgc aagaacccct accccaccaa gggcgagaag 240
atcatgctgg ccatcatcac caagatgacc ctcacccagg tgtccacctg gttcgccaac 300
gcgcgccggc gcctcaagaa agagaataaa atgacgtgga cgccgcggaa ccgcagcgag 360
gacgaggaag aggaggagaa cattgacctg gagaagaacg acgaggacga gccccagaag 420
cccgaggaca agggcgaccc cgagggcccc gaagcaggag gagctgagca gaaggcggct 480
tcgggctgcg aacggcttca gggaccaccc acccctgcag gcaaggagac ggagggcagc 540
ctcagcgact cggattttaa ggagccgccc tcggagggcc gcctcgacgc gctgcagggc 600
ccccccccgca ccggcgggcc ctccccggct gggccagcgg cggcgcggct ggcggaggac 660
ccggcccctc actaccccgc cggagcgccg gcgcccggcc cgcatccagc cgcgggcgag 720
gtgcctccgg gtcccggcgg gccctcggtt atccattcgc cgcctccgcc gccgcctcct 780

gcggtgctcg ccaagcccaa actgtggtct ttggcagaga tcgccacatt gtcggacaag 840

gtcaaggacg ggggcggcgg gaacgagggc tctccatgcc caccgtgtcc cgggcccata 900

gccgggcaag ccctaggagg cagccgggcg tcgccggccc cggcgccgtc acgctcgccc 960

tcggcgcagt gtccttttcc aggcgggacg gtgctgtccc ggcctctcta ctacaccgcg 1020

cccttctatc ccggctacac gaactatggc tccttcggac accttcatgg ccacccgggg 1080

cccgggccag gccccacaac cggtccgggg tctcatttca atggattaaa ccagaccgtg 1140

ttgaaccgag cggacgcttt ggctaaagac ccgaaaatgt tgcggagcca gtctcagcta 1200

gacctgtgca aagactctcc ctatgaattg aagaaaggta tgtccgacat ttaa 1254

<210> 39
<211> 2560
<212> DNA
<213> human

<400> 39
gaattccggc cagaagaaat ctggcctcgg aacacgccat tctccgcgcc gcttccaata 60

accactaaca tccctaacga gcatccgagc cgagggctct gctcggaaat cgtcctggcc 120

caactcggcc cttcgagctc tcgaagatta ccgcatctat tttttttttc tttttttttct 180

tttcctagcg cagataaagt gagcccggaa agggaaggag gggcgggga caccattgcc 240

ctgaaagaat aaataagtaa ataaacaaac tggctcctcg ccgcagctgg acgcggtcgg 300

ttgagtccag gttgggtcgg acctgaaccc ctaaaagcgg aaccgcctcc cgccctcgcc 360

atcccggagc tgagtcgccg gcggcggtgg ctgctgccag acccggagtt tcctctttca 420

ctggatggag ctgaactttg ggcggccaga gcagcacagc tgtccgggga tcgctgcacg 480

ctgagctccc tcggcaagac ccagcggcgg ctcgggattt ttttgggggg gcggggacca 540

gccccgcgcc ggcaccatgt tcctggcgac cctgtacttc gcgctgccgc tcttggactt 600

gctcctgtcg gccgaagtga gcggcggaga ccgcctggat tgcgtgaaag ccagtgatca 660

gtgcctgaag gagcagagct gcagcaccaa gtaccgcacg ctaaggcagt gcgtggcggg 720

caaggagacc aacttcagcc tggcatccgg cctggaggcc aaggatgagt gccgcagcgc 780

catggaggcc ctgaagcaga agtcgctcta caactgccgc tgcaagcggg gtatgaagaa 840

ggagaagaac tgcctgcgca tttactggag catgtaccag agcctgcagg gaaatgatct 900

gctggaggat tccccatatg aaccagttaa cagcagattg tcagatatat tccgggtggt 960

cccattcata tcagatgttt ttcagcaagt ggagcacatt cccaaaggga acaactgcct 1020

ggatgcagcg aaggcctgca acctcgacga catttgcaag aagtacaggt cggcgtacat 1080
caccccgtgc accaccagcg tgtccaacga tgtctgcaac cgccgcaagt gccacaaggc 1140
cctccggcag ttctttgaca aggtcccggc caagcacagc tacggaatgc tcttctgctc 1200
ctgccgggac atcgcctgca cagagcggag gcgacagacc atcgtgcctg tgtgctccta 1260
tgaagagagg gagaagccca actgtttgaa tttgcaggac tcctgcaaga cgaattacat 1320
ctgcagatct cgccttgcgg atttttttac caactgccag ccagagtcaa ggtctgtcag 1380
cagctgtcta aaggaaaact acgctgactg cctcctcgcc tactcggggc ttattggcac 1440
agtcatgacc cccaactaca tagactccag tagcctcagt gtggccccat ggtgtgactg 1500
cagcaacagt gggaacgacc tagaagagtg cttgaaattt ttgaatttct tcaaggacaa 1560
tacatgtctt aaaaatgcaa ttcaagcctt tggcaatggc tccgatgtga ccgtgtggca 1620
gccagccttc ccagtacaga ccaccactgc cactaccacc actgccctcc gggttaagaa 1680
caagcccctg gggccagcag ggtctgagaa tgaaattccc actcatgttt tgccaccgtg 1740
tgcaaattta caggcacaga agctgaaatc caatgtgtcg ggcaatacac acctctgtat 1800
ttccaatggt aattatgaaa aagaaggtct cggtgcttcc agccacataa ccacaaaatc 1860
aatggctgct cctccaagct gtggtctgag cccactgctg gtcctggtgg taaccgctct 1920
gtccacccta ttatctttaa cagaaacatc atagctgcat taaaaaaata caatatggac 1980
atgtaaaaag acaaaaacca agttatctgt ttcctgttct cttgtatagc tgaaattcca 2040
gtttaggagc tcagttgaga aacagttcca ttcaactgga acattttttt ttttcctttt 2100
aagaaagctt cttgtgatcc ttcggggctt ctgtgaaaaa cctgatgcag tgctccatcc 2160
aaactcagaa ggctttggga tatgctgtat tttaaaggga cagtttgtaa cttgggctgt 2220
aaagcaaact ggggctgtgt tttcgatgat gatgatcatc atgatcatga tgattttaac 2280
agttttactt ctggcctttc ctagctagag aaggagttaa tatttctaag gtaactccca 2340
tatctccttt aatgacattg atttctaatg atataaattt cagcctacat tgatgccaag 2400
ctttttttgcc acaaagaaga ttcttaccaa gagtgggctt tgtggaaaca gctggtactg 2460
atgttcacct ttatatatgt actagcattt tccacgctga tgtttatgta ctgtaaacag 2520
ttctgcactc ttgtacaaaa gaaaaaacca cccggaattc 2560

<210> 40
<211> 2560
<212> DNA

<213> human

<400> 40
gaattccggc cagaagaaat ctggcctcgg aacacgccat tctccgcgcc gcttccaata 60

accactaaca tccctaacga gcatccgagc cgagggctct gctcggaaat cgtcctggcc 120

caactcggcc cttcgagctc tcgaagatta ccgcatctat ttttttttc tttttttct 180

tttcctagcg cagataaagt gagcccggaa agggaaggag ggggcgggga caccattgcc 240

ctgaaagaat aaataagtaa ataaacaaac tggctcctcg ccgcagctgg acgcggtcgg 300

ttgagtccag gttgggtcgg acctgaaccc ctaaaagcgg aaccgcctcc cgccctcgcc 360

atcccggagc tgagtcgccg gcggcggtgg ctgctgccag acccggagtt tcctctttca 420

ctggatggag ctgaactttg ggcggccaga gcagcacagc tgtccgggga tcgctgcacg 480

ctgagctccc tcggcaagac ccagcggcgg ctcgggattt ttttgggggg gcggggacca 540

gccccgcgcc ggcaccatgt tcctggcgac cctgtacttc gcgctgccgc tcttggactt 600

gctcctgtcg gccgaagtga gcggcggaga ccgcctggat tgcgtgaaag ccagtgatca 660

gtgcctgaag gagcagagct gcagcaccaa gtaccgcacg ctaaggcagt gcgtggcggg 720

caaggagacc aacttcagcc tggcatccgg cctggaggcc aaggatgagt gccgcagcgc 780

catggaggcc ctgaagcaga agtcgctcta caactgccgc tgcaagcggg gtatgaagaa 840

ggagaagaac tgcctgcgca tttactggag catgtaccag agcctgcagg gaaatgatct 900

gctggaggat tccccatatg aaccagttaa cagcagattg tcagatatat tccgggtggt 960

cccattcata tcagatgttt ttcagcaagt ggagcacatt cccaaaggga acaactgcct 1020

ggatgcagcg aaggcctgca acctcgacga catttgcaag aagtacaggt cggcgtacat 1080

caccccgtgc accaccagcg tgtccaacga tgtctgcaac cgccgcaagt gccacaaggc 1140

cctccggcag ttctttgaca aggtcccggc caagcacagc tacggaatgc tcttctgctc 1200

ctgccgggac atcgcctgca cagagcggag gcgacagacc atcgtgcctg tgtgctccta 1260

tgaagagagg gagaagccca actgtttgaa tttgcaggac tcctgcaaga cgaattacat 1320

ctgcagatct cgccttgcgg atttttttac caactgccag ccagagtcaa ggtctgtcag 1380

cagctgtcta aaggaaaact acgctgactg cctcctcgcc tactcggggc ttattggcac 1440

agtcatgacc cccaactaca tagactccag tagcctcagt gtggccccat ggtgtgactg 1500

cagcaacagt gggaacgacc tagaagagtg cttgaaattt ttgaatttct tcaaggacaa 1560

tacatgtctt aaaaatgcaa ttcaagcctt tggcaatggc tccgatgtga ccgtgtggca 1620

gccagccttc ccagtacaga ccaccactgc cactaccacc actgccctcc gggttaagaa 1680

caagcccctg gggccagcag ggtctgagaa tgaaattccc actcatgttt tgccaccgtg 1740

tgcaaattta caggcacaga agctgaaatc caatgtgtcg ggcaatacac acctctgtat 1800

ttccaatggt aattatgaaa aagaaggtct cggtgcttcc agccacataa ccacaaaatc 1860

aatggctgct cctccaagct gtggtctgag cccactgctg gtcctggtgg taaccgctct 1920

gtccacccta ttatctttaa cagaaacatc atagctgcat taaaaaaata caatatggac 1980

atgtaaaaag acaaaaacca agttatctgt ttcctgttct cttgtatagc tgaaattcca 2040

gtttaggagc tcagttgaga aacagttcca ttcaactgga acattttttt ttttcctttt 2100

aagaaagctt cttgtgatcc ttcggggctt ctgtgaaaaa cctgatgcag tgctccatcc 2160

aaactcagaa ggctttggga tatgctgtat tttaaaggga cagtttgtaa cttgggctgt 2220

aaagcaaact ggggctgtgt tttcgatgat gatgatcatc atgatcatga tgattttaac 2280

agttttactt ctggcctttc ctagctagag aaggagttaa tatttctaag gtaactccca 2340

tatctccttt aatgacattg atttctaatg atataaattt cagcctacat tgatgccaag 2400

ctttttttgcc acaaagaaga ttcttaccaa gagtgggctt tgtggaaaca gctggtactg 2460

atgttcacct ttatatatgt actagcattt tccacgctga tgtttatgta ctgtaaacag 2520

ttctgcactc ttgtacaaaa gaaaaaacca cccggaattc 2560

<210> 41
<211> 2439
<212> DNA
<213> human

<400> 41
cagcacccag ctccccgcca ccgccatggt ccccgacacc gcctgcgttc ttctgctcac 60

cctggctgcc ctcggcgcgt ccggacaggg ccagagcccg ttgggctcag acctgggccc 120

gcagatgctt cgggaactgc aggaaaccaa cgcggcgctg caggacgtgc gggactggct 180

gcggcagcag gtcagggaga tcacgttcct gaaaaacacg gtgatggagt gtgacgcgtg 240

cgggatgcag cagtcagtac gcaccggcct acccagcgtg cggcccctgc tccactgcgc 300

gcccggcttc tgcttccccg gcgtggcctg catccagacg gagagcggcg gccgctgcgg 360

cccctgcccc gcgggcttca cgggcaacgg ctcgcactgc accgacgtca acgagtgcaa 420

cgcccacccc tgcttccccc gagtccgctg tatcaacacc agcccggggt tccgctgcga 480

ggcttgcccg ccggggtaca gcggccccac ccaccagggc gtggggctgg ctttcgccaa 540

ggccaacaag caggtttgca cggacatcaa cgagtgtgag accgggcaac ataactgcgt 600
ccccaactcc gtgtgcatca acacccgggg ctccttccag tgcggcccgt gccagcccgg 660
cttcgtgggc gaccaggcgt ccggctgcca gcgcggcgca cagcgcttct gccccgacgg 720
ctcgcccagc gagtgccacg agcatgcaga ctgcgtccta gagcgcgatg gctcgcggtc 780
gtgcgtgtgt cgcgttggct gggccggcaa cgggatcctc tgtggtcgcg acactgacct 840
agacggcttc ccggacgaga agctgcgctg cccggagccg cagtgccgta aggacaactg 900
cgtgactgtg cccaactcag ggcaggagga tgtggaccgc gatggcatcg gagacgcctg 960
cgatccggat gccgacgggg acggggtccc caatgaaaag gacaactgcc cgctggtgcg 1020
gaacccagac cagcgcaaca cggacgagga caagtggggc gatgcgtgcg acaactgccg 1080
gtcccagaag aacgacgacc aaaaggacac agaccaggac ggccggggcg atgcgtgcga 1140
cgacgacatc gacggcgacc ggatccgcaa ccaggccgac aactgcccta gggtacccaa 1200
ctcagaccag aaggacagtg atggcgatgg tataggggat gcctgtgaca actgtcccca 1260
gaagagcaac ccggatcagg cggatgtgga ccacgacttt gtgggagatg cttgtgacag 1320
cgatcaagac caggatggag acggacatca ggactctcgg gacaactgtc ccacggtgcc 1380
taacagtgcc caggaggact cagaccacga tggccagggt gatgcctgcg acgacgacga 1440
cgacaatgac ggagtccctg acagtcggga caactgccgc ctggtgccta accccggcca 1500
ggaggacgcg gacagggacg gcgtgggcga cgtgtgccag gacgactttg atgcagacaa 1560
ggtggtagac aagatcgacg tgtgtccgga gaacgctgaa gtcacgctca ccgacttcag 1620
ggccttccag acagtcgtgc tggacccgga gggtgacgcg cagattgacc ccaactgggt 1680
ggtgctcaac cagggaaggg agatcgtgca gacaatgaac agcgacccag gcctggctgt 1740
gggttacact gccttcaatg gcgtggactt cgagggcacg ttccatgtga acacggtcac 1800
ggatgacgac tatgcgggct tcatctttgg ctaccaggac agctccagct tctacgtggt 1860
catgtggaag cagatggagc aaacgtattg gcaggcgaac cccttccgtg ctgtggccga 1920
gcctggcatc caactcaagg ctgtgaagtc ttccacaggc cccggggaac agctgcggaa 1980
cgctctgtgg catacaggag acacagagtc ccaggtgcgg ctgctgtgga aggacccgcg 2040
aaacgtgggt tggaaggaca agaagtccta tcgttggttc ctgcagcacc ggccccaagt 2100
gggctacatc agggtgcgat tctatgaggg ccctgagctg gtggccgaca gcaacgtggt 2160
cttggacaca accatgcggg gtggccgcct gggggtcttc tgcttctccc aggagaacat 2220
catctgggcc aacctgcgtt accgctgcaa tgacaccatc ccagaggact atgagaccca 2280

tcagctgcgg caagcctagg gaccagggtg aggacccgcc ggatgacagc caccctcacc 2340

gcggctggat gggggctctg cacccagccc aaggggtggc cgtcctgagg gggaagtgag 2400

aagggctcag agaggacaaa ataaagtgtg tgtgcaggg 2439

<210> 42
<211> 2439
<212> DNA
<213> human

<400> 42
cagcacccag ctccccgcca ccgccatggt ccccgacacc gcctgcgttc ttctgctcac 60

cctggctgcc ctcggcgcgt ccggacaggg ccagagcccg ttgggctcag acctgggccc 120

gcagatgctt cgggaactgc aggaaaccaa cgcggcgctg caggacgtgc gggactggct 180

gcggcagcag gtcagggaga tcacgttcct gaaaaacacg gtgatggagt gtgacgcgtg 240

cgggatgcag cagtcagtac gcaccggcct acccagcgtg cggcccctgc tccactgcgc 300

gcccggcttc tgcttccccg gcgtggcctg catccagacg gagagcggcg gccgctgcgg 360

cccctgcccc gcgggcttca cgggcaacgg ctcgcactgc accgacgtca acgagtgcaa 420

cgcccacccc tgcttccccc gagtccgctg tatcaacacc agcccggggt tccgctgcga 480

ggcttgcccg ccggggtaca gcggccccac ccaccagggc gtggggctgg ctttcgccaa 540

ggccaacaag caggtttgca cggacatcaa cgagtgtgag accgggcaac ataactgcgt 600

ccccaactcc gtgtgcatca acacccgggg ctccttccag tgcggcccgt gccagcccgg 660

cttcgtgggc gaccaggcgt ccggctgcca gcgcggcgca cagcgcttct gccccgacgg 720

ctcgcccagc gagtgccacg agcatgcaga ctgcgtccta gagcgcgatg gctcgcggtc 780

gtgcgtgtgt cgcgttggct gggccggcaa cgggatcctc tgtggtcgcg acactgacct 840

agacggcttc ccggacgaga agctgcgctg cccggagccg cagtgccgta aggacaactg 900

cgtgactgtg cccaactcag ggcaggagga tgtggaccgc gatggcatcg gagacgcctg 960

cgatccggat gccgacgggg acggggtccc caatgaaaag gacaactgcc cgctggtgcg 1020

gaacccagac cagcgcaaca cggacgagga caagtggggc gatgcgtgcg acaactgccg 1080

gtcccagaag aacgacgacc aaaaggacac agaccaggac ggccggggcg atgcgtgcga 1140

cgacgacatc gacggcgacc ggatccgcaa ccaggccgac aactgcccta gggtacccaa 1200

ctcagaccag aaggacagtg atggcgatgg tataggggat gcctgtgaca actgtcccca 1260

gaagagcaac ccggatcagg cggatgtgga ccacgacttt gtgggagatg cttgtgacag 1320

cgatcaagac caggatggag acggacatca ggactctcgg gacaactgtc ccacggtgcc 1380

taacagtgcc caggaggact cagaccacga tggccagggt gatgcctgcg acgacgacga 1440

cgacaatgac ggagtccctg acagtcggga caactgccgc ctggtgccta accccggcca 1500

ggaggacgcg gacagggacg gcgtgggcga cgtgtgccag gacgactttg atgcagacaa 1560

ggtggtagac aagatcgacg tgtgtccgga gaacgctgaa gtcacgctca ccgacttcag 1620

ggccttccag acagtcgtgc tggacccgga gggtgacgcg cagattgacc ccaactgggt 1680

ggtgctcaac cagggaaggg agatcgtgca gacaatgaac agcgacccag gcctggctgt 1740

gggttacact gccttcaatg gcgtggactt cgagggcacg ttccatgtga acacggtcac 1800

ggatgacgac tatgcgggct tcatctttgg ctaccaggac agctccagct tctacgtggt 1860

catgtggaag cagatggagc aaacgtattg gcaggcgaac cccttccgtg ctgtggccga 1920

gcctggcatc caactcaagg ctgtgaagtc ttccacaggc cccggggaac agctgcggaa 1980

cgctctgtgg catacaggag acacagagtc ccaggtgcgg ctgctgtgga aggacccgcg 2040

aaacgtgggt tggaaggaca agaagtccta tcgttggttc ctgcagcacc ggccccaagt 2100

gggctacatc agggtgcgat tctatgaggg ccctgagctg gtggccgaca gcaacgtggt 2160

cttggacaca accatgcggg gtggccgcct gggggtcttc tgcttctccc aggagaacat 2220

catctgggcc aacctgcgtt accgctgcaa tgacaccatc ccagaggact atgagaccca 2280

tcagctgcgg caagcctagg gaccagggtg aggacccgcc ggatgacagc caccctcacc 2340

gcggctggat gggggctctg cacccagccc aaggggtggc cgtcctgagg gggaagtgag 2400

aagggctcag agaggacaaa ataaagtgtg tgtgcaggg 2439

<210> 43
<211> 1385
<212> DNA
<213> human

<400> 43
ctccagccat tggtgtctgt gtcattacta atagagtctt gtaaacactc gttaatcacg 60

gaagccgccg gcctggggct ccgcacgcca gcctgtgcgg gtcttccccg cctctgcagc 120

ctagtgggaa ggaggtggga ggaaagaagg aagaaaggga gggagggagg aggcaggcca 180

gagggaggga ccgcctcgga ggcagaagag ccgcgaggag ccagcggagc accgcgggct 240

ggggcgcagc cacccgccgc tcctcgagtc ccctcgcccc tttcccttcg tgccccccgg 300

cagcctccag cgtcggtccc caggcagcat ggtgaggtct gctcccggtc cctcgccacc 360

atgtacgtga gctacctcct ggacaaggac gtgagcatgt accctagctc cgtgcgccac 420
tctggcggcc tcaacctggc gccgcagaac ttcgtcagcc ccccgcagta cccggactac 480
ggcggttacc acgtggcggc cgcagctgca gcggcagcga acttggacag cgcgcagtcc 540
ccggggccat cctggccggc agcgtatggc gccccactcc gggaggactg gaatggctac 600
gcgcccggag gcgccgcggc cgccgccaac gccgtggctc acggcctcaa cggtggctcc 660
ccggccgcag ccatgggcta cagcagcccc gcagactacc atccgcacca ccacccgcat 720
caccacccgc accacccggc cgcggcgcct tcctgcgctt ctgggctgct gcaaacgctc 780
aaccccggcc ctcctgggcc cgccgccacc gctgccgccg agcagctgtc tcccggcggc 840
cagcggcgga acctgtgcga gtggatgcgg aagccggcgc agcagtccct cggcagccaa 900
gtgaaaacca ggacgaaaga caaatatcga gtggtgtaca cggaccacca gcggctggag 960
ctggagaagg agtttcacta cagtcgctac atcaccatcc ggaggaaagc cgagctagcc 1020
gccacgctgg ggctctctga gaggcaggtt aaaatctggt ttcagaaccg cagagcaaag 1080
gagaggaaaa tcaacaagaa gaagttgcag cagcaacagc agcagcagcc accacagccg 1140
cctccgccgc caccacagcc tccccagcct cagccaggtc ctctgagaag tgtcccagag 1200
cccttgagtc cggtgtcttc cctgcaagcc tcagtgtctg gctctgtccc tggggttctg 1260
gggccaactg ggggggtgct aaaccccacc gtcacccagt gacccaccgg ggttctgcag 1320
cggcagagca attccaggct gagccatgag gagcgtggac tctgctagac tcctcaggag 1380
agacc 1385

<210> 44
<211> 4098
<212> DNA
<213> human

<400> 44
ggtggcctct gtggccgtcc aggctagcgg cggcccgcag gcggcgggga gaaagactct 60
ctcacctggt cttgcggctg tggccaccgc cggccagggg tgtggagggc gtgctgccgg 120
agacgtccgc cgggctctgc agttccgccg ggggtcgggc agctatggag ccgcggccca 180
cggcgccctc ctccggcgcc ccgggactgg ccggggtcgg ggagacgccg tcagccgctg 240
cgctggccgc agccagggtg gaactgcccg gcacggctgt gccctcggtg ccggaggatg 300
ctgcgcccgc gagccgggac ggcggcgggg tccgcgatga gggccccgcg gcggccgggg 360
acgggctggg cagacccttg ggcccaccc cgagccagag ccgtttccag gtggacctgg 420

tttccgagaa cgccgggcgg gccgctgctg cggcggcggc ggcggcggcg gcagcggcgg 480
cggctggtgc tggggcgggg gccaagcaga cccccgcgga cggggaagcc agcggcgaga 540
gcgagccagc taaaggcagc gaggaagcca agggccgctt ccgcgtgaac ttcgtggacc 600
cagctgcctc ctcgtcggct gaagacagcc tgtcagatgc tgccggggtc ggagtcgacg 660
ggcccaacgt gagcttccag aacggcgggg acacggtgct gagcgagggc agcagcctgc 720
actccggcgg cggcggcggc agtgggcacc accagcacta ctattatgat acccacacca 780
acacctacta cctgcgcacc ttcggccaca acaccatgga cgctgtgccc aggatcgatc 840
actaccggca cacagccgcg cagctgggcg agaagctgct ccggcctagc ctggcggagc 900
tccacgacga gctggaaaag gaaccttttg aggatggctt tgcaaatggg gaagaaagta 960
ctccaaccag agatgctgtg gtcacgtata ctgcagaaag taaaggagtc gtgaagtttg 1020
gctggatcaa gggtgtatta gtacgttgta tgttaaacat ttggggtgtg atgcttttca 1080
ttagattgtc atggattgtg ggtcaagctg gaataggtct atcagtcctt gtaataatga 1140
tggccactgt tgtgacaact atcacaggat tgtctacttc agcaatagca actaatggat 1200
ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgatttcga gaggaagaga 1620
ctttcttttc tgtatttgcc atcttttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100

ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880
tatttcatga tgcttttgac atacaatatg gagtagtggt tattcgccta aaagaaggtc 2940
tggatatatc tcatcttcaa ggacaagaag aattattgtc atcacaagag aaatctcctg 3000
gcaccaagga tgtggtagta agtgtggaat atagtaaaaa gtccgattta gatacttcca 3060
aaccactcag tgaaaaacca attacacaca aagttgagga agaggatggc aagactgcaa 3120
ctcaaccact gttgaaaaaa gaatccaaag gccctattgt gcctttaaat gtagctgacc 3180
aaaagcttct tgaagctagt acacagtttc agaaaaaaca aggaaagaat actattgatg 3240
tctggtggct tttgatgat ggaggtttga ccttattgat accttacctt ctgacgacca 3300
agaaaaaatg gaaagactgt aagatcagag tattcattgg tggaaagata aacagaatag 3360
accatgaccg gagagcgatg gctactttgc ttagcaagtt ccggatagac ttttctgata 3420
tcatggttct aggagatatc aataccaaac caaagaaaga aaatattata gcttttgagg 3480
aaatcattga gccatacaga cttcatgaag atgataaaga gcaagatatt gcagataaaa 3540
tgaaagaaga tgaaccatgg cgaataacag ataatgagct tgaactttat aagaccaaga 3600
cataccggca gatcaggtta aatgagttat taaaggaaca ttcaagcaca gctaatatta 3660
ttgtcatgag tctcccagtt gcacgaaaag gtgctgtgtc tagtgctctc tacatggcat 3720
ggttagaagc tctatctaag gacctaccac caatcctcct agttcgtggg aatcatcaga 3780
gtgtccttac cttctattca taaatgttct atacagtgga cagccctcca gaatggtact 3840

tcagtgccta gtgtagtaac ctgaaatctt caatgacaca ttaacatcac aatggcgaat 3900

ggtgactttt ctttcacgat ttcattaatt tgaaagcaca caggaaagct tgctccattg 3960

ataacgtgta tggagacttc ggttttagtc aattccatat ctcaatctta atggtgattc 4020

ttctctgttg aactgaagtt tgtgagagta gttttccttt gctacttgaa tagcaataaa 4080

agcgtgttaa ctttttgg 4098

<210> 45
<211> 4098
<212> DNA
<213> human

<400> 45
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agctatggag ccgcggccca 180

cggcgccctc ctccggcgcc ccgggactgg ccggggtcgg ggagacgccg tcagccgctg 240

cgctggccgc agccagggtg gaactgcccg gcacggctgt gccctcggtg ccggaggatg 300

ctgcgcccgc gagccgggac ggcggcgggg tccgcgatga gggccccgcg gcggccgggg 360

acgggctggg cagacccttg ggcccaccc cgagccagag ccgtttccag gtggacctgg 420

tttccgagaa cgccgggcgg gccgctgctg cggcggcggc ggcggcggcg gcagcggcgg 480

cggctggtgc tggggcgggg gccaagcaga cccccgcgga cggggaagcc agcggcgaga 540

gcgagccagc taaaggcagc gaggaagcca agggccgctt ccgcgtgaac ttcgtggacc 600

cagctgcctc ctcgtcggct gaagacagcc tgtcagatgc tgccggggtc ggagtcgacg 660

ggcccaacgt gagcttccag aacggcgggg acacggtgct gagcgagggc agcagcctgc 720

actccggcgg cggcggcggc agtgggcacc accagcacta ctattatgat acccacacca 780

acacctacta cctgcgcacc ttcggccaca acaccatgga cgctgtgccc aggatcgatc 840

actaccggca cacagccgcg cagctgggcg agaagctgct ccggcctagc ctggcggagc 900

tccacgacga gctggaaaag gaaccttttg aggatggctt tgcaaatggg gaagaaagta 960

ctccaaccag agatgctgtg gtcacgtata ctgcagaaag taaaggagtc gtgaagtttg 1020

gctggatcaa gggtgtatta gtacgttgta tgttaaacat ttggggtgtg atgcttttca 1080

ttagattgtc atggattgtg ggtcaagctg gaataggtct atcagtcctt gtaataatga 1140

tggccactgt tgtgacaact atcacaggat tgtctacttc agcaatagca actaatggat 1200

ttgtaagagg aggaggagca tattatttaa tatctagaag tctagggcca gaatttggtg 1260
gtgcaattgg tctaatcttc gcctttgcca acgctgttgc agttgctatg tatgtggttg 1320
gatttgcaga aaccgtggtg gagttgctta aggaacattc catacttatg atagatgaaa 1380
tcaatgatat ccgaattatt ggagccatta cagtcgtgat tcttttaggt atctcagtag 1440
ctggaatgga gtgggaagca aaagctcaga ttgttctttt ggtgatccta cttcttgcta 1500
ttggtgattt cgtcatagga acatttatcc cactggagag caagaagcca aaagggtttt 1560
ttggttataa atctgaaata tttaatgaga actttgggcc cgattttcga gaggaagaga 1620
ctttcttttc tgtatttgcc atctttttc ctgctgcaac tggtattctg gctggagcaa 1680
atatctcagg tgatcttgca gatcctcagt cagccatacc caaaggaaca ctcctagcca 1740
ttttaattac tacattggtt tacgtaggaa ttgcagtatc tgtaggttct tgtgttgttc 1800
gagatgccac tggaaacgtt aatgacacta tcgtaacaga gctaacaaac tgtacttctg 1860
cagcctgcaa attaaacttt gatttttcat cttgtgaaag cagtccttgt tcctatggcc 1920
taatgaacaa cttccaggta atgagtatgg tgtcaggatt tacaccacta atttctgcag 1980
gtatattttc agccactctt tcttcagcat tagcatccct agtgagtgct cccaaaatat 2040
ttcaggctct atgtaaggac aacatctacc cagctttcca gatgtttgct aaaggttatg 2100
ggaaaaataa tgaacctctt cgtggctaca tcttaacatt cttaattgca cttggattca 2160
tcttaattgc tgaactgaat gttattgcac caattatctc aaacttcttc cttgcatcat 2220
atgcattgat caatttttca gtattccatg catcacttgc aaaatctcca ggatggcgtc 2280
ctgcattcaa atactacaac atgtggatat cacttcttgg agcaattctt tgttgcatag 2340
taatgttcgt cattaactgg tgggctgcat tgctaacata tgtgatagtc cttgggctgt 2400
atatttatgt tacctacaaa aaaccagatg tgaattgggg atcctctaca caagccctga 2460
cttacctgaa tgcactgcag cattcaattc gtctttctgg agtggaagac cacgtgaaaa 2520
actttaggcc acagtgtctt gttatgacag gtgctccaaa ctcacgtcca gctttacttc 2580
atcttgttca tgatttcaca aaaaatgttg gtttgatgat ctgtggccat gtacatatgg 2640
gtcctcgaag acaagccatg aaagagatgt ccatcgatca agccaaatat cagcgatggc 2700
ttattaagaa caaaatgaag gcattttatg ctccagtaca tgcagatgac ttgagagaag 2760
gtgcacagta tttgatgcag gctgctggtc ttggtcgtat gaagccaaac acacttgtcc 2820
ttggatttaa gaaagattgg ttgcaagcag atatgaggga tgtggatatg tatataaact 2880

tatttcatga tgcttttgac atacaatatg gagtagtggt tattcgccta aaagaaggtc 2940

tggatatatc tcatcttcaa ggacaagaag aattattgtc atcacaagag aaatctcctg 3000

gcaccaagga tgtggtagta agtgtggaat atagtaaaaa gtccgattta gatacttcca 3060

aaccactcag tgaaaaacca attacacaca aagttgagga agaggatggc aagactgcaa 3120

ctcaaccact gttgaaaaaa gaatccaaag gccctattgt gcctttaaat gtagctgacc 3180

aaaagcttct tgaagctagt acacagtttc agaaaaaaca aggaaagaat actattgatg 3240

tctggtggct ttttgatgat ggaggtttga ccttattgat accttacctt ctgacgacca 3300

agaaaaaatg gaaagactgt aagatcagag tattcattgg tggaaagata aacagaatag 3360

accatgaccg gagagcgatg gctactttgc ttagcaagtt ccggatagac ttttctgata 3420

tcatggttct aggagatatc aataccaaac caaagaaaga aaatattata gcttttgagg 3480

aaatcattga gccatacaga cttcatgaag atgataaaga gcaagatatt gcagataaaa 3540

tgaaagaaga tgaaccatgg cgaataacag ataatgagct tgaactttat aagaccaaga 3600

cataccggca gatcaggtta aatgagttat taaaggaaca ttcaagcaca gctaatatta 3660

ttgtcatgag tctcccagtt gcacgaaaag gtgctgtgtc tagtgctctc tacatggcat 3720

ggttagaagc tctatctaag gacctaccac caatcctcct agttcgtggg aatcatcaga 3780

gtgtccttac cttctattca taaatgttct atacagtgga cagccctcca gaatggtact 3840

tcagtgccta gtgtagtaac ctgaaatctt caatgacaca ttaacatcac aatggcgaat 3900

ggtgactttt ctttcacgat ttcattaatt tgaaagcaca caggaaagct tgctccattg 3960

ataacgtgta tggagacttc ggttttagtc aattccatat ctcaatctta atggtgattc 4020

ttctctgttg aactgaagtt tgtgagagta gttttccttt gctacttgaa tagcaataaa 4080

agcgtgttaa ctttttgg 4098

<210> 46
<211> 3311
<212> DNA
<213> human

<400> 46
tgctaatgct tttggtacaa atggatgtgg aatataattg aatattttct tgtttaaggg 60

gagcatgaag aggtgttgag gttatgtcaa gcatctggca cagctgaagg cagatggaaa 120

tatttacaag tacgcaattt gagactaaga tattgttatc attctcctat tgaagacaag 180

agcaatagta aaacacatca ggtcaggggg ttaaagacct gtgataaacc acttccgata 240

agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800
cagcgctcca tccagcttga gagtaaggga ttaaccctcc agaacagcca gtggatgaat 1860
ggcacagtga tcgtggacag caccgtggga aaggacactt tgtttcttat cacctggaca 1920
acgcagcctc cccaaatcct tctctgggat cccagtggac agaagcaagg tggctttgta 1980

gtggacaaaa acaccaaaat ggcctacctc caaatcccag gcattgctaa ggttggcact 2040

tggaaataca gtctgcaagc aagctcacaa accttgaccc tgactgtcac gtcccgtgcg 2100

tccaatgcta ccctgcctcc aattacagtg acttccaaaa cgaacaagga caccagcaaa 2160

ttccccagcc ctctggtagt ttatgcaaat attcgccaag gagcctcccc aattctcagg 2220

gccagtgtca cagccctgat tgaatcagtg aatggaaaaa cagttacctt ggaactactg 2280

gataatggag caggtgctga tgctactaag gatgacggtg tctactcaag gtatttcaca 2340

acttatgaca cgaatggtag atacagtgta aaagtgcggg ctctgggagg agttaacgca 2400

gccagacgga gagtgatacc ccagcagagt ggagcactgt acatacctgg ctggattgag 2460

aatgatgaaa tacaatggaa tccaccaaga cctgaaatta ataaggatga tgttcaacac 2520

aagcaagtgt gtttcagcag aacatcctcg ggaggctcat ttgtggcttc tgatgtccca 2580

aatgctccca tacctgatct cttcccacct ggccaaatca ccgacctgaa ggcggaaatt 2640

cacgggggca gtctcattaa tctgacttgg acagctcctg gggatgatta tgaccatgga 2700

acagctcaca agtatatcat tcgaataagt acaagtattc ttgatctcag agacaagttc 2760

aatgaatctc ttcaagtgaa tactactgct ctcatcccaa aggaagccaa ctctgaggaa 2820

gtctttttgt ttaaaccaga aaacattact tttgaaaatg gcacagatct tttcattgct 2880

attcaggctg ttgataaggt cgatctgaaa tcagaaatat ccaacattgc acgagtatct 2940

ttgtttattc ctccacagac tccgccagag acacctagtc ctgatgaaac gtctgctcct 3000

tgtcctaata ttcatatcaa cagcaccatt cctggcattc acattttaaa aattatgtgg 3060

aagtggatag gagaactgca gctgtcaata gcctagggct gaatttttgt cagataaata 3120

aaataaatca ttcatccttt ttttgattat aaaattttct aaaatgtatt ttagacttcc 3180

tgtagggggc gatatactaa atgtatatag tacatttata ctaaatgtat tcctgtaggg 3240

ggcgatatac taaatgtatt ttagacttcc tgtagggggc gataaaataa aatgctaaac 3300

aactgggtaa a 3311

<210> 47
<211> 3311
<212> DNA
<213> human

<400> 47
tgctaatgct tttggtacaa atggatgtgg aatataattg aatattttct tgtttaaggg 60

gagcatgaag aggtgttgag gttatgtcaa gcatctggca cagctgaagg cagatggaaa 120

tatttacaag tacgcaattt gagactaaga tattgttatc attctcctat tgaagacaag 180
agcaatagta aaacacatca ggtcaggggg ttaaagacct gtgataaacc acttccgata 240
agttggaaac gtgtgtctat attttcatat ctgtatatat ataatggtaa agaaagacac 300
cttcgtaacc cgcattttcc aaagagagga atcacaggga gatgtacagc aatggggcca 360
tttaagagtt ctgtgttcat cttgattctt caccttctag aaggggccct gagtaattca 420
ctcattcagc tgaacaacaa tggctatgaa ggcattgtcg ttgcaatcga ccccaatgtg 480
ccagaagatg aaacactcat tcaacaaata aaggacatgg tgacccaggc atctctgtat 540
ctgtttgaag ctacaggaaa gcgattttat ttcaaaaatg ttgccatttt gattcctgaa 600
acatggaaga caaaggctga ctatgtgaga ccaaaacttg agacctacaa aaatgctgat 660
gttctggttg ctgagtctac tcctccaggt aatgatgaac cctacactga gcagatgggc 720
aactgtggag agaagggtga aaggatccac ctcactcctg atttcattgc aggaaaaaag 780
ttagctgaat atggaccaca aggtaaggca tttgtccatg agtgggctca tctacgatgg 840
ggagtatttg acgagtacaa taatgatgag aaattctact tatccaatgg aagaatacaa 900
gcagtaagat gttcagcagg tattactggt acaaatgtag taaagaagtg tcagggaggc 960
agctgttaca ccaaaagatg cacattcaat aaagttacag gactctatga aaaaggatgt 1020
gagtttgttc tccaatcccg ccagacggag aaggcttcta taatgtttgc acaacatgtt 1080
gattctatag ttgaattctg tacagaacaa aaccacaaca aagaagctcc aaacaagcaa 1140
aatcaaaaat gcaatctccg aagcacatgg gaagtgatcc gtgattctga ggactttaag 1200
aaaaccactc ctatgacaac acagccacca aatcccacct tctcattgct gcagattgga 1260
caaagaattg tgtgtttagt ccttgacaaa tctggaagca tggcgactgg taaccgcctc 1320
aatcgactga atcaagcagg ccagcttttc ctgctgcaga cagttgagct ggggtcctgg 1380
gttgggatgg tgacatttga cagtgctgcc catgtacaaa gtgaactcat acagataaac 1440
agtggcagtg acagggacac actcgccaaa agattacctg cagcagcttc aggagggacg 1500
tccatctgca gcgggcttcg atcggcattt actgtgatta ggaagaaata tccaactgat 1560
ggatctgaaa ttgtgctgct gacggatggg gaagacaaca ctataagtgg gtgctttaac 1620
gaggtcaaac aaagtggtgc catcatccac acagtcgctt tggggccctc tgcagctcaa 1680
gaactagagg agctgtccaa aatgacagga ggtttacaga catatgcttc agatcaagtt 1740
cagaacaatg gcctcattga tgcttttggg gccctttcat caggaaatgg agctgtctct 1800

cagcgctcca tccagcttga gagtaaggga ttaaccctcc agaacagcca gtggatgaat 1860

ggcacagtga tcgtggacag caccgtggga aaggacactt tgtttcttat cacctggaca 1920

acgcagcctc cccaaatcct tctctgggat cccagtggac agaagcaagg tggctttgta 1980

gtggacaaaa acaccaaaat ggcctacctc caaatcccag gcattgctaa ggttggcact 2040

tggaaataca gtctgcaagc aagctcacaa accttgaccc tgactgtcac gtcccgtgcg 2100

tccaatgcta ccctgcctcc aattacagtg acttccaaaa cgaacaagga caccagcaaa 2160

ttccccagcc ctctggtagt ttatgcaaat attcgccaag gagcctcccc aattctcagg 2220

gccagtgtca cagccctgat tgaatcagtg aatggaaaaa cagttacctt ggaactactg 2280

gataatggag caggtgctga tgctactaag gatgacggtg tctactcaag gtatttcaca 2340

acttatgaca cgaatggtag atacagtgta aaagtgcggg ctctgggagg agttaacgca 2400

gccagacgga gagtgatacc ccagcagagt ggagcactgt acatacctgg ctggattgag 2460

aatgatgaaa tacaatggaa tccaccaaga cctgaaatta ataaggatga tgttcaacac 2520

aagcaagtgt gtttcagcag aacatcctcg ggaggctcat ttgtggcttc tgatgtccca 2580

aatgctccca tacctgatct cttcccacct ggccaaatca ccgacctgaa ggcggaaatt 2640

cacgggggca gtctcattaa tctgacttgg acagctcctg gggatgatta tgaccatgga 2700

acagctcaca agtatatcat tcgaataagt acaagtattc ttgatctcag agacaagttc 2760

aatgaatctc ttcaagtgaa tactactgct ctcatcccaa aggaagccaa ctctgaggaa 2820

gtctttttgt ttaaaccaga aaacattact tttgaaaatg gcacagatct tttcattgct 2880

attcaggctg ttgataaggt cgatctgaaa tcagaaatat ccaacattgc acgagtatct 2940

ttgtttattc ctccacagac tccgccagag acacctagtc ctgatgaaac gtctgctcct 3000

tgtcctaata ttcatatcaa cagcaccatt cctggcattc acattttaaa aattatgtgg 3060

aagtggatag gagaactgca gctgtcaata gcctagggct gaatttttgt cagataaata 3120

aaataaatca ttcatccttt ttttgattat aaaattttct aaaatgtatt ttagacttcc 3180

tgtagggggc gatatactaa atgtatatag tacatttata ctaaatgtat tcctgtaggg 3240

ggcgatatac taaatgtatt ttagacttcc tgtagggggc gataaaataa aatgctaaac 3300

aactgggtaa a 3311

<210> 48
<211> 3697
<212> DNA
<213> human

<400> 48

```
agggagtgtt cccgggggag atactccagt cgtagcaaga gtctcgacca ctgaatggaa     60
gaaaaggact tttaaccacc attttgtgac ttacagaaag gaatttgaat aaagaaaact    120
atgatacttc aggcccatct tcactccctg tgtcttctta tgctttattt ggcaactgga    180
tatggccaag aggggaagtt tagtggaccc ctgaaaccca tgacattttc tatttatgaa    240
ggccaagaac cgagtcaaat tatattccag tttaaggcca atcctcctgc tgtgactttt    300
gaactaactg gggagacaga caacatattt gtgatagaac gggagggact tctgtattac    360
aacagagcct tggacaggga aacaagatct actcacaatc tccaggttgc agccctggac    420
gctaatggaa ttatagtgga gggtccagtc cctatcacca tagaagtgaa ggacatcaac    480
gacaatcgac ccacgtttct ccagtcaaag tacgaaggct cagtaaggca gaactctcgc    540
ccaggaaagc ccttcttgta tgtcaatgcc acagacctgg atgatccggc cactcccaat    600
ggccagcttt attaccagat tgtcatccag cttcccatga tcaacaatgt catgtacttt    660
cagatcaaca acaaaacggg agccatctct cttacccgag agggatctca ggaattgaat    720
cctgctaaga atccttccta taatctggtg atctcagtga aggacatggg aggccagagt    780
gagaattcct tcagtgatac cacatctgtg gatatcatag tgacagagaa tatttggaaa    840
gcaccaaaac ctgtggagat ggtggaaaac tcaactgatc ctcaccccat caaaatcact    900
caggtgcggt ggaatgatcc cggtgcacaa tattccttag ttgacaaaga gaagctgcca    960
agattcccat tttcaattga ccaggaagga gatatttacg tgactcagcc cttggaccga   1020
gaagaaaagg atgcatatgt tttttatgca gttgcaaagg atgagtacgg aaaaccactt   1080
tcatatccgc tggaaattca tgtaaaagtt aaagatatta atgataatcc acctacatgt   1140
ccgtcaccag taaccgtatt tgaggtccag gagaatgaac gactgggtaa cagtatcggg   1200
acccttactg cacatgacag ggatgaagaa aatactgcca acagttttct aaactacagg   1260
attgtggagc aaactcccaa acttcccatg gatggactct tcctaatcca aacctatgct   1320
ggaatgttac agttagctaa acagtccttg aagaagcaag atactcctca gtacaactta   1380
acgatagagg tgtctgacaa agatttcaag acccctttgtt ttgtgcaaat caacgttatt   1440
gatatcaatg atcagatccc catctttgaa aaatcagatt atggaaacct gactcttgct   1500
gaagacacaa acattgggtc caccatctta accatccagg ccactgatgc tgatgagcca   1560
tttactggga gttctaaaat tctgtatcat atcataaagg gagacagtga gggacgcctg   1620
ggggttgaca cagatcccca taccaacacc ggatatgtca taattaaaaa gcctcttgat   1680
```

tttgaaacag cagctgtttc caacattgtg ttcaaagcag aaaatcctga gcctctagtg 1740
tttggtgtga agtacaatgc aagttctttt gccaagttca cgcttattgt gacagatgtg 1800
aatgaagcac ctcaattttc ccaacacgta ttccaagcga aagtcagtga ggatgtagct 1860
ataggcacta aagtgggcaa tgtgactgcc aaggatccag aaggtctgga cataagctat 1920
tcactgaggg gagacacaag aggttggctt aaaattgacc acgtgactgg tgagatcttt 1980
agtgtggctc cattggacag agaagccgga agtccatatc gggtacaagt ggtggccaca 2040
gaagtagggg ggtcttcctt gagctctgtg tcagagttcc acctgatcct tatggatgtg 2100
aatgacaacc ctcccaggct agccaaggac tacacgggct tgttcttctg ccatcccctc 2160
agtgcacctg gaagtctcat tttcgaggct actgatgatg atcagcactt atttcggggt 2220
ccccatttta cattttccct cggcagtgga agcttacaaa acgactggga agtttccaaa 2280
atcaatggta ctcatgcccg actgtctacc aggcacacag agtttgagga gagggagtat 2340
gtcgtcttga tccgcatcaa tgatggggt cggccaccct tggaaggcat tgtttcttta 2400
ccagttacat tctgcagttg tgtggaagga agttgtttcc ggccagcagg tcaccagact 2460
gggataccca ctgtgggcat ggcagttggt atactgctga ccacccttct ggtgattggt 2520
ataattttag cagttgtgtt tatccgcata aagaaggata aaggcaaaga taatgttgaa 2580
agtgctcaag catctgaagt caaacctctg agaagctgaa tttgaaaagg aatgtttgaa 2640
tttatatagc aagtgctatt tcagcaacaa ccatctcatc ctattacttt tcatctaacg 2700
tgcattataa tttttaaac agatattccc tcttgtcctt taatatttgc taaatatttc 2760
tttttgagg tggagtcttg ctctgtcgcc caggctggag tacagtggtg tgatcccagc 2820
tcactgcaac ctccgcctcc tgggttcaca tgattctcct gcctcagctt cctaagtagc 2880
tgggtttaca ggcacccacc accatgccca gctaattttt gtatttttaa tagagacggg 2940
gtttcgccat ttggccaggc tggtcttgaa ctcctgacgt caagtgatct gcctgccttg 3000
gtctcccaat acaggcatga accactgcac ccacctactt agatatttca tgtgctatag 3060
acattagaga gatttttcat ttttccatga catttttcct ctctgcaaat ggcttagcta 3120
cttgtgtttt tcccttttgg ggcaagacag actcattaaa tattctgtac attttttctt 3180
tatcaaggag atatatcagt gttgtctcat agaactgcct ggattccatt tatgtttttt 3240
ctgattccat cctgtgtccc cttcatcctt gactcctttg gtatttcact gaatttcaaa 3300
catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 49
<211> 3697
<212> DNA
<213> human

<400> 49
agggagtgtt cccgggggag atactccagt cgtagcaaga gtctcgacca ctgaatggaa 60

gaaaaggact tttaaccacc attttgtgac ttacagaaag gaatttgaat aaagaaaact 120

atgatacttc aggcccatct tcactccctg tgtcttctta tgctttattt ggcaactgga 180

tatggccaag aggggaagtt tagtggaccc ctgaaaccca tgacattttc tatttatgaa 240

ggccaagaac cgagtcaaat tatattccag tttaaggcca atcctcctgc tgtgactttt 300

gaactaactg gggagacaga caacatattt gtgatagaac gggagggact tctgtattac 360

aacagagcct tggacaggga aacaagatct actcacaatc tccaggttgc agccctggac 420

gctaatggaa ttatagtgga gggtccagtc cctatcacca tagaagtgaa ggacatcaac 480

gacaatcgac ccacgtttct ccagtcaaag tacgaaggct cagtaaggca gaactctcgc 540

ccaggaaagc ccttcttgta tgtcaatgcc acagacctgg atgatccggc cactcccaat 600

ggccagcttt attaccagat tgtcatccag cttcccatga tcaacaatgt catgtacttt 660

cagatcaaca acaaaacggg agccatctct cttacccgag agggatctca ggaattgaat 720

cctgctaaga atccttccta taatctggtg atctcagtga aggacatggg aggccagagt 780

gagaattcct tcagtgatac cacatctgtg gatatcatag tgacagagaa tatttggaaa 840

gcaccaaaac ctgtggagat ggtggaaaac tcaactgatc ctcaccccat caaaatcact 900

caggtgcggt ggaatgatcc cggtgcacaa tattccttag ttgacaaaga gaagctgcca 960

agattcccat tttcaattga ccaggaagga gatatttacg tgactcagcc cttggaccga 1020

gaagaaaagg atgcatatgt tttttatgca gttgcaaagg atgagtacgg aaaaccactt 1080

tcatatccgc tggaaattca tgtaaaagtt aaagatatta atgataatcc acctacatgt 1140

ccgtcaccag taaccgtatt tgaggtccag gagaatgaac gactgggtaa cagtatcggg 1200

acccttactg cacatgacag ggatgaagaa aatactgcca acagttttct aaactacagg 1260

attgtggagc aaactcccaa acttcccatg gatggactct tcctaatcca aacctatgct 1320

ggaatgttac agttagctaa acagtccttg aagaagcaag atactcctca gtacaactta 1380

acgatagagg tgtctgacaa agatttcaag acccttttgtt ttgtgcaaat caacgttatt 1440

gatatcaatg atcagatccc catctttgaa aaatcagatt atggaaacct gactcttgct 1500

gaagacacaa acattgggtc caccatctta accatccagg ccactgatgc tgatgagcca 1560

tttactggga gttctaaaat tctgtatcat atcataaagg gagacagtga gggacgcctg 1620

ggggttgaca cagatcccca taccaacacc ggatatgtca taattaaaaa gcctcttgat 1680

tttgaaacag cagctgtttc caacattgtg ttcaaagcag aaaatcctga gcctctagtg 1740

tttggtgtga agtacaatgc aagttctttt gccaagttca cgcttattgt gacagatgtg 1800

aatgaagcac ctcaattttc ccaacacgta ttccaagcga aagtcagtga ggatgtagct 1860

ataggcacta aagtgggcaa tgtgactgcc aaggatccag aaggtctgga cataagctat 1920

tcactgaggg gagacacaag aggttggctt aaaattgacc acgtgactgg tgagatcttt 1980

agtgtggctc cattggacag agaagccgga agtccatatc gggtacaagt ggtggccaca 2040

gaagtagggg ggtcttcctt gagctctgtg tcagagttcc acctgatcct tatggatgtg 2100

aatgacaacc ctcccaggct agccaaggac tacacgggct tgttcttctg ccatcccctc 2160

agtgcacctg gaagtctcat tttcgaggct actgatgatg atcagcactt atttcggggt 2220

ccccatttta cattttccct cggcagtgga agcttacaaa acgactggga agtttccaaa 2280

atcaatggta ctcatgcccg actgtctacc aggcacacag agtttgagga gagggagtat 2340

gtcgtcttga tccgcatcaa tgatgggggt cggccaccct tggaaggcat tgtttcttta 2400

ccagttacat tctgcagttg tgtggaagga agttgtttcc ggccagcagg tcaccagact 2460

gggataccca ctgtgggcat ggcagttggt atactgctga ccacccttct ggtgattggt 2520

ataattttag cagttgtgtt tatccgcata aagaaggata aaggcaaaga taatgttgaa 2580

agtgctcaag catctgaagt caaacctctg agaagctgaa tttgaaaagg aatgtttgaa 2640

tttatatagc aagtgctatt tcagcaacaa ccatctcatc ctattacttt tcatctaacg 2700

tgcattataa ttttttaaac agatattccc tcttgtcctt taatatttgc taaatatttc 2760

ttttttgagg tggagtcttg ctctgtcgcc caggctggag tacagtggtg tgatcccagc 2820

tcactgcaac ctccgcctcc tgggttcaca tgattctcct gcctcagctt cctaagtagc 2880

tgggtttaca ggcacccacc accatgccca gctaattttt gtatttttaa tagagacggg 2940

gtttcgccat ttggccaggc tggtcttgaa ctcctgacgt caagtgatct gcctgccttg 3000

gtctcccaat acaggcatga accactgcac ccacctactt agatatttca tgtgctatag 3060

acattagaga gatttttcat ttttccatga catttttcct ctctgcaaat ggcttagcta 3120

cttgtgtttt tccctttggg ggcaagacag actcattaaa tattctgtac atttttcttt 3180

tatcaaggag atatatcagt gttgtctcat agaactgcct ggattccatt tatgtttttt 3240

ctgattccat cctgtgtccc cttcatcctt gactcctttg gtatttcact gaatttcaaa 3300

catttgtcag agaagaaaaa cgtgaggact caggaaaaat aaataaataa aagaacagcc 3360

ttttccctta gtattaacag aaatgtttct gtgtcattaa ccatctttaa tcaatgtgac 3420

atgttgctct ttggctgaaa ttcttcaact tggaaatgac acagacccac agaaggtgtt 3480

caaacacaac ctactctgca aaccttggta aaggaaccag tcagctggcc agatttcctc 3540

actacctgcc atgcatacat gctgcgcatg ttttcttcat tcgtatgtta gtaaagtttt 3600

ggttattata tatttaacat gtggaagaaa acaagacatg aaaagagtgg tgacaaatca 3660

agaataaaca ctggttgtag tcagttttgt ttgttaa 3697

<210> 50
<211> 3803
<212> DNA
<213> human

<400> 50
ccatggtagg agcgctcgcc tcgctgcggt gcccgctgag gccatgccgg ggccccggcg 60

ccccgctggc tcccgcctgc gcctgctcct gctcctgctg ctgccgccgc tgctgctgct 120

gctccggggc agccacgcgg gcaacctgac ggtagccgtg gtactgccgc tggccaatac 180

ctcgtacccc tggtcgtggg cgcgcgtggg acccgccgtg gagctggccc tggcccaggt 240

gaaggcgcgc cccgacttgc tgccgggctg gacggtccgc acggtgctgg gcagcagcga 300

aaacgcgctg ggcgtctgct ccgacaccgc agcgcccctg gccgcggtgg acctcaagtg 360

ggagcacaac cccgctgtgt tcctgggccc cggctgcgtg tacgccgccg ccccagtggg 420

gcgcttcacc gcgcactggc gggtcccgct gctgaccgcc ggcgccccgg cgctgggctt 480

cggtgtcaag gacgagtatg cgctgaccac ccgcgcgggg cccagctacg ccaagctggg 540

ggacttcgtg gcggcgctgc accgacggct gggctgggag cgccaagcgc tcatgctcta 600

cgcctaccgg ccgggtgacg aagagcactg cttcttcctc gtggaggggc tgttcatgcg 660

ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380
gtaccctcct cctgacatcc ccaaatgtgg ctttgacaac gaagacccag catgcaacca 1440
agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740
gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg gggctatctg ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160
cctgcgaatg gcttcacccc ctgtgcgggg ctcccaggct ggtgacgtat acagctttgg 2220
gatcatcctt caggagattg ccctgaggag tggggtcttc cacgtggaag gtttggacct 2280
gagccccaaa gagatcatcg agcgggtgac tcggggtgag cagcccccct tccggccctc 2340

cctggccctg cagagtcacc tggaggagtt ggggctgctc atgcagcggt gctgggctga 2400

ggacccacag gagaggccac cattccagca gatccgcctg acgttgcgca aatttaacag 2460

ggagaacagc agcaacatcc tggacaacct gctgtcccgc atggagcagt acgcgaacaa 2520

tctggaggaa ctggtggagg agcggaccca ggcatacctg gaggagaagc gcaaggctga 2580

ggccctgctc taccagatcc tgcctcactc agtggctgag cagctgaagc gtggggagac 2640

ggtgcaggcc gaagcctttg acagtgttac catctacttc agtgacattg tgggtttcac 2700

agcgctgtcg gcggagagca cacccatgca ggtggtgacc ctgctcaatg acctgtacac 2760

ttgctttgat gctgtcatag acaactttga tgtgtacaag gtggagacaa ttggcgatgc 2820

ctacatggtg gtgtcagggc tccctgtgcg gaacgggcgg ctacacgcct gcgaggtagc 2880

ccgcatggcc ctggcactgc tggatgctgt gcgctccttc cgaatccgcc accggcccca 2940

ggagcagctg cgcttgcgca ttggcatcca cacaggacct gtgtgtgctg gagtggtggg 3000

actgaagatg ccccgttact gtctctttgg ggatacagtc aacacagcct caagaatgga 3060

gtctaatggg gaagccctga agatccactt gtcttctgag accaaggctg tcctggagga 3120

gtttggtggt ttcgagctgg agcttcgagg ggatgtagaa atgaagggca aaggcaaggt 3180

tcggacctac tggctccttg ggagaggggg gagtagcacc cgaggctgac ctgcctcctc 3240

tcctatccct ccacacctcc cctaccctgt gccagaagca acagaggtgc caggcctcag 3300

cctcacccac agcagcccca tcgccaaagg atggaagtaa tttgaatagc tcaggtgtgc 3360

tgaccccagt gaagacacca gataggacct ctgagagggg actggcatgg ggggatctca 3420

gagcttacag gctgagccaa gcccacggcc atgcacaggg acactcacac aggcacacgc 3480

acctgctctc cacctggact caggccgggc tgggctgtgg atccttgatc ccctcccctc 3540

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gacccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 51
<211> 3803
<212> DNA
<213> human

<400> 51

ccatggtagg agcgctcgcc tcgctgcggt gcccgctgag gccatgccgg ggccccggcg 60
ccccgctggc tcccgcctgc gcctgctcct gctcctgctg ctgccgccgc tgctgctgct 120
gctccggggc agccacgcgg gcaacctgac ggtagccgtg gtactgccgc tggccaatac 180
ctcgtacccc tggtcgtggg cgcgcgtggg acccgccgtg gagctggccc tggcccaggt 240
gaaggcgcgc cccgacttgc tgccgggctg gacggtccgc acggtgctgg gcagcagcga 300
aaacgcgctg ggcgtctgct ccgacaccgc agcgcccctg gccgcggtgg acctcaagtg 360
ggagcacaac cccgctgtgt tcctgggccc cggctgcgtg tacgccgccg ccccagtggg 420
gcgcttcacc gcgcactggc gggtcccgct gctgaccgcc ggcgccccgg cgctgggctt 480
cggtgtcaag gacgagtatg cgctgaccac ccgcgcgggg cccagctacg ccaagctggg 540
ggacttcgtg gcggcgctgc accgacggct gggctgggag cgccaagcgc tcatgctcta 600
cgcctaccgg ccgggtgacg aagagcactg cttcttcctc gtggaggggc tgttcatgcg 660
ggtccgcgac cgcctcaata ttacggtgga ccacctggag ttcgccgagg acgacctcag 720
ccactacacc aggctgctgc ggaccatgcc gcgcaaaggc cgagttatct acatctgcag 780
ctcccctgat gccttcagaa ccctcatgct cctggccctg gaagctggct tgtgtgggga 840
ggactacgtt ttcttccacc tggatatctt tgggcaaagc ctgcaaggtg gacagggccc 900
tgctccccgc aggccctggg agagagggga tgggcaggat gtcagtgccc gccaggcctt 960
tcaggctgcc aaaatcatta catataaaga cccagataat cccgagtact tggaattcct 1020
gaagcagtta aaacacctgg cctatgagca gttcaacttc accatggagg atggcctggt 1080
gaacaccatc ccagcatcct tccacgacgg gctcctgctc tatatccagg cagtgacgga 1140
gactctggca catgggggaa ctgttactga tggggagaac atcactcagc ggatgtggaa 1200
ccgaagcttt caaggtgtga caggatacct gaaaattgat agcagtggcg atcgggaaac 1260
agacttctcc ctctgggata tggatcccga gaatggtgcc ttcagggttg tactgaacta 1320
caatgggact tcccaagagc tggtggctgt gtcggggcgc aaactgaact ggcccctggg 1380
gtaccctcct cctgacatcc ccaaatgtgg ctttgacaac gaagacccag catgcaacca 1440
agatcacctt tccaccctgg aggtgctggc tttggtgggc agcctctcct tgctcggcat 1500
tctgattgtc tccttcttca tatacaggaa gatgcagctg gagaaggaac tggcctcgga 1560
gctgtggcgg gtgcgctggg aggacgttga gcccagtagc cttgagaggc acctgcggag 1620
tgcaggcagc cggctgaccc tgagcgggag aggctccaat tacggctccc tgctaaccac 1680
agagggccag ttccaagtct ttgccaagac agcatattat aagggcaacc tcgtggctgt 1740

gaaacgtgtg aaccgtaaac gcattgagct gacacgaaaa gtcctgtttg aactgaagca 1800
tatgcgggat gtgcagaatg aacacctgac caggtttgtg ggagcctgca ccgacccccc 1860
caatatctgc atcctcacag agtactgtcc ccgtgggagc ctgcaggaca ttctggagaa 1920
tgagagcatc accctggact ggatgttccg gtactcactc accaatgaca tcgtcaaggg 1980
catgctgttt ctacacaatg ggctatctg ttcccatggg aacctcaagt catccaactg 2040
cgtggtagat gggcgctttg tgctcaagat caccgactat gggctggaga gcttcaggga 2100
cctggaccca gagcaaggac acaccgttta tgccaaaaag ctgtggacgg cccctgagct 2160
cctgcgaatg gcttcacccc ctgtgcgggg ctcccaggct ggtgacgtat acagctttgg 2220
gatcatcctt caggagattg ccctgaggag tggggtcttc cacgtggaag gtttggacct 2280
gagccccaaa gagatcatcg agcgggtgac tcggggtgag cagcccccct tccggccctc 2340
cctggccctg cagagtcacc tggaggagtt ggggctgctc atgcagcggt gctgggctga 2400
ggacccacag gagaggccac cattccagca gatccgcctg acgttgcgca aatttaacag 2460
ggagaacagc agcaacatcc tggacaacct gctgtcccgc atggagcagt acgcgaacaa 2520
tctggaggaa ctggtggagg agcggaccca ggcatacctg gaggagaagc gcaaggctga 2580
ggccctgctc taccagatcc tgcctcactc agtggctgag cagctgaagc gtggggagac 2640
ggtgcaggcc gaagcctttg acagtgttac catctacttc agtgacattg tgggtttcac 2700
agcgctgtcg gcggagagca cacccatgca ggtggtgacc ctgctcaatg acctgtacac 2760
ttgctttgat gctgtcatag acaactttga tgtgtacaag gtggagacaa ttggcgatgc 2820
ctacatggtg gtgtcagggc tccctgtgcg gaacgggcgg ctacacgcct gcgaggtagc 2880
ccgcatggcc ctggcactgc tggatgctgt gcgctccttc cgaatccgcc accggcccca 2940
ggagcagctg cgcttgcgca ttggcatcca cacaggacct gtgtgtgctg gagtggtggg 3000
actgaagatg ccccgttact gtctctttgg ggatacagtc aacacagcct caagaatgga 3060
gtctaatggg gaagccctga agatccactt gtcttctgag accaaggctg tcctggagga 3120
gtttggtggt ttcgagctgg agcttcgagg ggatgtagaa atgaagggca aaggcaaggt 3180
tcggacctac tggctccttg gggagagggg gagtagcacc cgaggctgac ctgcctcctc 3240
tcctatccct ccacacctcc cctaccctgt gccagaagca acagaggtgc caggcctcag 3300
cctcacccac agcagcccca tcgccaaagg atggaagtaa tttgaatagc tcaggtgtgc 3360
tgaccccagt gaagacacca gataggacct ctgagagggg actggcatgg ggggatctca 3420

gagcttacag gctgagccaa gcccacggcc atgcacaggg acactcacac aggcacacgc 3480

acctgctctc cacctggact caggccgggc tgggctgtgg atccttgatc ccctcccctc 3540

cccatgctct cctccctcag ccttgctacc ctgtgactta ctgggaggag agtcacctga 3600

aggggaacat gaaaagagac taggtgaaga gagggcaggg gagcccacat ctggggctgg 3660

cccacaatac ctgctccccc gaccccctcc acccagcagt agacacagtg cacaggggag 3720

aagaggggtg gcgcagaagg gttgggggcc tgtatgcctt gcttctacca tgagcagaga 3780

caattaaaat ctttattcca gtg 3803

<210> 52
<211> 1155
<212> DNA
<213> human

<400> 52
atggattgca gtaacggatc ggcagagtgt accggagaag gaggatcaaa agaggtggtg 60

gggactttta aggctaaaga cctaatagtc acaccagcta ccattttaaa ggaaaaacca 120

gaccccaata atctggtttt tggaactgtg ttcacggatc atatgctgac ggtggagtgg 180

tcctcagagt ttggatggga gaaacctcat atcaagcctc ttcagaacct gtcattgcac 240

cctggctcat cagctttgca ctatgcagtg gaattatttg aaggattgaa ggcatttcga 300

ggagtagata ataaaattcg actgtttcag ccaaacctca acatggatag aatgtatcgc 360

tctgctgtga gggcaactct gccggtattt gacaaagaag agctcttaga gtgtattcaa 420

cagcttgtga aattggatca agaatgggtc ccatattcaa catctgctag tctgtatatt 480

cgtcctgcat tcattggaac tgagccttct cttggagtca agaagcctac caaagccctg 540

ctctttgtac tcttgagccc agtgggacct tatttttcaa gtggaacctt taatccagtg 600

tccctgtggg ccaatcccaa gtatgtaaga gcctggaaag gtggaactgg ggactgcaag 660

atgggaggga attacggctc atctcttttt gcccaatgtg aagacgtaga taatgggtgt 720

cagcaggtcc tgtggctcta tggcagagac catcagatca ctgaagtggg aactatgaat 780

ctttttcttt actggataaa tgaagatgga gaagaagaac tggcaactcc tccactagat 840

ggcatcattc ttccaggagt gacaaggcgg tgcattctgg acctggcaca tcagtggggt 900

gaatttaagg tgtcagagag atacctcacc atggatgact tgacaacagc cctggagggg 960

aacagagtga gagagatgtt tagctctggt acagcctgtg ttgtttgccc agtttctgat 1020

atactgtaca aaggcgagac aatacacatt ccaactatgg agaatggtcc taagctggca 1080

agccgcatct tgagcaaatt aactgatatc cagtatggaa gagaagagag cgactggaca 1140

attgtgctat cctga 1155

<210> 53
<211> 2511
<212> DNA
<213> human

<400> 53
cttttcacac tggccttaaa gaggatatat tagaagttga agtaggaagg gagccagaga 60

ggccgatggc gcaaaggtac gacgatctac cccattacgg gggcatggat ggagtaggca 120

tcccctccac gatgtatggg gacccgcatg cagccaggtc catgcagccg gtccaccacc 180

tgaaccacgg gcctcctctg cactcgcatc agtacccgca cacagctcat accaacgcca 240

tggcccccag catgggctcc tctgtcaatg acgctttaaa gagagataaa gatgccattt 300

atggacaccc cctcttccct ctcttagcac tgatttttga gaaatgtgaa ttagctactt 360

gtacccccccg cgagccgggg gtggcgggcg gggacgtctg ctcgtcagag tcattcaatg 420

aagatatagc cgtgttcgcc aaacagattc gcgcagaaaa acctctattt tcttctaatc 480

cagaactgga taacttgatg attcaagcca tacaagtatt aaggtttcat ctattggaat 540

tagagaaggt acacgaatta tgtgacaatt tctgccaccg gtatattagc tgtttgaaag 600

ggaaaatgcc tatcgatttg gtgatagacg atagagaagg aggatcaaaa tcagacagtg 660

aagatataac aagatcagca aatctaactg accagccctc ttggaacaga gatcatgatg 720

acacggcatc tactcgttca ggaggaaccc caggcccttc cagcggtggc cacacgtcac 780

acagtgggga caacagcagt gagcaaggtg atggcttgga caacagtgta gcttccccca 840

gcacaggtga cgatgatgac cctgataagg acaaaaagcg tcacaaaaag cgtggcatct 900

ttcccaaagt agccacaaat atcatgaggg cgtggctgtt ccagcatcta acacaccctt 960

acccttctga agaacagaaa aagcagttgg cacaagacac gggactcacc atccttcaag 1020

tgaacaattg gtttattaat gcccggagaa gaatagtgca gcccatgata gaccagtcca 1080

accgagcagt aagtcaagga acaccttata atcctgatgg acagcccatg ggaggtttcg 1140

taatggacgg tcagcaacat atgggaatta gagcaccagg acctatgagt ggaatgggca 1200

tgaatatggg catggagggg cagtggcact acatgtaacc ttcatctagt taaccaatcg 1260

caaagcaagg gggaaggctg caaagtatgc caggggagta tgtagcccgg ggtggtccaa 1320

tgggtgtgag tatgggacag ccaagttata cccaacccca gatgcccccc catcctgctc 1380

agctgcgtca tgggcccccc atgcatacgt acattcctgg acaccctcac cacccaacag 1440

tgatgatgca tggaggaccg ccccaccctg gaatgccaat gtcagcatca agccccacag 1500

ttcttaatac aggagaccca acaatgagtg gacaagtcat ggacattcat gctcagtagc 1560

ttaagggaat atgcattgtc tgcaatggtg actgatttca aatcatgttt tttctgcaat 1620

gactgtggag ttccattctt ggcatctact ctggaccaag gagcatccct aattcttcat 1680

agggaccttt aaaaagcagg aaataccaac tgaagtcaat ttgggggaca tgctaaataa 1740

ctatataaga cattaagaga acaaagagtg aaatattgta aatgctatta tactgttatc 1800

catattacgt tgtttcttat agatttttta aaaaaaatgt gaaatttttc cacactatgt 1860

gtgttgtttc catagctctt cacttcctcc agaagcctcc ttacattaaa aagccttaca 1920

gttatcctgc aagggacagg aaggtctgat ttgcaggatt tttagagcat taaaataact 1980

atcaggcaga agaatctttc ttctcgccta ggatttcagc catgcgcgcg ctctctctct 2040

ttctctctct tttcctctct ctccctcttt ctagcctggg gcttgaattt gcatgtctaa 2100

ttcatttact caccatattt gaattggcct gaacagatgt aaatcgggaa ggatgggaaa 2160

aactgcagtc atcaacaatg attaatcagc tgttgcaggc agtgtcttaa ggagactggt 2220

aggaggaggc atggaaacca aaaggccgtg tgtttagaag cctaattgtc acatcaagca 2280

tcattgtccc catgcaacaa ccaccacctt atacatcact tcctgtttta agcagctcta 2340

aaacatagac tgaagattta tttttaatat gttgacttta tttctgagca aagcatcggt 2400

catgtgtgta ttttttcata gtcccacctt ggagcattta tgtagacatt gtaaataaat 2460

tttgtgcaaa aaggactgga aaaatgaact gtattattgc aattttttttt t 2511

<210> 54
<211> 1887
<212> DNA
<213> human

<400> 54
tcgcccaatt ccgggctcag acactgggct cccagctggg gactgctcca tggccatgga 60

gatagacagc aggcctgggg ggctccccgg cagtagctgc aacctaggtg cagcccgaga 120

acacatgcag gcggtcaccc gaaactacat cacccacccc cgtgtcacct acaggactgt 180

gtgcagcgtg aacgggcccc tggtggtgct ggaccgggtc aagtttgccc agtatgcgga 240

gatcgtccac ttcaccctcc cagatgggac tcagaggagc gggcaggtgc ttgaggtggc 300

tggcaccaag gcgattgttc aggtgtttga agggacatca gggatcgatg ccaggaagac 360

cacttgcgaa tttacagggg acatcctacg aactccggtg tcagaggaca tgctgggtcg 420

ggttttcaat ggctccggca agcccattga caaggggcca gtggtcatgg cggaggactt 480

tctggatatc aatggccagc ccatcaaccc gcactcccgc atctaccccg aggagatgat 540

tcagacgggc atttctccta ttgacgtcat gaacagcatt gcccgcggcc agaagatccc 600

catcttctca gcagccgggc tcccccacaa tgagattgcc gctcagatct gccgccaggc 660

ggggctggtg aagaagtcca aggctgtgct ggattaccat gacgacaact tcgccatcgt 720

ctttgcagcc atgggggtga acatggagac agccagattc ttcaagtctg actttgagca 780

gaatggaacc atggggaacg tctgcctctt cctgaacttg gccaatgacc ccacgatcga 840

gcggatcatc accccgcgcc tggcgctgac cactgctgaa ttccttgcct accagtgtga 900

gaagcatgtg ctggtcatac tgacggacat gagttcctat gcagaggcct tgcgggaggt 960

ctctgctgct agagaggagg tgcctgggcg ccgagggttt cctggatata tgtacacaga 1020

cctggccacc atctacgagc gggcgggccg tgtggagggt cggggaggat ccatcacaca 1080

gatccccatc ctcaccatgc ccaacgacga tatcacccac cctatcccag acttgacggg 1140

cttcatcaca gagggacaga tctacgtgga cagacagctt cacaacagac agatctaccc 1200

ccccatcaac gtgctccctt ccctgtcgcg gctgatgaag tcagccattg ggaaggcat 1260

gacaagaaag gaccatggag atgtctccaa ccagctgtac gcctgctatg ccatcgggaa 1320

ggacgtgcag gccatgaagg cagtagttgg ggaggaggcg ctcacctctg aggacctgct 1380

ctacctggaa ttcctgcaga agtttgagaa gaacttcatc aatcagggcc cctacgagaa 1440

ccgctcgatg ttcgagtcgc tggaccttag ctggaagctg ctgcgcatct tccccaagga 1500

gatgctgaag cgcattccgc aggccgtgat cgacgagttc tattcccgcg aggggcggct 1560

gcaggacctc gcgcctgaca ctgcgctcta gccccgcgcg ccgtggcacc ccaacaccgg 1620

caggaaccta ccctcggctc ccgggtctcc ccgtccctcg ccacccctaa ccagcggctt 1680

tcgcgccgcc ctccgccctc cgtggctccg aggtggtggg gggcgccgca gtcatccctt 1740

tcctcgctcg attccttttc ccgcgctcca tgcctccccc tcagctcccg gtgctgcgga 1800

agaactgaag gttcatgcct actctgacgg gagcatctgt attttttatg ttaaaagccc 1860

acaaaataaa aataaaaatg aactgag 1887

<210> 55
<211> 1506
<212> DNA
<213> human

<400> 55
aggcggacaa agcccgattg ttcctgggcc ctttccccat cgcgcctggg cctgctcccc 60
agcccggggc aggggcgggg gccagtgtgg tgacacacgc tgtagctgtc tccccggctg 120
gctggctcgc tctctcctgg ggacacagag gtcggcaggc agcacacaga gggacctacg 180
ggcagctgtt ccttcccccg actcaagaat ccccggaggc ccggaggcct gcagcaggag 240
cggccatgaa gaagctgatg gtggtgctga gtctgattgc tgcagcctgg gcagaggagc 300
agaataagtt ggtgcatggc ggaccctgcg acaagacatc tcacccctac caagctgccc 360
tctacacctc gggccacttg ctctgtggtg gggtccttat ccatccactg tgggtcctca 420
cagctgccca ctgcaaaaaa ccgaatcttc aggtcttcct ggggaagcat aaccttcggc 480
aaagggagag ttcccaggag cagagttctg ttgtccgggc tgtgatccac cctgactatg 540
atgccgccag ccatgaccag gacatcatgc tgttgcgcct ggcacgccca gccaaactct 600
ctgaactcat ccagcccctt cccctggaga gggactgctc agccaacacc accagctgcc 660
acatcctggg ctggggcaag acagcagatg gtgatttccc tgacaccatc cagtgtgcat 720
acatccacct ggtgtcccgt gaggagtgtg agcatgccta ccctggccag atcacccaga 780
acatgttgtg tgctggggat gagaagtacg ggaaggattc ctgccagggt gattctgggg 840
gtccgctggt atgtggagac cacctccgag gccttgtgtc atggggtaac atcccctgtg 900
gatcaaagga gaagccagga gtctacacca acgtctgcag atacacgaac tggatccaaa 960
aaaccattca ggccaagtga ccctgacatg tgacatctac ctcccgacct accaccccac 1020
tggctggttc cagaacgtct ctcacctaga ccttgcctcc cctcctctcc tgcccagctc 1080
tgaccctgat gcttaataaa cgcagcgacg tgagggtcct gattctccct ggttttaccc 1140
cagctccatc cttgcatcac tggggaggac gtgatgagtg aggacttggg tcctcggtct 1200
taccccccacc actaagagaa tacaggaaaa tcccttctag gcatctcctc tccccaaccc 1260
ttccacacgt ttgatttctt cctgcagagg cccagccacg tgtctggaat cccagctccg 1320
ctgcttactg tcggtgtccc cttgggatgt acctttcttc actgcagatt tctcacctgt 1380
aagatgaaga taaggatgat acagtctcca tcaggcagtg gctgttggaa agatttaaga 1440
tttcacacct atgacataca tgggatagca cctgggccgc catgcactca ataaagaatg 1500
tatttt 1506

<210> 56
<211> 2907

<212> DNA
<213> human

<400> 56
gccatctggg cccaggcccc atgccccgag gaggggtggt ctgaagccca ccagagcccc 60

ctgccagact gtctgcctcc cttctgactg tggccgcttg gcatggccag caacagcagc 120

tcctgcccga cacctggggg cgggcacctc aatgggtacc cggtgcctcc ctacgccttc 180

ttcttccccc ctatgctggg tggactctcc ccgccaggcg ctctgaccac tctccagcac 240

cagcttccag ttagtggata tagcacacca tccccagcca ccattgagac ccagagcagc 300

agttctgaag agatagtgcc cagccctccc tcgccacccc ctctaccccg catctacaag 360

ccttgctttg tctgtcagga caagtcctca ggctaccact atggggtcag cgcctgtgag 420

ggctgcaagg gcttcttccg ccgcagcatc cagaagaaca tggtgtacac gtgtcaccgg 480

gacaagaact gcatcatcaa caaggtgacc cggaaccgct gccagtactg ccgactgcag 540

aagtgctttg aagtgggcat gtccaaggag tctgtgagaa acgaccgaaa caagaagaag 600

aaggaggtgc ccaagcccga gtgctctgag agctacacgc tgacgccgga ggtgggggag 660

ctcattgaga aggtgcgcaa agcgcaccag gaaaccttcc ctgccctctg ccagctgggc 720

aaatacacta cgaacaacag ctcagaacaa cgtgtctctc tggacattga cctctgggac 780

aagttcagtg aactctccac caagtgcatc attaagactg tggagttcgc caagcagctg 840

cccggcttca ccaccctcac catcgccgac cagatcaccc tcctcaaggc tgcctgcctg 900

gacatcctga tcctgcggat ctgcacgcgg tacacgcccg agcaggacac catgaccttc 960

tcggacgggc tgaccctgaa ccggacccag atgcacaacg ctggcttcgg cccccctcacc 1020

gacctggtct ttgccttcgc caaccagctg ctgcccctgg agatggatga tgcggagacg 1080

gggctgctca gcgccatctg cctcatctgc ggagaccgcc aggacctgga gcagccggac 1140

cgggtggaca tgctgcagga gccgctgctg gaggcgctaa aggtctacgt gcggaagcgg 1200

aggcccagcc gcccccacat gttccccaag atgctaatga agattactga cctgcgaagc 1260

atcagcgcca agggggctga gcgggtgatc acgctgaaga tggagatccc gggctccatg 1320

ccgcctctca tccaggaaat gttggagaac tcagagggcc tggacactct gagcggacag 1380

ccgggggggtg gggggcggga cgggggtggc ctggcccccc cgccaggcag ctgtagcccc 1440

agcctcagcc ccagctccaa cagaagcagc ccggccaccc actccccgtg accgcccacg 1500

ccacatggac acagccctcg ccctccgccc cggcttttct ctgcctttct accgaccatg 1560

tgaccccgca ccagccctgc ccccacctgc cctcccgggc agtactgggg accttccctg 1620

```
ggggacgggg agggaggagg cagcgactcc ttggacagag gcctgggccc tcagtggact   1680

gcctgctccc acagcctggg ctgacgtcag aggccgaggc caggaactga gtgaggcccc   1740

tggtcctggg tctcaggatg ggtcctgggg gcctcgtgtt catcaagaca cccctctgcc   1800

cagctcacca catcttcatc accagcaaac gccaggactt ggctcccccca tcctcagaac  1860

tcacaagcca ttgctcccca gctggggaac ctcaacctcc cccctgcctc ggttggtgac   1920

agagggggtg ggacaggggc gggggttcc ccctgtacat accctgccat accaacccca    1980

ggtattaatt ctcgctggtt ttgtttttat tttaattttt ttgttttgat tttttaata    2040

agaattttca ttttaagcac atttatactg aaggaatttg tgctgtgtat tgggggggagc  2100

tggatccaga gctggagggg gtgggtccgg gggagggagt ggctcggaag gggcccccac   2160

tctcctttca tgtccctgtg ccccccagtt ctcctcctca gccttttcct cctcagtttt   2220

ctctttaaaa ctgtgaagta ctaactttcc aaggcctgcc ttcccctccc tcccactgga   2280

gaagccgcca gccccttttct ccctctgcct gaccactggg tgtggacggt gtggggcagc  2340

cctgaaagga caggctcctg gccttggcac ttgcctgcac ccaccatgag gcatggagca   2400

gggcagagca agggccccgg gacagagttt tcccagacct ggctcctcgg cagagctgcc   2460

tcccgtcagg gcccacatca tctaggctcc ccagccccca ctgtgaaggg gctggccagg   2520

ggcccgagct gcccccaccc ccggcctcag ccaccagcac ccccataggg cccccagaca   2580

ccacacacat gcgcgtgcgc acacacacaa acacacacac actggacagt agatgggccg   2640

acacacactt ggcccgagtt cctccatttc cctggcctgc cccccacccc caacctgtcc   2700

cacccccgtg ccccctcctt accccgcagg acgggcctac agggggggtct cccctcaccc  2760

ctgcaccccc agctggggga gctggctctg ccccgacctc cttcaccagg ggttggggcc   2820

ccttcccctg gagcccgtgg gtgcacctgt tactgttggg ctttccactg agatctactg   2880

gataaagaat aaagttctat ttattct                                       2907
```

```
<210>  57
<211>  2907
<212>  DNA
<213>  human

<400>  57
gccatctggg cccaggcccc atgccccgag gaggggtggt ctgaagccca ccagagcccc     60

ctgccagact gtctgcctcc cttctgactg tggccgcttg gcatggccag caacagcagc    120

tcctgcccga cacctggggg cgggcacctc aatgggtacc cggtgcctcc ctacgccttc    180
```

ttcttccccc ctatgctggg tggactctcc ccgccaggcg ctctgaccac tctccagcac 240
cagcttccag ttagtggata tagcacacca tccccagcca ccattgagac ccagagcagc 300
agttctgaag agatagtgcc cagccctccc tcgccacccc ctctaccccg catctacaag 360
ccttgctttg tctgtcagga caagtcctca ggctaccact atggggtcag cgcctgtgag 420
ggctgcaagg gcttcttccg ccgcagcatc cagaagaaca tggtgtacac gtgtcaccgg 480
gacaagaact gcatcatcaa caaggtgacc cggaaccgct gccagtactg ccgactgcag 540
aagtgctttg aagtgggcat gtccaaggag tctgtgagaa acgaccgaaa caagaagaag 600
aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg cccccctcacc 1020
gacctggtct ttgccttcgc caaccagctg ctgcccctgg agatggatga tgcggagacg 1080
gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca aggggggctga gcgggtgatc acgctgaaga tggagatccc gggctccatg 1320
ccgcctctca tccaggaaat gttggagaac tcagagggcc tggacactct gagcggacag 1380
ccggggggtg gggggcggga cgggggtggc ctggcccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680
gcctgctccc acagcctggg ctgacgtcag aggccgaggc caggaactga gtgaggcccc 1740
tggtcctggg tctcaggatg ggtcctgggg gcctcgtgtt catcaagaca cccctctgcc 1800
cagctcacca catcttcatc accagcaaac gccaggactt ggctccccca tcctcagaac 1860

tcacaagcca ttgctcccca gctggggaac ctcaacctcc cccctgcctc ggttggtgac 1920

agagggggtg ggacaggggc gggggttcc ccctgtacat accctgccat accaacccca 1980

ggtattaatt ctcgctggtt ttgtttttat tttaattttt ttgttttgat ttttttaata 2040

agaattttca ttttaagcac atttatactg aaggaatttg tgctgtgtat tggggggagc 2100

tggatccaga gctggagggg gtgggtccgg gggagggagt ggctcggaag gggcccccac 2160

tctcctttca tgtccctgtg ccccccagtt ctcctcctca gccttttcct cctcagtttt 2220

ctctttaaaa ctgtgaagta ctaactttcc aaggcctgcc ttcccctccc tcccactgga 2280

gaagccgcca gccccctttct ccctctgcct gaccactggg tgtggacggt gtggggcagc 2340

cctgaaagga caggctcctg gccttggcac ttgcctgcac ccaccatgag gcatggagca 2400

gggcagagca agggccccgg gacagagttt tcccagacct ggctcctcgg cagagctgcc 2460

tcccgtcagg gcccacatca tctaggctcc ccagccccca ctgtgaaggg gctggccagg 2520

ggcccgagct gcccccaccc ccggcctcag ccaccagcac ccccataggg cccccagaca 2580

ccacacacat gcgcgtgcgc acacacacaa acacacacac actggacagt agatgggccg 2640

acacacactt ggcccgagtt cctccatttc cctggcctgc cccccacccc caacctgtcc 2700

cacccccgtg ccccctcctt accccgcagg acgggcctac agggggggtct cccctcaccc 2760

ctgcaccccc agctggggga gctggctctg ccccgacctc cttcaccagg ggttggggcc 2820

ccttcccctg gagcccgtgg gtgcacctgt tactgttggg ctttccactg agatctactg 2880

gataaagaat aaagttctat ttattct 2907

<210> 58
<211> 5026
<212> DNA
<213> human

<400> 58
agaggaggaa attgttcctc gtctgataag acaacagtgg agaaaggacg catgctgttt 60

cttagggaca cggctgactt ccagatatga ccatgtattt gtggcttaaa ctcttggcat 120

ttggctttgc ctttctggac acagaagtat ttgtgacagg gcaaagccca acaccttccc 180

ccactggatt gactacagca aagatgccca gtgttccact ttcaagtgac cccttaccta 240

ctcacaccac tgcattctca cccgcaagca cctttgaaag agaaaatgac ttctcagaga 300

ccacaacttc tcttagtcca gacaatactt ccacccaagt atccccggac tctttggata 360

atgctagtgc ttttaatacc acaggtgttt catcagtaca gacgcctcac cttcccacgc 420

acgcagactc gcagacgccc tctgctggaa ctgacacgca gacattcagc ggctccgccg 480
ccaatgcaaa actcaaccct accccaggca gcaatgctat ctcagatgtc ccaggagaga 540
ggagtacagc cagcaccttt cctacagacc cagtttcccc attgacaacc accctcagcc 600
ttgcacacca cagctctgct gccttacctg cacgcacctc caacaccacc atcacagcga 660
acacctcaga tgcctacctt aatgcctctg aaacaaccac tctgagccct tctggaagcg 720
ctgtcatttc aaccacaaca atagctacta ctccatctaa gccaacatgt gatgaaaaat 780
atgcaaacat cactgtggat tacttatata acaaggaaac taaattattt acagcaaagc 840
taaatgttaa tgagaatgtg gaatgtggaa acaatacttg cacaaacaat gaggtgcata 900
accttacaga atgtaaaaat gcgtctgttt ccatatctca taattcatgt actgctcctg 960
ataagacatt aatattagat gtgccaccag gggttgaaaa gtttcagtta catgattgta 1020
cacaagttga aaaagcagat actactattt gtttaaaatg gaaaaatatt gaaaccttta 1080
cttgtgatac acagaatatt acctacagat ttcagtgtgg taatatgata tttgataata 1140
aagaaattaa attagaaaac cttgaacccg aacatgagta taagtgtgac tcagaaatac 1200
tctataataa ccacaagttt actaacgcaa gtaaaattat taaaacagat tttgggagtc 1260
caggagagcc tcagattatt ttttgtagaa gtgaagctgc acatcaagga gtaattacct 1320
ggaatccccc tcaaagatca tttcataatt ttaccctctg ttatataaaa gagacagaaa 1380
aagattgcct caatctggat aaaaacctga tcaaatatga tttgcaaaat ttaaaacctt 1440
atacgaaata tgttttatca ttacatgcct acatcattgc aaaagtgcaa cgtaatggaa 1500
gtgctgcaat gtgtcatttc acaactaaaa gtgctcctcc aagccaggtc tggaacatga 1560
ctgtctccat gacatcagat aatagtatgc atgtcaagtg taggcctccc agggaccgta 1620
atggccccca tgaacgttac catttggaag ttgaagctgg aaatactctg gttagaaatg 1680
agtcgcataa gaattgcgat ttccgtgtaa aagatcttca atattcaaca gactacactt 1740
ttaaggccta ttttcacaat ggagactatc ctggagaacc ctttatttta catcattcaa 1800
catcttataa ttctaaggca ctgatagcat ttctggcatt tctgattatt gtgacatcaa 1860
tagccctgct tgttgttctc tacaaaatct atgatctaca taagaaaaga tcctgcaatt 1920
tagatgaaca gcaggagctt gttgaaaggg atgatgaaaa acaactgatg aatgtggagc 1980
caatccatgc agatattttg ttggaaactt ataagaggaa gattgctgat gaaggaagac 2040
tttttctggc tgaatttcag agcatcccgc gggtgttcag caagtttcct ataaaggaag 2100
ctcgaaagcc ctttaaccag aataaaaacc gttatgttga cattcttcct tatgattata 2160

accgtgttga actctctgag ataaacggag atgcagggtc aaactacata aatgccagct 2220
atattgatgg tttcaaagaa cccaggaaat acattgctgc acaaggtccc agggatgaaa 2280
ctgttgatga tttctggagg atgatttggg aacagaaagc cacagttatt gtcatggtca 2340
ctcgatgtga agaaggaaac aggaacaagt gtgcagaata ctggccgtca atggaagagg 2400
gcactcgggc ttttggagat gttgttgtaa agatcaacca gcacaaaaga tgtccagatt 2460
acatcattca gaaattgaac attgtaaata aaaaagaaaa agcaactgga agagaggtga 2520
ctcacattca gttcaccagc tggccagacc acggggtgcc tgaggatcct cacttgctcc 2580
tcaaactgag aaggagagtg aatgccttca gcaatttctt cagtggtccc attgtggtgc 2640
actgcagtgc tggtgttggg cgcacaggaa cctatatcgg aattgatgcc atgctagaag 2700
gcctggaagc cgagaacaaa gtggatgttt atggttatgt tgtcaagcta aggcgacaga 2760
gatgcctgat ggttcaagta gaggcccagt acatcttgat ccatcaggct ttggtggaat 2820
acaatcagtt tggagaaaca gaagtgaatt tgtctgaatt acatccatat ctacataaca 2880
tgaagaaaag ggatccaccc agtgagccgt ctccactaga ggctgaattc cagagacttc 2940
cttcatatag gagctggagg acacagcaca ttggaaatca agaagaaaat aaaagtaaaa 3000
acaggaattc taatgtcatc ccatatgact ataacagagt gccacttaaa catgagctgg 3060
aaatgagtaa agagagtgag catgattcag atgaatcctc tgatgatgac agtgattcag 3120
aggaaccaag caaatacatc aatgcatctt ttataatgag ctactggaaa cctgaagtga 3180
tgattgctgc tcagggacca ctgaaggaga ccattggtga cttttggcag atgatcttcc 3240
aaagaaaagt caaagttatt gttatgctga cagaactgaa acatggagac caggaaatct 3300
gtgctcagta ctggggagaa ggaaagcaaa catatggaga tattgaagtt gacctgaaag 3360
acacagacaa atcttcaact tataccttc gtgtctttga actgagacat tccaagagga 3420
aagactctcg aactgtgtac cagtaccaat atacaaactg gagtgtggag cagcttcctg 3480
cagaacccaa ggaattaatc tctatgattc aggtcgtcaa acaaaaactt ccccagaaga 3540
attcctctga agggaacaag catcacaaga gtacacctct actcattcac tgcagggatg 3600
gatctcagca aacgggaata ttttgtgctt tgttaaatct cttagaaagt gcggaaacag 3660
aagaggtagt ggatattttt caagtggtaa aagctctacg caaagctagg ccaggcatgg 3720
tttccacatt cgagcaatat caattcctat atgacgtcat tgccagcacc taccctgctc 3780
agaatggaca agtaaagaaa aacaaccatc aagaagataa aattgaattt gataatgaag 3840

tggacaaagt aaagcaggat gctaattgtg ttaatccact tggtgcccca gaaaagctcc 3900

ctgaagcaaa ggaacaggct gaaggttctg aacccacgag tggcactgag gggccagaac 3960

attctgtcaa tggtcctgca agtccagctt taaatcaagg ttcataggaa aagacataaa 4020

tgaggaaact ccaaacctcc tgttagctgt tatttctatt tttgtagaag taggaagtga 4080

aaataggtat acagtggatt aattaaatgc agcgaaccaa tatttgtaga agggttatat 4140

tttactactg tggaaaaata tttaagatag ttttgccaga acagtttgta cagacgtatg 4200

cttattttaa aattttatct cttattcagt aaaaaacaac ttctttgtaa tcgttatgtg 4260

tgtatatgta tgtgtgtatg ggtgtgtgtt tgtgtgagag acagagaaag agagagaatt 4320

ctttcaagtg aatctaaaag cttttgcttt tcctttgttt ttatgaagaa aaaatacatt 4380

ttatattaga agtgttaact tagcttgaag gatctgtttt taaaaatcat aaactgtgtg 4440

cagactcaat aaaatcatgt acatttctga aatgacctca agatgtcctc cttgttctac 4500

tcatatatat ctatcttata tacttactat tttacttcta gagatagtac ataaaggtgg 4560

tatgtgtgtg tatgctacta caaaaaagtt gttaactaaa ttaacattgg gaaatcttat 4620

attccatata ttagcattta gtccaatgtc tttttaagct tatttaatta aaaaatttcc 4680

agtgagctta tcatgctgtc tttacatggg gttttcaatt ttgcatgctc gattattccc 4740

tgtacaatat ttaaaattta ttgcttgata cttttgacaa caaattaggt tttgtacaat 4800

tgaacttaaa taaatgtcat taaaataaat aaatgcaata tgtattaata ttcattgtat 4860

aaaaatagaa gaatacaaac atatttgtta aatatttaca tatgaaattt aatatagcta 4920

tttttatgga atttttcatt gatatgaaaa atatgatatt gcatatgcat agttcccatg 4980

ttaaatccca ttcataactt tcattaaagc atttactttg aatttc 5026

<210> 59
<211> 625
<212> DNA
<213> human

<400> 59
aggcgggccg ctcccacttc ggcacgaggg gcacgaggta aatcttttct gcttactgaa 60

aaggaagagt ctgatgatta gttactgatc ctctttgcat ttgtaaagct ttggagatat 120

tgaatcatgt taccatttct gttttttcc accctgtttt cttccatatt tactgaagct 180

cagaagcagt attgggtctg caactcatcc gatgcaagta tttcatacac ctactgtgat 240

aaaatgcaat acccaatttc aattaatgtt aacccctgta tagaattgaa aggatccaaa 300

ggattattgc acattttcta cattccaagg agagatttaa agcaattata tttcaatctc 360

tatataactg tcaacaccat gaatcttcca aagcgcaaag aagttatttg ccgaggatct 420

gatgacgatt actctttttg cagagctctg aagggagaga ctgtgaatac aacaatatca 480

ttctccttca agggaataaa attttctaag ggaaaataca aatgtgttgt tgaagctatt 540

tctgggagcc cagaagaaat gctcttttgc ttggagtttg tcatcctaca ccaacctaat 600

tcaaattaga ataaattgag tattt 625

<210> 60
<211> 2088
<212> DNA
<213> human

<400> 60
gaattcggca cgagcgcgcg gcgaatctca acgctgcgcc gtctgcgggc gcttccgggc 60

caccagtttc tctgctttcc accctggcgc cccccagccc tggctcccca gctgcgctgc 120

cccgggcgtc cacgccctgc gggcttagcg ggttcagtgg gctcaatctg cgcagcgcca 180

cctccatgtt gaccaagcct ctacaggggc ctcccgcgcc ccccgggacc cccacgccgc 240

cgccaggagg caaggatcgg gaagcgttcg aggccgagta tcgactcggc cccctcctgg 300

gtaagggggg ctttggcacc gtcttcgcag gacaccgcct cacagatcga ctccaggtgg 360

ccatcaaagt gattccccgg aatcgtgtgc tgggctggtc cccccttgtca gactcagtca 420

catgcccact cgaagtcgca ctgctatgga aagtgggtgc aggtggtggg caccctggcg 480

tgatccgcct gcttgactgg tttgagacac aggaaggctt catgctggtc ctcgagcggc 540

ctttgcccgc ccaggatctc tttgactata tcacagagaa gggcccactg ggtgaaggcc 600

caagccgctg cttctttggc caagtagtgg cagccatcca gcactgccat tcccgtggag 660

ttgtccatcg tgacatcaag gatgagaaca tcctgataga cctacgccgt ggctgtgcca 720

aactcattga ttttggttct ggtgccctgc ttcatgatga accctacact gactttgatg 780

ggacaagggt gtacagcccc ccagagtgga tctctcgaca ccagtaccat gcactcccgg 840

ccactgtctg gtcactgggc atcctcctct atgacatggt gtgtggggac attccctttg 900

agagggacca ggagattctg gaagctgagc tccacttccc agcccatgtc tccccagact 960

gctgtgccct aatccgccgg tgcctggccc ccaaaccttc ttcccgaccc tcactggaag 1020

agatcctgct ggacccctgg atgcaaacac cagccgagga tgttacccct caacccctcc 1080

aaaggaggcc ctgccccttt ggcctggtcc ttgctaccct aagcctggcc tggcctggcc 1140

tggcccccaa tggtcagaag agccatccca tggccatgtc acagggatag atggacattt 1200
gttgacttgg ttttacaggt cattaccagt cattaaagtc cagtattact aaggtaaggg 1260
attgaggatc aggggttaga agacataaac caagtttgcc cagttccctt cccaatccta 1320
caaaggagcc ttcctcccag aacctgtggt ccctgatttt ggagggggaa cttcttgctt 1380
ctcattttgc taaggaagtt tattttggtg aagttgttcc cattttgagc cccgggactc 1440
ttattttgat gatgtgtcac cccacattgg cacctcctac taccaccaca caaacttagt 1500
tcatatgctt ttacttgggc aagggtgctt tccttccaat accccagtag cttttatttt 1560
agtaaaggga ccctttcccc tagcctaggg tcccatattg ggtcaagctg cttacctgcc 1620
tcagcccagg atttttttatt ttgggggagg taatgccctg ttgttacccc aaggcttctt 1680
tttttttttt tttttttttg ggtgagggga ccctactttg ttatcccaag tgctcttatt 1740
ctggtgagaa gaaccttaat tccataattt gggaaggaat ggaagatgga caccaccgga 1800
caccaccaga caataggatg ggatggatgg tttttttgggg gatgggctag gggaaataag 1860
gcttgctgtt tgttttcctg gggcgctccc tccaattttg cagatttttg caacctcctc 1920
ctgagccggg attgtccaat tactaaaatg taaataatca cgtattgtgg ggaggggagt 1980
tccaagtgtg ccctcctttt ttttcctgcc tggattattt aaaaagccat gtgtggaaac 2040
ccactattta ataaaagtaa tagaatcaga aaaaaaaaaa aaaaaaaa 2088

<210> 61
<211> 2270
<212> DNA
<213> human

<400> 61
ctcctccagc ctctcacact ctcctcagct ctctcatctc ctggaaccat ggccagcaca 60
tccaccacca tcaggagcca cagcagcagc cgccggggtt tcagtgccaa ctcagccagg 120
ctccctgggg tcagccgctc tggcttcagc agcgtctccg tgtcccgctc caggggcagt 180
ggtggcctgg gtggtgcatg tggaggagct ggctttggca gccgcagtct gtatggcctg 240
gggggctcca agaggatctc cattggaggg ggcagctgtg ccatcagtgg cggctatggc 300
agcagagccg gaggcagcta tggctttggt ggcgccggga gtggatttgg tttcggtggt 360
ggagccggca ttggctttgg tctgggtggt ggagccggcc ttgctggtgg ctttgggggc 420
cctggcttcc ctgtgtgccc ccctggaggc atccaagagg tcaccgtcaa ccagagtctc 480
ctgactcccc tcaacctgca aatcgatccc accatccagc gggtgcgggc tgaggagcgt 540

gaacagatca agaccctcaa caacaagttt gcctccttca tcgacaaggt gcggttcctg 600

gagcagcaga acaaggttct ggaaacaaag tggaccctgc tgcaggagca gggcaccaag 660

actgtgaggc agaacctgga gccgttgttc gagcagtaca tcaacaacct caggaggcag 720

ctggacagca ttgtcgggga acggggccgc ctggactcag agctcagagg catgcaggac 780

ctggtggagg acttcaagaa caaatatgag gatgaaatca acaagcgcac agcagcagag 840

aatgaatttg tgactctgaa gaaggatgtg gatgctgcct acatgaacaa ggttgaactg 900

caagccaagg cagacactct cacagacgag atcaacttcc tgagagcctt gtatgatgca 960

gagctgtccc agatgcagac ccacatctca gacacatctg tggtgctgtc catggacaac 1020

aaccgcaacc tggacctgga cagcatcatc gctgaggtca aggcccaata tgaggagatt 1080

gctcagagaa gccgggctga ggctgagtcc tggtaccaga ccaagtacga ggagctgcag 1140

gtcacagcag gcagacatgg ggacgacctg cgcaacacca agcaggagat tgctgagatc 1200

aaccgcatga tccagaggct gagatctgag atcgaccacg tcaagaagca gtgcgccaac 1260

ctgcaggccg ccattgctga tgctgagcag cgtggggaga tggccctcaa ggatgccaag 1320

aacaagctgg aagggctgga ggatgccctg cagaaggcca agcaggacct ggcccggctg 1380

ctgaaggagt accaggagct gatgaatgtc aagctggccc tggacgtgga gatcgccacc 1440

taccgcaagc tgctggaggg tgaggagtgc aggctgaatg gcgaaggcgt tggacaagtc 1500

aacatctctg tggtgcagtc caccgtctcc agtggctatg gcggtgccag tggtgtcggc 1560

agtggcttag gcctgggtgg aggaagcagc tactcctatg gcagtggtct tggcgttgga 1620

ggtggcttca gttccagcag tggcagagcc attgggggtg gcctcagctc tgttggaggc 1680

ggcagttcca ccatcaagta caccaccacc tcctcctcca gcaggaagag ctataagcac 1740

taaagtgcgt ctgctagctc tcggtcccac agtcctcagg cccctctctg gctgcagagc 1800

cctctcctca ggttgcctgt cctctcctgg cctccagtct cccctgctgt cccaggtaga 1860

gctggggatg aatgcttagt gccctcactt cttctctctc tctctatacc atctgagcac 1920

ccattgctca ccatcagatc aacctctgat tttacatcat gatgtaatca ccactggagc 1980

ttcactgtta ctaaattatt aatttcttgc ctccagtgtt ctatctctga ggctgagcat 2040

tataagaaaa tgacctctgc tccttttcat tgcagaaaat tgccaggggc ttatttcaga 2100

acaacttcca cttactttcc actggctctc aaactctcta acttataagt gttgtgaacc 2160

cccacccagg cagtatccat gaaagcacaa gtgactagtc ctatgatgta caaagcctgt 2220

atctctgtga tgatttctgt gctcttcact gtttgcaatt gctaaataaa 2270

<210> 62
<211> 2048
<212> DNA
<213> human

<400> 62
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tatttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 63
<211> 2048
<212> DNA
<213> human

<400> 63
atgtgaaggc acaagctgct gttatataca acagagtgaa ctgagcatca gtcagaaaaa 60

gtctatgttt gcagaaatac agatccaaga caaagacagg atgggcactg ctggaaaagt 120

tattaaatgc aaagcagctg tgctttggga gcagaagcaa cccttctcca ttgaggaaat 180

agaagttgcc ccaccaaaga ctaaagaagt tcgcattaag attttggcca caggaatctg 240

tcgcacagat gaccatgtga taaaaggaac aatggtgtcc aagtttccag tgattgtggg 300

acatgaggca actgggattg tagagagcat tggagaagga gtgactacag tgaaaccagg 360

tgacaaagtc atccctctct ttctgccaca atgtagagaa tgcaatgctt gtcgcaaccc 420

agatggcaac ctttgcatta ggagcgatat tactggtcgt ggagtactgg ctgatggcac 480

caccagattt acatgcaagg gcaaaccagt acaccacttc atgaacacca gtacatttac 540

cgagtacaca gtggtggatg aatcttctgt tgctaagatt gatgatgcag ctcctcctga 600

gaaagtctgt ttaattggct gtgggttttc cactggatat ggcgctgctg ttaaaactgg 660

caaggtcaaa cctggttcca cttgcgtcgt ctttggcctg ggaggagttg gcctgtcagt 720

catcatgggc tgtaagtcag ctggtgcatc taggatcatt gggattgacc tcaacaaaga 780

caaatttgag aaggccatgg ctgtaggtgc cactgagtgt atcagtccca aggactctac 840

caaacccatc agtgaggtgc tgtcagaaat gacaggcaac aacgtgggat acacctttga 900

agttattggg catcttgaaa ccatgattga tgccctggca tcctgccaca tgaactatgg 960

gaccagcgtg gttgtaggag ttcctccatc agccaagatg ctcacctatg acccgatgtt 1020

gctcttcact ggacgcacat ggaagggatg tgtctttgga ggtttgaaaa gcagagatga 1080

tgtcccaaaa ctagtgactg agttcctggc aaagaaattt gacctggacc agttgataac 1140

tcatgtttta ccatttaaaa aaatcagtga aggatttgag ctgctcaatt caggacaaag 1200

cattcgaacg gtcctgacgt tttgagatcc aaagtggcag gaggtctgtg ttgtcatggt 1260

gaactggagt ttctcttgtg agagttccct catctgaaat catgtatctg tctcacaaat 1320

acaagcataa gtagaagatt tgttgaagac atagaaccct tataaagaat tattaacctt 1380

tataaacatt taaagtcttg tgagcacctg ggaattagta taataacaat gttaatattt 1440

ttgatttaca ttttgtaagg ctataattgt atcttttaag aaaacataca cttggatttc 1500

tatgttgaaa tggagatttt taagagtttt aaccagctgc tgcagatata taactcaaaa 1560

cagatatagc gtataaagat atagtaaatg catctcccag agtaatattc acttaacaca 1620

ttgaaactat tatttttttag atttgaatat aaatgtattt tttaaacact tgttatgagt 1680

taacttggat tacattttga aatcagttca ttccatgatg catattactg gattagatta 1740

agaaagacag aaaagattaa gggacgggca catttttcaa cgattaagaa tcatcattac 1800

ataacttggt gaaactgaaa aagtatatca tatgggtaca caaggctatt tgccagcata 1860

tattaatatt ttagaaaata ttccttttgt aatactgaat ataaacatag agctagagtc 1920

atattatcat acttatcata atgttcaatt tgatacagta gaattgcaag tccctaagtc 1980

cctattcact gtgcttagta gtgactccat ttaataaaaa gtgtttttag tttttaacaa 2040

ctaaaccg 2048

<210> 64
<211> 2816
<212> DNA
<213> human

<400> 64
tcgttgatat caaagacagt tgaaggaaat gaattttgaa acttcacggt gtgccacccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

```
agcatggact gtatccgcat gcaggactcg gacctgagtg accccatgtg gccacagtac     360
acgaacctgg ggctcctgaa cagcatggac cagcagattc agaacggctc ctcgtccacc     420
agtccctata acacagacca cgcgcagaac agcgtcacgg cgccctcgcc ctacgcacag     480
cccagctcca ccttcgatgc tctctctcca tcacccgcca tcccctccaa caccgactac     540
ccaggcccgc acagtttcga cgtgtccttc cagcagtcga gcaccgccaa gtcggccacc     600
tggacgtatt ccactgaact gaagaaactc tactgccaaa ttgcaaagac atgccccatc     660
cagatcaagg tgatgacccc acctcctcag ggagctgtta tccgcgccat gcctgtctac     720
aaaaaagctg agcacgtcac ggaggtggtg aagcggtgcc ccaaccatga gctgagccgt     780
gaattcaacg agggacagat tgcccctcct agtcatttga ttcgagtaga ggggaacagc     840
catgcccagt atgtagaaga tcccatcaca ggaagacaga gtgtgctggt accttatgag     900
ccaccccagg ttggcactga attcacgaca gtcttgtaca atttcatgtg taacagcagt     960
tgtgttggag ggatgaaccg ccgtccaatt ttaatcattg ttactctgga aaccagagat    1020
gggcaagtcc tgggccgacg ctgctttgag gcccggatct gtgcttgccc aggaagagac    1080
aggaaggcgg atgaagatag catcagaaag cagcaagttt cggacagtac aaagaacggt    1140
gatggtacga agcgcccgtt tcgtcagaac acacatggta tccagatgac atccatcaag    1200
aaacgaagat ccccagatga tgaactgtta tacttaccag tgaggggccg tgagacttat    1260
gaaatgctgt tgaagatcaa agagtccctg gaactcatgc agtaccttcc tcagcacaca    1320
attgaaacgt acaggcaaca gcaacagcag cagcaccagc acttacttca gaaacatctc    1380
ctttcagcct gcttcaggaa tgagcttgtg gagccccgga gagaaactcc aaaacaatct    1440
gacgtcttct ttagacattc caagccccca aaccgatcag tgtacccata gagccctatc    1500
tctatatttt aagtgtgtgt gttgtatttc catgtgtata tgtgagtgtg tgtgtgtgta    1560
tgtgtgtgcg tgtgtatcta gccctcataa acaggacttg aagacacttt ggctcagaga    1620
cccaactgct caaaggcaca aagccactag tgagagaatc ttttgaaggg actcaaacct    1680
ttacaagaaa ggatgttttc tgcagatttt gtatccttag accggccatt ggtgggtgag    1740
gaaccactgt gtttgtctgt gagctttctg ttgtttcctg ggagggaggg gtcaggtggg    1800
gaaaggggca ttaagatgtt tattggaacc cttttctgtc ttcttctgtt gtttttctaa    1860
aattcacagg gaagcttttg agcaggtctc aaacttaaga tgtcttttta agaaaaggag    1920
aaaaaagttg ttattgtctg tgcataagta agttgtaggt gactgagaga ctcagtcaga    1980
```

ccctttttaat gctggtcatg taataatatt gcaagtagta agaaacgaag gtgtcaagtg 2040

tactgctggg cagcgaggtg atcattacca aaagtaatca actttgtggg tggagagttc 2100

tttgtgagaa cttgcattat ttgtgtcctc ccctcatgtg taggtagaac atttcttaat 2160

gctgtgtacc tgcctctgcc actgtatgtt ggcatctgtt atgctaaagt ttttcttgta 2220

catgaaaccc tggaagacct actacaaaaa aactgttgtt tggcccccat agcaggtgaa 2280

ctcattttgt gcttttaata gaaagacaaa tccaccccag taatattgcc cttacgtagt 2340

tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 65
<211> 2816
<212> DNA
<213> human

<400> 65
tcgttgatat caaagacagt tgaaggaaat gaattttgaa acttcacggt gtgccacccct 60

acagtactgc cctgaccctt acatccagcg tttcgtagaa acccagctca tttctcttgg 120

aaagaaagtt attaccgatc caccatgtcc cagagcacac agacaaatga attcctcagt 180

ccagaggttt tccagcatat ctgggatttt ctggaacagc ctatatgttc agttcagccc 240

attgacttga actttgtgga tgaaccatca gaagatggtg cgacaaacaa gattgagatt 300

agcatggact gtatccgcat gcaggactcg gacctgagtg accccatgtg gccacagtac 360

acgaacctgg ggctcctgaa cagcatggac cagcagattc agaacggctc ctcgtccacc 420

agtccctata acacagacca cgcgcagaac agcgtcacgg cgccctcgcc ctacgcacag 480

cccagctcca ccttcgatgc tctctctcca tcacccgcca tcccctccaa caccgactac 540

ccaggcccgc acagtttcga cgtgtccttc cagcagtcga gcaccgccaa gtcggccacc 600

tggacgtatt ccactgaact gaagaaactc tactgccaaa ttgcaaagac atgccccatc 660

cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagcccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt tttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400

aatttgctta attagagctt ctatccctca agcctaccta ccataaaacc agccatatta 2460

ctgatactgt tcagtgcatt tagccaggag acttacgttt tgagtaagtg agatccaagc 2520

agacgtgtta aaatcagcac tcctggactg gaaattaaag attgaaaggg tagactactt 2580

ttcttttttt tactcaaaag tttagagaat ctctgtttct ttccatttta aaaacatatt 2640

ttaagataat agcataaaga ctttaaaaat gttcctcccc tccatcttcc cacacccagt 2700

caccagcact gtattttctg tcaccaagac aatgatttct tgttattgag gctgttgctt 2760

ttgtggatgt gtgattttaa ttttcaataa acttttgcat cttggtttaa aagaaa 2816

<210> 66
<211> 5838
<212> DNA
<213> human

<400> 66
ccgggcaggt ggctcatgct cgggagcgtg gttgagcggc tggcgcggtt gtcctggagc 60

aggggcgcag gaattctgat gtgaaactaa cagtctgtga gccctggaac ctccgctcag 120

agaagatgaa ggatatcgac ataggaaaag agtatatcat ccccagtcct gggtatagaa 180

gtgtgaggga gagaaccagc acttctggga cgcacagaga ccgtgaagat tccaagttca 240

ggagaactcg accgttggaa tgccaagatg ccttggaaac agcagcccga gccgagggcc 300

tctctcttga tgcctccatg cattctcagc tcagaatcct ggatgaggag catcccaagg 360

gaaagtacca tcatggcttg agtgctctga agcccatccg gactacttcc aaacaccagc 420

acccagtgga caatgctggg ctttttcct gtatgacttt ttcgtggctt tcttctctgg 480

cccgtgtggc ccacaagaag gggagctct caatggaaga cgtgtggtct ctgtccaagc 540

acgagtcttc tgacgtgaac tgcagaagac tagagagact gtggcaagaa gagctgaatg 600

aagttgggcc agacgctgct tccctgcgaa gggttgtgtg gatcttctgc cgcaccaggc 660

tcatcctgtc catcgtgtgc ctgatgatca cgcagctggc tggcttcagt ggaccagcct 720

tcatggtgaa acacctcttg gagtataccc aggcaacaga gtctaacctg cagtacagct 780

tgttgttagt gctgggcctc ctcctgacgg aaatcgtgcg gtcttggtcg cttgcactga 840

cttgggcatt gaattaccga accggtgtcc gcttgcgggg ggccatccta accatggcat 900

ttaagaagat ccttaagtta aagaacatta aagagaaatc cctgggtgag ctcatcaaca 960

tttgctccaa cgatgggcag agaatgtttg aggcagcagc cgttggcagc ctgctggctg 1020

gaggacccgt tgttgccatc ttaggcatga tttataatgt aattattctg ggaccaacag 1080

gcttcctggg atcagctgtt tttatcctct tttacccagc aatgatgttt gcatcacggc 1140

tcacagcata tttcaggaga aaatgcgtgg ccgccacgga tgaacgtgtc cagaagatga 1200

atgaagttct tacttacatt aaatttatca aaatgtatgc ctgggtcaaa gcattttctc 1260

agagtgttca aaaaatccgc gaggaggagc gtcggatatt ggaaaaagcc gggtacttcc 1320

agggtatcac tgtgggtgtg gctcccattg tggtggtgat tgccagcgtg gtgaccttct 1380

ctgttcatat gaccctgggc ttcgatctga cagcagcaca ggctttcaca gtggtgacag 1440

tcttcaattc catgactttt gctttgaaag taacaccgtt ttcagtaaag tccctctcag 1500

aagcctcagt ggctgttgac agatttaaga gtttgtttct aatggaagag gttcacatga 1560

taaagaacaa accagccagt cctcacatca agatagagat gaaaaatgcc accttggcat 1620

gggactcctc ccactccagt atccagaact cgcccaagct gacccccaaa atgaaaaaag 1680

acaagagggc ttccaggggc aagaaagaga aggtgaggca gctgcagcgc actgagcatc 1740

aggcggtgct ggcagagcag aaaggccacc tcctcctgga cagtgacgag cggcccagtc 1800

ccgaagagga agaaggcaag cacatccacc tgggccacct gcgcttacag aggacactgc 1860

acagcatcga tctggagatc caagagggta aactggttgg aatctgcggc agtgtgggaa 1920

gtggaaaaac ctctctcatt tcagccattt taggccagat gacgcttcta gagggcagca 1980

ttgcaatcag tggaaccttc gcttatgtgg cccagcaggc ctggatcctc aatgctactc 2040

tgagagacaa catcctgttt gggaaggaat atgatgaaga aagatacaac tctgtgctga 2100

acagctgctg cctgaggcct gacctggcca ttcttcccag cagcgacctg acggagattg 2160

gagagcgagg agccaacctg agcggtgggc agcgccagag gatcagcctt gcccgggcct 2220

tgtatagtga caggagcatc tacatcctgg acgacccccct cagtgcctta gatgcccatg 2280

tgggcaacca catcttcaat agtgctatcc ggaaacatct caagtccaag acagttctgt 2340

ttgttaccca ccagttacag tacctggttg actgtgatga agtgatcttc atgaaagagg 2400

gctgtattac ggaaagaggc acccatgagg aactgatgaa tttaaatggt gactatgcta 2460

ccatttttaa taacctgttg ctgggagaga caccgccagt tgagatcaat tcaaaaaagg 2520

aaaccagtgg ttcacagaag aagtcacaag acaagggtcc taaaacagga tcagtaaaga 2580

aggaaaaagc agtaaagcca gaggaagggc agcttgtgca gctggaagag aaagggcagg 2640

gttcagtgcc ctggtcagta tatggtgtct acatccaggc tgctgggggc cccttggcat 2700

tcctggttat tatggccctt ttcatgctga atgtaggcag caccgccttc agcacctggt 2760

```
ggttgagtta ctggatcaag caaggaagcg ggaacaccac tgtgactcga gggaacgaga   2820
cctcggtgag tgacagcatg aaggacaatc ctcatatgca gtactatgcc agcatctacg   2880
ccctctccat ggcagtcatg ctgatcctga aagccattcg aggagttgtc tttgtcaagg   2940
gcacgctgcg agcttcctcc cggctgcatg acgagctttt ccgaaggatc cttcgaagcc   3000
ctatgaagtt ttttgacacg acccccacag ggaggattct caacaggttt tccaaagaca   3060
tggatgaagt tgacgtgcgg ctgccgttcc aggccgagat gttcatccag aacgttatcc   3120
tggtgttctt ctgtgtggga atgatcgcag gagtcttccc gtggttcctt gtggcagtgg   3180
ggccccttgt catcctcttt tcagtcctgc acattgtctc cagggtcctg attcgggagc   3240
tgaagcgtct ggacaatatc acgcagtcac ctttcctctc ccacatcacg tccagcatac   3300
agggccttgc caccatccac gcctacaata aagggcagga gtttctgcac agataccagg   3360
agctgctgga tgacaaccaa gctccttttt ttttgtttac gtgtgcgatg cggtggctgg   3420
ctgtgcggct ggacctcatc agcatcgccc tcatcaccac cacggggctg atgatcgttc   3480
ttatgcacgg gcagattccc ccagcctatg cgggtctcgc catctcttat gctgtccagt   3540
taacggggct gttccagttt acggtcagac tggcatctga gacagaagct cgattcacct   3600
cggtggagag gatcaatcac tacattaaga ctctgtcctt ggaagcacct gccagaatta   3660
agaacaaggc tccctcccct gactggcccc aggagggaga ggtgaccttt gagaacgcag   3720
agatgaggta ccgagaaaac ctccctcttg tcctaaagaa agtatccttc acgatcaaac   3780
ctaaagagaa gattggcatt gtggggcgga caggatcagg gaagtcctcg ctggggatgg   3840
ccctcttccg tctggtggag ttatctggag gctgcatcaa gattgatgga gtgagaatca   3900
gtgatattgg ccttgccgac ctccgaagca aactctctat cattcctcaa gagccggtgc   3960
tgttcagtgg cactgtcaga tcaaatttgg accccttcaa ccagtacact gaagaccaga   4020
tttgggatgc cctggagagg acacacatga aagaatgtat tgctcagcta cctctgaaac   4080
ttgaatctga agtgatggag aatggggata acttctcagt gggggaacgg cagctcttgt   4140
gcatagctag agccctgctc cgccactgta agattctgat tttagatgaa gccacagctg   4200
ccatggacac agagacagac ttattgattc aagagaccat ccgagaagca tttgcagact   4260
gtaccatgct gaccattgcc catcgcctgc acacggttct aggctccgat aggattatgg   4320
tgctggccca gggacaggtg gtggagtttg acaccccatc ggtccttctg tccaacgaca   4380
gttcccgatt ctatgccatg tttgctgctg cagagaacaa ggtcgctgtc aagggctgac   4440
tcctccctgt tgacgaagtc tcttttcttt agagcattgc cattccctgc ctggggcggg   4500
```

cccctcatcg cgtcctccta ccgaaacctt gcctttctcg attttatctt tcgcacagca 4560

gttccggatt ggcttgtgtg tttcactttt agggagagtc atattttgat tattgtattt 4620

attccatatt catgtaaaca aaatttagtt tttgttctta attgcactct aaaaggttca 4680

gggaaccgtt attataattg tatcagaggc ctataatgaa gctttatacg tgtagctata 4740

tctatatata attctgtaca tagcctatat ttacagtgaa aatgtaagct gtttatttta 4800

tattaaaata agcactgtgc taataacagt gcatattcct ttctatcatt tttgtacagt 4860

ttgctgtact agagatctgg ttttgctatt agactgtagg aagagtagca tttcattctt 4920

ctctagctgg tggtttcacg gtgccaggtt ttctgggtgt ccaaaggaag acgtgtggca 4980

atagtgggcc ctccgacagc cccctctgcc gcctccccac agccgctcca ggggtggctg 5040

gagacgggtg ggcggctgga gaccatgcag agcgccgtga gttctcaggg ctcctgcctt 5100

ctgtcctggt gtcacttact gtttctgtca ggagagcagc ggggcgaagc ccaggcccct 5160

tttcactccc tccatcaaga atggggatca cagagacatt cctccgagcc ggggagtttc 5220

tttcctgcct tcttcttttt gctgttgttt ctaaacaaga atcagtctat ccacagagag 5280

tcccactgcc tcaggttcct atggctggcc actgcacaga gctctccagc tccaagacct 5340

gttggttcca agccctggag ccaactgctg ctttttgagg tggcactttt tcatttgcct 5400

attcccacac ctccacagtt cagtggcagg gctcaggatt tcgtgggtct gttttccttt 5460

ctcaccgcag tcgtcgcaca gtctctctct ctctctcccc tcaaagtctg caactttaag 5520

cagctcttgc taatcagtgt ctcacactgg cgtagaagtt tttgtactgt aaagagacct 5580

acctcaggtt gctggttgct gtgtggtttg gtgtgttccc gcaaaccccc tttgtgctgt 5640

ggggctggta gctcaggtgg gcgtggtcac tgctgtcatc agttgaatgg tcagcgttgc 5700

atgtcgtgac caactagaca ttctgtcgcc ttagcatgtt tgctgaacac cttgtggaag 5760

caaaaatctg aaaatgtgaa taaaattatt ttggattttg taaaaaaaaa aaaaaaaaaa 5820

aaaaaaaaaa aaaaaaa 5838

<210> 67
<211> 1181
<212> DNA
<213> human

<400> 67
ggcacgaggc ccgggccccc caaagtcccg gccgggccga gggtcggcgg ccgccggcgg 60

gccgggcccg cgcacagcgc ccgcatgtac aacatgatgg agacggagct gaagccgccg 120

ggcccgcagc aaacttcggg gggcggcggc ggcaactcca ccgcggcggc ggccggcggc 180

aaccagaaaa acagcccgga ccgcgtcaag cggcccatga atgccttcat ggtgtggtcc 240

cgcgggcagc ggcgcaagat ggcccaggag aaccccaaga tgcacaactc ggagatcagc 300

aagcgcctgg gcgccgagtg gaaacttttg tcggagacgg agaagcggcc gttcatcgac 360

gaggctaagc ggctgcgagc gctgcacatg aaggagcacc cggattataa ataccggccc 420

cggcggaaaa ccaagacgct catgaagaag gataagtaca cgctgcccgg cgggctgctg 480

gcccccggcg gcaatagcat ggcgagcggg gtcggggtgg gcgccggcct gggcgcgggc 540

gtgaaccagc gcatggacag ttacgcgcac atgaacggct ggagcaacgg cagctacagc 600

atgatgcagg accagctggg ctacccgcag cacccgggcc tcaatgcgca cggcgcagcg 660

cagatgcagc ccatgcaccg ctacgacgtg agcgccctgc agtacaactc catgaccagc 720

tcgcagacct acatgaacgg ctcgcccacc tacagcatgt cctactcgca gcagggcacc 780

cctggcatgg ctcttggctc catgggttcg gtggtcaagt ccgaggccag ctccagcccc 840

cctgtggtta cctcttcctc ccactccagg gcgccctgcc aggccgggga cctccgggac 900

atgatcagca tgtatctccc cggcgccgag gtgccggaac ccgccgcccc cagcagactt 960

cacatgtccc agcactacca gagcggcccg gtgcccggca cggccattaa cggcacactg 1020

cccctctcac acatgtgagg gccggacagc gaactggagg ggggagaaat tttcaaagaa 1080

aaacgaggga aatgggaggg gtgcaaaaga ggagagtaag aaacagcatg gagaaaaccc 1140

ggtacgctca aaaaaaaaaa aaaaaaaaaa aaaaaaaaaa a 1181

<210> 68
<211> 4755
<212> DNA
<213> human

<400> 68
gctgctctcg ttcgcttggc tcagctcagc tcagctcagc gcagctccgc ggccgccaag 60

ccgaggcggg cacggtctcc gagtcgcgga cgccagctcc gagctccctc tctccgccgc 120

gcctccgcca ggtcgcgcct tcgtcgggac cacttcgggc aggagtcgcg tggcgaaggc 180

ctgcggccgc ggcacaaagt tgggggccgc gaagatgagg ctgtccccgg cgcccctgaa 240

gctgagccgg actccggcac tgctggccct ggcgctgccc ctggccgcgg cgctggcctt 300

ctccgacgag accctggaca aagtgcccaa gtcagagggc tactgcagcc gtatcctgcg 360

cgcccagggc acgcggcgcg agggctacac cgagttcagc ctccgcgtgg agggcgaccc 420

cgacttctac aagccgggaa ccagctaccg cgtaacactt tcagctgctc ctccctccta 480
cttcagagga ttcacattaa ttgccctcag agagaacaga gagggtgata aggaagaaga 540
ccatgctggg accttccaga tcatagacga agaagaaact cagtttatga gcaattgccc 600
tgttgcagtc actgaaagca ctccacggag gaggacccgg atccaggtgt tttggatagc 660
accaccagcg ggaacaggct gcgtgattct gaaggccagc atcgtacaaa aacgcattat 720
ttattttcaa gatgagggct ctctgaccaa gaaactttgt gaacaagatt ccacatttga 780
tggggtgact gacaaaccca tcttagactg ctgtgcctgc ggaactgcca agtacagact 840
cacattttat gggaattggt ccgagaagac acacccaaag gattaccctc gtcgggccaa 900
ccactggtct gcgatcatcg gaggatccca ctccaagaat tatgtactgt gggaatatgg 960
aggatatgcc agcgaaggcg tcaaacaagt tgcagaattg ggctcacccg tgaaaatgga 1020
ggaagaaatt cgacaacaga gtgatgaggt cctcaccgtc atcaaagcca aagcccagtg 1080
gccagcctgg cagcctctca acgtgagagc agcaccttca gctgaatttt ccgtggacag 1140
aacgcgccat ttaatgtcct tcctgaccat gatgggccct agtcccgact ggaacgtagg 1200
cttatctgca gaagatctgt gcaccaagga atgtggctgg gtccagaagg tggtgcaaga 1260
cctgattccc tgggacgctg gcaccgacag cggggtgacc tatgagtcac ccaacaaacc 1320
caccattccc caggagaaaa tccggcccct gaccagcctg gaccatcctc agagtccttt 1380
ctatgaccca gagggtgggt ccatcactca agtagccaga gttgtcatcg agagaatcgc 1440
acggaagggt gaacaatgca atattgtacc tgacaatgtc gatgatattg tagctgacct 1500
ggctccagaa gagaaagatg aagatgacac ccctgaaacc tgcatctact ccaactggtc 1560
cccatggtcc gcctgcagct cctccacctg tgacaaaggc aagaggatgc gacagcgcat 1620
gctgaaagca cagctggacc tcagcgtccc ctgccctgac acccaggact tccagccctg 1680
catgggccct ggctgcagtg acgaagacgg ccccacctgc accatgtccg agtggatcac 1740
ctggtcgccc tgcagcatct cctgcggcat gggcatgagg tcccgggaga ggtatgtgaa 1800
gcagttcccg gaggacggct ccgtgtgcac gctgcccact gaggaaacgg agaagtgcac 1860
ggtcaacgag gagtgctctc ccagcagctg cctgatgacc gagtggggcg agtgggacga 1920
gtacagcgcc acctgcggca tgggcatgaa gaagcggcac cgcatgatca agatgaaccc 1980
cgcagatggc tccatgtgca aagccgagac atcacaggca gagaagcgca tgatgccaga 2040
gtgccacacc atcccatgct tgctgtcccc atggtccgag tggagtgact gcagcgtgac 2100

ctgcgggaag ggcatgcgaa cccgacagcg gatgctcaag tctctggcag aacttggaga 2160
ctgcaatgag gatctggagc aggtggagaa gtgcatgctc cctgaatgcc ccattgactg 2220
tgagctcacc gagtggtccc agtggtcgga atgtaacaag tcatgtggga aaggccacgt 2280
gattcgaacc cggatgatcc aaatggagcc tcagtttgga ggtgcaccct gcccagagac 2340
tgtgcagcga aaaaagtgcc gcatccgaaa atgccttcga aatccatcca tccaaaagct 2400
acgctggagg gaggcccgag agagccggcg gagtgagcag ctgaaggaag agtctgaagg 2460
ggagcagttc ccaggttgta ggatgcgccc atggacggcc tggtcagaat gcaccaaact 2520
gtgcggaggt ggaattcagg aacgttacat gactgtaaag aagagattca aaagctccca 2580
gtttaccagc tgcaaagaca agaaggagat cagagcatgc aatgttcatc cttgttagca 2640
agggtacgag ttccccaggg ctgcactcta gattccagag tcaccaatgg ctggattatt 2700
tgcttgttta agacaattta aattgtgtac gctagttttc atttttgcag tgtggttcgc 2760
ccagtagtct tgtggatgcc agagacatcc tttctgaata cttcttgatg ggtacaggct 2820
gagtggggcg ccctcacctc cagccagcct cttcctgcag aggagtagtg tcagccacct 2880
tgtactaagc tgaaacatgt ccctctggag cttccacctg gccagggagg acggagactt 2940
tgacctactc cacatggaga ggcaaccatg tctggaagtg actatgcctg agtcccaggg 3000
tgcggcaggt aggaaacatt cacagatgaa gacagcagat tccccacatt ctcatctttg 3060
gcctgttcaa tgaaaccatt gtttgcccat ctcttcttag tggaacttta ggtctctttt 3120
caagtctcct cagtcatcaa tagttcctgg ggaaaaacag agctggtaga cttgaagagg 3180
agcattgatg ttgggtggct tttgttcttt cactgagaaa ttcggaatac atttgtctca 3240
cccctgatat tggttcctga tgccccccca acaaaaataa ataaataaat tatggctgct 3300
ttatttaaat ataaggtagc tagtttttac acctgagata aataataagc ttagagtgta 3360
tttttccctt gcttttgggg gttcagagga gtatgtacaa ttcttctggg aagccagcct 3420
tctgaacttt ttggtactaa atccttattg gaaccaagac aaaggaagca aaattggtct 3480
ctttagagac caatttgcct aaattttaaa atcttcctac acacatctag acgttcaagt 3540
ttgcaaatca gtttttagca agaaaacatt tttgctatac aaacattttg ctaagtctgc 3600
ccaaagcccc cccaatgcat tccttcaaca aaatacaatc tctgtacttt aaagttattt 3660
tagtcatgaa attttatatg cagagagaaa aagttaccga gacagaaaac aaatctaagg 3720
gaaaggaata ttatgggatt aagctgagca agcaattctg gtggaaagtc aaacctgtca 3780
gtgctccaca ccagggctgt ggtcctccca gacatgcata ggaatggcca caggtttaca 3840

ctgccttccc agcaattata agcacaccag attcagggag actgaccacc aagggatagt 3900

gtaaaaggac attttctcag ttgggtccat cagcagtttt tcttcctgca tttattgttg 3960

aaaactattg tttcatttct tcttttatag gccttattac tgcttaatcc aaatgtgtac 4020

cattggtgag acacatacaa tgctctgaat acactacgaa tttgtattaa acacatcaga 4080

atatttccaa atacaacata gtatagtcct gaatatgtac ttttaacaca agagagacta 4140

ttcaataaaa actcactggg tctttcatgt ctttaagcta agtaagtgtt cagaaggttc 4200

ttttttatat tgtcctccac ctccatcatt ttcaataaaa gatagggctt ttgctccctt 4260

gttcttggag ggaccattat tacatctctg aactaccttt gtatccaaca tgttttaaat 4320

ccttaaatga attgctttct cccaaaaaaa gcacagtata aagaaacaca agatttaatt 4380

atttttctac ttggggggaa aaaagtcctc atgtagaagc acccactttt gcaatgttgt 4440

tctaagctat ctatctaact ctcagcccat gataaagttc cttaagctgg tgattcctaa 4500

tcaaggacaa gccaccctag tgtctcatgt ttgtatttgg tcccagttgg gtacatttta 4560

aaatcctgat tttggagact taaaaccagg ttaatggcta agaatgggta acatgactct 4620

tgttggattg ttattttttg tttgcaatgg ggaatttata agaagcatca agtctctttc 4680

ttaccaaagt cttgttaggt ggtttatagt tcttttggct aacaaatcat tttggaaata 4740

aagatttttt actac 4755

<210> 69
<211> 6841
<212> DNA
<213> human

<400> 69
gccggagggc gcccgagggg ccccgggccg cggcgctcag ggcccgggcg gccggcggcg 60

gccccggggc tgggggagt ccagcccgga tattgagtgc agccattgag aaaagccaaa 120

ctcttgtgtg tgcgcgtctc gatagccccc aagatggccg ccaatgtggg atcgatgttt 180

caatattgga agcgatttga tctacggcga ctccagaagg agcttaattc cgtcgcttct 240

gagctgtctg cacggcagga ggagagtgaa cattctcata aacatttaat tgaactccgc 300

cgggaattta agaaaaatgt acctgaggaa atcagagaga tggtggctcc tgtattaaaa 360

agcttccaag ccgaggtggt ggcccttagt aagagaagtc aggaggcgga ggctgctttt 420

ctgagtgttt acaagcaatt aattgaagca ccagaccccg tgcctgtgtt tgaggcggca 480

cgcagcctag acgacagact gcagcccccc agctttgacc ccagtgggca gccccggcga 540

gacctccaca cttcgtggaa gaggaacccc gagctcctca gccccaaaga gcagagagag 600

gggacgtcgc ctgccgggcc cacgctgacc gagggaagcc gcctcccagg cattcccggg 660

aaagccctcc tgacagaaac cttgctgcag agaaatgagg cggaaaaaca aaagggcctt 720

caagaagtac agatcacttt ggcggccaga ctgggggagg cagaggagaa aatcaaagtc 780

ctacattcag cgctaaaggc tacgcaggca gagctgctag agctgcggcg gaagtacgac 840

gaggaggcag catccaaggc agatgaagtc ggcctgatca tgaccaacct ggagaaagct 900

aatcagcgag ctgaggctgc ccagcgggag gtggaaagtc tccgggaaca gctggcctct 960

gtcaacagct ccatccgcct ggcttgctgc tctccccagg ggcccagtgg ggataaggtg 1020

aacttcactc tgtgctcggg ccctcggctg gaggccgcgc tggcctccaa ggacagggag 1080

atcctgcggc tgctgaagga cgtgcagcac ctccagagct cactgcagga gctggaggag 1140

gcatccgcca accagatcgc cgacctggag cggcagctca cggccaagtc cgaggccata 1200

gaaaagctgg aagagaagct ccaggcccag tctgactatg aggaaattaa aacggagctg 1260

agcatcctga aagccatgaa gctggcctcc agcacctgca gcctccccca gggcatggcc 1320

aagcctgaag actcactgct tattgcaaag gaggccttct tccccacgca gaaattcctt 1380

ctggagaagc ccagcctcct ggccagccct gaggaagacc catcagagga cgattccatc 1440

aaggattcac tgggcacgga gcagtcctac ccctcccctc agcagctccc acctccacca 1500

gggccagaag accccctgtc tcccagcccc gggcagcccc tgctgggccc cagcttgggg 1560

cctgacggca ctcggacttt ctcgctgtcc cccttcccca gcctggcatc aggggagaga 1620

ctgatgatgc ccccagccgc cttcaaggga gaggcgggcg gcctgctggt gttcccccca 1680

gccttctatg gcgccaagcc ccccacagcc cctgccaccc cggcccctgg ccctgagcca 1740

ctgggcggtc ctgagcccgc ggatggtggt gggggcggag cggcggggcc cggggcagag 1800

gaggagcagc tggacacggc agagatcgcc ttccaggtga aggagcagct gctgaaacac 1860

aacatcgggc agcgggtgtt tgggcattac gtgctggggc tgtcgcaggg ctcggtcagc 1920

gagatcctag cccggcccaa gccctggcgc aagctcacgg tgaagggcaa ggagcccttc 1980

atcaagatga agcagttcct gtcggatgag cagaatgtac tggcgctcag gaccatccaa 2040

gtgcggcagc gaggcagcat caccccgaga atccgcacgc ctgagacagg ctcagacgac 2100

gccatcaaga gcattctaga gcaggccaag aaggagatcg agtcgcagaa gggcggcgag 2160

cccaagacct cggtggcccc gctgagcatc gccaacggca cgacccccgc cagcacctcg 2220

gaggacgcca tcaagagcat cctggagcag gcacgccgtg agatgcaggc gcaacagcag 2280
gcgctgctgg agatggaggt ggcgcccagg ggccgctcgg tgcccccctc gcccccggag 2340
cggccatcac tggccaccgc gagccagaac ggggccccgg ccttggtgaa gcaggaggag 2400
ggcagcgggg gccccgcgca ggcgccgctc ccggtcctgt cccccgccgc cttcgtgcag 2460
agcatcatcc gcaaggtcaa gtccgagatc ggcgacgccg gctacttcga ccaccactgg 2520
gcctccgacc gcggcctgct cagccgcccc tacgcctccg tgtcgccctc gctgtcctcc 2580
tcctcctcct ctggctactc tggccagccc aacggccgcg cctggccccg cggggacgag 2640
gcccctgtgc cccccgagga cgaggcggcg gcaggggcgg aggacgaacc ccccaggacg 2700
ggcgagctca aggctgaggg cgcgacggcc gaggcgggcg cgcggctgcc ctactacccg 2760
gcctacgtgc cgcgcaccct gaagcccacc gtgccgccgc tgacccccga gcagtacgag 2820
ctgtacatgt accgtgaggt agacacgctg gagctcaccc gccaggtcaa ggagaagctg 2880
gccaagaacg gcatctgcca gaggatcttc ggggagaagg tgctgggcct gtcacagggc 2940
agcgtgagcg acatgctgtc ccggccgaag ccatggagca agctgacgca gaaggggcgg 3000
gagcccttca tccgcatgca gctgtggctc tctgaccagc tcggccaggc agtgggccag 3060
cagcctggtg cctcccaggc cagtcccaca gaaccaaggt cctcaccatc cccacccccc 3120
agccccacag agcctgagaa gagctcccag gagccgttga gcctgtccct ggagagcagc 3180
aaggagaacc agcagccaga gggccgctcc agctcctcgt tgagcgggaa gatgtactca 3240
ggcagccagg ccccaggggg catccaggag atcgtggcca tgtcccccga gctggacacg 3300
tactccatca ccaagagggt gaaggaggtc ctcacagaca acaatctagg gcagcggctg 3360
tttggggaaa gcatcctggg tctgacacag ggctccgtgt ctgacctgct gtcccggccc 3420
aaaccctggc acaagctgag cctgaagggg cgggagcctt ttgtccgcat gcagctgtgg 3480
ctcaatgacc cccataacgt ggagaagctg agggatatga agaagctgga gaagaaagcc 3540
tacctgaaac gtcgctatgg cctcatcagc accggctcag acagtgagtc cccggccacc 3600
cgctcagagt gccccagccc ctgcctgcag ccccaggacc tgagcctcct gcagatcaag 3660
aagccccggg tggtgctggc acccgaggag aaggaggcac tgcggaaggc ctatcagctg 3720
gaaccctacc cctcgcagca gaccatcgag ctcctctcct tccagctcaa cctcaagacc 3780
aacaccgtca tcaactggtt ccacaactac aggtcccgga tgcgccggga gatgttggtg 3840
gaggggaccc aggatgagcc agaccttgat ccaagcgggg gtcctggaat cctaccgcca 3900
ggccactccc acccagaccc caccccgcag agccctgact ctgagactga ggaccagaag 3960

ccaaccgtga aggaactgga gcttcaggag ggccctgagg agaacagcac acccctgacc 4020
acccaggaca aggcccaagt gaggatcaag caggaacaga tggaggagga tgctgaggaa 4080
gaggcaggca gccagcccca ggactcaggg gagctggaca aaggccaagg tccccccaaa 4140
gaggagcatc ccgaccctcc gggtaatgat ggactcccaa aagtggctcc cgggcccctc 4200
cttccaggtg gatccacccc agactgtccc tcacttcatc cccaacagga gagtgaggcc 4260
ggggagcgac ttcacccgga ccctttaagt tttaagtcag cctcagagtc ctcacgctgc 4320
agcctggagg tgtcactgaa ctcgccctcg gccgcctcct caccaggcct catgatgtct 4380
gtgtcacctg tcccctcctc ctcagctccc atctccccat ccccacctgg cgccccccct 4440
gccaaagtgc cgagtgccag ccccactgct gacatggctg gagccttgca ccccagtgcc 4500
aaggtgaacc ccaacttgca gcggcggcat gagaagatgg ccaatctgaa caacatcatt 4560
taccgactag agcgggctgc caatcgggag gaggccctgg agtgggagtt ctgaaggcag 4620
ggtgaggggg caagggacat accctggtaa ctaccttcct tctcgcactt actctcctca 4680
acaggatggg gtaagggagg gaggaactca accatcaaaa tgtggacagc aatgttatgc 4740
cgtttacgtt ttttgttgta atcctagttc tatgaagctg tgtgagcagg tgggtcaaat 4800
gccattgcct ccacttttct gcacccccct gctcctcttc accctgaccc ctctgcagga 4860
ggcagaagca aaatggcacc acatattcac ctgaaaactc caaactcttt tagaaaaata 4920
aataaatatt tatagacctc ttttagatat tttaataaag gatcctttgg aatttatccc 4980
agctgatgct gttttgatat tacagagagt tataaaatca ggatgctgtc acaactgttg 5040
cgaagtatac actgaagttg tgtcgttttt gccactagat gagattaaaa gaagacaatt 5100
attcaaagcc atcacaaaac actataagac tgaccaaaat ttagataacc tttgaaccac 5160
gatttttttc cacatctgtc tgtgagacac agcgcaatgc tactgccctt ccagaaactg 5220
tgctaaaaag agaaagtcca aaagactcta aacaaaaacc tcgacgccgt tgaggatgtg 5280
tttcattctg gtggtctgtt ttgcaagctt gataacagaa tgtccgtgcc attgtaaatg 5340
ttgtagagat gtgggccgtg gcccaaccgt cctatatgag atgtagcatg gtacagaaca 5400
aactgcttac acaggtctca ctagttagaa acctgtgggc catggaggtc agacatccat 5460
cttgtccatc tataggcaag aagtgtttcc agatcctttg gaaaggtggg catggggcag 5520
gtgcttggag agtggcgttt gagccagagc gaccccattt cccgtgtgaa ccataggcac 5580
aacccaggaa gtttccccac ttgtaggagt gtgggtattc cagagcaaga ctgtggccac 5640

catcttcccc tcttggtgtt ttccgaaagt gacagtgttg gtcatcccat gaccactgaa 5700

gcttagtaac cagcgccaaa aagtagattc atcaaactag agaccccagc tccccttctc 5760

gccatcttct ttctcaagtt gaccgtggtg ctgtttctgg aaggcatctg caactccaag 5820

tccatgcaga actctggaag gccaagttca tcgcagcatg ttcaccatat cccagcctcc 5880

aaatctatcc tcctaccttc caacgcatga cctgttgggg agcagagact taaccccaa 5940

ctcagaggaa cccttcctcc agcgtctttg gcatggtttc tagggtgaga gttcccaatt 6000

tggatagaac ggccaccata ttggttactg aatctctctc ccttgttttt attacgtttc 6060

ctttttcaaa ctgtccatgg gaaggctgaa ttgagtgact ccccagaatg aagatgagaa 6120

ggtgaatata atcaatgcca atgtaatgcc agcgggtgag atggccgatg gaggtttcaa 6180

agatgtagct agcattttga aaccatatgg gcaaaacccg gcaaccagaa ggggacagat 6240

aaggaccgtt ccagaaatcc caactctcac acccagccca ggctgcagtc tccacaccaa 6300

acagtcaaca aaacacaaac cctgaaggaa aacctttcc atacacccag gctatgcatt 6360

gaagagtttt ccactgtata catttttatc cagatgaagg tatttttata ttttgacaat 6420

aggaaacagt gaccattttc agagtaatca aatctggaac aaatgaaaca tcttttagcc 6480

accaccaccc tgttgcaatt aagacaaccg tgggggaaca caccactttt tactgttgaa 6540

accaacacaa cgttgaaatc caggcttata cgcagactcc gattcctaga gaactaaatt 6600

tggctttagt gtgacgggat ttgattaagc acttagtata gtcttttgaa cacggaaatc 6660

ctgttgtact taaagctagc ggacccgtga acaactttgt caggttcacg tcctataacg 6720

gttaaaaaac acacacacac atacacaaac cgtttctatg agagattgat gaactttgtt 6780

taaaatttta aaaaaaggaa cacgttctgt aaacgagtcg ctaaatacag aattgtataa 6840

t 6841

<210> 70
<211> 443
<212> DNA
<213> human

<400> 70
tttttttttt tttttccatt aacccaacac aagtttattt actaagtcaa gtcaaactga 60

ggagtatttg tttctttggt agttggtaga caaagaatac atatacatat tctatttccc 120

attaagcatt cgatcatgtt acaaaacaat ggcctagaga actatcattg aagatttacc 180

aaatctgcct gaagcaaaat gtgataaact gcagaaatgg tggagaatga cactggaagt 240

cataaagttg attctcaaac acgggtttga actgcatagg accgcttata tgcatatttt 300

gataaatata ttgaaaattt ttttggaaat ttgcaacaat ttaaaaaact tgcagatgca 360

ccatgtagct tagaaatact gaaaaactaa gaaaaaggtg tgtcatgaat tcatataata 420

tatgtagaca ctagtctatt tta 443

<210> 71
<211> 2432
<212> DNA
<213> human

<400> 71
ggcgagtggc gagtggcgag tgtcaggggg gcggccggcg ggggcggggc ggccggagga 60

ggcgttggca gcgggctcgg acccacgcgg cgccgcggcc cgcctggcct gcagcgctcc 120

cacccccggc ggcggcacga tgccctttga cttcaggagg tttgacatct acaggaaggt 180

gcccaaggac cttacgcagc caacgtacac cggggccatt atctccatct gctgctgcct 240

cttcatcctc ttcctcttcc tctcggagct caccggattt ataacgacag aagttgtgaa 300

cgagctctat gtcgatgacc cagacaagga cagcggtggc aagatcgacg tcagtctgaa 360

catcagttta cccaatctgc actgcgagtt ggttgggctt gacattcagg atgagatggg 420

caggcacgaa gtgggccaca tcgacaactc catgaagatc ccgctgaaca atggggcagg 480

ctgccgcttc gaggggcagt tcagcatcaa caaggtcccc ggcaacttcc acgtgtccac 540

acacagtgcc acagcccagc cacagaaccc agacatgacg catgtcatcc acaagctctc 600

ctttggggac acgctacagg tccagaacat ccacggagct ttcaatgctc tcgggggagc 660

agacagactc acctccaacc ccctggcctc ccacgactac atcctgaaga ttgtgcccac 720

ggtttatgag gacaagagtg gcaagcagcg gtactcctac cagtacacgg tggccaacaa 780

ggaatacgtc gcctacagcc acacgggccg catcatccct gcaatctggt tccgctacga 840

cctcagcccc atcacggtca agtacacaga gagacggcag ccgctgtaca gattcatcac 900

cacgatctgt gccatcattg gcgggacctt caccgtcgcc ggcatcctgg actcatgcat 960

cttcacagcc tctgaggcct ggaagaagat ccagctgggc aagatgcatt gacgccacac 1020

ccagcctaat ggccgaggac cctgggcatc gccagccttg cctccagtgc cctgtctcct 1080

ttggccctca atctggtccc aaatctggct gtgtcccaaa gggtgtgtgg gaagtggggg 1140

gaaagtagag gatggctcga tgttttgcag ctacctcttt tccccgtgtt tctttttaga 1200

caaattacac tgcctgaagt tgcagttccc ctttccctgg ggagccccaa gaacagagtc 1260

aggcaagggg tggggagtcc agggatcttg gggacccctc ctaggagagc tgcagtctct 1320

tccctcaggg gaacatccca gaatgcatat cgatcagctc tcagccaggc ttcgacaatc 1380

tcgcagcccc cactaggtgg acacattaat gatttggttt ctcccctggg cagccaacct 1440

gccccagagg caccagacct gggctttcag ctttgggacc aggctgccca aaggtactcc 1500

tttatacacc cggcaccttc cacgaaagat ggtacttccc aagcaagccc ctatgatttg 1560

tcactataga tggaaccctg acttctgccc catcccttcc tgcccaacct agaacccagg 1620

cctcaagtct ttaccccacc cctttcttgt tcttccaaga agcagatgcc cagttgctca 1680

gcagcagcgg tagagacttg aatctgccca ccagtcacaa ggcgggtcac agattcctct 1740

tcctctcttc tcctcgttcc tctgaaccct ccaccaatgt gcctcagcct gtgtgctgtg 1800

tggcaacagc attctggttc ccactgccaa gatctcccac cactctgctg ggatctgcag 1860

tggcagggag tgggggttgt gtaaagggga agtcatcttt tgagatccag atagacatgg 1920

tttgtgcact tacgtccaga tgggaagcat ccttcctgca accctaaaat aatcatgcag 1980

cctctcagac ggacgccatc ggtcccaagg ccttaggtgg aggaagcaaa gcaggccagg 2040

cctgtcctgt ccgtggacct ctaccttctg gactccctac gggtgcagag cacttgggtt 2100

tctctacagc catcgtggcc cacttgacac tgtgctcctc catcagctgg tcacatgcca 2160

acacgttccc agcccctgag gcagctccag ggtgccccac ctgctcctga ggtgggtccc 2220

taccctgctg ctcctcttca tcctttccct tttgtcctga aagggaggag caatggtcca 2280

ggcattaatt ccacccaggg aattttagct atgccctcat gtcccaggga gagagccaca 2340

cgcctgtttt ccatttatag caagattgtt tgcatacttt tgtaatgaag gggagtgtcc 2400

agtggaagga tttttaaaat tatcttatgg at 2432

<210> 72
<211> 2782
<212> DNA
<213> human

<400> 72
gcggccgcgg cggggcggcg cgggaaccgg gccccggggg gagtcggccg ggctgctgct 60

gctgctgctc caggtgctgc cgcccggggc tacggcaggg ccgggacgcc ggggcacgcg 120

gggcgctggc ggcggcggcg tctgctggcc cggcgcggcc ccagcctttc cccgggacgc 180

gcggctgctg ctgctcctgc cgccgctgcc gccactgtcg ccgccgccgc cgagctccgc 240

gcccgcagcc tccgcctccc ggatggacgc tctgccccgc agcgggctga acctgaagga 300

ggagccgctg ctgcccgccg gcctgggctc agtgcgctcc tggatgcagg gcgcgggcat 360
cctggacgcc agcaccgcgg cgcagagtgg cgtgggtctg gcacgagcac attttgagaa 420
gcagcctccc tccaacctca ggaaatccaa cttcttccac ttcgtgctgg ccatgtacga 480
ccggcagggg cagcccgtgg aggtggagcg cacagccttc atcgacttcg tggaaaagga 540
ccgagagccc ggggcggaaa agactaacaa tgggatccat taccgcctcc ggctggtgta 600
taacaatgga ctgcggacag agcaagacct ctacgtgcgt ctcatcgact ccatgtccaa 660
acaggccatc atctatgagg ggcaggacaa gaaccccgaa atgtgccgag tgctgctcac 720
ccatgagatc atgtgcagcc ggtgctgtga ccggaagagc tgtggcaacc ggaatgagac 780
gccctcagac cccgtcatca ttgacaggtt cttcctcaag ttcttcctca aatgcaacca 840
gaactgcctg aagaatgcgg ggaatcccag agacatgcgc cgcttccagg tggtggtgtc 900
cacgacggtg agcgtggacg gacacgtgct ggccgtgtcc gacaacatgt ttgtgcacaa 960
caactccaag catggccgca gggcgcgccg cctggacccc tccgaagctg ccaccccctg 1020
catcaaggcc atcagccccg gggagggctg gaccacgggc ggcgccaccg tcattgtcat 1080
cggcgacaac ttcttcgacg ggttgcaggt cgtgttcgga aacgtgctcg tgtggagcga 1140
gctcatcacg ccccacgcca tccgggtgca gacgcccccg cggcacatcc ccggggtggt 1200
ggaggtgacc ctctcctaca agtccaagca gttttgcaag ggatgccccg gccgctttgt 1260
ctacacagct ctgaacgagc ccaccattga ctacggattc cagaggctac agaaagtcat 1320
tcccagacac ccccggagacc ccgagaggct gcccaaggaa gtgctgctga agcgggcggc 1380
cgacttggca gaagccctgt acggagtgcc cggcagtaac caggagctgc tcctgaagcg 1440
cgcggcggac gtggccgagg ctctgtacag cacccccgc gcacccgggc cgctcgcacc 1500
cctggccccg agccacccac actccgccgt cgtgggcatc aacgccttca gcagcccgct 1560
ggccatcgcc gtcggggacg ccaccccggg gcccgagccg ggctacgcgc gcagctgcag 1620
cagcgcgtcc ccccgcgggt tcgcgcccag ccccggctcg cagcagagcg gctacggcgg 1680
cggcctcgga gctggcctgg gcggctacgg cgcgccgggc gtggccggcc tcggcgtgcc 1740
tgggtccccc agcttcctca atggctccac cgccacctcg cccttcgcca aggagcgcct 1800
tcgcccccgt gctgcgcccc ccaagctccc caccccaggc ctgccccaga gcccacggag 1860
aggggcttcc agaccagtct tttgaggatt ctgacaagtt ccactctcca gcccgggggc 1920
ttcagggcct ggcatactcc taattacggt ctgcagctgt tcccatggag cccggactgg 1980
aggtccctct gggattcaca gccacacccc ggatggtggc acagacagat gcagggccag 2040

ggccatgggc ggacctcaac ccgtgagctg aacggggaga ggccttcacc ccatgctcaa 2100
gcctccccgc tagcagcccc acaggcttct ctcgcctccc tgtcttgggg tagtcagaag 2160
ccccagcact gtgcagatgc tcttggcagg acagcatcgc agggaggtgc tgggattctg 2220
ggcctcactg tctgggtctt ggttcctctg aaagagatgg atcttgtgca gaccagggtt 2280
gttgagtgag gggagcgtgg gatggggacc gtgggaaaga ggacagctca gggagaagtg 2340
acctggaaag gtcctgtttg catctgaccc atctcaactg gcccagcatc ccaacttctc 2400
tgcagcgaaa gggtggcgcc ccgcagcctc gggaggcctg cccaggctcc cgtggagctt 2460
ccaacagctg cttggccccg cagctgcccc cacttccttt gagacctgca ctctcatgct 2520
tgccgcatca tgcctccctg tgggggcttt gggcatggag gaggcagaag agggggtgcc 2580
aggcctcctg tatttggggt cttcccccag tggatgtctc atggactctg gccccacaca 2640
ctcacaatga ctctggctgg ccccacgcag cgggcccagc cgccccccag gtggcctcac 2700
attctgctct gctaagtttg gagaaaacag aacaataaac cagatgcagg tggtgcccgc 2760
ccggcctctc acctgcctcc tt 2782

<210> 73
<211> 1722
<212> DNA
<213> human

<400> 73
ggggaaaaga gctaggaaag agctgcaaag cagtgtgggc tttttccctt tttttgctcc 60
ttttcattac ccctcctccg ttttcaccct tctccggact tcgcgtagaa cctgcgaatt 120
tcgaagagga ggtggcaaag tgggagaaaa gaggtgttag ggtttggggt ttttttgttt 180
ttgtttttgt tttttaattt cttgatttca acattttctc ccaccctctc ggctgcagcc 240
aacgcctctt acctgttctg cggcgccgcg caccgctggc agctgagggt tagaaagcgg 300
ggtgtatttt agattttaag caaaaatttt aaagataaat ccatttttct ctcccacccc 360
caacgccatc tccactgcat ccgatctcat tatttcggtg gttgcttggg ggtgaacaat 420
tttgtggctt tttttcccct ataattctga cccgctcagg cttgagggtt tctccggcct 480
ccgctcactg cgtgcacctg gcgctgccct gcttccccca acctgttgca aggctttaat 540
tcttgcaact gggacctgct cgcaggcacc ccagccctcc acctctctct acatttttgc 600
aagtgtctgg gggagggcac ctgctctacc tgccagaaat tttaaaacaa aaacaaaaac 660
aaaaaaatct ccgggggccc tcttggcccc tttatccctg cactctcgct ctcctgcccc 720

accccgaggt aaagggggcg actaagagaa gatggtgttg ctcaccgcgg tcctcctgct 780

gctggccgcc tatgcggggc cggcccagag cctgggctcc ttcgtgcact gcgagccctg 840

cgacgagaaa gccctctcca tgtgcccccc cagccccctg ggctgcgagc tggtcaagga 900

gccgggctgc ggctgctgca tgacctgcgc cctggccgag ggcagtcgt gcggcgtcta 960

caccgagcgc tgcgcccagg ggctgcgctg cctcccccgg caggacgagg agaagccgct 1020

gcacgccctg ctgcacggcc gcggggtttg cctcaacgaa aagagctacc gcgagcaagt 1080

caagatcgag agagactccc gtgagcacga ggagcccacc acctctgaga tggccgagga 1140

gacctactcc cccaagatct tccggcccaa acacacccgc atctccgagc tgaaggctga 1200

agcagtgaag aaggaccgca gaaagaagct gacccagtcc aagtttgtcg gggagccga 1260

gaacactgcc cacccccgga tcatctctgc acctgagatg agacaggagt ctgagcaggg 1320

cccctgccgc agacacatgg aggcttccct gcaggagctc aaagccagcc cacgcatggt 1380

gccccgtgct gtgtacctgc ccaattgtga ccgcaaagga ttctacaaga gaaagcagtg 1440

caaaccttcc cgtggccgca agcgtggcat ctgctggtgc gtggacaagt acgggatgaa 1500

gctgccaggc atggagtacg ttgacgggga ctttcagtgc cacaccttcg acagcagcaa 1560

cgttgagtga tgcgtccccc cccaaccttt ccctcacccc ctcccacccc cagccccgac 1620

tccagccagc gcctccctcc accccaggac gccactcatt tcatctcatt taagggaaaa 1680

atatatatct atctatttga ggaaaaaaaa aaaaaaaaaa aa 1722

<210> 74
<211> 2626
<212> DNA
<213> human

<400> 74
gggtacggct gcgagaagac gacagaaggg gatgtcacct gctttatttc tggctttggc 60

ctgtggtctg tgatacccat cctgcttgat gttctgcaga atggcacttg actgctgggc 120

atgcatgaag ttaagggcaa gaaacagtat gccatgtgtt ctgtaccatc atgtgtctct 180

tcttgcttct gggcccttct actggtgaac tttcatcaag atctgcgcca tgccgtgtca 240

ctatcaagcc attaagtttt gtctgggttg ctgtcagccc cagttggctt cctggtcaac 300

aaggacctca agaactgcct gtggaccgag gccccctacc agtgcatgag acacacacct 360

accctcccca gctttccagg aaccctactg gctgccagac tgatggcgg gctggtatgt 420

gtggacatgt gttcactgtc attatgctgt ggctccaggt gagggtgagg actgggccta 480

```
tatagaatcc agataccatt gtcaacttcc cttattcccg tctaagatgt gagcagagtg     540
ccatagtagg ggttctggga agaggtattt ctgatttgtg ggcctctgct tgcttgactt     600
caggtcactt atacttctta ttttgcttgc ctgccttcat ccctcatttc ctccctctca     660
ttcttctttc ctccctccct ttcctggtag cctcctttcc tccccttctg ccttcccctt     720
ccttctttcc ttattctttt ttattttgtt taaatagtac cacagagaaa acaactgaaa     780
aaccacattt ttctacatac agctggggag gtagctgaga acttggcact gcgcacacat     840
actaggttga aagagagttg aggaaaccag aaggccaagt ggatctgctg gcaaaccctg     900
aacctgtctc ctgcgcttgc tctacagttc tgaagttgaa aatcgttttc atgcctagca     960
tctgcttgag ttataaaccc caaggcagcc atgtcataga ctagtgttta ctcttgtttt    1020
gactttgttt taatgcttcc taagacccaa gtgcctcctg ctgtttcctc ctttgtggta    1080
gcctctggcc atctggacct caatccccag ctttcccact ttcagcagtc ctttgctctc    1140
tttgcttcta cctcaaatag ccccaggagt gggctttagt ctccaatatg gagcatctca    1200
agcttctcct gggggatggg gattgggatg ggcggaatct gttttggatc tccgggttat    1260
ttccagtggg tgtaaaagca gagctgggcc tttccctctc ttatccctga gggtgggtaa    1320
gaaggactgt atctacacct gttcttccct accttctctt ttgttaggga ggcctcattc    1380
taagttcctc aagagagtcc ttggcttaaa gctgtagcaa gggtgtgcta ggtgggggat    1440
ttggagcaaa accgtcgagt aggcatgata ctggtatgga gtgggcctgc aaaatcagac    1500
agaaatggct tgagaagccg caggggggagc atgcctgtct ctcagtgata gagtatggga    1560
gggacctccc tagcttggaa aatgagaatt gaaggggtta tgaacaaata ggatgcctag    1620
ttgaggatgt tcccaaagtt ttgtccaatc ttatcattag tagattttat aagccacaga    1680
gacaaaccag aaacggaata atgttacttt ggatgcttta tttttttgtt ctaggtgtgg    1740
ctttgtacat gcagaagaat gctatatgct gcacattttg cctttaaagt cttacgactt    1800
tccccatttt agtctaatgg gaagatacag atgtgcaagt ctgctttttt gtttttttgtt    1860
attatttttt tttttgctct gtgttatgga cattttcaga catgcacaga agtggagagg    1920
atggtccttg gaccccatgt gtccatcacc tagctgcatc acttatcagc tatggtcaac    1980
ctggtttcat ctgtatctct ctcttttcac ctgtattgtt tattgaaaat ccaagacact    2040
atgccaatgc aaccgtgact actttgggag attggtagtc tcttttgatg gtgatagtga    2100
tggggtgcac tatcataatc acatcaggtc tgctttttgc ttttaatgtt aactaatgaa    2160
```

```
gttccagaga tgggccttag aaatgtgttt taagaattaa caaggagtct caaaaagaaa    2220
tgagagggat gcttcctttc ccttgcatct acaaaacaag agagagactg ttctgttgta    2280
aaactctttc aaaaattctg atatggtaag gtacttgaga cccttcacca gaatgtcaat    2340
cttttttct gtgtaacatg gaaacttgtg tgaccattag cattgttatc agcttgtact    2400
ggtctcataa ctctggtttt ggaagaataa tttggaaatt gttgctgtgt tctgtgaaaa    2460
taacctcccc aaaataatta gtaactggtt gttctacttg gtaatttgac accctgttaa    2520
taacgcaatt atttctgtgt tcttaaacag tataaatagt tgtaagtttg catgcatgat    2580
ggaaaaataa aaacctgtat ctctgtcaaa aaaaaaaaaa aaaaaa                   2626
```

```
<210>  75
<211>  3337
<212>  DNA
<213>  human

<400>  75
gtcgagcctc tagcccgccc gggtttcctt cgcagtcgcg caccgacgct caaacgcgcg      60
ctccaacccg cagcctcctc ctgcctcacc gcccgaagat ggcggctctc aaactcctct     120
cctccgggct tcggctctgc gcctctgccc gcggatctgg ggcaacctgg tacaagggat     180
gtgtttgttc cttttccacc agtgctcatc gccataccaa gttttataca gatccagtag     240
aagctgtaaa agacatccct gatggtgcca cggttttggt tggtggtttt gggctatgtg     300
gaattccaga gaatcttata gatgctttac tgaaaactgg agtaaaagga ctaactgcag     360
tcagcaacaa tgcaggggtt gacaattttg gtttggggct tttgcttcgg tcgaagcaga     420
taaaacgcat ggtctcttca tatgtgggag aaaatgcaga atttgaacga cagtacttat     480
ctggtgaatt agaagtggag ctgacaccac agggcacact tgcagagagg atccgtgcag     540
gcggggctgg agttcctgca ttttacaccc caacagggta tgggaccctg gtacaagaag     600
gaggatcgcc catcaaatac aacaaagatg gcagtgttgc cattgccagt aagccaagag     660
aggtgaggga gttcaatggt cagcacttta ttttggagga agcaattaca ggggattttg     720
ctttggtgaa agcctggaag gcggaccgag caggaaacgt gattttcagg aaaagtgcaa     780
ggaatttcaa cttgccaatg tgcaaagctg cagaaaccac agtggtagag gttgaagaaa     840
ttgtggatat tggagcattt gctccagaag acatccatat tcctcagatt tatgtacatc     900
gccttataaa gggagaaaaa tatgagaaaa gaattgagcg tttatcaatc cggaaagagg     960
gagatgggga agccaaatct gctaaacctg gagatgacgt aagggaacga atcatcaaga    1020
```

gggccgctct tgagtttgag gatggcatgt atgctaattt gggcatagga atccctctcc 1080
tggccagcaa ttttatcagc ccaaatataa ctgttcatct tcaaagtgaa aatggagttc 1140
tgggtttggg tccatatcca cgacaacatg aagctgatgc agatctcatc aatgcaggca 1200
aggaaacagt tactattctt ccaggagcct ctttttctc cagcgatgaa tcatttgcaa 1260
tgattagagg tggacacgtc gatctgacaa tgctaggagc gatgcaggtt tccaaatatg 1320
gtgacctggc taactggatg atacctggga agatggtgaa aggaatggga ggtgctatgg 1380
atttagtgtc cagtgcgaaa accaaagtgg tggtcaccat ggagcattct gcaaagggaa 1440
atgcacataa aatcatggag aaatgtacat taccattgac tggaaagcaa tgtgtcaacc 1500
gcattattac tgaaaaggct gtgtttgatg tggacaagaa gaaagggttg actctgattg 1560
agctctggga aggcctgaca gtggatgacg tacaaaagag tactgggtgt gattttgcag 1620
tttcaccaaa actcatgcca atgcagcaga tcgcaaattg aaatatggat atttgtacca 1680
ggctgcgtgt ttttcatttt aaacacacaa gatttaattg aaaggacatc aataatcata 1740
attgtgtatt taacaggtgg ttttttatta gttttcttgt gtttcagact ttatgcagcc 1800
atataaactg ttctctaggc atgctgtgac attttaataa aaagcaaaag gagcatttat 1860
aattatctca tttgttaagg ctgagaaggt tgtttttata ataggtaatt atattgaatg 1920
cattttcact gaatatggta tgtatgctaa attatatgaa cctttcccca agaagggccc 1980
tagaaattga tgtggctttc ctcttaaata ttaattatta gtcctgaaag aaagataaca 2040
tatgtgattt ttgtggttag gagagttgct gtcatgattg tttttcttc agcctcctct 2100
gacttttctt ttggggcttc agattttatg attacatctt gtccccctag aacatccccc 2160
ttcctcccat actgctttta aacagatgcc caagaaggca agcaggaatg cctcttgtgg 2220
gggagggcag ggagaaataa ctagttcaaa ccaactatct atctatgctt tgcaaagact 2280
aaggcgtatt ataggaagag ggctagaaac ctaactgatt cttctcagtt ttctcatttt 2340
aaaacagccc agtattcctt tgtatcctca agggtccttg agaatacttc tgttattgaa 2400
accctgtggg ctacttgtac tgtacctcct ctcaagccaa gaagggctgt gggataattt 2460
accatgaatc cttagtagca atgacagcag agttaaaaaa taaaaggtgt tttactttca 2520
ggctcttgtt ttggttcaga ggagatttta aatattgaat gacacttcta cagaacaacg 2580
gtttttcttc tgccaaggct acttccttta acgaagtgcc tttaattcag ccttatccaa 2640
ctagggaaaa taatgttgga caagtctagg atttgaagag tcagtgaact tttagtgtca 2700
gggaataaac atggtgggta gattaggttt gaaaaaaact tccttagagg tatttattct 2760

caatacctga caggggccca tgggaatgac ttcagaagca tcccggataa tagatgggta 2820

aaaagtctag gcaccctgaa gaacaggtga gacagctggc ctctggacag aggtaggcat 2880

agtacagtac gatatatcat tcctctggtc ctaaatatac aaacttattc atgtttttag 2940

gtgatgatgg tcattgaaac tcacttcttt tcaggtgtag ctacaattgt gtaatgtaca 3000

atattagaga aaggacaggc tttttatgag taacacacac catatataaa acagcctttc 3060

tggctgacca catggttaaa tgcatacctt cccagtactg gggggaaaat gacccttctt 3120

agaatgtgca agttccatag agtaatatat tgatatgatt ttgaaaagaa ttgttgatag 3180

ttacatcttc aaacttatca ttccagtatg catctttaag ataatgtgat tctaagtaga 3240

tgactttata ttcttgatta aagagtgcta tacatgttaa gaaatgcatt aaggaataca 3300

ataaatattc taaactgatg aaaaaaaaaa aaaaaaa 3337

<210> 76
<211> 2460
<212> DNA
<213> human

<400> 76
aaagtcaaac cccgacaccg cggcgggccg gtgagctcac tagctgaccc ggcaggtcag 60

gatctggctt agcggcgccg cgagctccag tgcgcgcacc cgtggccgcc tcccagccct 120

ctttgccgga cgagctctgg gccgccacaa gactaaggaa tggccacccc gcccaagaga 180

agctgcccgt ctttctcagc cagctctgag gggacccgca tcaagaaaat ctccatcgaa 240

gggaacatcg ctgcagggaa gtcaacattt gtgaatatcc ttaaacaatt gtgtgaagat 300

tgggaagtgg ttcctgaacc tgttgccaga tggtgcaatg ttcaaagtac tcaagatgaa 360

tttgaggaac ttacaatgtc tcagaaaaat ggtgggaatg ttcttcagat gatgtatgag 420

aaacctgaac gatggtcttt taccttccaa acatatgcct gtctcagtcg aataagagct 480

cagcttgcct ctctgaatgg caagctcaaa gatgcagaga aacctgtatt atttttgaa 540

cgatctgtgt atagtgacag gtatattttt gcatctaatt tgtatgaatc tgaatgcatg 600

aatgagacag agtggacaat ttatcaagac tggcatgact ggatgaataa ccaatttggc 660

caaagccttg aattggatgg aatcatttat cttcaagcca ctccagagac atgcttacat 720

agaatatatt tacggggaag aaatgaagag caaggcattc ctcttgaata tttagagaag 780

cttcattata aacatgaaag ctggctcctg cataggacac tgaaaaccaa cttcgattat 840

cttcaagagg tgcctatctt aacactggat gttaatgaag actttaaaga caaatatgaa 900

```
agtctggttg aaaaggtcaa agagtttttg agtactttgt gatcttgctg aagactacag     960
gcagccaaat ggttccagat acttcagctt tgtgtatctt cgtaacttca tattaatata    1020
agtttcttta gaaaacccaa gtttttaatc gtttttgttt taaggaaaaa agatttttaa    1080
aatgaatctt atgcaaaact ttttgatcag tttcttttct tttgtttttt ttttaaaaaa    1140
gacatttaaa gacaaagaca ttatttctca tagcaggaaa tgtagaggta gatggttcca    1200
gtatcagcat agtgactaaa ctacattata aaagatccag cttccttctg tcattcccct    1260
cttttgtctt cctcagcagg ttggcttttt tccctggtgc ctctcacttc gttggtgacc    1320
agtttcttaa actgaaagct ttaatgttac atagtaaatg gtagtgtgtc ctgtgtaaat    1380
tagtgtacct attaaaagtt gcaaagtgga attaaaggaa tccctagaat aaggattctg    1440
aagttttatt ttaaattatt atcttcttaa cagtttagtc ccacctctta cttcctgcct    1500
cagtctgctt tctctactgt ctggattaat taggcagcct gctataaagt taaagtcaca    1560
catttctatt ttgcaaacac tgtgattact ctttgctttg tagtttgctt tgctttgtag    1620
ggttctgctt ttaagttttt ctctttttca gacaaattac tgataaaaat gatattgctc    1680
tatatgtaat atatcctgaa agcattattt tttgttgaat aggaaataaa attaatgaag    1740
acagaggcta gaaagcatcc attaattaat gagacacact taactactta tctctaaacc    1800
atctatgtga atatttgtaa aaataatgaa tggactcatc ttagttctgt atataaatat    1860
attttctttc tagtttgttt agttaaggtg tgcagtgttt ttcctgtgta ttaaaccttt    1920
ccattttacg ttttagaaaa ttttatgtat tttaaaataa ggggaagagt cattttcacc    1980
tttaaactac tatttttctt tccaagtcat ttttgttttt ggtttcttat tcaaagatga    2040
taatttagtg gattaaccag tccagacgca ctgatctttg caaaggagac ttaatttcaa    2100
atctgtaatt accatacata aactgtctca ttatacgtat gcattttttt agtttgtttt    2160
tgtttggtat aaattaattt gttaattaaa tatttcttaa gtataaacct tatgaactac    2220
agtggagcta cactcattga aatgtaattt cagttctaaa aagatgtaat aatcatttta    2280
gaattaaaat ttattctact tttaaataaa ttatgaatat taaaggtgaa aattgtataa    2340
attactttga ttccatttta agtggagaca tatttcagtg atttttagta acctttaaaa    2400
atgtataatg acttttaaaa tttgtagaat tgaaaagacg ctaataaaaa tttattattt    2460
```

<210> 77
<211> 7680
<212> DNA

<213> human

<400> 77

```
gcggacactc ctctcggctc ctccccggca gcggcggcgg ctcggagcgg gctccggggc      60
tcgggtgcag cggccagcgg gcctggcggc gaggattacc cggggaagtg gttgtctcct     120
ggctggagcc gcgagacggg cgctcagggc gcggggccgg cggcggcgaa cgagaggacg     180
gactctggcg gccgggtcgt tggccggggg agcgcgggca ccgggcgagc aggccgcgtc     240
gcgctcacca tggtcagcta ctgggacacc ggggtcctgc tgtgcgcgct gctcagctgt     300
ctgcttctca caggatctag ttcaggttca aaattaaaag atcctgaact gagtttaaaa     360
ggcacccagc acatcatgca agcaggccag acactgcatc tccaatgcag gggggaagca     420
gcccataaat ggtctttgcc tgaaatggtg agtaaggaaa gcgaaaggct gagcataact     480
aaatctgcct gtggaagaaa tggcaaacaa ttctgcagta ctttaacctt gaacacagct     540
caagcaaacc acactggctt ctacagctgc aaatatctag ctgtacctac ttcaaagaag     600
aaggaaacag aatctgcaat ctatatattt attagtgata caggtagacc tttcgtagag     660
atgtacagtg aaatccccga aattatacac atgactgaag gaagggagct cgtcattccc     720
tgccgggtta cgtcacctaa catcactgtt actttaaaaa agtttccact tgacactttg     780
atccctgatg gaaaacgcat aatctgggac agtagaaagg gcttcatcat atcaaatgca     840
acgtacaaag aaatagggct tctgacctgt gaagcaacag tcaatgggca tttgtataag     900
acaaactatc tcacacatcg acaaaccaat acaatcatag atgtccaaat aagcacacca     960
cgcccagtca aattacttag aggccatact cttgtcctca attgtactgc taccactccc    1020
ttgaacacga gagttcaaat gacctggagt taccctgatg aaaaaaataa gagagcttcc    1080
gtaaggcgac gaattgacca aagcaattcc catgccaaca tattctacag tgttcttact    1140
attgacaaaa tgcagaacaa agacaaagga ctttatactt gtcgtgtaag gagtggacca    1200
tcattcaaat ctgttaacac ctcagtgcat atatatgata aagcattcat cactgtgaaa    1260
catcgaaaac agcaggtgct tgaaaccgta gctggcaagc ggtcttaccg gctctctatg    1320
aaagtgaagg catttccctc gccggaagtt gtatggttaa aagatgggtt acctgcgact    1380
gagaaatctg ctcgctattt gactcgtggc tactcgttaa ttatcaagga cgtaactgaa    1440
gaggatgcag ggaattatac aatcttgctg agcataaaac agtcaaatgt gtttaaaaac    1500
ctcactgcca ctctaattgt caatgtgaaa ccccagattt acgaaaaggc cgtgtcatcg    1560
tttccagacc cggctctcta cccactgggc agcagacaaa tcctgacttg taccgcatat    1620
```

ggtatccctc aacctacaat caagtggttc tggcacccct gtaaccataa tcattccgaa 1680

gcaaggtgtg acttttgttc caataatgaa gagtccttta tcctggatgc tgacagcaac 1740

atgggaaaca gaattgagag catcactcag cgcatggcaa taatagaagg aaagaataag 1800

atggctagca ccttggttgt ggctgactct agaatttctg gaatctacat ttgcatagct 1860

tccaataaag ttgggactgt gggaagaaac ataagctttt atatcacaga tgtgccaaat 1920

gggtttcatg ttaacttgga aaaaatgccg acggaaggag aggacctgaa actgtcttgc 1980

acagttaaca agttcttata cagagacgtt acttggattt tactgcggac agttaataac 2040

agaacaatgc actacagtat tagcaagcaa aaaatggcca tcactaagga gcactccatc 2100

actcttaatc ttaccatcat gaatgtttcc ctgcaagatt caggcaccta tgcctgcaga 2160

gccaggaatg tatacacagg ggaagaaatc ctccagaaga aagaaattac aatcagagat 2220

caggaagcac catacctcct gcgaaacctc agtgatcaca cagtggccat cagcagttcc 2280

accactttag actgtcatgc taatggtgtc cccgagcctc agatcacttg gtttaaaaac 2340

aaccacaaaa tacaacaaga gcctggaatt attttaggac caggaagcag cacgctgttt 2400

attgaaagag tcacagaaga ggatgaaggt gtctatcact gcaaagccac caaccagaag 2460

ggctctgtgg aaagttcagc atacctcact gttcaaggaa cctcggacaa gtctaatctg 2520

gagctgatca ctctaacatg cacctgtgtg gctgcgactc tcttctggct cctattaacc 2580

ctccttatcc gaaaaatgaa aaggtcttct tctgaaataa agactgacta cctatcaatt 2640

ataatggacc cagatgaagt tcctttggat gagcagtgtg agcggctccc ttatgatgcc 2700

agcaagtggg agtttgcccg ggagagactt aaactgggca aatcacttgg aagaggggct 2760

tttggaaaag tggttcaagc atcagcattt ggcattaaga aatcacctac gtgccggact 2820

gtggctgtga aaatgctgaa agagggggcc acggccagcg agtacaaagc tctgatgact 2880

gagctaaaaa tcttgaccca cattggccac catctgaacg tggttaacct gctgggagcc 2940

tgcaccaagc aaggagggcc tctgatggtg attgttgaat actgcaaata tggaaatctc 3000

tccaactacc tcaagagcaa acgtgactta tttttctca acaaggatgc agcactacac 3060

atggagccta agaaagaaaa aatggagcca ggcctggaac aaggcaagaa accaagacta 3120

gatagcgtca ccagcagcga aagctttgcg agctccggct ttcaggaaga taaaagtctg 3180

agtgatgttg aggaagagga ggattctgac ggtttctaca aggagcccat cactatggaa 3240

gatctgattt cttacagttt tcaagtggcc agaggcatgg agttcctgtc ttccagaaag 3300

tgcattcatc gggacctggc agcgagaaac attcttttat ctgagaacaa cgtggtgaag 3360

atttgtgatt ttggccttgc ccgggatatt tataagaacc ccgattatgt gagaaaagga 3420
gatactcgac ttcctctgaa atggatggct cccgaatcta tctttgacaa aatctacagc 3480
accaagagcg acgtgtggtc ttacggagta ttgctgtggg aaatcttctc cttaggtggg 3540
tctccatacc caggagtaca aatggatgag gacttttgca gtcgcctgag ggaaggcatg 3600
aggatgagag ctcctgagta ctctactcct gaaatctatc agatcatgct ggactgctgg 3660
cacagagacc caaaagaaag gccaagattt gcagaacttg tggaaaaact aggtgatttg 3720
cttcaagcaa atgtacaaca ggatggtaaa gactacatcc caatcaatgc catactgaca 3780
ggaaatagtg ggtttacata ctcaactcct gccttctctg aggacttctt caaggaaagt 3840
atttcagctc cgaagtttaa ttcaggaagc tctgatgatg tcagatatgt aaatgctttc 3900
aagttcatga gcctggaaag aatcaaaacc tttgaagaac ttttaccgaa tgccacctcc 3960
atgtttgatg actaccaggg cgacagcagc actctgttgg cctctcccat gctgaagcgc 4020
ttcacctgga ctgacagcaa acccaaggcc tcgctcaaga ttgacttgag agtaaccagt 4080
aaaagtaagg agtcggggct gtctgatgtc agcaggccca gtttctgcca ttccagctgt 4140
gggcacgtca gcgaaggcaa gcgcaggttc acctacgacc acgctgagct ggaaaggaaa 4200
atcgcgtgct gctccccgcc cccagactac aactcggtgg tcctgtactc caccccaccc 4260
atctagagtt tgacacgaag ccttatttct agaagcacat gtgtatttat acccccagga 4320
aactagcttt tgccagtatt atgcatatat aagtttacac ctttatcttt ccatgggagc 4380
cagctgcttt ttgtgatttt tttaatagtg cttttttttt ttgactaaca agaatgtaac 4440
tccagataga gaaatagtga caagtgaaga acactactgc taaatcctca tgttactcag 4500
tgttagagaa atccttccta aacccaatga cttccctgct ccaacccccg ccacctcagg 4560
gcacgcagga ccagtttgat tgaggagctg cactgatcac ccaatgcatc acgtacccca 4620
ctgggccagc cctgcagccc aaaacccagg gcaacaagcc cgttagcccc aggggatcac 4680
tggctggcct gagcaacatc tcgggagtcc tctagcaggc ctaagacatg tgaggaggaa 4740
aaggaaaaaa agcaaaaagc aagggagaaa agagaaaccg ggagaaggca tgagaaagaa 4800
tttgagacgc accatgtggg cacggagggg gacggggctc agcaatgcca tttcagtggc 4860
ttcccagctc tgacccttct acatttgagg gcccagccag gagcagatgg acagcgatga 4920
ggggacattt tctggattct gggaggcaag aaaaggacaa atatcttttt tggaactaaa 4980
gcaaatttta gacctttacc tatggaagtg gttctatgtc cattctcatt cgtggcatgt 5040

tttgatttgt agcactgagg gtggcactca actctgagcc catacttttg gctcctctag 5100
taagatgcac tgaaaactta gccagagtta ggttgtctcc aggccatgat ggccttacac 5160
tgaaaatgtc acattctatt ttgggtatta atatatagtc cagacactta actcaatttc 5220
ttggtattat tctgttttgc acagttagtt gtgaaagaaa gctgagaaga atgaaaatgc 5280
agtcctgagg agagttttct ccatatcaaa acgagggctg atggaggaaa aaggtcaata 5340
aggtcaaggg aagaccccgt ctctatacca accaaaccaa ttcaccaaca cagttgggac 5400
ccaaaacaca ggaagtcagt cacgtttcct tttcatttaa tggggattcc actatctcac 5460
actaatctga aaggatgtgg aagagcatta gctggcgcat attaagcact ttaagctcct 5520
tgagtaaaaa ggtggtatgt aatttatgca aggtatttct ccagttggga ctcaggatat 5580
tagttaatga gccatcacta gaagaaaagc ccattttcaa ctgctttgaa acttgcctgg 5640
ggtctgagca tgatgggaat agggagacag ggtaggaaag ggcgcctact cttcagggtc 5700
taaagatcaa gtgggccttg gatcgctaag ctggctctgt ttgatgctat ttatgcaagt 5760
tagggtctat gtatttagga tgcgcctact cttcagggtc taaagatcaa gtgggccttg 5820
gatcgctaag ctggctctgt ttgatgctat ttatgcaagt tagggtctat gtatttagga 5880
tgtctgcacc ttctgcagcc agtcagaagc tggagaggca acagtggatt gctgcttctt 5940
ggggagaaga gtatgcttcc ttttatccat gtaatttaac tgtagaacct gagctctaag 6000
taaccgaaga atgtatgcct ctgttcttat gtgccacatc cttgtttaaa ggctctctgt 6060
atgaagagat gggaccgtca tcagcacatt ccctagtgag cctactggct cctggcagcg 6120
gcttttgtgg aagactcact agccagaaga gaggagtggg acagtcctct ccaccaagat 6180
ctaaatccaa acaaaagcag gctagagcca gaagagagga caaatctttg ttgttcctct 6240
tctttacaca tacgcaaacc acctgtgaca gctggcaatt ttataaatca ggtaactgga 6300
aggaggttaa actcagaaaa aagaagacct cagtcaattc tctacttttt tttttttttt 6360
tccaaatcag ataatagccc agcaaatagt gataacaaat aaaaccttag ctgttcatgt 6420
cttgatttca ataattaatt cttaatcatt aagagaccat aataaatact ccttttcaag 6480
agaaaagcaa aaccattaga attgttactc agctccttca aactcaggtt tgtagcatac 6540
atgagtccat ccatcagtca aagaatggtt ccatctggag tcttaatgta gaaagaaaaa 6600
tggagacttg taataatgag ctagttacaa agtgcttgtt cattaaaata gcactgaaaa 6660
ttgaaacatg aattaactga taatattcca atcatttgcc atttatgaca aaaatggttg 6720
gcactaacaa agaacgagca cttcctttca gagtttctga gataatgtac gtggaacagt 6780

ctgggtggaa tggggctgaa accatgtgca agtctgtgtc ttgtcagtcc aagaagtgac 6840

accgagatgt taattttagg gacccgtgcc ttgtttccta gcccacaaga atgcaaacat 6900

caaacagata ctcgctagcc tcatttaaat tgattaaagg aggagtgcat ctttggccga 6960

cagtggtgta actgtgtgtg tgtgtgtgtg tgtgtgtgtg tgtgtgtgtg tgtgggtgtg 7020

ggtgtatgtg tgttttgtgc ataactattt aaggaaactg gaattttaaa gttactttta 7080

tacaaaccaa gaatatatgc tacagatata agacagacat ggtttggtcc tatatttcta 7140

gtcatgatga atgtattttg tataccatct tcatataata tacttaaaaa tatttcttaa 7200

ttgggatttg taatcgtacc aacttaattg ataaacttgg caactgcttt tatgttctgt 7260

ctccttccat aaatttttca aaatactaat tcaacaaaga aaaagctctt ttttttccta 7320

aaataaactc aaatttatcc ttgtttagag cagagaaaaa ttaagaaaaa ctttgaaatg 7380

gtctcaaaaa attgctaaat attttcaatg gaaaactaaa tgttagttta gctgattgta 7440

tggggttttc gaacctttca ctttttgttt gttttaccta tttcacaact gtgtaaattg 7500

ccaataattc ctgtccatga aaatgcaaat tatccagtgt agatatattt gaccatcacc 7560

ctatggatat tggctagttt tgcctttatt aagcaaattc atttcagcct gaatgtctgc 7620

ctatatattc tctgctcttt gtattctcct ttgaacccgt taaaacatcc tgtggcactc 7680

<210> 78
<211> 3160
<212> DNA
<213> human

<400> 78
attctcgccg cgcccgggcg gacgatccag cgaacagccc cgcttctaac ccgagatgct 60

gctgccggcg cccgcgctcc gccgcgccct gctgtcccgc ccctggaccg gggccggcct 120

gcggtggaag cacacctcct ccctgaaggt ggccaacgag cccgtcttag ccttcacgca 180

gggcagccct gagcgagatg ccctgcaaaa ggccttgaag gacctgaagg gccggatgga 240

agccatccca tgcgtggtgg gggatgagga ggtgtggacg tcggacgtgc agtaccaagt 300

gtcgcctttt aaccatggac ataaggtggc caagttctgt tatgcagaca agagcctgct 360

caacaaagcc attgaggctg ccctggctgc ccggaaagag tgggacctga agcctattgc 420

agaccgggcc cagatcttcc tgaaggcggc agacatgctg agtgggccgc gcagggctga 480

gatcctcgcc aagaccatgg tgggacaggg taagaccgtg atccaagcgg agattgacgc 540

tgcagcggaa ctcatcgact tcttccggtt caatgccaag tatgcggtgg agctggaggg 600

gcagcagccc atcagcgtgc ccccgagcac caacagcacg gtgtaccggg gtctggaggg 660

cttcgtggcg gccatctcgc cctttaactt cactgcaatc ggcggcaacc tggcgggggc 720

accggccctg atgggcaacg tggtcctatg gaagcccagt gacactgcca tgctggccag 780

ctatgctgtc taccgcatcc ttcgggaggc tggcctgccc cccaacatca tccagtttgt 840

gccagctgat gggcccctat ttggggacac tgtcaccagc tcagagcacc tctgtggcat 900

caacttcaca ggcagtgtgc ccaccttcaa acacctgtgg aagcaggtgg cccagaacct 960

ggaccggttc cacaccttcc cacgcctggc tggagagtgc ggcggaaaga acttccactt 1020

cgtgcaccgc tcggccgacg tggagagcgt ggtgagcggg accctccgct cagccttcga 1080

gtacggtggc cagaagtgtt ccgcctgctc gcgtctctac gtgccgcact cgctgtggcc 1140

gcagatcaaa gggcggctgc tggaggagca cagtcggatc aaagtgggcg accctgcaga 1200

ggattttggg accttcttct ctgcagtgat tgatgccaag tcctttgccc gtatcaagaa 1260

gtggctggag cacgcgcgct cctcgcccag cctcaccatc ctggctgggg gcaagtgtga 1320

tgactccgtg ggctactttg tggagccctg catcgtggag agcaaggacc ctcaggagcc 1380

catcatgaag gaggagatct tcgggcctgt actgtctgtg tacgtctacc cggacgacaa 1440

gtacaaggag acgctgcagc tggttgacag caccaccagc tatggcctca cgggggcagt 1500

gttctcccag gataaggacg tcgtgcagga ggccacaaag gtgctgagga atgctgccgg 1560

caacttctac atcaacgaca agtccactgg ctcgatagtg ggccagcagc cctttggggg 1620

ggcccgagcc tctggaacca atgacaagcc agggggccca cactacatcc tgcgctggac 1680

gtcgccgcag gtcatcaagg agacacataa gcccctgggg gactggagct acgcgtacat 1740

gcagtgagcc cctctcgggc tccaccgtcc agctgtctgt ccgtccaggt ggccgacctc 1800

actgcacaga ccccactcca gcccctccac cccttcttca tgcacagctg cctttctata 1860

atccgggctt gactcccttc ttaccactgt attctggcct ctcccatgcc tcaggctctg 1920

gtttgagatc gtgctgggga ggaacatggc cactacccct tatcccatcg gccatgtggg 1980

aggtatgacc ctggtgcctg gcaggttctc cctctgccct ccactgggcc cagtggctca 2040

gggacctggg gaaaggagat ggagcagctc ttgggatcct ttggggaaaa ggaggccatt 2100

ctgggcccct tggcaaacct caccactcac agaggctcct ggccttgatc cctgcccctc 2160

caggtgtcca gggtaaagtg taactcagac tgacctgtgg ggcacagggg gcaccagctg 2220

gccttgccct ctctggtctg ggctgtctac cttcctcact gtatctttgc ccagacccac 2280

ctgggccagt aggcccctgt ccccagccac acaccttaga tgctggcatg ccttactcca 2340

ggtgcctgtg tttggccgag gcctgtgtga ttcccggtct gcaccacatg gcggggttgg 2400

ggggccgctg gaggccacct gccaaggcgt gggatgggat ggtcctgccg gtttaggccg 2460

tgattctgga aaaccttgga tgggccttcg tcctatgtca gccttccctt tgatcctcag 2520

gccctacctg tagagacctc cactcctaga gccagtctca gggtctggga tttccctgca 2580

ggagctcagc caccactgtg ccatggtgac acaggccaag gcagacattg gccctccctt 2640

ctcccagccc ccagaggcct ggccttgggt tcgtcagcat gggccgagga cgttgcctgt 2700

agaatcctcc tctgcctggg agtggctctg tgtggaccag tccctcactg gcccattctt 2760

tttttgacgc agccaatctg tgaccacgat tcctcccaca gatgcctcct gcttggattc 2820

tgagtggtca gagatctgta aagcatgact ttcaaggatg gttcttaggg gactgtgaaa 2880

gtgttgggtc ttcctccagg atgcctgcat gggaccccac ccggagctgg tgtggccatt 2940

ccccaagtgc cactggccca tggatggggg tgggtgctgg tgccagctgg gctgggtgtg 3000

ggttctgtgt ccttccagga tatgtgtcat ttcccatgag gggccggggc aggtggctgg 3060

gtgggggcac aggctggagt attcttagtt ctactggttc tacactgtga ggtggcaatg 3120

ggatttgctc agatgccacc caataaaatg cctgttactt 3160

<210> 79
<211> 1546
<212> DNA
<213> human

<400> 79
cacggccgga gagacgcgga ggaggagaca tgagccggcg ggcgcccaga cggagcggcc 60

gtgacgcttt cgcgctgcag ccgcgcgccc cgaccccgga gcgctgaccc ctggccccac 120

gcagctccgc gcccgggccg gagagcgcaa ctcggcttcc agacccgccg cgcatgctgt 180

ccccggactg agccgggcag ccagcctccc acggacgccc ggacggccgg ccggccagca 240

gtgagcgagc ttccccgcac cggccaggcg cctcctgcac agcggctgcc gccccgcagc 300

ccctgcgcca gcccggaggg cgcagcgctc gggaggagcc gcgcggggcg ctgatgccgc 360

agggcgcgcc gcggagcgcc ccggagcagc agagtctgca gcagcagcag ccggcgagga 420

gggagcagca gcagcggcgg cggcggcggc ggcggcggcg gaggcgcccg gtcccggccg 480

cgcggagcgg acatgtgcag gctgggctag gagccgccgc ctccctcccg cccagcgatg 540

tattcagcgc cctccgcctg cacttgcctg tgtttacact tcctgctgct gtgcttccag 600

```
gtacaggtgc tggttgccga ggagaacgtg gacttccgca tccacgtgga gaaccagacg     660
cgggctcggg acgatgtgag ccgtaagcag ctgcggctgt accagctcta cagccggacc     720
agtgggaaac acatccaggt cctgggccgc aggatcagtg cccgcggcga ggatggggac     780
aagtatgccc agctcctagt ggagacagac accttcggta gtcaagtccg gatcaagggc     840
aaggagacgg aattctacct gtgcatgaac cgcaaaggca agctcgtggg gaagcccgat     900
ggcaccagca aggagtgtgt gttcatcgag aaggttctgg agaacaacta cacggccctg     960
atgtcggcta agtactccgg ctggtacgtg ggcttcacca agaaggggcg gccgcggaag    1020
ggccccaaga cccgggagaa ccagcaggac gtgcatttca tgaagcgcta ccccaagggg    1080
cagccggagc ttcagaagcc cttcaagtac acgacggtga ccaagaggtc ccgtcggatc    1140
cggcccacac accctgccta ggccaccccg ccgcggcccc tcaggtcgcc ctggccacac    1200
tcacactccc agaaaactgc atcagaggaa tatttttaca tgaaaaataa ggaagaagct    1260
ctatttttgt acattgtgtt taaaagaaga caaaaactga accaaaactc ttgggggag     1320
gggtgataag gattttattg ttgacttgaa acccccgatg acaaaagact cacgcaaagg    1380
gactgtagtc aacccacagg tgcttgtctc tctctaggaa cagacaactc taaactcgtc    1440
cccagaggag gacttgaatg aggaaaccaa cactttgaga aaccaaagtc ctttttccca    1500
aaggttctga aaggaaaaaa aaaaaaaaac aaaaaaaaaa aaaaaa                   1546
```

<210> 80
<211> 2819
<212> DNA
<213> human

<400> 80

```
ctgggcccag ctcccccgag aggtggtcgg atcctctggg ctgctcggtc gatgcctgtg      60
ccactgacgt ccaggcatga ggtggttcct gccctggacg ctggcagcag tgacagcagc     120
agccgccagc accgtcctgg ccacggccct ctctccagcc cctacgacca tggactttac     180
cccagctcca ctggaggaca cctcctcacg cccccaattc tgcaagtggc catgtgagtg     240
cccgccatcc ccaccccgct gcccgctggg ggtcagcctc atcacagatg gctgtgagtg     300
ctgtaagatg tgcgctcagc agcttgggga caactgcacg gaggctgcca tctgtgaccc     360
ccaccggggc ctctactgtg actacagcgg ggaccgcccg aggtacgcaa taggagtgtg     420
tgcacaggtg gtcggtgtgg gctgcgtcct ggatggggtg cgctacaaca acggccagtc     480
cttccagcct aactgcaagt acaactgcac gtgcatcgac ggcgcggtgg gctgcacacc     540
```

actgtgcctc cgagtgcgcc ccccgcgtct ctggtgcccc cacccgcggc gcgtgagcat 600
acctggccac tgctgtgagc agtgggtatg tgaggacgac gccaagaggc cacgcaagac 660
cgcaccccgt gacacaggag ccttcgatgc tgtgggtgag gtggaggcat ggcacaggaa 720
ctgcatagcc tacacaagcc cctggagccc ttgctccacc agctgcggcc tgggggtctc 780
cactcggatc tccaatgtta acgcccagtg ctggcctgag caagagagcc gcctctgcaa 840
cttgcggcca tgcgatgtgg acatccatac actcattaag gcagggaaga agtgtctggc 900
tgtgtaccag ccagaggcat ccatgaactt cacacttgcg ggctgcatca gcacacgctc 960
ctatcaaccc aagtactgtg gagtttgcat ggacaatagg tgctgcatcc cctacaagtc 1020
taagactatc gacgtgtcct tccagtgtcc tgatgggctt ggcttctccc gccaggtcct 1080
atggattaat gcctgcttct gtaacctgag ctgtaggaat cccaatgaca tctttgctga 1140
cttggaatcc taccctgact tctcagaaat tgccaactag gcaggcacaa atcttgggtc 1200
ttggggacta acccaatgcc tgtgaagcag tcagccctta tggccaataa cttttcacca 1260
atgagcctta gttaccctga tctggaccct tggcctccat ttctgtctct aaccattcaa 1320
atgacgcctg atggtgctgc tcaggcccat gctatgagtt ttctccttga tatcattcag 1380
catctactct aaagaaaaat gcctgtctct agctgttctg gactacaccc aagcctgatc 1440
cagcctttcc aagtcactag aagtcctgct ggatcttgcc taaatcccaa gaaatggaat 1500
caggtagact tttaatatca ctaatttctt ctttagatgc caaaccacaa gactctttgg 1560
gtccattcag atgaatagat ggaatttgga acaatagaat aatctattat ttggagcctg 1620
ccaagaggta ctgtaatggg taattctgac gtcagcgcac caaaactatc ctgattccaa 1680
atatgtatgc acctcaaggt catcaaacat ttgccaagtg agttgaatag ttgcttaatt 1740
ttgattttta atggaaagtt gtatccatta acctgggcat tgttgaggtt aagtttctct 1800
tcacccctac actgtgaagg gtacagatta ggtttgtccc agtcagaaat aaaatttgat 1860
aaacattcct gttgatggga aaagccccca gttaatactc cagagacagg gaaaggtcag 1920
cccgtttcag aaggaccaat tgactctcac actgaatcag ctgctgactg gcagggcttt 1980
gggcagttgg ccaggctctt ccttgaatct tctcccttgt cctgcttggg gttcatagga 2040
attggtaagg cctctggact ggcctgtctg gcccctgaga gtggtgccct ggaacactcc 2100
tctactctta cagagccttg agagacccag ctgcagacca tgccagaccc actgaaatga 2160
ccaagacagg ttcaggtagg ggtgtgggtc aaaccaagaa gtgggtgccc ttggtagcag 2220
cctggggtga cctctagagc tggaggctgt gggactccag ggcccccgt gttcaggaca 2280

catctattgc agagactcat ttcacagcct ttcgttctgc tgaccaaatg gccagttttc 2340

tggtaggaag atggaggttt accggttgtt tagaaacaga aatagactta ataaaggttt 2400

aaagctgaag aggttgaagc taaaaggaaa aggttgttgt taatgaatat caggctatta 2460

tttattgtat taggaaaata taatatttac tgttagaatt cttttattta gggccttttc 2520

tgtgccagac attgctctca gtgctttgca tgtattagct cactgaatct tcacgacaat 2580

gttgagaagt tcccattatt atttctgttc ttacaaatgt gaaacggaag ctcatagagg 2640

tgagaaaact caaccagagt cacccagttg gtgactggga aagttaggat tcagatcgaa 2700

attggactgt ctttataacc catattttcc ccctgttttt agagcttcca aatgtgtcag 2760

aataggaaaa cattgcaata aatggcttga ttttttaaaa aaaaaaaaaa aaaaaaaaa 2819

<210> 81
<211> 2584
<212> DNA
<213> human

<400> 81
tggcggcggc ggcggcggtt gtcccggctg tgccggttgg tgtggcccgt cagcccgcgt 60

accacagcgc ccgggccgcg tcgagcccag tacagccaag ccgctgcggc cgggtccggc 120

gcgggcggcg cgcgcagacg gagggcggcg gccgcggcca gggcggcccg tgggaccgcg 180

ggcccccggc gcagcgctgc ccggctcccg gccctgccgg cctcctccct tggcgccgcg 240

gccatggcgg ccagcgcgaa gcggaagcag gaggagaagc acctgaagat gctgcgggac 300

atgaccggcc tcccgcgcaa ccgaaagtgc ttcgactgcg accagcgcgg ccccacctac 360

gttaacatga cggtcggctc cttcgtgtgt acctcctgct ccggcagcct gcgaggatta 420

aatccaccac acagggtgaa atctatctcc atgacaacat tcacacaaca ggaaattgaa 480

ttcttacaaa aacatggaaa tgaagtctgt aaacagattt ggctaggatt atttgatgat 540

agatcttcag caattccaga cttcagggat ccacaaaaag tgaaagagtt tctacaagaa 600

aagtatgaaa agaaaagatg gtatgtcccg ccagaacaag ccaaagtcgt ggcatcagtt 660

catgcatcta tttcagggtc ctctgccagt agcacaagca gcacacctga ggtcaaacca 720

ctgaaatctc ttttagggga ttctgcacca acactgcact taaataaggg cacacctagt 780

cagtccccag ttgtaggtcg ttctcaaggg cagcagcagg agaagaagca atttgacctt 840

ttaagtgatc tcggctcaga catctttgct gctccagctc ctcagtcaac agctacagcc 900

aattttgcta actttgcaca tttcaacagt catgcagctc agaattctgc aaatgcagat 960

```
tttgcaaact ttgatgcatt tggacagtct agtggttcga gtaattttgg aggtttcccc   1020
acagcaagtc actctccttt tcagccccaa actacaggtg gaagtgctgc atcagtaaat   1080
gctaattttg ctcattttga taacttcccc aaatcctcca gtgctgattt tggaaccttc   1140
aatacttccc agagtcatca aacagcatca gctgttagta aagtttcaac gaacaaagct   1200
ggtttacaga ctgcagacaa atatgcagca cttgctaatt tagacaatat cttcagtgcc   1260
gggcaaggtg gtgatcaggg aagtggcttt gggaccacag gtaaagctcc tgttggttct   1320
gtggtttcag ttcccagtca gtcaagtgca tcttcagaca agtatgcagc tctggcagaa   1380
ctagacagcg ttttcagttc tgcagccacc tccagtaatg cgtatacttc cacaagtaat   1440
gctagcagca atgtttttgg aacagtgcca gtggttgctt ctgcacagac acagcctgct   1500
tcatcaagtg tgcctgctcc atttggacgt acgccttcca caaatccatt tgttgctgct   1560
gctggtcctt ctgtggcatc ttctacaaac ccatttcaga ccaatgccag aggagcaaca   1620
gcggcaacct ttggcactgc atccatgagc atgcccacgg gattcggcac tcctgctccc   1680
tacagtcttc ccaccagctt tagtggcagc tttcagcagc ctgcctttcc agcccaagca   1740
gctttccctc aacagacagc tttttctcaa cagcccaatg gtgcaggttt tgcagcattt   1800
ggacaaacaa agccagtagt aaccccttt ggtcaagttg cagctgctgg agtatctagt    1860
aatcctttta tgactggtgc accaacagga caatttccaa caggaagctc atcaaccaat   1920
cctttcttat agccttatat agacaattta ctggaacgaa cttttatgtg gtcacattac   1980
atctctccac ctcttgcact gttgtcttgt ttcactgatc ttagctttaa acacaagaga   2040
agtctttaaa aagcctgcat tgtgtattaa acaccaggta atatgtgcaa aaccgagggc   2100
tccagtaaca ccttctaacc tgtgaattgg cagaaaaggg tagcggtatc atgtatatta   2160
aaattggcta atattaagtt attgcagata ccacattcat tatgctgcag tactgtacat   2220
atttttctta gaaattagct atttgtgcat atcagtattt gtaactttaa cacattgtta   2280
tgtgagaaat gttactgggg aaatagatca gccactttta aggtgctgtc atatatcttg   2340
gaatgaatga cctaaaatca ttttaaccat tgctactgga aagtaacaga gtcaaaattg   2400
gaaggtttta ttcattcttg aatttttcct ttctaaagag ctcttctatt tatacatgcc   2460
taaattcttt taaaatgtag agggatacct gtctgcataa taaagctgat catgttttgc   2520
tacagtttgc aggtgaaaaa aaataaatat tataaaataa aaaaaaaaaa aaagaaaaaa   2580
aaaa                                                                2584
```

<210> 82
<211> 2115
<212> DNA
<213> human

<400> 82
gaaatgaacc tctcttattg atttttattg gcctagagcc aggagtactg cattcagttg 60

actttcaggg taaaaagaaa acagtcctgg ttgttgtcat cataaacata tggaccagtg 120

tgatggtgaa atgagatgag gctccgcaat ggaactgtag ccactgcttt agcatttatc 180

acttccttcc ttactttgtc ttggtatact acatggcaaa atgggaaaga aaaactgatt 240

gcttatcaac gagaattcct tgctttgaaa gaacgtcttc gaatagctga acacagaatc 300

tcacagcgct cttctgaatt aaatacgatt gtgcaacagt tcaagcgtgt aggagcagaa 360

acaaatggaa gtaaggatgc gttgaataag ttttcagata ataccctaaa gctgttaaag 420

gagttaacaa gcaaaaaatc tcttcaagtg ccaagtattt attatcattt gcctcattta 480

ttgaaaaatg aaggaagtct tcaacctgct gtacagattg gcaacggaag aacaggagtt 540

tcaatagtca tgggcattcc cacagtgaag agagaagtta aatcttacct catagaaact 600

cttcattccc ttattgataa cctgtatcct gaagagaagt tggactgtgt tatagtagtc 660

ttcataggag agacagatat tgattatgta catggtgttg tagccaacct ggagaaagaa 720

ttttctaaag aaatcagttc tggcttggtg gaagtcatat caccccctga aagctattat 780

cctgacttga caaacctaaa ggagacattt ggagactcca aagaaagagt aagatggaga 840

acaaagcaaa acctagatta ctgttttcta atgatgtatg ctcaagaaaa gggcatatat 900

tacattcagc ttgaagatga tattattgtc aaacaaaatt attttaatac cataaaaaat 960

tttgcacttc aactttcttc tgaggaatgg atgattctag agttttccca gctgggcttc 1020

attggtaaaa tgtttcaagc gccggatctt actctgattg tagaattcat attcatgttt 1080

tacaaggaga aacccattga ttggctcctg gaccatattc tctgggtgaa agtctgcaac 1140

cctgaaaaag atgcaaaaca ttgtgataga cagaaagcaa atctgcgaat tcgcttcaga 1200

ccttcccttt tccaacatgt tggtctgcac tcatcactat caggaaaaat ccaaaaactc 1260

acggataaag attatatgaa accattactt cttaaaatcc atgtaaaccc acctgcggag 1320

gtatctactt ccttgaaggt ctaccaaggg catacgctgg agaaaactta catgggagag 1380

gatttcttct gggctatcac accgatagct ggagactaca tcttgtttaa atttgataaa 1440

ccagtcaatg tagaaagtta tttgttccat agcggcaacc aagaacatcc tggagatatt 1500

ctgctaaaca caactgtgga agttttgcct tttaagagtg aaggtttgga aataagcaaa 1560

gaaaccaaag acaaacgatt agaagatggc tatttcagaa taggaaaatt tgagaatggt 1620

gttgcagaag gaatggtgga tccaagtctc aatcccattt cagcctttcg actttcagtt 1680

attcagaatt ctgctgtttg ggccattctt aatgagattc atattaaaaa agccaccaac 1740

tgatcatctg agaaaccaac acatttttc ctgtgaattt gttaattaaa gatagttaag 1800

catgtatctt tttttattt ctacttgaac actacctctt gtgaagtcta ctgtagataa 1860

gacgattgtc atttccactt ggaaagtgaa tctcccataa taattgtatt tgtttgaaac 1920

taagctgtcc tcagatttta acttgactca aacattttc aattatgaca gcctgttaat 1980

atgacttgta ctattttggt attatactaa tacataagag ttgtacatat tgttacattc 2040

tttaaatttg agaaaaacta atgttacata cattttatga aggggtact tttgaggttc 2100

acttatttta ctatt 2115

<210> 83
<211> 1635
<212> DNA
<213> human

<400> 83
gggggtggcg gggacgcgag tggcggccgc ggggccccgg acaagggtcc gcagagctgc 60

agccttcgag ggccagccct ctccgagtcc ggggctgggt cccaccagtg acaaggcggc 120

agccccgcgc acaccaaaga gaaagcggct gtggcggcag cggcagcccc agccatgctg 180

tgttatgtga cgaggccgga cgcggtgctg atggaggtgg aggtggaggc gaaagccaac 240

ggcgaggact gcctcaacca ggtgtgcagg cgactgggaa tcatagaagt tgactatttt 300

ggactgcaat ttacgggtag caaaggtgaa agtttatggc taaacctgag aaaccggatc 360

tcccagcaga tggatgggct agccccttac aggcttaaac ttagagtcaa gttcttcgtg 420

gagcctcatc tcatcttaca ggagcagact aggcatatct tttcttgca catcaaggag 480

gccctcttgg caggccacct cttgtgttcc ccagagcagg cagtggaact cagtgccctc 540

ctggcccaga ccaagtttgg agactacaac cagaacactg ccaagtataa ctatgaggag 600

ctctgtgcca aggagctctc ctctgccacc ttgaacagca ttgttgcaaa acataaggag 660

ttggagggga ccagccaggc ttcagctgaa taccaagttt tgcagattgt gtcggcaatg 720

gaaaactatg gcatagaatg gcattctgtg cgggatagcg aagggcagag actgctcatt 780

ggggttggac ctgaaggaat ctcaatttgt aaagatgact ttagcccaat taataggata 840

gcttatcctg tggtgcagat ggccacccag tcaggaaaga atgtatattt gacggtcacc 900

aaggaatctg ggaacagcat cgtgctcttg tttaaaatga tcagcaccag ggcggccagc 960

gggctctacc gagcgataac agagacgcac gcattctaca ggtgtgacac agtgaccagc 1020

gccgtgatga tgcagtatag ccgtgacttg aagggccact tggcatctct gtttctgaat 1080

gaaaacatta accttggcaa gaaatatgtc tttgatatta aaagaacatc aaaggaggtg 1140

tatgaccatg ccaggagggc tctgtacaat gctggcgttg tggacctcgt ttcaagaagc 1200

aaccagagcc cttcacactc gcctctgaag tcctcagaaa gcagcatgaa ctgcagcagc 1260

tgcgagggcc tcagctgcca gcagacccgg gtgctgcagg agaagctacg caagctgaag 1320

gaagccatgc tgtgcatggt gtgctgcgag gaggagatca actccacctt ctgtccctgt 1380

ggccacactg tgtgctgtga gagctgcgcc gcccagctac agtcatgtcc cgtctgcagg 1440

tcgcgtgtgg agcatgtcca gcacgtctat ctgccaacgc acaccagtct tctcaatctg 1500

actgtaatct aatctgttgt gcttttgttg gacttggcat gtttccatga actgcactat 1560

tataaactat taaaatgata gatgttggag aaagtaatta ttccaacacc catctgccca 1620

tgcgatgtta aaaaa 1635

<210> 84
<211> 2118
<212> DNA
<213> human

<400> 84
ctgtgaagat ggcgctctcc agggtgtgct gggctcggtc ggctgtgtgg ggctcggcag 60

tcacccctgg acattttgtc acccggaggc tgcaacttgg tcgctctggc ctggcttggg 120

gggcccctcg gtcttcaaag cttcaccttt ctccaaaggc agatgtgaag aacttgatgt 180

cttatgtggt aaccaagaca aaagcgatta atgggaaata ccatcgtttc ttgggtcgtc 240

atttccccg cttctatatc ctgtacacaa tcttcatgaa aggattgcag atgttatggg 300

ctgatgccaa aaaggctaga agaataaaga caaatatgtg gaagcacaat ataaagtttc 360

atcaacttcc ataccgggag atggagcatt tgagacagtt ccgccaagac gtcaccaagt 420

gtcttttcct aggtattatt tccattccac cttttgccaa ctacctggtc ttcttgctaa 480

tgtacctgtt tcccaggcaa ctactgatca ggcatttctg gaccccaaaa caacaaactg 540

atttcttaga tatctatcat gctttccgga agcagtccca cccagaaatt attagttatt 600

tagaaaaggt catccctctc atttctgatg caggactccg gtggcgtctg acagatctgt 660

gcaccaagat acagcgtggt acccacccag caatacatga tatcttggct ctgagagagt 720
gtttctctaa ccatcctctg ggcatgaacc aactccaggc tttgcacgtg aaagccttga 780
gccgggccat gcttctcaca tcttacctgc ctcctccctt gttgagacat cgtttgaaga 840
ctcatacaac tgtgattcac caactggaca aggctttggc aaagctgggg attggccagc 900
tgactgctca ggaagtaaaa tcggcttgtt atctccgtgg cctgaattct acgcatattg 960
gtgaagatag gtgtcgaact tggctgggag aatggctgca gatttcctgc agcctgaaag 1020
aagctgagct gtctctcttg ctgcacaacg tggtcctgct ctccaccaac taccttggga 1080
caaggcgctg aatgaaccat ggagcggatg gcattgtcct gcagtcgtat agtatagcag 1140
tgcaggaaca aacagcactt gccagcaaag tctgtgtgta ctgttaagtg tgtgggaggc 1200
agagagagga gcaggggcca tgggcttcac agcatggcac acctgtggga actgcagaca 1260
ttcctctcac agctagaact gaaacaaacc ctcttgctag gggtggtccg tgtgaggtgt 1320
catcctgtcc ccctcataat tactaatagc tggaactggc agcagcctct actgggcttt 1380
tactgtgatg tgttcagttc atgtcctagg aagtcagctt ttgccccagg tgggaatcct 1440
tatttggctt aggactgatc cacttccatg ttacttacat ctgtgggttt ttgttgttgc 1500
tgttagaaaa tttttggctg gtgaaaacag cactcctttg gctggagcac ttgtgtccat 1560
gcatgtactt gggtgtttcc ctccatcctt tctgatatga ccaaaaatca agttgttttg 1620
ttttttgtca ccttcactgg catgggctaa ccacttcttt ttcaaaccct ctgaacacct 1680
ttttctgatg ggtaacttgc aggaatattc tattggaaaa gataacagga agtacaagtg 1740
cttcttgacc ccttcctcaa tgtttctagc cttcactctc cattgtcttt tctgggctgt 1800
attacagccc tctgtggatc ttcaactctg ctgcctccac tgtgatgcag cagtccaact 1860
gtaactgaca gtggctgcct tctctgggcc atggatcaca cctgtaaggt actaattact 1920
gcccagcctg ggagatcag gagaggtctg catagttagt aagttgggtt tagcttttgt 1980
gtgtgcatca gtgacttaga gttctgtaat aacttattgt aaatgcatga agcactgttt 2040
ttaaacccaa gtaaagactg cttgaaacct gttgatggaa aaaaaaaaaa aaaaaaaaaa 2100
aaaaaaaaaa aaaaaaa 2118

<210> 85
<211> 4221
<212> DNA
<213> human

<400> 85

cgataacgat ttgtgttgtg agaggcgcaa gctgcgattt ctgctgaact tggaggcatt 60
tctacgactt ttctctcagc tgaggctttt cctccgaccc tgatgctctt caattcggtg 120
ctccgccagc cccagcttgg cgtcctgaga aatggatggt cttcacaata ccctcttcaa 180
tcccttctga ctggttatca gtgcagtggt aatgatgaac acacttctta tggagaaaca 240
ggagtcccag ttcctccttt tggatgtacc ttctcttctg ctcccaatat ggaacatgta 300
ctagcagttg ccaatgaaga aggctttgtt cgattgtata acacagaatc acaaagtttc 360
agaaagaagt gcttcaaaga atggatggct cactggaatg ccgtctttga cctggcctgg 420
gttcctggtg aacttaaact tgttacagca gcaggtgatc aaacagccaa attttgggac 480
gtaaaagctg gtgagctgat tggaacatgc aaaggtcatc aatgcagcct caagtcagtt 540
gccttttcta agtttgagaa agctgtattc tgtacgggtg gaagagatgg caacattatg 600
gtctgggata ccaggtgcaa caaaaaagat gggttttata ggcaagtgaa tcaaatcagt 660
ggagctcaca atacctcaga caagcaaacc ccttcaaaac ccaagaagaa acagaattca 720
aaaggacttg ctccttctgt ggatttccag caaagtgtta ctgtggtcct ctttcaagac 780
gagaatacct tagtctcagc aggagctgtg gatgggataa tcaaagtatg ggatttacgt 840
aagaattata ctgcttatcg acaagaaccc atagcatcca agtctttcct gtacccaggt 900
agcagcactc gaaaacttgg atattcaagt ctgattttgg attccactgg ctctacttta 960
tttgctaatt gcacagacga taacatctac atgtttaata tgactgggtt gaagacttct 1020
ccagtggcta ttttcaatgg acaccagaac tctacctttt atgtaaaatc cagccttagt 1080
ccagatgacc agtttttagt cagtggctca agtgatgaag ctgcctacat atggaaggtc 1140
tccacaccct ggcaacctcc tactgtgctc ctgggtcatt ctcaagaggt cacgtctgtg 1200
tgctggtgtc catctgactt cacaaagatt gctacctgtt ctgatgacaa tacactaaaa 1260
atctggcgct tgaatagagg cttagaggag aaaccaggag gtgataaact ttccacggtg 1320
ggttgggcct ctcagaagaa aaaagagtca agacctggcc tagtaacagt aacgagtagc 1380
cagagtactc ctgccaaagc ccccagggta aagtgcaatc catccaattc ttccccgtca 1440
tccgcagctt gtgccccaag ctgtgctgga gacctccctc ttccttcaaa tactcctacg 1500
ttctctatta aaacctctcc tgccaaggcc cggtctccca tcaacagaag aggctctgtc 1560
tcctccgtct ctcccaagcc accttcatct ttcaagatgt cgattagaaa ctgggtgacc 1620
cgaacacctt cctcatcacc acccatcact ccacctgctt cggagaccaa gatcatgtct 1680
ccgagaaaag cccttattcc tgtgagccag aagtcatccc aagcagaggc ttgctctgag 1740

tctagaaata gagtaaagag gaggctagac tcaagctgtc tggagagtgt gaaacaaaag 1800
tgtgtgaaga gttgtaactg tgtgactgag cttgatggcc aagttgaaaa tcttcatttg 1860
gatctgtgct gccttgctgg taaccaggaa gaccttagta aggactctct aggtcctacc 1920
aaatcaagca aaattgaagg agctggtacc agtatctcag agcctccgtc tcctatcagt 1980
ccgtatgctt cagaaagctg tggaacgcta cctcttcctt tgagaccttg tggagaaggg 2040
tctgaaatgg taggcaaaga gaatagttcc ccagagaata aaaactggtt gttggccatg 2100
gcagccaaac ggaaggctga gaatccatct ccacgaagtc cgtcatccca gacacccaat 2160
tccaggagac agagcggaaa gacattgcca agcccggtca ccatcacgcc cagctccatg 2220
aggaaaatct gcacatactt ccatagaaag tcccaggagg acttctgtgg tcctgaacac 2280
tcaacagaat tatagattct aatctgagtg agttactgag ctttggtcca ctaaaacaag 2340
ctgagctttg gtccactaaa acaagatgaa aaatacaaga gtgactctat aactctggtc 2400
tttaagaaag ctgccttttc atttttagac aaaatctttt caacgctgaa atgtacctaa 2460
tctggttcta ctaccataat gtatatgcag cttcccgagg atgaatgctg tgtttaaatt 2520
tcataaagta aatttgtcac tctagcattt tgaatgaata gtcttcactt tttaaattat 2580
tcatcttctc tataataatg acatcccagt tcatggaggc aaaaaacaag tttcttgtta 2640
tcctgaaact ttctatgctc agtggaaagt atctgccagc cacagcatga ggcctgtgaa 2700
ggctgactga gaaatcctct gctgaagacc cctggttctg ttctgcctcc aacatgtata 2760
attttatttg aaatacataa tcttttcact atgcttttgt ggggtttttt ttaagtatgt 2820
gtaaaaatgt gatgctcaga taagtacatt tatatcagtt cagtgttaaa atgcagtctc 2880
ttgagttaaa gtcatcttta ttttaaatgc agtgataaat gtcaactctt cggagaaact 2940
aggagaacaa caacagaaag ctgtgtttgt ctttttttctc tcaaatatat ctcccgtatg 3000
agatttcagg tccccatgtt ttcaccaagc aatctgctat gtcagccaac ccaacatcac 3060
tttctacagg aggttatgat ttttgccatt tactagagga agatgtttta tgaaatcaat 3120
ttggggtttg aattcaggtg cagtcatcag ttctttaggg gctgcaatgt tttaaaaaaa 3180
ataagtcatc agattttaag aaaaaagtga tgatttctta ttgatatttt tgtaacagaa 3240
tatagctctt aactgaaaat ccagaaccag aaacataaat cttgagtttc ttttcatgta 3300
cataaaaagc aatagccttt tagtatagat agccctgagc caaaaagtaa tagaattttc 3360
tctagatatt taatacagag agtgtataga ctgactctaa gttaataatg tgcaaaatat 3420

cttaaacatc cctcccctta ttcaacaatt atgtatcagt gatcttgaac cattgtttta 3480

tatttttcac ctttgtaacc tcatggaaag aggctttaca tactttctat gtactattta 3540

cttagaaggg agccccttc cagtcatgaa acttcatttg ttttatccat atccctgagg 3600

actgtgtaga ctttatgtca gttctgtgta gactttatgt cagtttttgt cattatttga 3660

aaatctattc tgacaacttt ttaattcctt tgatcttata agttaaagct gtaacaactg 3720

aaattgcatg gatcaagtaa gcatagtttt atccagggag aaaaataaaa ggaagccata 3780

gaattgctct ggtcaaaacc aagcacacca tagccttaac tgaatattta ggaaatctgc 3840

ctaatctgct tatatttggt gtttgttttt tgactgttgg gctttgggaa gatgttattt 3900

atgaccaata tctgccagta acgctgttta tctcacttgc tttgaaagcc aatgggggaa 3960

aaaaatccat gaaaaaaaaa agattgataa agtagatgat tttgtttgta tccctaccca 4020

tctcctggca gccctactga gtgaaattgg gatacatttg gctgtcagaa attataccga 4080

gtctactggg tataacatgt ctcacttgga aagctagtac ttttaaatgg gtgccaaagg 4140

tcaactgtaa tgagataatt atccctgcct gtgtccatgt cagactttga gctgatcctg 4200

aataataaag ccttttacct t 4221

<210> 86
<211> 867
<212> DNA
<213> human

<400> 86
cgtttcagcg tggcggcgct ggtgctggcg ttggccctgg aggacggccc cgagtgatgg 60

ctggcgcctg cctcccgggt gtctcccggg tacagatgga gtcgtcccgc ggccgccggc 120

ggcaaggtcg gcagctgcga ggccaagaga gaccccagga cacacacagc tgcctcccgg 180

tgcgagaaga agaccccggc ttgagagtga gatggcgttt aatgattgct tcagtttgaa 240

ctaccctggc aacccctgcc caggggactt gatcgaagtg ttccgtcctg gctatcagca 300

ctgggccctg tacttgggtg atggttacgt tatcaacata gcacctgtag atggcattcc 360

tgcgtccttt acaagcgcca agtctgtatt cagcagtaag gccctggtga aaatgcagct 420

cttgaaggat gttgtgggaa atgacacata cagaataaac aataaatacg atgaaacgta 480

ccccccctctc cctgtggaag aaatcataaa gcggtcagag tttgtaattg gacaggaggt 540

ggcctataac ttacttgtca acaactgtga acattttgtg acattgcttc gctatggaga 600

aggagtttca gagcaggcca accgagcgat aagtaccgtt gagtttgtga cagctgctgt 660

tggtgtcttc tcattcctgg gcttgtttcc aaaaggacaa agagcaaaat actattaaca 720

atttaccaaa gagatattga tattgaagga atttgggagg aggaaaagaa acctggggtg 780

aatacttatt ttcagtgcat cattactgtt ccagattcct atgatggatg gcagactctt 840

taataaattg cttactgata ttatctt 867

<210> 87
<211> 561
<212> DNA
<213> human

<220>
<221> misc_feature
<222> (534)..(534)
<223> any kind of base

<400> 87
tttttttttc aaattttatt ttttgtactt ttattgaaaa ggtacattta aaaaaataca 60

cagacatttt accatttaca ggttgcagat atagatgctc taaaagagtc cactctattt 120

tgttgttcta tgataactct tgcccctgat atcacaaaca ttccagtctt gttgatatcg 180

gcttaaaaag gggggcatgg gagcatgacc tgcaatatat tcagcgaaca gaaagacaaa 240

ttgttcaatt ataaattttt ttatcttctg tacattattg cattagggag ccacaaaatt 300

atgtagcatc attacaaatg aaaacaggtt aaaaatgaag aagatactta tatagaaata 360

catggattca ttgtcttctt gcagaatgca caagaggtgc aaaaatgtgc aatttaggaa 420

gctctttttc tgtttgtata cgtttgctta gcaacacaaa ccagtgagga agctacaaaa 480

taagttaaac aaaaatagca aacaggtagt aattatagct atgttatatg gctntctatt 540

tcatttaaat atctccaaat a 561

<210> 88
<211> 471
<212> DNA
<213> human

<400> 88
tttcatgaat aattttattt ttattttggt catgactttt taaaattaat ggaaagtctc 60

agtctgctca aatgataaac caaaaaatgg ggtcatgaag caaattcagt ctttgcattt 120

cttcaacaac caactcacat ttctctgctc cttgactcag aggctggaca tgtgctaaca 180

gctttttttgc ttctgtatat ccttaatagg atggcagaat cccggtgtta aaagaatctt 240

aaagtcagcc gtgtcaccat gggacctcga ttagcaaaca gatgtagaca ttccttccaa 300

attcagtcaa agaaaaaact ctaataccag cagccatgtt cagggtgtgc acgacatgct 360

gagcgcttgg gatacatccc cttgtttaat tctcacagaa actttgtgag acaagtacta 420

taatccctgt ttaacagatg ggcaaactga ggtttggaaa aacactcatt t 471

<210> 89
<211> 727
<212> DNA
<213> human

<400> 89
taaaatatta ccttttattt tctcaggcac acaagtgatt tggataccaa ttatcaagac 60

atttactga tttccctttta gaatgatcta ttttaaatct tcagccacct tagaaaaagt 120

ttgcagcaat cactttgcaa ttcataagta tcaggttaga atttagttgt ggaataagtt 180

aacagtttat gttcaatatg tgaggttatt tgaactcctg agttttaaat atcctgtgga 240

acagtgattc ctctcccttc taatggcttt tcagattaaa gcaatgactt taaaaagatt 300

acatcctaaa tacttgatta caacagaaat cgaccaatct aaaaatcaga tagtgttata 360

ctgaacatca ttctgatata atgagtagcc tctggctgaa acaaaattcc accaccaagg 420

ccatcaacca ggttagtact gtttttcctg ggtctatgt aaactctcct tttctctgca 480

aatgctgctt ggctgtgaac agcatggatt tacctgcacc aatgtggcac acacctagca 540

actttctcaa gcattctaaa gatatcccca gagctacaat attgacatat gcacagcact 600

ttctctagac agtccaatca gcgtgcacat cacacacaca gaatgctggg atatgctata 660

ctgcacactt agtacacagg tggaatagag tacaatgact aaagctcaca gaaaatgttt 720

tagtctt 727

<210> 90
<211> 460
<212> DNA
<213> human

<400> 90
tttttgatat aaagggcttt tattttcttt acagttttct gtattttcca aatttctaca 60

taaacataca cataaaagtc ataaaaatgt ccctcaaaat gacactccct ccaatgcagg 120

gagtgaggag gtgtgtgctg ggacgccaca gggtggctgc tgcagctaat ccctgtgcag 180

gtgcagccac tgctacactc cagcgtctga ggcccctctg cactggcagt gttagaatgg 240

ctgtctgaga gccaaggctg ttcacccgag cagagagtca tggagctggt acaggcagga 300

gccaaggtaa gcactaggct ggtgtgtgcc ctccaggggg caccctcctt gaagcaggcc 360

ctccaaggta ctcgcccccct gaggcagccg ataacagccg gaagccttcc cctgggggga 420

cctggccatt tgtggagcag ggaaaggctc cactgccagt 460

<210> 91
<211> 392
<212> DNA
<213> human

<400> 91
tttttttttt tttttttgag ttgctgaaaa gtttactttt gagttttaaa ctgtactttg 60

aaaactatat tgtgaactgt gttgacatga ggaaaggctg ggctccctga aaacatccag 120

ttccacagca gggcgggccc aagcccagcc aatccccagg caatgcaggg cagggatccc 180

acctggtata agtacgggca gagggcagag ccaggctgta tcgaggggcg gcgctgggac 240

cctcctcgcc cagaattcct actcatcccc agcacagaag tgctctgtag ggccagctga 300

ggacaccccg gttcactgag ggtggcccac agtaagtcgc cgtctggcag taagttagct 360

ctgcaggtgt ggaccccaag accacacacg gc 392

<210> 92
<211> 496
<212> DNA
<213> human

<400> 92
tttttgtat ttttctcaat ataatgttgc aagatgtcat tatattctca ttacccataa 60

cttcccatcc aaatcttaat ggcacatttg atattttttc aaatggcttt gagatataat 120

tcacatacca cacaattcat gtatctaaag tatacagttc tgtagttctg tggttgttgg 180

ttacatccac cagactgagg gctccctgag ggttggtgcc tcagctttcc cttcacttac 240

tcattcacca aacattcagc gcctactgag tactggggtt atgatggcca acaggagaga 300

cagtctctgc ctactgtttg gtgggggtgg agtacttagt aaccgtcgtc attattgagt 360

gcttacctgt gctgggcaca gtgctgctac tgggtgactg tgtccccagg gctgtcccag 420

gctgggggtc tggaggagta gttatcagtt gaactgagtt aatccacagt ggaaggtaac 480

ccactccctg ccctag 496

<210> 93
<211> 472
<212> DNA
<213> human

<400> 93
ttaacacaaa agcttttact tggaaactgg caaatactgg actagaatac tgacatgctc 60

acgctctggc gggagctcgg atgcagaagc tattgcacaa agcccctctg attgccttgc 120

tcctctttgg catgtatcag cagagcccca agggccaatt gcccacaggt ggggactgtt 180

ctccatcaag gtatggggac ccctacttcc ttgttttgtt aaaaagtgca ggtaggcgaa 240

gaaagcccag gcagttgacc cagtctttga aacagctgac tccccagagc tggggccagg 300

ggagcctggt cttgaggggt aagggctgca gggccaggct gatggcctgt gtcatggcat 360

tggccatctc ctctccagct tctcctcagc catcgcccgt ccgtcatggt ggtgtcggct 420

gcacctggac cttcctgcct ctccgctcag ggcagcagca gtgagtgcag ca 472

<210> 94
<211> 585
<212> DNA
<213> human

<400> 94
tttttatatt atagtaaatt tatttggtat aattacatat taacattatt tacaatgcta 60

atttttttat ttataaagta tctttatatg gacaaaaaga tacaaactgt tatcatttta 120

agtacaaagt atttctagaa atacattata agccattaaa aaaaaaaacg atatttcaag 180

attggttctt acatgctatg accaactcat atgaaagagc aagttgctcc cccttcattc 240

cttcccccaa ctccaaaggg aaaggaattt gatatttagg ctttaaaaaa tttcctacta 300

cctttatctt ttaaaaaacc ctactcaaaa caactatctc ttataaggga aaatatcata 360

gataagattt tcctttagaa aatgacatta aaagtggcat gagccctaga atgatatgtg 420

tattagaggc acttaaaaaa aatcagaagg gatccatagg aaggaattta attcagcaaa 480

tactgagtgt ccactgcatg caaggtaccc tgccagaaat ctcaaatgag taagttgtcc 540

ttagggatag aaggtgctaa gcatcatgca aaagatatga actga 585

<210> 95
<211> 400
<212> DNA
<213> human

<400> 95
ttattgtttt ttaaaaatac aattttgaaa tttattgttg aaaatggaca catggaacaa 60

accaaacctt gttttatcat gtaattttca gaaaatatgt gatccataaa gattaaaaga 120

aagttgtatt aagtctggca gctttagtat taacttgaaa taaaatatgg caagctttcc 180

acgtcctcct ttatttccac aatccatatg tacgagctag attccagtca gaacttccac 240

aaatacttca ctctttggta gcagcggtta taaattacgc ctttgctaat ttgcgttgtt 300

cccaaccagg agaaacatta ccacaaaaaa agtcagtttc atcctgcagt gttcccgcag 360

caaccatatt aaagctgaag aataaagctc ctttgtagta 400

<210> 96
<211> 461
<212> DNA
<213> human

<400> 96
ttatgccatg aattcctttt tactgaaata ccgtaggact cactaccaca ataagtactt 60

aagctgaaag agtttctaat gggagccaag taaattcagc tctccactgt gcaaagcatc 120

ttgtcatttc tataataaaa gtctttccat gttcagtcca ctttggctct ggaacctgga 180

tgagtcatgc ttggggccca gggtgcctgt gaggatgctg catgagaatt tcagctgtgg 240

tggcagtggc tgggagtccc actgactcag ggggaggcca ggcgagatga gctggaactt 300

ttaggggaga gctggcactt agggacatca ttgattgtgc ttttcttagc ctagttctgt 360

cctgcaattt atttttctta tcatgtgact gtcggctgaa gtctggggtt atttggtttc 420

tttttcttct tccttgctgg acagtctcca tggggtcacg g 461

<210> 97
<211> 542
<212> DNA
<213> human

<400> 97
taaatccagt aaagcatagt actagattca tcatacttgt acaatacaac gggcgacatg 60

aaaatggcaa aggctctcct ttttgtgaac aatttaatac aactggtggt ccaataactc 120

tacaatcagc cttgtagagg tcattaaaga cagaatcctg aaagtccgtg actacaaata 180

cattttcaaa ttccggagaa tccaaacctt caaattcttc cactgactcc atctttacaa 240

agcccacttt aatgtccttt aaggctttta taagttcttc ttgttttcca gcttcttgaa 300

ccaatatcac tcttgtttca atctgaggca tctcttcttc tacatatgaa gtagatccaa 360

taagtaagtt ttccttggaa atctcagtaa ctttagaatc aaaaatggaa gagtctgcca 420

agctagtcct cccagtagtg gatgttaata cactattttc agccatgatt tgtattcttc 480

taaatcagca ctctcaaaaa agccctagga gttccacctc ttcaaacgcc gactcctctc 540

ac 542

<210> 98
<211> 1017
<212> DNA
<213> human

<400> 98
atgggcacct ggcttctggc ctgcacctgc gtctgcacct gtgtctgctc gggagtctct 60

gtctcagggg atggacgagg gccaagggct ggaacctcca cctgcctcac caacaacatt 120

ctcaggattg attgccactg gtctgcccca gagctgggtc agggctccag ccccgggctc 180

cccttcatca gccccgtggt gctgacacat gccctttca gcaaccaggc tgctggtggc 240

acacagaagt gcatctggca gggcagtgag tgcactgtag tgttgccgcc caaggcagca 300

ctcctgccat ctgacaattt catcatcact ttctaccact gcatgtccgg gagggatcag 360

agcacgtcag ctcgccactg catcctgacc tggagcctca gtcctgcctt ggagtcaatg 420

accacacttc tcagctatga gctggacttc aagaggcagg aagaggcctg ggagcgggcc 480

cagcacaggg atcacattgt cggggtgacc tggctcatac ttgaagcctt tgagctggac 540

cctggcttta tccttgaggc caggctgcgt gtccagacgg ccatgctggg ggatgacggg 600

gcacaggagg agcgagggga gccagcccat gggaagagtg aggcccagga gtgtggttca 660

cacaaggtcc ttcagcaggt gacacaaacc tccaaggccc atcacaaggt ccttcagcag 720

atgacacaaa cctccaaggc tcatcacaaa ccttccactt tggcccaggg cactaaaggg 780

cgcacttttg ccagccctgg gcccttcctg cccacggacc ctctgatccc accctggggg 840

tggccaggca acacctttgt tgctgtgtcc atctttctcc tgctgactgg cccgacctac 900

ctcctgttca agctgtcgcc cagactcctc actttgggca aaggacaaga agcaactcgg 960

atgggggccc acagggctgg tgtgctgctg agccaggact gtgctggcac ccgatga 1017

<210> 99
<211> 1099
<212> DNA
<213> human

<400> 99
agccccaagc ttaccacctg cacccggaga gctgtgtcac catgtgggtc ccggttgtct 60

tcctcaccct gtccgtgacg tggattggtg agaggggcca tggttggggg gatgcaggag 120

agggagccag ccctgactgt caagctgagg ctctttcccc cccaacccag caccccagcc 180

cagacaggga gctgggctct tttctgtctc tcccagcccc actccaagcc catacccccca 240

gcccctccat attgcaacag tcctcactcc cacaccaggt ccccgctccc tcccacttac 300

cccagaactt tctccccatt gcccagccag ctccctgctc ccagctgctt tactaaaggg 360

gaagttcctg ggcatctccg tgtttctctt tgtggggctc aaaacctcca aggacctctc 420

tcaatgccat tggttccttg gaccgtatca ctggtccacc tcctgagccc ctcaatccta 480

tcacagtcta ctgacttttc ccattcagct gtgagtgccc aaccctatcc cagagacctt 540

gatgcttggc ctcccaatct tgccctagga tacccagatg ccaaccagac acctccttct 600

tcctagccag gctatctggc ctgagacaac aaatgggtcc ctcagtctgg caatgggact 660

ctgagaactc ctcattccct gactcttagc cccagactct tcattcagtg gcccacattt 720

tccttaggaa aaacatgagc atccccagcc acaactgcca gctctctgat tccccaaatc 780

tgcatccttt tcaaaaccta aaaacaaaaa gaaaaacaaa taaaacaaaa ccaactcaga 840

ccagaactgt tttctcaacc tgggacttcc taaactttcc aaaaccttcc tcttccagca 900

actgaacctc gccataaggc acttatccct ggttcctagc accccttatc ccctcagaat 960

ccacaacttg taccaagttt cccttctccc agtccaagac cccaaatcac cacaaaggac 1020

ccaatcccca gactcaagat atggtctggg cgctgtcttg tgtctcctac cctgatccct 1080

gggttcaact ctgctccca 1099

<210> 100
<211> 1095
<212> DNA
<213> human

<400> 100
ccaagcttac cacctgcacc cggagagctg tgtcaccatg tgggtcccgg ttgtcttcct 60

caccctgtcc gtgacgtgga ttggtgagag gggccatggt tggggggatg caggagaggg 120

agccagccct gactgtcaag ctgaggctct ttcccccccca acccagcacc ccagcccaga 180

cagggagctg ggctcttttc tgtctctccc agccccactt caagcccata cccccagccc 240

ctccatattg caacagtcct cactcccaca ccaggtcccc gctccctccc acttacccca 300

gaactttctc cccattgccc agccagctcc ctgctcccag ctgctttact aaaggggaag 360

ttcctgggca tctccgtgtt tctctttgtg gggctcaaaa cctccaagga cctctctcaa 420

tgccattggt tccttggacc gtatcactgg tccatctcct gagcccctca atcctatcac 480

agtctactga cttttcccat tcagctgtga gtgtccaacc ctatcccaga gaccttgatg 540

cttggcctcc caatcttgcc ctaggatacc cagatgccaa ccagacacct ccttcttcct 600

```
agccaggcta tctggcctga gacaacaaat gggtccctca gtctggcaat gggactctga    660
gaactcctca ttccctgact cttagcccca gactcttcat tcagtggccc acattttcct    720
taggaaaaac atgagcatcc ccagccacaa ctgccagctc tctgagtccc caaatctgca    780
tccttttcaa aacctaaaaa caaaaagaaa aacaaataaa acaaaaccaa ctcagaccag    840
aactgttttc tcaacctggg acttcctaaa ctttccaaaa ccttcctctt ccagcaactg    900
aacctcgcca taaggcactt atccctggtt cctagcaccc cttatcccct cagaatccac    960
aacttgtacc aagtttccct tctcccagtc caagacccca aatcaccaca aaggacccaa   1020
tccccagact caagatatgg tctgggcgct gtcttgtgtc tcctaccctg atccctgggt   1080
tcaactctgc tccca                                                    1095
```

## Description

## BACKGROUND

This application claims the benefit of U.S Provisional Application No. 60/368,667 filed on March 29, 2002.

The invention relates to the selection of portfolios of diagnostic markers.

A few single gene diagnostic markers such as her-2-neu are currently in use. Usually, however, diseases are not easily diagnosed with molecular diagnostics for one particular gene. Multiple markers are often required and the number of such markers that may be included in a assay based on differential gene modulation can be large, even in the hundreds of genes. It is desirable to group markers into portfolios so that the most reliable results are obtained using the smallest number of markers necessary to obtain such a result. This is particularly true in assays that contain multiple steps such as nucleic acid amplification steps.

## SUMMARY OF THE INVENTION

The invention is a method of cancer by identifying differential modulation of each gene (relative to the expression of the same genes in a normal population) in a combination of genes selected from the group consisting of Seq. ID. No. 1-30, Seq. ID No. 32, Seq. ID No. 34 and Seq. ID No. 98. In another embodiment the combination is selected from the group consisting of Seq. ID No. 32-67 , Seq. ID No. 69, and Seq ID. No. 98-100.

Gene expression portfolios and kits for employing the method are further aspects of the invention.

## DETAILED DESCRIPTION

The methods of this invention can be used in conjunction with any method for determining the gene expression patterns of relevant cells as well as protein based methods of determining gene expression. Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify copy DNA (cDNA) or copy RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637; the disclosures of which are incorporated herein by reference.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al., the disclosure of each of which is incorporated herein by reference.

Analysis of the expression levels is conducted by comparing such intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased colon tissue sample vs. normal colon tissue sample). A ratio of these expression intensities indicates the fold-change in gene expression between the test and control samples.

Modulated genes are those that are differentially expressed as up regulated or down regulated in non-normal cells. Up regulation and down regulation are relative terms meaning that a detectable difference (beyond the contribution of noise in the system used to measure it) is found in the amount of expression of the genes relative to some baseline. In this case, the baseline is the measured gene expression of a normal cell. The genes of interest in the non-normal cells are then either up regulated or down regulated relative to the baseline level using the same measurement method.

Preferably, levels of up and down regulation are distinguished based on fold changes of the intensity measurements of hybridized microarray probes. For example, in the case in which a 1.5 fold or more difference is used to make such distinctions, the diseased cell is found to yield at least 1.5 times more, or 1.5 times less intensity than the normal cells.

Other methods of making distinctions are available. For example, statistical tests can be used to find the genes most significantly different between diverse groups of samples. The Student's t-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, there is likelihood to see small p-values just by chance and adjustments for this using a Sidak correction as well as a randomization/ permutation experiment can be made.

A p-value less than .05 by the t-test is evidence that the gene is significantly different. More compelling evidence is a p-value less then .05 after the Sidak correct is factored in. For a large number of samples in each group, a p-value less than 0.05 after the randomization/ permutation test is the most compelling evidence of a significant difference.

Genes can be grouped so that information obtained about the set of genes in the group provides a sound basis for making clinically relevant judgments such as a diagnosis, prognosis, or treatment choice. These sets of genes make up the portfolios of the invention. As with most diagnostic markers, it is often desirable to use the fewest number of markers sufficient to make a correct medical judgment. This prevents a delay in treatment pending further analysis as well as inappropriate use of time and resources. Preferred optimal portfolio is one that employs the fewest number of markers for making such judgments while meeting conditions that maximize the probability that such judgments are indeed correct. These conditions will generally include sensitivity and specificity requirements. In the context of microarray based detection methods, the sensitivity of the portfolio can be reflected in the fold differences exhibited by a gene's expression in the diseased or aberrant state relative to the normal state. The detection of the differential expression of a gene is sensitive if it exhibits a large fold change relative to the expression of the gene in another state. Another aspect of sensitivity is the ability to distinguish signal from noise. For example, while the expression of a set of genes may show adequate sensitivity for defining a given disease state, if the signal that is generated by one (e.g., intensity measurements in microarrays) is below a level that easily distinguished from noise in a given setting (e.g., a clinical laboratory) then that gene should be excluded from the optimal portfolio. A procedure for setting conditions such as these that define the optimal portfolio can be incorporated into the inventive methods.

Specificity can be reflected in statistical measurements of the correlation of the signaling of gene expression with the condition of interest. If the differential expression of a set of genes is observed to produce a large fold change but they do so for a number of conditions other than the condition of interest (e.g. multiple disease states) then the gene expression profile for that set of genes is non-specific. Statistical measurements of correlation of data or the degree of consistency of data such as standard deviation, correlation coefficients, and the like can be a used as such measurements. In considering a group of genes for inclusion in a portfolio, a small standard deviation in expression measurements correlates with greater specificity. Genes that display similar expression patterns may be co-regulated by an identical factor that pushes the genes in the same direction. If this factor is sufficient but not necessary for classifying a sample, then these genes will fail to correctly identify a sample if the markers are all related to this single factor. Diversification then results in selecting as few markers as possible, yet covers as many different optimal expression patterns that are contained in the data set.

In the method of the invention, a group of genetic markers is selected for use in diagnostic applications. These groups of markers are "portfolios". Diagnostic applications include the detection or identification of a disease state or condition of a subject, determining the likelihood that a subject will contract a given disease or condition, determining the likelihood that a subject with a disease or condition will respond to therapy, determining the prognosis of a subject with a disease or condition (or its likely progression or regression), and determining the effect of a treatment on a subject with a disease or condition. For example, the method can be used to establish portfolios for detecting the presence or likelihood of a subject contracting colon cancer or the likelihood that such a subject will respond favorably to cytotoxic drugs.

The portfolios selected by the method of the invention contain a number and type of markers that assure accurate and precise results and are economized in terms of the number of genes that comprise the portfolio. The method of the invention can be used to establish optimal gene expression portfolios for any disease, condition, or state that is concomitant with the expression of multiple genes. An optimal portfolio in the context of the instant invention refers to a gene expression profile that provides an assessment of the condition of a subject (based upon the condition for which the analysis was undertaken) according to predetermined standards of at least two of the following parameters: accuracy, precision, and number of genes comprising the portfolio.

Most preferably, the markers employed in the portfolio are nucleic acid sequences that express mRNA ("genes"). Expression of the markers may occur ordinarily in a healthy subject and be more highly expressed or less highly expressed when an event that is the object of the diagnostic application occurs. Alternatively, expression may not occur except when the event that is the object of the diagnostic application occurs.

Marker attributes, features, indicia, or measurements that can be compared to make diagnostic judgments are diagnostic parameters used in the method. Indicators of gene expression levels are the most preferred diagnostic parameters. Such indicators include intensity measurements read from microarrays, as described above. Other diagnostic parameters are also possible such as indicators of the relative degree of methylation of the markers.

Distinctions are made among the diagnostic parameters through the use of mathematical/statistical values that are related to each other. The preferred distinctions are mean signal readings indicative of gene expression and measurements of the variance of such readings. The most preferred distinctions are made by use of the mean of signal ratios between different group readings (e.g., microarray intensity measurements) and the standard deviations of the signal ratio measurements. A great number of such mathematical/statistical values can be used in their place such as return at a given percentile.

A relationship among diagnostic parameter distinctions is used to optimize the selection of markers useful for the diagnostic application. Typically, this is done through the use of linear or quadratic programming algorithms. However, heuristic approaches can also be applied or can be used to supplement input data selection or data output. The most preferred relationship is a mean-variance relationship such as that described in *Mean-Variance Analysis in Portfolio Choice and Capital Markets* by Harry M. Markowitz (Frank J. Fabozzi Associates, New Hope, PA: 2000, ISBN: 1-883249-75-9) which is incorporated herein by reference. The relationship is best understood in the context of the selection of stocks for a financial investment portfolio. This is the context for which the relationship was developed and elucidated.

The investor looking to optimize a portfolio of stocks can select from a large number of possible stocks, each having a historical rate of return and a risk factor. The mean variance method uses a critical line algorithm of linear programming or quadratic programming to identify all feasible portfolios that minimize risk (as measured by variance or standard deviation) for a given level of expected return and maximize expected return for a given level of risk. When standard deviation is plotted against expected return an efficient frontier is generated. Selection of stocks along the efficient frontier results in a diversified stock portfolio optimized in terms of return and risk.

When the mean variance relationship is used in the method of the instant invention, diagnostic parameters such as microarray signal intensity and standard deviation replace the return and risk factor values used in the selection of financial portfolios. Most preferably, when the mean variance relationship is applied, a commercial computer software application such as the "Wagner Associates Mean-Variance Optimization Application", referred to as "Wagner Software" throughout this specification. This software uses functions from the "Wagner Associates Mean-Variance Optimization Library" to determine an efficient frontier and optimal portfolios in the Markowitz sense. Since such applications are made for financial applications, it may be necessary to preprocess input data so that it can conform to conventions required by the software. For example, when Wagner Software is employed in conjunction with microarray intensity measurements the following data transformation method is employed.

A relationship between each genes baseline and experimental value must first be established. The preferred process is conducted as follows. A baseline class is selected. Typically, this will comprise genes from a population that does not have the condition of interest. For example, if one were interested in selecting a portfolio of genes that are diagnostic for breast cancer, samples from patients without breast cancer can be used to make the baseline class. Once the baseline class is selected, the arithmetic mean and standard deviation is calculated for the indicator of gene expression of each gene for baseline class samples. This indicator is typically the fluorescent intensity of a microarray reading. The statistical data computed is then used to calculate a baseline value of (X*Standard Deviation + Mean) for each gene. This is the baseline reading for the gene from which all other samples will be compared. X is a stringency variable selected by the person formulating the portfolio. Higher values of X are more stringent than lower. Preferably, X is in the range of .5 to 3 with 2 to 3 being more preferred and 3 being most preferred.

Ratios between each experimental sample (those displaying the condition of interest) versus baseline readings are then calculated. The ratios are then transformed to base 10 logarithmic values for ease of data handling by the software. This enables down regulated genes to display negative values necessary for optimization according to the Markman mean-variance algorithm using the Wagner Software.

The preprocessed data comprising these transformed ratios are used as inputs in place of the asset return values that are normally used in the Wagner Software when it is used for financial analysis purposes.

Once an efficient frontier is formulated, an optimized portfolio is selected for a given input level (return) or variance that corresponds to a point on the frontier. These inputs or variances are the predetermined standards set by the person formulating the portfolio. Stated differently, one seeking the optimum portfolio determines an acceptable input level (indicative of sensitivity) or a given level of variance (indicative of specificity) and selects the genes that lie along the efficient frontier that correspond to that input level or variance. The Wagner Software can select such genes when an input level or variance is selected. It can also assign a weight to each gene in the portfolio as it would for a stock in a stock portfolio.

Determining whether a sample has the condition for which the portfolio is diagnostic can be conducted by comparing the expression of the genes in the portfolio for the patient sample with calculated values of differentially expressed genes used to establish the portfolio. Preferably, a portfolio value is first generated by summing the multiples of the intensity value of each gene in the portfolio by the weight assigned to that gene in the portfolio selection process.

A boundary value is then calculated by (Y*standard deviation + mean of the portfolio value for baseline groups) where Y is a stringency value having the same meaning as X described above. A sample having a portfolio value greater than the boundary value of the baseline class is then classified as having the condition. If desired, this process can be conducted iteratively in accordance with well known statistical methods for improving confidence levels.

Optionally one can reiterate this process until best prediction accuracy is obtained.

The process of portfolio selection and characterization of an unknown is summarized as follows:

1. Choose baseline class.
2. Calculate mean, and standard deviation of each gene for baseline class samples.
3. Calculate (X*Standard Deviation + Mean) for each gene. This is the baseline reading from which all other samples will be compared. X is a stringency variable with higher values of X being more stringent than lower.
4. Calculate ratio between each Experimental sample versus baseline reading calculated in step 3.
5. Transform ratios such that ratios less than 1 are negative (eg.using Log base 10). (Down regulated genes now correctly have negative values necessary for MV optimization).
6. These transformed ratios are used as inputs in place of the asset returns that are normally used in the software application.
7. The software will plot the efficient frontier and return an optimized portfolio at any point along the efficient frontier.
8. Choose a desired return or variance on the efficient frontier.
9. Calculate the Portfolio's Value for each sample by summing the multiples of each gene's intensity value by the weight generated by the portfolio selection algorithm.
10. Calculate a boundary value by adding the mean Portfolio Value for Baseline groups to the multiple of Y and the Standard Deviation of the Baseline's Portfolio Values. Values greater than this boundary value shall be classified as the Experimental Class.
11. Optionally one can reiterate this process until best prediction accuracy is obtained.

A second portfolio can optionally be created by reversing the baseline and experimental calculation. This creates a new portfolio of genes which are up-regulated in the original baseline class. This second portfolio's value can be subtracted from the first to create a new classification value based on multiple portfolios.

Another useful method of pre-selecting genes from gene expression data so that it can be used as input for a process for selecting a portfolio is based on a threshold given by $1 \le \left|\frac{(\mu_t - \mu_n)}{(\sigma_t + \sigma_n)}\right|$, where $\mu_t$ is the mean of the subset known to possess the disease or condition, $\mu_n$ is the mean of the subset of normal samples, and $\sigma_t + \sigma_n$ represent the combined standard deviations. A signal to noise cutoff can also be used by pre-selecting the data according to a relationship such as $0.5 \le \left|\frac{(\mu_t - MAX_n)}{(\sigma_t + \sigma_n)}\right|$. This ensures that genes that are pre-selected based on their differential modulation are differentiated in a clinically significant way. That is, above the noise level of instrumentation appropriate to the task of measuring the diagnostic parameters. For each marker pre-selected according to these criteria, a matrix is established in which columns represents samples, rows represent markers and each element is a normalized intensity measurement for the expression of that marker according to the relationship: $\left|\frac{(\mu_t - I)}{\mu_t}\right|$ where I is the intensity measurement.

Using this process of creating input for financial portfolio software make also allows one to set additional boundary conditions to define the optimal portfolios. For example, portfolio size can be limited to a fixed range or number of markers. This can be done either by making data pre-selection criteria more stringent (e.g, $.8 \le \left|\frac{(\mu_t - MAX_n)}{(\sigma_t + \sigma_n)}\right|$ instead of $0.5 \le \left|\frac{(\mu_t - MAX_n)}{(\sigma_t + \sigma_n)}\right|$) or by using programming features such as restricting portfolio size. One could, for example, set the boundary condition that the efficient frontier is to be selected from among only the optimal 10 genes.

One could also use all of the genes pre-selected for determining the efficient frontier and then limit the number of genes selected (e.g., no more than 10).

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with breast cancer. Output from the method would be an optimized set of genes that could include some genes that are expressed in peripheral blood as well as in diseased breast tissue. If sample used in the testing method are obtained from peripheral blood and certain genes differentially expressed in instances of breast cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply the rule that only a given percentage of the portfolio can be represented by a particular gene or genes. Commercially available software such as the Wagner Software readily accommodates these types of heuristics. This can be useful, for example, when factors other than accuracy and precision (e.g., anticipated licensing fees) have an impact on the desirability of including one or more genes.

Other relationships aside from the mean-variance relationship can be used in the method of the invention provided that they optimize the portfolio according to predetermined attributes such as assay accuracy and precision. Two examples are the Martin simultaneous equation approach (Elton, Edwin J. and Martin J. Gruber (1987), *Modern Portfolio Theory Investment Analysis,* Third Edition, John Wiley, New York, 1987) and Genetic Algorithms (Davis, L., (1989), *Adapting Operator Probabilities in Genetic Algorithms,* in *Proceedings of the Third International Conference on Genetic Algorithms,* Morgan Kaufmann: San Mateo, pp. 61-69). There are also many ways to adapt the mean-variance relationship to handle skewed data such as where a marker detection technology exhibits a known bias. These include, for example, the Semi-Deviation method in which the square root of the average squared (negative) deviation from a reference signal and includes only those signal values that fall below the reference signal.

Articles of this invention include representations of the gene expression profiles that make up the portfolios useful for treating, diagnosing, prognosticating, and otherwise assessing diseases. These representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. When such a microarray contains an optimized portfolio great savings in time, process steps, and resources are attained by minimizing the number of cDNA or oligonucleotides that must be applied to the substrate, reacted with the sample, read by an analyzer, processed for results, and (sometimes) verified.

Other articles according to the invention can be fashioned into reagent kits for conducting hybridization, amplification, and signal generation indicative of the level of expression of the genes in the portfolios established through the method of the invention. Kits made according to the invention include formatted assays for determining the gene expression profiles. These can include all or some of the materials needed to conduct the assays such as reagents and instructions.

## EXAMPLES

### Example 1: Producing an Optimized Portfolio

Gene expression data was recently produced from tissue samples representative of eleven different types of cancers. The data was published in *Cancer Research* 61: 7388-7393, 2001 and http://carrier.gnf.org/welsh/epican/. *See,* Andrew I. Su et al., "Molecular Classification of Human Carcinomas by Use of Gene Expression Signatures."The data included intensity measurements obtained with the use of an "U95"a oligonucleotide microarray (commercially available from Affymetrix, Inc.).

Measurements of the expression of genes from the published data (fluorescent intensity measurements) was used to select optimum gene expression portfolios for a panel of markers to determine whether a circulating cell is indicative of the presence of breast cancer, prostate cancer, ovarian cancer, colorectal cancer, or lung cancer. Such circulating cells would preferably be epithelial cells.

The data in the study was collected from the following samples: 24 adenocarcinomas, 12 infilitrating ductal breast adenocarcenomas, 21 colorectal adenocarcinomas, 23 ovarian adenocarcinomas, 25 lung carcinomas, and data from the following additional samples: 19 prostate adenocarcinomas, 12 breast carinomas, 13 colon carcinomas, 13 ovarian carcinomas, 13 ovarian carcinomas, and 89 lung carcenomas.

Using intensity readings from a collection of normal samples as the baseline class, the arithmetic mean, and standard deviation of each gene were calculated followed by a calculation of the value (X*Standard Deviation + Mean) for each gene. The stringency variable, X, was assigned a value of 3 in this case. Ratios were then calculated between each Experimental sample described in the study versus the baseline value calculations. The ratios were transformed into common logarithms. These values were then used as the input values for the Wagner Software.

This procedure selected an efficient frontier along which a minimum set of markers for each tumor type that have the lowest amount of variation for a selected level of differential (chosen at the best signal to noise ratio point). Optimization by the software resulted in the selection of a portfolio of 24 genes including 2 for prostate cancer, 5 for breast cancer, 6 for colon cancer, 2 for ovarian cancer, and 9 for lung cancer markers (Table 1).

**Table 1.**

| Cancer Type | Accession | Name | Description | Seq, ID No. |
|---|---|---|---|---|
| PR | NM_001648 | KLK3 | kallikrein 3, (prostate specific antigen) | Seq. ID No. I |
| PR | NM_005551 | KLK2 | kallikrein 2, prostatic | Seq. ID No. 2 |
| BR | NM_004064 | CDKNIB | cyclin-dependent kinase inhibitor 1B (p27, Kipl) | Seq. ID No. 34 |
| BR | NM_002411 | MGB1 | mammaglobin I | Seq. ID No. 3 |
| BR | NM_005264 | GFRA1 | GDNF family receptor alpha I | Seq. ID No. 4 |
| BR | none | C18ORF1 | chromosome 18 open reading frame I | Seq. ID No. 98 |
| BR | NM_000095 | COMP | cartilage oligomeric matrix protein | Seq. ID No. 6 |
| CO | NM_001804 | CDX1 | caudal type homeo box transcription factor I | Seq. ID No. 8 |
| CO | NM_001046 | SLC12A2 | solute carrier family 12 (sodium/potassium/ chloride transporters), member 2 | Seq. ID No. 9 |
| CO | NM_001285 | CLCA1 | chloride channel, calcium activated, family member I | Seq. ID No. 11 |
| CO | NM_007052 | NOX1 | NADPH oxidase I | Seq. ID No. 13 |
| CO | NM_002457 | MUC2 | mucin 2, intestinal/tracheal | Seq. ID No. 14 |
| CO | NM_004063 | CDH17 | cadherin 17, LI cadherin | Seq. ID No. 15 |
| LU_A | NM_021950 | MS4A2 | membrane-spanning 4-domains, subfamily A, member 2 | Seq. ID No. 17 |
| LU_A | NM_000964 | ASAHL | N-acylsphingosine amidohydrolase (acid ceramidase)-like | Seq. ID No. 18 |
| LU_A | NM_006495 | EVI2B | ecotropic viral integration site 2B | Seq. ID No. 20 |
| LU_A | NM_006864 | LILRB3 | leukocyte immunoglobulin-like receptor, subfamily B | Seq. ID No. 21 |
| LU_A | X67301 | None | H.sapiens mRNA for IgM heavy chain constant region (Ab63) | Seq. ID No. 22 |
| LU_A | NM_002123 | HLA-DQB1 | major histocompatibility complex, class II, DQ beta I | Seq. ID No.23 |
| LU_S | NM_000673 | ADH7 | alcohol dehydrogenase 7 (class IV), mu or sigma polypeptide | Seq. ID No. 24 |
| LU_S | NM_003722 | TP63 | tumor protein 63 kDa with strong homology to p53 | Seq. ID No. 26 |

Table continued

| **Cancer Type** | **Accession** | **Name** | **Description** | **Seq, ID No.** |
|---|---|---|---|---|
| LU_S | none | SOX2 | SRY (sex determining region Y)-box 2 | Seq. ID No. 32 |
| OV | NM_000906 | NPR1 | natriuretic peptide receptor A/guanylate cyclase A | Seq. ID No. 28, 29 |
| OV | NM_000378 | WTI | Wilms tumor I | Seq. ID No. 30 |

**Example 2: Heuristic Step**

A heuristic rule was further applied to the portfolio obtained in Example 1. That is, the rule stated that if the gene/marker identified would likely be expressed in peripheral blood or were well-characterized tissue markers (e.g. PSA, mammaglobin, etc.), then such genes/marker would be removed from the portfolio. Application of the rule enabled the establishment of a portfolio of genes/markers that are optimized for use in a screening application in which the patient sample is obtained by assaying components found in the peripheral blood such as epithelial cells. The result of the selected portfolio contains 31 genes as shown in Table 2.

**Table 2.**

| Cancer Type | Accession | Name | Description | Seq. ID No. |
|---|---|---|---|---|
| PR | Hs.12784 | KIAA0293 | KIAA0293 protein | Seq. ID No. 67 |
| PR | NM_006562 | LBXI | transcription factor similar to D. melanogaster homeodomain protein lady bird late | Seq. ID No. 33 |
| PR | NM_016026 | LOC51109 | CGI-82 protein | Seq. ID No. 34 |
| PR | HG2261-HT2352 | None | Antigen | Seq. ID No. 99 |
| PR | NM_012449 | STEAP | six transmembrane epithelial antigen of the prostate | Seq. ID No. 35 |
| PR | NM_001634 | AMD1 | S-adenosylmethionine decarboxylase I | Seq. ID No. 36 |
| PR | HG2261-HT2351 | None | Antigen | Seq. ID No. 100 |
| PR | NM_006457 | LIM | LIM protein (similar to rat protein kinase C-binding enigma) | Seq. ID No. 37 |
| BR | NM_005853 | IRX5 | iroquois homeobox protein 5 | Seq. ID No. 38 |
| BR | NM_005264 | GFRA I | GDNF family receptor alpha I | Seq. ID No. 39, 40 |
| BR | none | C180RF1 I | chromosome 18 open reading frame I | Seq. ID No. 98 |
| BR | NM_000095 | COMP | cartilage oligomemc matrix protein (pseudoachondroplasia, epiphyseal dysplasia 1, multiple) | Seq. ID No. 41, 42 |
| CO | NM_001265 | CDX2 | caudal type homeo box transcription factor 2 | Seq. ID No. 43 |
| CO | NM_001046 | SLC12A2 | solute carrier family 12 (sodiuro/potassium/ chloride transporters), member 2 | Seq. ID No. 44, 45 |
| CO | NM_001285 | CLCA1 | chloride channel, calcium activated, family member I | Seq. ID No. 46, 47 |
| CO | NM_004063 | CDH17 | cadherin 17, LI cadherin (liver-intestine) | Seq. ID No.48, 49 |
| OV | NM_000906 | NPR1 | natriuretic peptide receptor A/guanylate cyclase A (atrionatriuretic peptide receptor A) | Seq. ID No. 50, 51 |

Table continued

| Cancer Type | Accession | Name | Description | Seq. ID No. |
|---|---|---|---|---|
| OV | NM_005504 | BCAT1 | branched chain aminotransferase 1, cytosolic | Seq. ID No. 52 |
| OV | NM_002398 | MEIS1 | Meis1 (mouse) homolog | Seq. ID No. 53 |
| OV | none | SPON1 | spondin I, (f-spondin) extracellular matrix protein | Seq. ID No. 69 |
| OV | NM_001692 | None | M25809:Human endomembrane proton pump subunit mRNA \|GenBank=M25809 | Seq. ID No. 54 |
| OV | NM_002774 | KLK6 | kallikrein 6 (neurosin, zyme) | Seq. ID No. 55 |
| LU_A | NM_000964 | ASAHL | N-acylsphingosine amidohydrolase (acid ceramidase)-like | Seq. ID No. 56, 57 |
| LU_A | NM_002838 | PTPRC | protein tyrosine phosphatase, receptor type, C | Seq. ID No. 58 |
| LU_A | NM_015364 | MD-2 | MD-2 protein | Seq. ID No. 59 |
| LU_A | NM_006875 | PIM2 | pim-2 oncogene | Seq. ID No. 60 |
| LU_S | NM_005554 | KRT6A | keratin 6A | Seq. ID No. 61 |
| LU_S | NM_000673 | ADH7 | alcohol dehydrogenase 7 (class IV), mu or sigma polypeptide | Seq. ID No. 62, 63 |
| LU_S | NM_003722 | TP63 | tumor protein 63 kDa with strong homology to p53 | Seq. ID No. 64, 65 |
| LU_S | none | SOX2 | SRY (sex determining region Y)-box 2 | Seq. ID No. 32 |
| LU_S | NM_005688 | ABCC5 | ATP-binding cassette, sub-family C (CFTR/MRP), member 5 | Seq. ID No. 66 |

**Example 3: Prognostic Portfolios**

A patient sample set with known clinical outcomes was used to test the portfolio selection method of the invention. The sample set is described in van't Veer, L. J et al . Gene Expression Profiling Predicts Clinical Outcome of Breast Cancer, *Nature*, 415, 530 - 536, (2002), incorporated herein by reference. In that study, breast tissue samples were obtained from 78 patients exhibiting sporadic breast tumors. The patients were all less than 55 years of age and presented with a tumor less than 5cm. All were lymph node negative. Thirty four of the patients presented with distant metastases in less than 5 years while 44 showed no distant metastases in the same period.

Sample preparation and expression profiling are described in the reference. A prognostic marker portfolio of 70 genes was selected from consideration of about 5,000 genes differentially expressed in patients with different prognoses (metastasis v. no metastasis). The selection was made based on unsupervised clustering followed by a correlation coefficient analysis. This was done by calculating the correlation coefficient of the expression of each gene with disease outcome. Those significantly associated with disease by this analysis were then rank ordered with successive groups of five compared using the "leave-one-out" method until an "optimized" panel of 70 genes was selected.

The data from the study were then processed according to the method of the invention. Sample number 54 was removed from further analysis due to a high percentage of missing values. The mean and standard deviation of the intensity measurements for each gene were calculated using the non-metastatic samples as the baseline. A discriminating value of X*(Standard Deviation + Mean) was then calculated for each baseline gene (X was assigned a value of 3). This value was used to ensure the resulting portfolio would be stringent. A ratio of the discriminating value to the baseline value was then calculated for each metastatic sample. This ratio was then converted to a common logarithm. This data was then imported into Wagner Software which produced an efficient frontier from which a portfolio of 16 genes was selected. The baseline and experimental values were then reversed and a second portfolio of 12 markers representing genes up-regulated in the non-metastatic cases was produced. The second portfolio's value is subtracted from the first portfolios value to create a combined portfolio value from all 28 genes. This final portfolio is comprised of genes from Seq. ID No.70 -97. 17 of the genes of this portfolio were also present in the 70 gene portfolio described in the reference.

The genes of the portfolio are identified below. (Seq. ID No. 70, Seq. ID No. 72, Seq. ID No. 73-77, Seq. ID No. 79, Seq. ID No. 80, Seq. ID No. 85, Seq. ID No. 87, Seq. ID No. 91-93, Seq. ID No. 95 and Seq. ID. No. 97.) 28 Gene list (2 Portfolios)

**Up in Metastatic Patients (Portfolio 1)**

| | |
|---|---|
| Contig53226_RC | Seq. ID No. 89 |
| NM_012214 | Seq. ID No. 82 |
| NM_020386 | Seq. ID No. 86 |
| NM_004504 | Seq. ID No. 81 |
| AA555029_RC | Seq. ID No. 70 |
| AL080059 | Seq. ID No.74 |
| AF055033 | Seq. ID No. 73 |
| NM_016448 | Seq. ID No. 85 |
| Contig40831_RC | Seq. ID No. 95 |
| Contig63649_RC | Seq. ID No. 91 |
| Contig24252_RC | Seq. ID No. 93 |
| NM_000436 | Seq. ID No. 75 |
| NM_002019 | Seq. ID No. 77 |
| Contig55313_RC | Seq. ID No. 90 |
| Contig25991 | Seq. ID No. 97 |
| NM_000788 | Seq. ID No. 76 |
| *Up in Non-Metastatic Patients (Portfolio 2)* AB033007 | Seq. ID No. 71 |
| Contig42421_RC | Seq. ID No. 96 |
| NM_003748 | Seq. ID No. 78 |
| NM_013262 | Seq. ID No. 83 |
| NM_003862 | Seq. ID No. 79 |
| NM_003882 | Seq. ID No. 80 |
| Contig48328_RC | Seq. ID No. 87 |
| NM_015416 | Seq. ID No. 84 |
| AB037863 | Seq. ID No. 72 |
| Contig27312_RC | Seq. ID No. 88 |
| Contig32125_RC | Seq. ID No. 92 |
| Contig49670_RC | Seq. ID No. 94 |

**17 Overlap**

| Systematic name | |
|---|---|
| NM_003862 | Seq. ID No. 79 |
| NM_003882 | Seq. ID No. 80 |
| Contig48328_RC | Seq. ID No. 87 |
| AA555029_RC | Seq. ID No. 70 |
| AL080059 | Seq. ID No. 74 |

Table continued

| Systematic name | |
|---|---|
| AF055033 | Seq. ID No.73 |
| AF055033 | Seq. ID No. 73 |
| NM_016448 | Seq. ID No. 85 |
| AB037863 | Seq. ID No. 72 |
| Contig40831_RC | Seq. ID No. 95 |
| Contig63649_RC | Seq. ID No. 91 |
| Contig24252_RC | Seq. ID No. 93 |
| NM 000436 | Seq. ID No. 75 |
| NM_002019 | Seq. ID No. 77 |
| Contig32125_RC | Seq. ID No. 92 |
| Contig25991 | Seq. ID No. 97 |
| NM_000788 | Seq. ID No. 76 |

The two portfolios were then used to determine the prognosis of the 78 original samples by comparing gene expression signatures from the microarray data according to the method for testing the classification accuracy described in the reference. In the case of the 70 gene portfolio, 81 % of the samples were properly characterized according to an optimized threshold biased to include ambiguous signatures as indicative of poor prognosis (85% for an absolute threshold). This portfolio misclassified 3 patients with a poor prognosis as having a good prognosis using the optimized threshold (5 for the absolute threshold). Twelve patients with a good prognosis were misclassified as having a good prognosis when they had a bad prognosis using the optimized threshold (8 for absolute).

In the case of the 28 gene portfolio, 94% of the samples were properly characterized according to an optimized threshold biased to include ambiguous signatures as indicative of poor prognosis (93% for an absolute threshold). This portfolio misclassified 3 patients with a poor prognosis as having a good prognosis using the optimized threshold (5 for the absolute threshold). Three patients with a good prognosis were misclassified as having a good prognosis when they had a bad prognosis using the optimized threshold (2 for absolute).

Comparing the two profiles, it is apparent that the profiles selected according to the method of the invention are much more economical and produce results that are more accurate and reliable than those of the comparative portfolio.

<u>Annex to the application documents - subsequently filed sequences listing</u>

## Claims

1. A method of diagnosing cancer comprising identifying differential modulation of each gene (relative to the expression of the same genes in a normal population) in a combination of genes selected from the group consisting of Seq. ID. No.32-67, Seq. ID No. 69, and Seq. ID No. 98-100.

2. The method of claim 1 wherein there is at least a 2 fold difference in the expression of the modulated genes.

3. The method of claim 1 wherein the p-value indicating differential modulation is less than .05.

4. A diagnostic portfolio comprising isolated nucleic acid sequences, their complements, or portions thereof of a combination of genes selected from the group consisting of Seq. ID. No. 32-67, Seq. ID No. 69, and Seq. ID No. 98-100.

5. The diagnostic portfolio of claim 4 in a matrix suitable for identifying the differential expression of the genes contained therein.

6. The diagnostic portfolio of claim 5 wherein said matrix is employed in a microarray.

7. The diagnostic portfolio of claim 6 wherein said microarray is a cDNA microarray.

8. The diagnostic portfolio of claim 7 wherein said microarray is an oligonucleotide microarray.

9. A kit for diagnosing cancer comprising reagents for the detection of the expression of genes selected from the group consisting of Seq. ID. No.32-67, Seq. ID No. 69, and Seq. ID No. 98-100.

10. The kit of claim 9 further comprising reagents for conducting a microarray analysis.

11. The kit of claim 9 further comprising a medium through which nucleic acid sequences within said genes, their compliments, or portions thereof are assayed.

12. The kit of claim 11 wherein said medium is a microarray.

13. The kit of claim 12 further comprising instructions.